# EUROPEAN PATENT SPECIFICATION

(11) **EP 3 350 313 B1**
(45) Date of publication and mention of the grant of the patent: **26.11.2025**
(21) Application number: 16846970.8
(22) Date of filing: 15.09.2016
(51) Int. Cl.: C12N 5/071, C12N 5/00, A61K 35/407, C12N 5/074, C12N 5/073, C12N 5/077

(54) **DERIVATION OF LIVER ORGANOIDS FROM HUMAN PLURIPOTENT STEM CELLS**
ABLEITUNG VON LEBERORGANOIDEN AUS MENSCHLICHEN PLURIPOTENTEN STAMMZELLEN
DÉRIVATION D'ORGANOÏDES HÉPATIQUES À PARTIR DE CELLULES SOUCHES PLURIPOTENTES HUMAINES

(30) Priority: 15.09.2015 SG 10201507675Y
(43) Date of publication of application: 25.07.2018
(73) Proprietor: Agency For Science, Technology And Research (A*star), Singapore 138632 (SG)
(72) Inventor: NG, Huck Hui, Singapore 138672 (SG); CHAN, Yun Shen, Singapore 138672 (SG); TNG, Weiquan John, Singapore 138672 (SG)
(74) Representative: Elkington and Fife LLP
(86) International application number: PCT/SG2016/050448
(87) International publication number: WO 2017/048193

(56) References cited:
- EP-A2- 2 718 422
- WO-A1-2014/127170
- WO-A1-2015/173425
- SUGIMOTO, S. ET AL.: "Hepatic organoid formation in collagen sponge of cells isolated from human liver tissues.", TISSUE ENGINEERING, vol. 11, no. 3-4, 2005, pages 626 - 633, XP008111267
- SAITO, M. ET AL.: "Reconstruction of liver organoid using a bioreactor.", WORLD JOURNAL OF GASTROENTEROLOGY, vol. 12, no. 12, 2006, pages 1881 - 1888, XP055372886
- SOTO-GUTIERREZ, A. ET AL.: "Engineering of an hepatic organoid to develop liver assist devices.", CELL TRANSPLANTATION, vol. 19, no. 6-7, 2010, pages 815 - 822, XP055372891
- MITAKA, T. ET AL.: "Reconstruction of hepatic organoid by rat small hepatocytes and hepatic nonparenchymal cells.", HEPATOLOGY, vol. 29, no. 1, 1999, pages 111 - 125, XP002941775
- THOMAS, R. ET AL.: "The effect of three-dimensional co-culture of hepatocytes and hepatic stellate cells on key hepatocyte functions in vitro.", CELLS TISSUES ORGANS, vol. 181, no. 2, 2005, pages 67 - 79, XP009185643
- MINGUET, S. ET AL.: "A population of c-Kit low ( CD 45/TER119)-hepatic cell progenitors of 11-day postcoitus mouse embryo liver reconstitutes cell -depleted liver organoids.", THE JOURNAL OF CLINICAL INVESTIGATION, vol. 112, no. 8, 2003, pages 1152 - 1163, XP055116468
- EBRAHIMKHANI, M. ET AL.: "Engineering in vitro human hepatic organoids.", HEPATOLOGY, vol. 60, no. 4, pages 249A - 250A, XP009509550
- BAPTISTA, P. ET AL.: "The use of whole organ decellularization for the generation of a vascularized liver organoid.", HEPATOLOGY, vol. 53, no. 2, 2011, pages 604 - 617, XP055268730
- LEMOINNE, S. ET AL.: "Origins and functions of liver myofibroblasts.", BIOCHIMICA ET BIOPHYSICA ACTA, vol. 1832, no. 7, 2013, pages 948 - 954, XP028589927
- HISATOMI, Y. ET AL.: "Flow cytometric isolation of endodermal progenitors from mouse salivary gland differentiate into hepatic and pancreatic lineages.", HEPATOLOGY, vol. 39, no. 3, 2004, pages 667 - 675, XP055519103
- ZULEWSKI, H. ET AL.: "Multipotential nestin-positive stem cells isolated from adult pancreatic islets differentiate ex vivo into pancreatic endocrine, exocrine, and hepatic phenotypes.", DIABETES, vol. 50, no. 3, 2001, pages 521 - 533, XP002955659

## Description

### BACKGROUND OF THE INVENTION

The liver is a major organ that is responsible for regulating a multitude of complex metabolic processes. Defects in the liver present a significant burden today, but the management of liver diseases remains inadequate due to two major factors: a shortage of liver donors (Vilarinho and Lifton, 2012) and an incomplete understanding of the underlying mechanisms of liver pathology. Research on liver diseases is hampered by the lack of faithful models for metabolic diseases of hepatic origin. There is still a heavy reliance on mouse models, which cannot fully depict human disease pathogenesis and response to drugs (Grompe and Strom, 2013; Seok et al., 2013; van der Worp et al., 2010). On the other hand, disease modelling in human hepatocyte cultures fails to recapitulate complex diseases that involve interactions between multiple cell types within an organ. Furthermore, primary human hepatocytes are defiant to long-term expansion in culture (Mitaka, 1998; Shan et al., 2013), while hepatocyte-like cells differentiated from pluripotent stem cells (PSCs) (Gieseck et al., 2014; Si-Tayeb et al., 2010b) are limited with low differentiation efficiencies and a lack of mature functional capabilities found in the liver. There is a substantial need for high quality liver model that resembles the in vivo liver tissue from basic research to industrial and medical applications.

### ORGANOIDS

These issues can potentially be overcomed using 3 dimensional in vitro culture systems to generate tissue-like organoids. Organoids are refered to as "mini-organ" defined by three specific criteria 1) Having at least 2 organ-specific cell types, 2) Cells self-organize in 3D to form structure resembling tissues in the organ and 3) is capable of manifesting organ-specific functionality (Lancaster and Knoblich, 2014). They can be created from cells of human origin to avoid surreptitious species-specific differences in disease pathogenesis and drug response, while mimicking in vivo disease presentation including interactions between various cell types involved in 3D space (Matano et al., 2015; van de Wetering et al., 2015); these properties make them ideal for the study of liver function and diseases in vitro. While no hepatic organoids with the above criteria have been describe, complex 3D liver cultures have been derived from human induced pluripotent stem cells (iPSCs) (Takebe et al, 2013) and adult liver stem cells (Huch et al., 2015). These technologies have not been shown to harbor functional interactions between the two major hepatic cell types: hepatocytes and cholangiocytes and do not exhibit any of liver specific function in vitro. In addition, no liver tissue structures have been observed in these 3D liver cultures in vitro. Current organoid generation protocols are also limited by extremely high costs for large scale expansion (Spence et al 2011, Lancaster et al 2013, Takasato et al 2016), which is necessary for downstream applications requiring a large number of cells such as engraftment (Fisher and Strom, 2006) and high-throughput screening. Scalability is dependent on the ability to propagate culture to large quantities via the proliferation of itself or its precursors. As such, self-renewing PSCs have been regarded as a promising source for terminally differentiated cells.

### SELF RENEWING ENDODERM PROGENITOR

However, PSC generation of organoids is complex and requires differentiation across multiple intermediate states to generate specific somatic cell types. For endodermal tissues such as the liver, mimicking human embryonic morphogenesis through the sequential exposure of PSCs to cytokines enables their derivation in vitro (Basma et al., 2009; D'Amour et al., 2006; Spence et al., 2011). However, these methods (1) utilize complex and lengthy differentiation protocols often with only low to moderate efficiency (Murry and Keller, 2008), (2) tend to yield cells with immature properties and incomplete functionality, and (3) harbour a risk for teratoma formation (Hentze et al., 2009). The number of intermediate states (steps) required in a protocol is an important factor for the overall efficiency of differentiation. At 80% differentiation efficiency for each step, a 2 step differentiation protocol would have 64% overall efficacy and 3 step protocol would be 51.2%. The low efficacy of 50% differentiation efficacy would also mean that 1 out of 2 cells are not desired and these contaminating cells results in many complications in downstream applications. In order to overcome this issues, several groups have created self-renewing endoderm progenitors that can be used as an alternative cell source (Cheng et al., 2012; Hannan et al., 2013), but these early endoderm progenitors remain relatively naive in the differentiation landscape and still requires much differentiation steps to generate desired endoderm cell types. There is a need to generate later endoderm progenitors that can give rise to organ cell type of interest in shorter time and lesser steps. Beside late endoderm progenitors, adult stem cells which are already committed to form specific organ lineage are desirable cell sources. In addition, these adult stem cells would generate cell types of adult phenotype compared to cells generated from PSC origins.

### BRIEF SUMMARY OF THE INVENTION

Described herein are multipotent endoderm spheroid progenitor cells (MESPs), a human pluripotent stem cell (hPSC)-derived self-renewing progenitor population that can serve as a source of human hepatic cells as well as other lineages from the posterior foregut such as the intestine and pancreas. By recapitulating the stepwise process of liver differentiation during development, the methods described herein enable the scalable production of MESP-derived hepatic organoids that contain the major parenchyma hepatic liver cell types, hepatocytes and cholangiocytes. These two cell types self-organize into structures resembling the human liver unit and possess many liver specific functions. Using Genome editing CRISPR/Cas technology, the instant inventors developed a liver organoid model of familiar hypercholesterolemia, and demonstrated the response of the diseased liver organoid to statins. In addition, the inventors generated the organoids in a high throughput manner which can be adapted for large scale screenings, demonstrating the applicability of the technology for both research and industrial applications. In addition, employing similar technology, liver organoids were generated from adult stem cells and these organoids exhibit similar structures and liver specific functions as organoids generated from MESP cells.

The inventors have surprisingly discovered that a single media can be used to generate both hepatocytes and cholangiocytes. This is contrary to what is known in the art. For example, TGFβ signaling promotes bile duct cell formation but inhibits hepatocyte formation. TGFβ signaling molecules are typically added to bile duct cell cultures, but are excluded from culture media used to generate hepatocytes, and in many methods inhibitors of the TGFβ pathway are added to the media used to generate hepatocytes.

The invention is set out in the appended claims. In one aspect, a liver organoid is provided, the liver organoid comprising at least two cell types selected from the group consisting of hepatocytes, and cholangiocytes,. In some embodiments wherein, the hepatocytes
a) express albumin (ALB);
b) express hepatocyte markers HNF4a (NCBI: 3172), FAH (NCBI: 2184), TAT (NCBI: 6898), GCK (NCBI: 2645), TTR (NCBI: 7276), MET (NCBI: 4233), GLUl/MGAM (NCBI: 8972), FAHD2A (NCBI: 51011), HNF1B (NCBI: 6928), HNF1A (NCBI: 6927), CYP3A4 (NCBI: 1576), CYP2C9 (NCBI: 1559), CYP2C19 (NCBI: 1557), CYP1A2 (NCBI: 1544), CYP2E1 (NCBI: 1571), CYP2D6 (NCBI: 1565), CYP3A7 (NCBI: 1551), CYP1A1 (NCBI: 1543), CYP3A5 (NCBI: 1577), CYP27A1 (NCBI: 1593), MRP2 (NCBI:1244), NTCP (NCBI: 6554), OATP1B3 (NCBI: 28234), UGT2B7 (NCBI: 7364), UGT2B15 (NCBI: 7366), UGT1A1 (NCBI: 54658), CEBP (NCBI: 1050), KRT8 (NCBI: 3856), NOTCH2 (NCBI: 4853) and CYP2B6 (NCBI: 1555); and
c) do not express the cholangiocyte marker Cytokeratin 7 (CK7)
(d) the organoids are capable of performing liver functions and exhibit spatially organized structure observed in liver.

In some embodiments, the hepatocyte markers comprise or consist of HNF4a (NCBI: 3172), FAH (NCBI: 2184), TAT (NCBI: 6898), GCK (NCBI: 2645), TTR (NCBI: 7276), MET (NCBI: 4233), GLUl/MGAM (NCBI: 8972), FAHD2A (NCBI: 51011), HNF1B (NCBI: 6928), HNF1A (NCBI: 6927), CYP3A4 (NCBI: 1576), CYP2C9 (NCBI: 1559), CYP2C19 (NCBI: 1557), CYP1A2 (NCBI: 1544), CYP2E1 (NCBI: 1571), CYP2D6 (NCBI: 1565), CYP3A7 (NCBI: 1551), CYP1A1 (NCBI: 1543), CYP3A5 (NCBI: 1577), CYP27A1 (NCBI: 1593), MRP2 (NCBI:1244), NTCP (NCBI: 6554), OATP1B3 (NCBI: 28234), UGT2B7 (NCBI: 7364), UGT2B15 (NCBI: 7366), UGT1A1 (NCBI: 54658), CEBP (NCBI: 1050), KRT8 (NCBI: 3856), NOTCH2 (NCBI: 4853) and CYP2B6 (NCBI: 1555).

In some embodiments, the cholangiocytes express CK7 but do not express albumin (ALB). In some embodiments, the cholangiocytes further express a marker selected from CFTR (NCBI: 1080), CK19 (NCBI: 3880), HNF1B (NCBI: 6928) or SOX9 (NCBI: 6662).

In some embodiments, the hepatoblasts express at least one marker selected from the group consisting of SOX9 (NCBI: 6662), CK19 (NCBI: 3880), CK18 (NCBI: 3875), HNF4a (NCBI: 3172), PROX1 (NCBI: 5629), ONECUT1 (NCBI: 3175), AFP (NCBI: 174), and ALB (NCBI: 213).

In some embodiments, the organoids further comprise at least one cell selected from the group consisting of liver specific endothelial cells (LSEC), stellate cells, hepatic myofibroblast and hepatoblasts. In some embodiments, the liver specific endothelial cells (LSEC) express at least one marker selected from the group consisting of CD45, CD80, CD86, CD11c, VAP1, STAB1 and CD31, wherein the CD31 expression that is mainly expressed in the cytoplasm and not on the cell surface.

In some embodiments, the stellate cells express at least one marker selected from the group consisting of GFAP, VIM, LHX2, LRAT, PDGFRb, HAND2, ICAM-1, VCAM-1, and N-CAM-1.

In some embodiments, the hepatic myofibroblast express a marker selected from the group consisting of COL1A1 and α-SMA.

In some embodiments, the parenchymal cell types originate from the same stem cell.

In some embodiments, the liver organoid cells are cultured in suspension without the use of extracellular matrices.

The organoids are capable of performing liver functions and exhibit a spatially organized structure observed in liver. In some embodiments, the liver functions are selected from the group consisting of liver specific metabolic activities, albumin secretion, glycogen storage, low density lipo-protein uptake, bile acid production, drug metabolism, and cytochrome enzymatic activities. In some embodiments, the spatially organized structure comprises a core of hepatocytes and peripheral bile duct-like structures formed by cholangiocytes around the core of hepatocytes. In some embodiments, the spatially organized structure comprises endogenous extracellular matrix adhesion molecules. In some cases, the spatially organized structure comprises liver parenchymal cells in both the interior and exterior of the organoid. In some embodiments, the hepatocytes are connected by a network of bile canaliculi to the cholangiocyte bile duct-like structures.

Also described herein is a media for generating hepatic organoids of the invention, the media comprising:
a) an activator of STAT3, GAB1 mediated cell adhesion and AKT/PI3K signaling pathways;
b) a molecule which is an repressor of NFκB activity and activator of mitogen-activated protein (MAP) kinase ERK, p38 and JNK;
c) a TGF-β inhibitor and/or SMAD2/3 inhibitor;
e) a steroid;
f) at least one molecule for inducing phosphorylation of SMAD1, SMADS and SMAD8 and activating MAPK signaling; and
g) a molecule that regulates bile acid synthesis and/or activates the FGF and MAPK pathway.

, The media may further comprising a WNT-signaling activator.

The media for generating hepatic organoids described herein, my further comprise:
a TGF-β inhibitor and/or SMAD2/3 inhibitor;
a pleiotropic cytokine that belongs to the interleukin 6 group of cytokines;
an inhibitor of γ-secretase; and
a steroid.

In another aspect, a method of deriving and maintaining a hepatic (liver) organoid is provided, the method comprising:
a) culturing an endoderm stem cell in a first cell culture medium to obtain an early hepatic progenitor;
b) transferring and culturing the cells obtained under a) in a suspension culture system in a second cell culture medium to obtain a late hepatic progenitor; and
c) culturing the late hepatic progenitors obtained under b) in a suspension culture system in a third cell culture medium to obtain a hepatic (liver) organoid, wherein the first medium comprises:
   an activator of STAT3, GAB1 mediated cell adhesion and AKT/PI3K signaling pathway;
   a molecule which is an repressor of NFκB activity and activator of mitogen-activated protein (MAP) kinase ERK, p38 and JNK;
   a TGF-β inhibitor and/or SMAD2/3 inhibitor;
   a WNT-signaling activator;
   a steroid;
   at least one molecule(s) inducing phosphorylation of SMAD1, SMADS and SMAD8 and activating MAPK signaling; and
   a molecule activating the FGF and MAPK pathway, wherein:
      i) the activator of STAT3, GAB1 mediated cell adhesion and AKT/PI3K signaling pathway in the first medium is a hepatocyte growth factor (HGF) at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, 70 ng/ml, or about 1, 2.5, 3.5 or 4.5 µg/ml; and
      ii) the molecule which is an repressor of NFκB activity and activator of mitogen-activated protein (MAP) kinase ERK, p38 and JNK in the first medium is a compound selected from the group consisting of nicotinamide, nicotinic acid, 5-fluoronicotinamide, isonicotinic acid hydrazide, and nikethamide; at a concentration of between about 0.1 mM to 1.0 M, or between about 2 mM to 0.8 M, or between about 4 mM to 0.6 M, or between about 6 mM to 0.4 M, or between about 8 mM to 0.2 M, or between about 10 mM to 800 mM, or between about 50 mM to 500 mM, or about 3, 5, 9, 15, 20, 30, 50, 80, 100, 120, 150, 200, 250, 300, 350, 400 or 450 mM; and
      iii) the TGF-β inhibitor in the first medium is selected from the group consisting of
         A83-01 3-(6-Methyl-2-pyridinyl)-N-phenyl-4-(4-quinolinyl)-1H-pyrazole-1-carbothioamide,
         A 77-01 4-(3-(6-methylpyridin-2-yl)-1H-pyrazol-4-yl)quinoline,
         SD-208 2-(5-chloro-2-fluorophenyl)-N-pyridin-4-ylpteridin-4-amine, LY2157299 4-[2-(6-methylpyridin-2-yl)-5,6-dihydro-4H-pyrrolo[1,2-b]pyrazol-3-yl]quinoline-6-carboxamide,
         SB 431542 4-[4-(1,3-benzodioxol-5-yl)-5-pyridin-2-yl-1H-imidazol-2-yl]benzamide, GW788388 N-(oxan-4-yl)-4-[4-(5-pyridin-2-yl-1H-pyrazol-4-yl)pyridin-2-yl]benzamide,
         SB505124 2-[4-(1,3-benzodioxol-5-yl)-2-tert-butyl-1H-imidazol-5-yl]-6-methylpyridine,
         SB525334 6-[2-tert-butyl-5-(6-methylpyridin-2-yl)-1H-imidazol-4-yl]quinoxaline, IN 1130 2-[3-[(4-amino-2-methylpyrimidin-5-yl)methyl]-4-methyl-1,3-thiazol-3-ium-5-yl]ethanol,
         ITD 1 (6,6-dimethyl-5,7-dihydroimidazo[2,1-b][1,3]thiazol-4-ium-3-yl)methyl N,N'-dicyclohexylcarbamimidothioate,
         LY2109761 4-[2-[4-(2-pyridin-2-yl-5,6-dihydro-4H-pyrrolo[1,2-b]pyrazol-3-yl)quinolin-7-yl]oxyethyl]morpholine,
         K02288 3-[6-amino-5-(3,4,5-trimethoxyphenyl)pyridin-3-yl]phenol,
         TGF-β RI kinase inhibitor [3-(Pyridin-2-yl)-4-(4-quinonyl)]-1H-pyrazole] and derivatives thereof; and
      iv) wherein the TGF-β inhibitor in the first medium is at a concentration of between about 0.5 nM to 20 µM, or 100 nM to 10 µM, or 250 nM to 5 µM, or 400 nM to 2.5 µM, or 0.5 nM to 1 µM, or 0.5 nM to 0.5 µM, or between about 1.5 nM to 0.4 µM, or between about 10 nM to 0.3 µM, or between about 30 nM to 0.2 µM, or between about 40 nM to 0.1 µM, or between about 50 nM to 85 nM, or about 1, 5, 15, 25, 30, 35, 45, 50, 65, 75, 130, 150, 170, 250, 350 or 450 nM; or about 0.5, 0.7, 0.9, 1, 1.5, 2, 2.5, 3, 3.5, 4, 4.5, 5, 5.5, 6, 8, 9, 10, 12, 14, 16, 18, or 20 µM; or
      v) the SMAD2/3 inhibitor is selected from the group consisting of a Smad2/3 phosphorylation inhibitor and siRNA targeting the mRNA of SMAD2 and SMAD3 transcript; and
      vi) the WNT-signaling activator in the first medium is a Glycogen synthase kinase 3 (GSK3) inhibitor; and the WNT-signaling activator in the first medium is at a concentration of between about 0.1 µM to 10 µM, or between about 0.5 µM to 8 µM, or between about 1 µM to 7 µM, or between about 2 µM to 6 µM, or between about 3 µM to 5 µM, or about 0.2, 0.3, 0.4,3, 4, 4.5, 5, 6, 7.5, 8.5, 9, 9.5 or 10 µM; and
      vii) the steroid in the first medium is a corticosteroid; and the steroid is at a concentration of between about 0.5 µM to 200 µM, or between about 1.5 µM to 150 µM, or between about 5 µM to 100 µM, or between about 10 µM to 90 µM, or between about 20 µM to 80 µM, or between about 30 µM to 70 µM, or between about 40 µM to 60 µM, or about 2, 8, 15, 25, 30, 35, 45, 65, 75, 110, 130, 140, 160, 170 or 190 µM; or
      viii) at least one molecule(s) in the first medium inducing phosphorylation of SMAD1, SMADS and SMAD8 and activating MAPK signaling is/are selected from the group consisting of BMP4, BMP2, BMP3, BMP5, BMP6, and BMP7; and the at least one molecule(s) in the first medium inducing phosphorylation of SMAD1, SMADS and SMAD8 and activating MAPK signaling is at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, 70 ng/ml, or about 1, 2.5, 3.5 or 4.5 µg/ml; and
      ix) the molecule in the first medium activating the FGF and MAPK pathway is selected from the group consisting of FGF7, FGF1, FGF3, FGF10, and FGF22.

In some embodiments, the endoderm stem cell is an early endoderm progenitor cell, a pluripotent stem cell, an induced pluripotent stem cell, a human embryonic stem cell, an MESP, or an adult liver stem cell. In some embodiments, the endoderm stem cell is an MESP or an adult liver stem cell.

In some embodiments, the culturing under a) is carried out together with a cellular support or an extracellular matrix. In some embodiments, the extracellular matrix promotes cell differentiation and is made of a material selected from the group consisting of matrigel, gelatine, methylcellulose, collagen, alginate, alginate beads, agarose, fibrin, fibrin glue, fibrinogen, blood plasma fibrin beads, whole plasma or components thereof, laminins, fibronectins, protecogylcans, HSP, chitosan, heparin, other synthetic polymer or polymer scaffolds and solid support materials.

In some embodiments, the culturing under a) is for 1 to 10 days or 1 to 8 days or 1 to 6 days. In some embodiments, the culturing under b) is for 6 to 12 days or 4 to 10 days or 6 to 8 days. In some embodiments, the culturing under c) is for 18 to 26 days or 20 to 24 days or 19 to 22 days.

As described herein, the second cell culture medium comprises:
an activator of STAT3, GAB1 mediated cell adhesion and AKT/PI3K signaling pathway;
a molecule which is an repressor of NFκBactivity and activator of mitogen-activated protein (MAP) kinase ERK, p38 and JNK;
a TGF-β inhibitor and/or SMAD2/3 inhibitor;
a steroid;
a molecule inducing phosphorylation of SMAD1 and SMADS and activating MAPK signaling; and
a molecule that regulates bile acid synthesis and activates a FGF and MAPK pathway.

As described herein, the second cell culture medium may further comprise a component to promote survival of late hepatic progenitors, wherein the component is selected from one or two or three or all of the following components:
an activator of AKT/PI3K signaling pathway and MAPK signaling pathway;
an activator of cAMP-dependent pathways or an activator of Protein Kinase A signaling pathway;
an activator of the Notch receptor;
an inhibitor of histone deacetylase (HDACs); and/or
a component for inducing late hepatic progenitor formation.

As described herein, the component for inducing late hepatic progenitor formation is a WNT-signaling activator, an inhibitor of γ-secretase; and/or a YAP inhibitor.

As described herein, the third cell culture medium comprises:
a TGF-β inhibitor and/or SMAD2/3 inhibitor;
a pleiotropic cytokine that belongs to the interleukin 6 group of cytokines;
an inhibitor of γ-secretase; and/or
a steroid.

As described herein, the third cell culture medium may further comprise:
a compound inducing phosphorylation of SMAD1, SMADS and SMAD8 and activating MAPK signaling;
an interleukin that acts as both a pro-inflammatory cytokine and an anti-inflammatory myokine;
a compound that acts as a hormone regulating bile acid synthesis and activates FGF and MAPK pathway;
an activator of cAMP-dependent pathways;
a YAP inhibitor;
a compound with biliary acid potency;
an activator of STAT3, GAB1 mediated cell adhesion and AKT/PI3K signaling pathway;
a glycosaminoglycan; and/or
an activator of AKT/PI3K signaling pathway and MAPK signaling pathway

Described herein but no longer claimed is a multipotent endoderm spheroid progenitor (MESP) cell is provided. , The MESP expresses one, two, three, four, five, six, seven, or more or all of the markers selected from the group consisting of HNF4A (NCBI Gene: 3172), PDX1 (NCBI Gene: 3651), CDX2 (NCBI Gene: 1045), SOX9 (NCBI Gene: 6662), KRT19 (NCBI Gene: 3880), AFP (NCBI Gene: 174), ONECUT2 (NCBI Gene: 9480), LGR5 (NCBI Gene: 8549), EPHB2 (NCBI Gene: 2048), LGR4 (NCBI Gene: 55366), NR5A2 (NCBI Gene: 2494), CDH1 (NCBI Gene: 999), KRT7 (NCBI Gene: 3855), ZNF503 (NCBI Gene: 84858), MSX2 (NCBI Gene: 4488), TRPS1 (NCBI Gene: 7227), ASCL2 (NCBI Gene: 430), IRF8 (NCBI Gene: 3394), HNF4G (NCBI Gene: 3174), ID2 (NCBI Gene: 3398), CD44 (NCBI Gene: 960), EPCAM (NCBI Gene: 4072), MET (NCBI Gene: 4233), IHH (NCBI Gene: 3549) and CLDN3 (NCBI Gene: 1365). In some embodiments, the MESP does not express a marker selected from the group consisting of SOX2 (NCBI Gene: 6657), CER1 (NCBI Gene: 9350), GATA4 (NCBI Gene: 2626), SOX17 (NCBI Gene: 64321), FOXA2 (NCBI Gene: 3170) and CXCR4 (NCBI Gene: 7852). In some embodiments, the karyotype of the MESP is normal for at least 10 passages in culture. In some embodiments, the MESP cells are polarized.

Also described herein but no longer claimed is a culture medium for deriving and maintaining endoderm spheroid progenitor cells is provided, the medium comprising:
a) a TGF-β inhibitor and/or SMAD2/3 inhibitor; and
b) a WNT-signaling activator.

As described herein, the medium may further comprise a steroid. As described herein, the WNT-signaling activator is a GSK3 inhibitor. As described herein, the medium further comprises an activator of AKT/PI3K signaling pathway and MAPK signaling pathway; an activator of STAT3, GAB1 mediated cell adhesion and AKT/PI3K signaling pathway; an activator of cAMP-dependent pathways and/or Protein Kinase A signaling pathway; a compound that activates the Notch receptor; a molecule which is an repressor of NFκB activity and activator of mitogen-activated protein (MAP) kinase ERK, p38 and JNK; a compound selected from the group consisting of nicotinamide, nicotinic acid, 5-fluoronicotinamide, isonicotinic acid hydrazide, and nikethamide; and an inhibitor of histone deacetylase.

Also described herein but no longer claimed isa method for producing a multipotent spheroid progenitor (MESP) cell is provided, the method comprising:
i) culturing an endoderm progenitor cell in a first medium on a cellular support under conditions suitable to differentiate the endoderm progenitor cell into a definitive endoderm (DE) cell;
ii) culturing the definitive endoderm cell in a second medium under conditions suitable to differentiate the definitive endoderm cell into a primitive gut cell; and
iii) culturing the primitive gut cell in a third medium on a cellular support under conditions suitable to differentiate the primitive gut cell into a MESP cell.

As described herein, the conditions suitable to differentiate the endoderm progenitor cell into a definitive endoderm (DE) cell, and the conditions suitable to differentiate the definitive endoderm cell into a primitive gut cell comprise two-dimensional monolayer culture. As described herein, the conditions suitable to differentiate the endoderm progenitor cell into a definitive endoderm (DE) cell, and the conditions suitable to differentiate the definitive endoderm cell into a primitive gut cell comprise three-dimensional monolayer culture. As described herein, the conditions suitable to differentiate the primitive gut cell into a MESP cell comprise three-dimensional culture.

As described herein, the first medium comprises an activator of TGF-β signaling pathway. As described herein, the second medium comprises an activator of BMP signaling pathway and an activator of FGF signaling pathway. In some embodiments, the third medium comprises an inhibitor of TGF-β signaling pathway, an activator of WNT signaling pathway, and an activator of Notch signaling pathway. In some embodiments, the third medium further comprises:
a) a SMAD2/3 inhibitor
c) a steroid
d) an activator of AKT/PI3K signaling pathway and/or MAPK signaling pathway
e) an activator of STAT3, GAB1 mediated cell adhesion and AKT/PI3K signaling pathway
f) an activator of cAMP-dependent pathways, such as an activator of Protein Kinase A signaling pathway
h) a repressor of NFκB activity and activator of mitogen-activated protein (MAP) kinase ERK, p38 and JNK, or
i) an inhibitor of histone deacetylase (HDACs).

As described herein, the cellular support comprises a material selected from the group consisting of matrigel, gelatine, methylcellulose, collagen, alginate, alginate beads, agarose, fibrin, fibrin glue, fibrinogen, blood plasma fibrin beads, whole plasma or components thereof, laminins, fibronectins, protecogylcans, HSP, chitosan, heparin, and synthetic polymers or polymer scaffolds.

### DEFINITIONS

The term "about," when modifying any amount, refers to the variation in that amount typically encountered by one of skill in the art, i.e., in the field of stem cell and organoid derivation and differentiation. For example, the term "about" refers to the normal variation encountered in measurements for a given analytical technique, both within and between batches or samples. Thus, the term about can include variation of 1-10% of the measured amount or value, such as +/- 1%, 2%, 3%, 4%, 5%, 6%, 7%, 8%, 9% or 10% variation. The amounts disclosed herein include equivalents to those amounts, including amounts modified or not modified by the term "about."

All numerical ranges disclosed herein include the lower and upper end points of the range, and all numerical values in between the end pionts, to the significant digit. For example, a range of 1 to 10 includes 1, 2, 3, 4, 5, 6, 7, 8, 9, and 10. A range of 0.1 to 5.0 includes 0.1, 0.2, 0.3, 0.4, 0.5, 0.6, 0.7, 0.8, 0.9, 1.0, 1.1, 1.2, ... 4.8, 4.9, and 5.0.

The term "substantially" when referring to expression of a gene, protein or cellular marker refers to the complete or nearly complete extent or degree of expression. For example, a cell population that is "substantially" negative of a particular cellular marker is either completely negative for the particular cellular marker or at least 95%, or at least 96%, or at least 97%, or at least 98%, or at least 99%, or at least 99.9% of the cell population is negative for the particular cellular marker. A cell culture system that is "substantially" free of a particular agent would mean that the cell culture system is either completely free of the agent or is at least 95%, or at least 96%, or at least 97%, or at least 98%, or at least 99%, or at least 99.9% free of the particular agent.

The term "organoids" refers to an *in vitro* 3 dimensional population of cells which resemble the vertebrate, mammalian or human organ. An organoid satisfies the following criteria; 1) contains multiple cell types of the organ, 2) different cell types are spatially organized into structures that resemble the organ tissue, 3) organoids should perform organ specific functions in vitro.

The term "spheroids" refers to an in vitro three-dimensional population of cells which form sphere-like structures. Unlike organoids, spheroids do not have multiple organ cell types, consist mainly of stem cells and do not form structures resembling the organs.

The term "parenchyma" refers to the functional cell types that compose the organ as compared to the connective and vascular supportive tissues.

The term "progenitor cell" refers to a cell state which has the ability to give rise to one or more daughter cells of a different cell state.

The term "early endoderm progenitor cell" refers to a cell that has ability to generate endodermal cell types in the organs of the human gut system. The early endoderm progenitor cell typically expresses early endoderm markers SOX17 and CXCR4.

The term "late endoderm progenitor cell" refers to a cell that has ability to generate endodermal cell types in the organs of the human gut system. The late endoderm progenitor cell typically expresses SOX9 but does not express early endoderm markers SOX17 and CXCR4.

The term "endoderm progenitor cell" refers to a cell that has the potential to generate all cell types found in the differentiated or adult liver tissue.

The term "endoderm spheroid progenitor cell" refers to a cell that is maintained in a spheroid culture system and has the potential to generate generate all cell types found in the differentiated or adult liver, intestine and pancreatic tissue.

The term "early hepatic progenitor" refers to a cell that has the potential to generate cell types found in the differentiated or adult liver tissue and expresses early hepatic progenitor markers such as AFP.

The term "late hepatic progenitor" refers to a cell that has the potential to generate cell types found in the differentiated or adult liver tissue and expresses late hepatic progenitor markers such as ALB.

The term "adult liver stem cell" refers to a cell that is isolated from adult liver and has the capacity to produce different cell types of the liver. In some embodiments, the adult liver stem cell is isolated from a mammal such as a rodent (e.g., mice or rats), bovine, porcine, or human.

The term "stem cell" refers to a cell state which can stably proliferate and maintain its cell state. A stem cell can undergo symmetrical cell division to give rise to 2 daughter cells of similar cell state or asymmetrical division to give rise to 1 daughter cell of similar cell state and 1 daughter cell of different cell state. The term includes an undifferentiated or unspecialized cell capable of perpetuating itself through cell division and having the potential to give rise to differentiated cells with specialized functions, such as liver cells, pancreatic cells, and intestinal cells.

The term "bile duct-like" refers to structures that resemble the bile ducts in a liver. The bile duct is formed by cholangiocytes which are organized to envelope a lumen.

The term "functionally connected" refers to a structural connection between two separate cell types, which, for example, facilitates the transportation of molecules between the two separate cell types, or provides conditions that promote maturation and differentiation of one or more cell types described herein. In some embodiments, the functional connection refers to transport of molecules between cells by diffusion, by active transport, or through a physical cellular structure such as a bile duct-like structure or bile canaliculi.

The term "not expressed" or "undetectable" refers to marker expression that is not more than 1.5 fold greater than the background expression or expression by a negative control. For example, if the assay is an immunofluorescence (IF) staining assay, then the protein is considered "not expressed" if the fluorescent signal is not greater than 1.5 fold the background signal when omitting the primary detection antibody, or is not greater than 1.5 fold the fluorescence signal of a control cell that does not express the marker (i.e., is negative for the marker). In microarray assays and quantitative RT-PCR assays, the transcript is not expressed when the RNA expression or relative intensity is less than 1.5 fold higher than a control cell that does not express the transcript.

The term "expressed" or "enriched" refers to the presence of more than 1.5-fold greater detectable marker expression when compared to background expression or expression by a negative control. For example, if the assay is quantitative PCR assay, then a marker is considered to be "expressed" or "enriched" if the expression level is greater than 1.5-fold the expression of a negative control sample. If the assay is an immunofluorescence (IF) staining assay, then the marker protein is considered "expressed" or "enriched" if the fluorescent signal is greater than 1.5 fold the background signal when omitting the primary detection antibody, or is greater than 1.5 fold the fluorescence signal of a control cell that does not express the marker (i.e., is negative for the marker).

The term "suspension culture" or "suspension culture system" refers to any culture conditions or system in which the cells are not embedded in a solid or semi-solid matrix and are free floating in the culture apparatus without resting on the bottom of the apparatus, or are not attached to a cellular feeder layer or cellular support layer.

The term "solid support materials" refers to solid or semi solid materials used in supporting cell growth where the cells are not in suspension culture.

The term "cellular support" refers a material that provides structural and nutritional support to cells in culture. The cellular support can provide both structural support and cytokines that play a part in maintaining liver stem cells in the undifferentiated state. The cellular support can comprise a material selected from the group consisting of matrigel, gelatine, methylcellulose, collagen, alginate, alginate beads, agarose, fibrin, fibrin glue, fibrinogen, blood plasma fibrin beads, whole plasma or components thereof, laminins, fibronectins, protecogylcans, HSP, chitosan, heparin, and synthetic polymers or polymer scaffolds. In some embodiments, the cellular support maintains the stem cells in a 3D structure such as a spheroid or organoid. In some embodiments, the cellular support comprises an extracellular matrix as further described herein.

The term "endogenous" refers to a component or molecule of a biological system that is produced by or synthesized by the cells or organoids described herein. The term "exogenous" refers to a component or molecule of a biological system that orginates from, or is produced or synthesized by an agent outside the biological system, for example, a molecule that is not produced by or synthesized by the cells or organoids described herein.

The term "genetically modified" refers to a cell that comprises an exogenouos nucleic acid that is not present in the unmodified cell, or that does not have the same structure as an endogenous nucleic acid or gene.

### BRIEF DESCRIPTION OF THE DRAWINGS

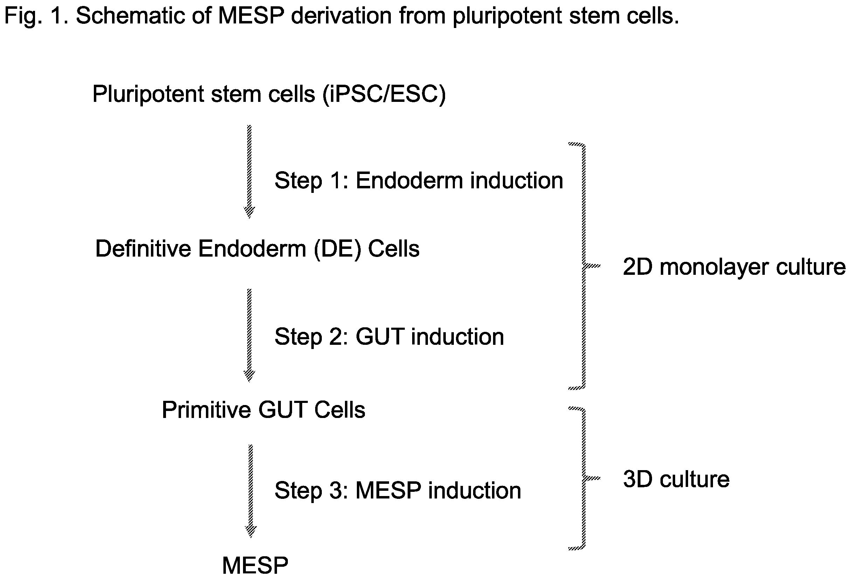

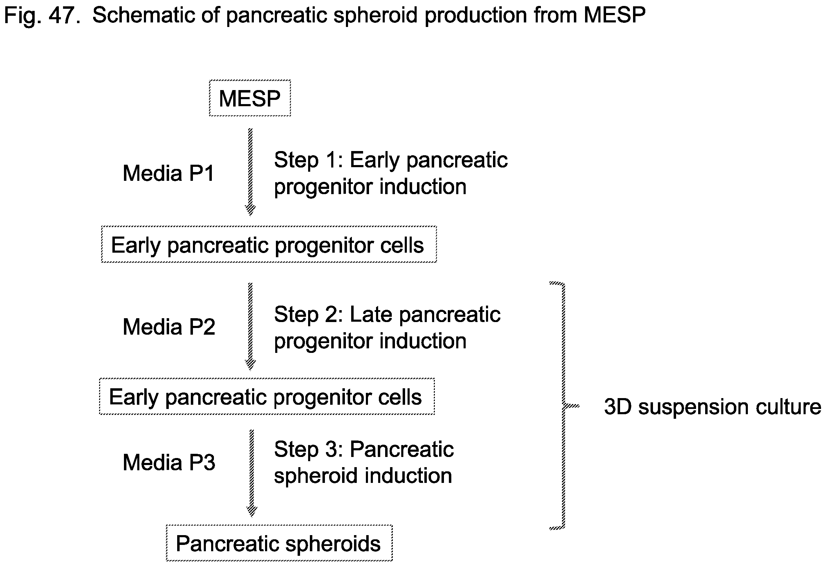

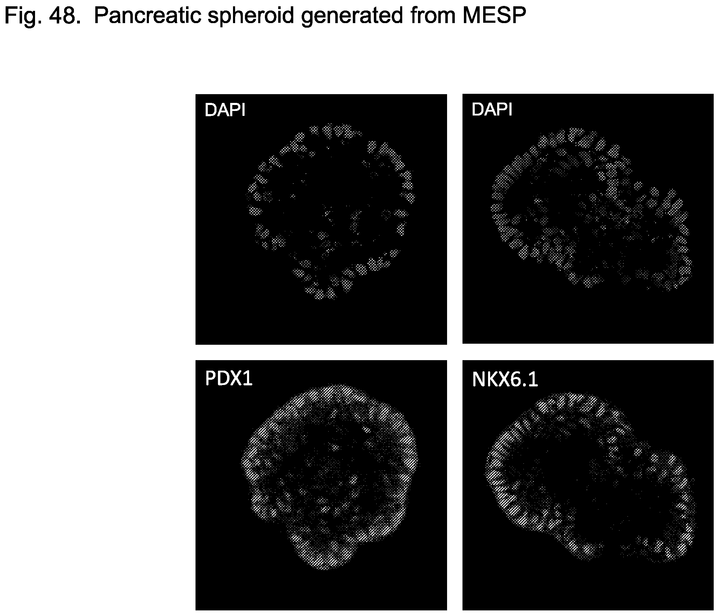

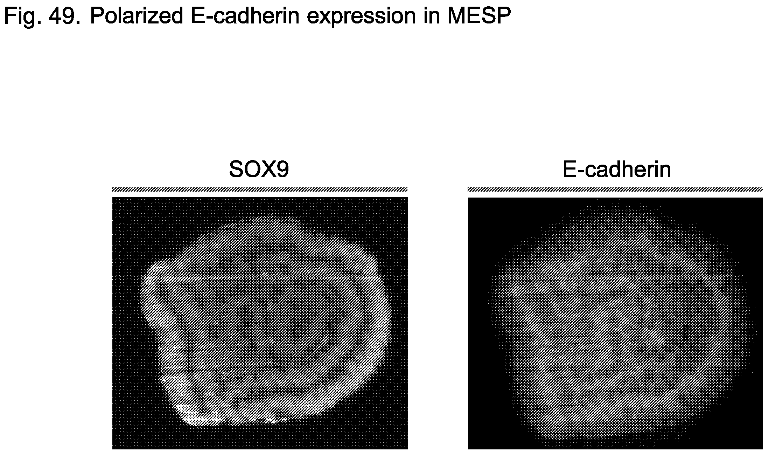

Figure 1. Schematic of MESP derivation from pluripotent stem cells. Stepwise differentiation of PSC along the endoderm lineage. PSC is differentiated to definitive endoderm cells and subsequently primitive gut cells in 2D before culturing in 3D to form MESP.

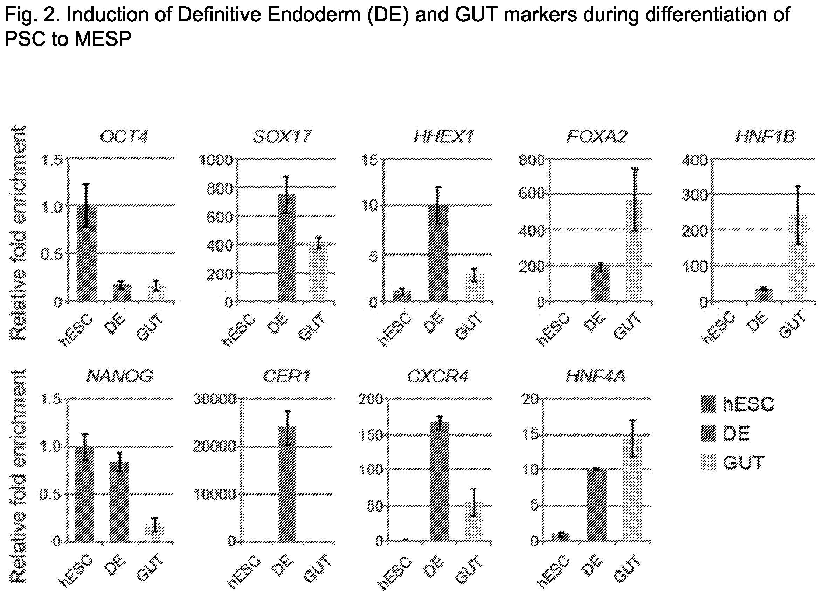

Figure 2. Induction of Definitive Endoderm (DE) and primitive GUT markers during differentiation of PSC to MESP. Pluripotency markers such as OCT4 and NANOG are downregulated in the process. Markers such as SOX17, HHEX1, CER1 and CXCR4 are upregulated in DE cells and subsequently downregulated as the cells further progress to form the primitive GUT cells. Markers such as HNF4A, HNF1B and FOXA2 are progressively upregulated. Results suggest a progressive commitment of the cells along the endoderm lineage and cells no longer resemble definitive endoderm progenitor.

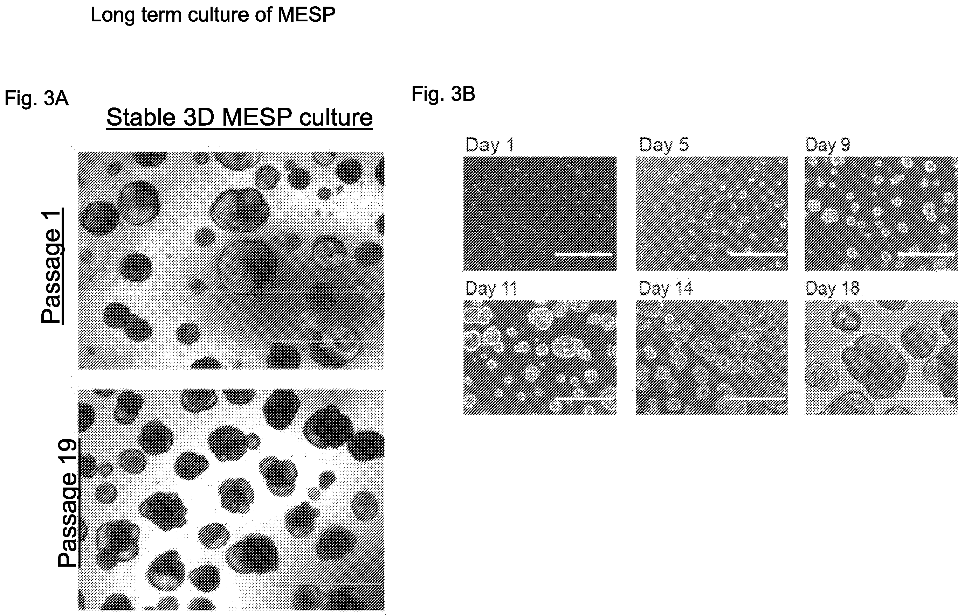

Figure 3A. Long term culture of MESP. MESP can be stably passage up to 19 times for approximately 260 days.
Figure 3B. Long term culture of MESP. MESP can form from single cells seeded during each passage. This reflects the clonal expansion potential of the cells and each stem cell within spheroids is capable of forming MESP.

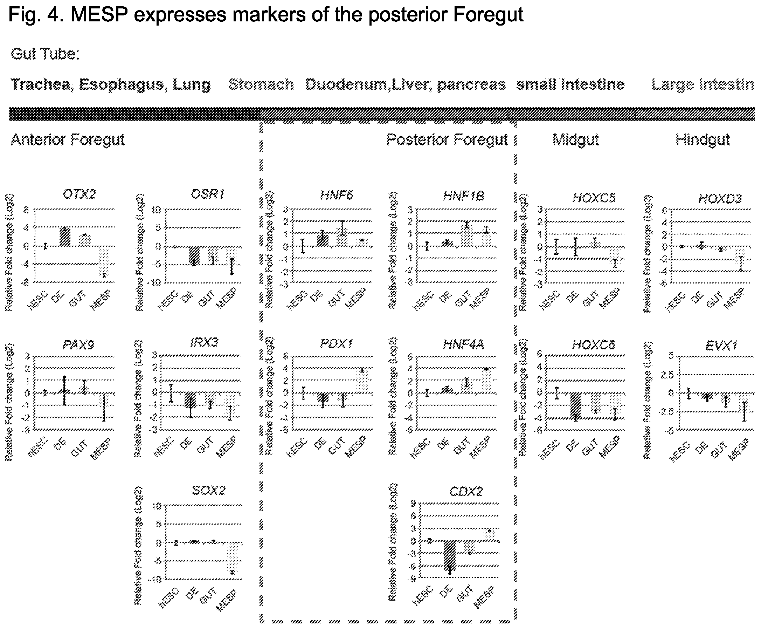

Figure 4. MESP expresses markers of the posterior Foregut. During development, various part of the gut tube give rise to major organs from the trachea to the large intestine. Specifically, the Anterior foregut give rise to the trachea, esophagus and lung, the posterior foregut give rise to the duodenum, liver and pancreas and the midgut and hindgut forms the small and large intestine respectively. Each regions of the gut tube expresses specific markers. MESP specifically expresses transcripts of genes that are enriched in the posterior foregut. Relative fold change was obtained by normalizing the level of gene expression to hESC. Data supports that these progenitors are capable of forming tissues of these 3 organs.

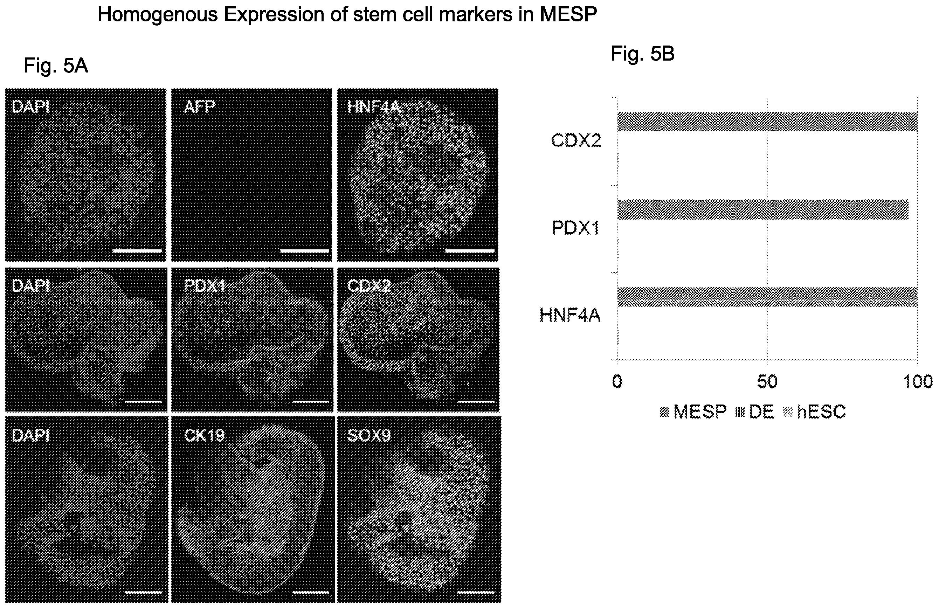

Figure 5A. Homogenous expression of stem cell markers in MESP. Protein markers such as HNF4A, CDX2 and PDX1 are homogenously expressed in all the cells. The cells also express adult stem cells markers such as CK19 and SOX9.
Figure 5B. Homogenous expression of stem cell markers in MESP. FACS analysis of MESP cells stained for the 3 markers PDX1, CDX2 and HNF4A. The result shows that the cells are homogenously stained for all 3 markers compared to the control DE cells and hESC which do not express these markers.

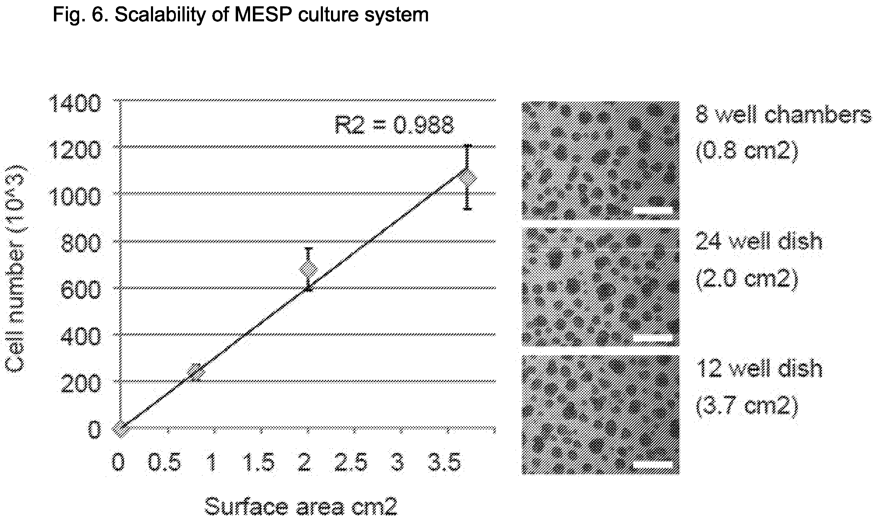

Figure 6. Scalability of MESP culture system. MESP can be culture in larger culture vessel from 8 well chamber slides to (surface area per well of 0.8cm² ) to 12 well dish (surface area per well 3.7cm²). The MESP cultured in different culture vessel is highly similar. The number of cells increases proportionally to the size of the culture vessel. The results underline the scalability of the culture system to generate large number of cells for downstream applications.

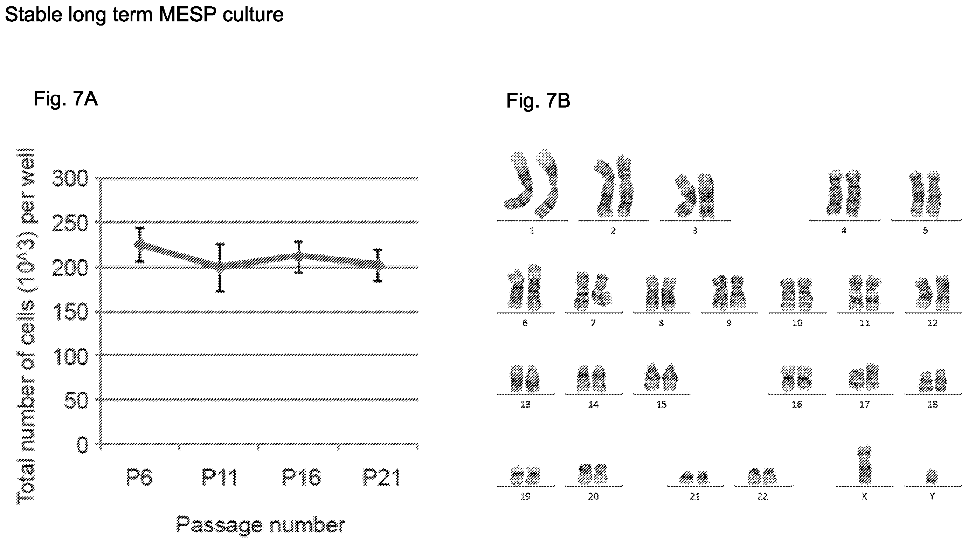

Figure 7A. Stable long term MESP culture. The number of cells generated per well (8 well chamber slide) remains consistent over long term culture. Total number of cells after 21 passages in a well is comparable to 6 passages. This data supports the long term self-renewing capacity of MESP and the cells do not senescence over long term culture up 280 days.
Figure 7B. Stable long term MESP culture. Cells in MESP are assayed for karyotypic abnormalities. The cells maintain a normal karyotype after long term culture for 10 passages (~140 days).

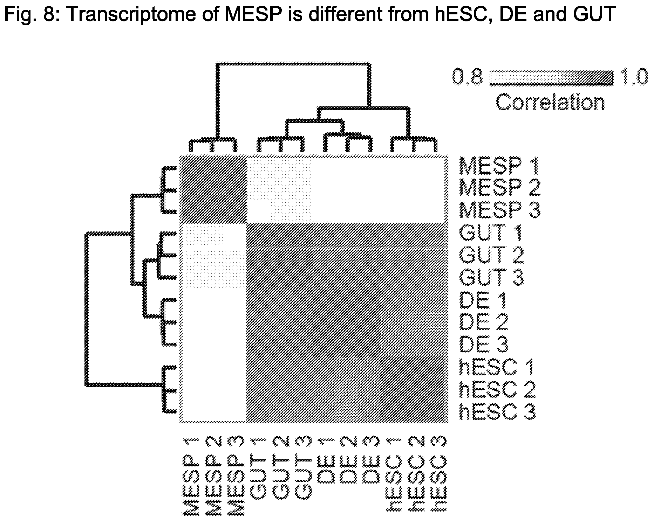

Figure 8. Transcriptome of MESP is different from hESC, DE and GUT. Global gene expression profile of hESCs, DE cells, GUT cells and MESPs are profiled using Whole genome microarrays. The transcriptome profile of MESP clusters distinctively from the other 3 cell states. The expression profile suggests that MESP is a unique stem cell state compared to DE and GUT endoderm cells.

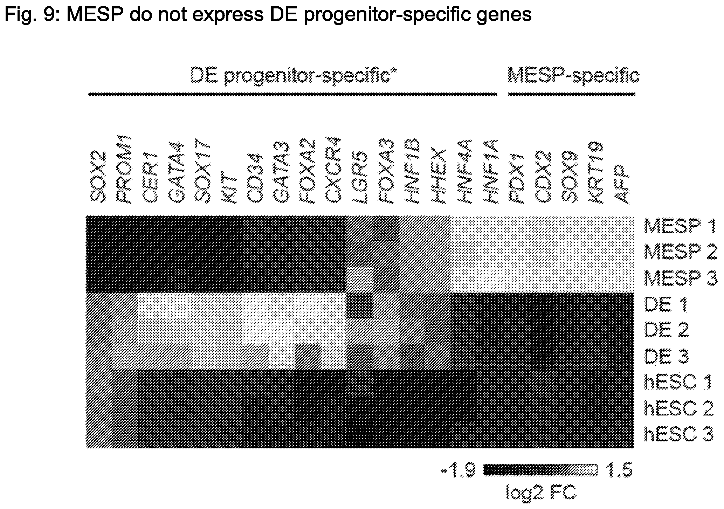

Figure 9: MESP do not express DE progenitor-specific genes. Expression of endoderm markers unique to DE progenitor cells (Cheng et al. and Hannan et al.) and MESP. DE progenitor markers identified in previous studies such as GATA3, GATA4, SOX17, FOXA2, CXCR4 are expressed in the DE cells profiled and not in MESP. Correspondingly, MESP specific markers such PDX1, CDX2, HNF4A, SOX9, and KRT19 are not expressed in the DE cells.

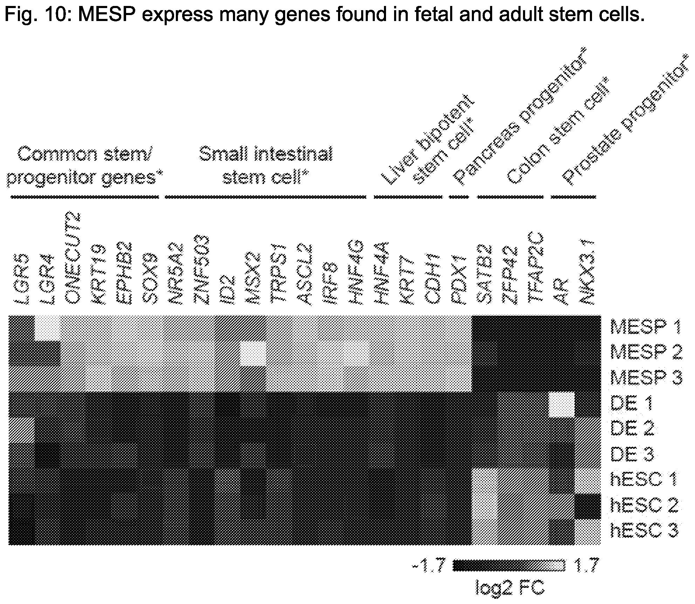

Figure 10: MESP expresses many genes found in fetal and adult stem cells. Expression of a list of the stem cell markers expressed in fetal and adult stem cells from various organs including intestine, liver, pancreas, colon and prostate. Many of these genes are expressed in MESP compared to DE and hESCs. Results underline the late endoderm cellular state of MESP.

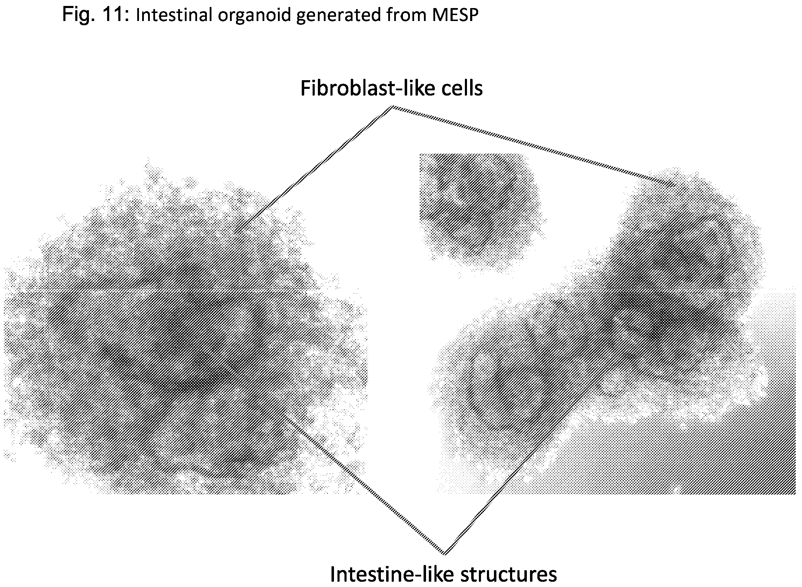

Figure 11: Intestinal organoid generated from MESP. MESP was differentiated to intestinal organoid using similar method describe in Spence et al. Within 3 weeks of differentiation, gut-like coiled structures which resemble the small intestine can be observed. The gut-like structures are envelope in a layer of fibroblast-like cells.

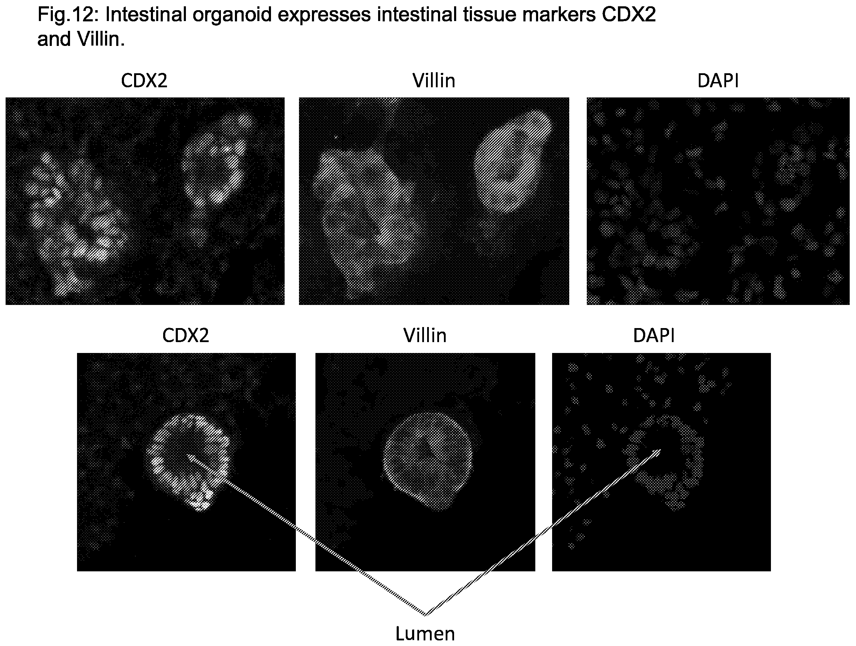

Figure 12. Intestinal organoid expresses key intestinal tissue markers CDX2 and Villin. All the cells in the intestinal organoids stains positive for the intestinal markers CDX2 and Villin. The cells self-organize in culture to form a lumen resembling the intestinal track. Asymmetrical distribution of Villin suggests the cells are polarized, similar to cells lining the small intestine.

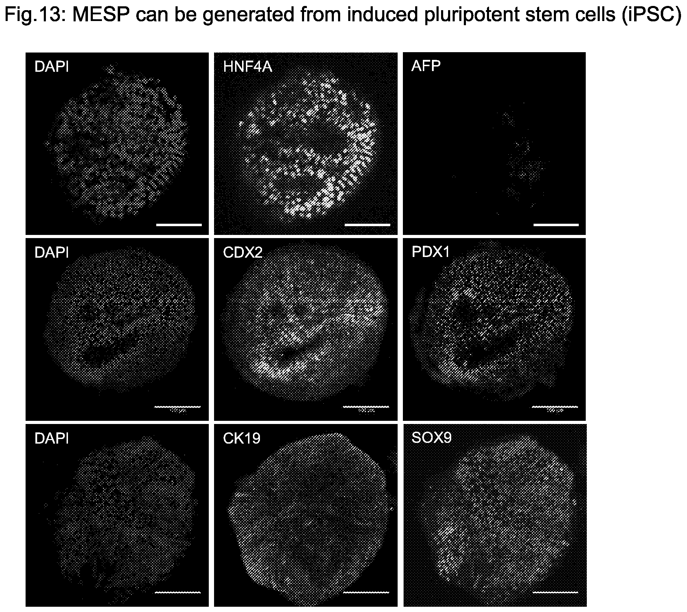

Figure 13: MESP can be generated from induced pluripotent stem cells (iPSC). While iPSCs closely resemble hESCs, these cells exhibits molecular differences and may not fully recapitulate hESCs characteristics. Results show that we can similarly apply the protocol to generate MESP from iPSCs. MESP generated from iPSCs also express key stem cell markers HNF4A, CDX2, PDX1, CK19 and SOX9.

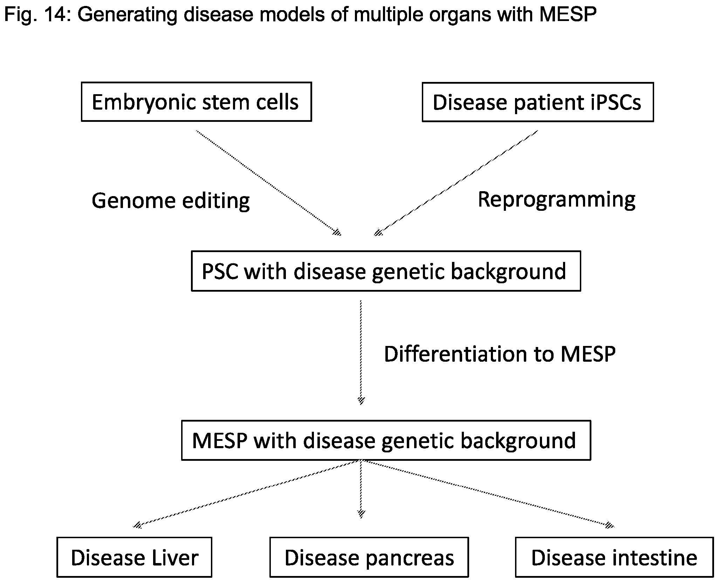

Figure 14: Generating disease models of multiple organs with MESP. Strategies to generate in vitro models of liver, pancreas and intestine diseases. Genetic disease of these organs can be modeled with stem cells which are amenable to genome editing tools such as CRISPR/Cas system. A large number of studies have shown that genetic mutations can be easily introduced into hESCs, and somatic cells harboring gene mutations for specific diseases can be reprogrammed to iPSCs. These 2 strategies enable the subsequent generation of MESP with disease genetic background. As such, the MESP culture system described herein would be useful for modeling disease of organs which the MESP is able to generate.

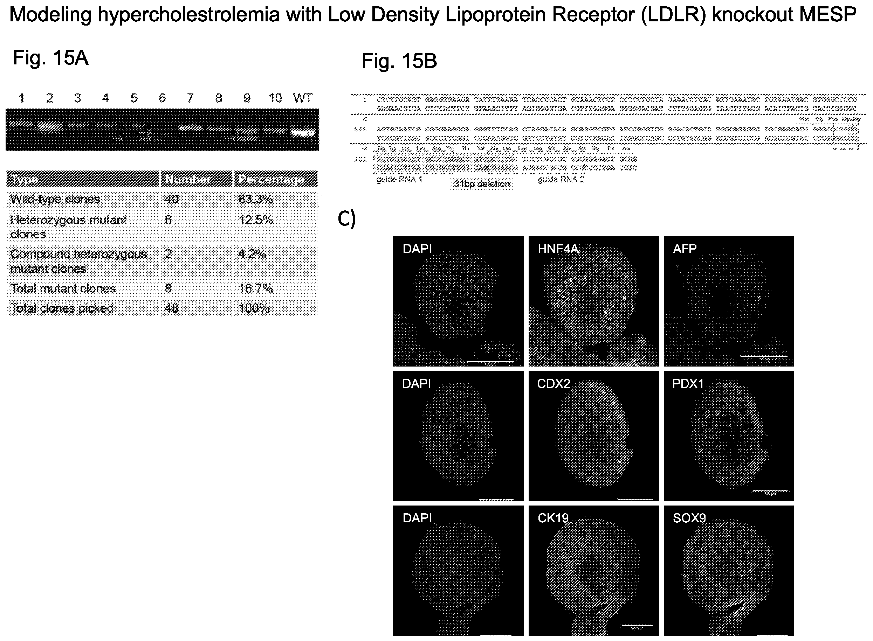

Figure 15A. Modeling hypercholesterolemia with Low Density Lipoprotein Receptor (LDLR) knockout MESP. The PCR amplification of the targeted LDLR exon 1 region from genomic DNA of 10 clones generated by genome editing with the CRISPR/Cas system. The red arrows demarcate shift in PCR products, indicating the presence of an insertion/deletion mutation which changes the size of the DNA. WT = wild-type control. The table outlines the mutation efficiency achieved for the LDLR loci genome editing using the CRISPR/Cas system.
Figure 15B. Modeling hypercholesterolemia with Low Density Lipoprotein Receptor (LDLR) knockout MESP. Sequence of exon 1 of LDLR gene locus (SEQ ID NOS:95-96). Highlighted region corresponds to the deleted sequence (SEQ ID NO:97) in the mutant allele from a clone with homozygous mutation. Guide RNA binding regions are highlighted in the dotted box (SEQ ID NO:98 (gRNA 1); SEQ ID NO:99 (gRNA 2).
Figure 15C. Modeling hypercholesterolemia with Low Density Lipoprotein Receptor (LDLR) knockout MESP. MESP generated from LDLR KO hESC. LDLR deficient MESPs express all the key stem cell markers similar to the wild type MESP.

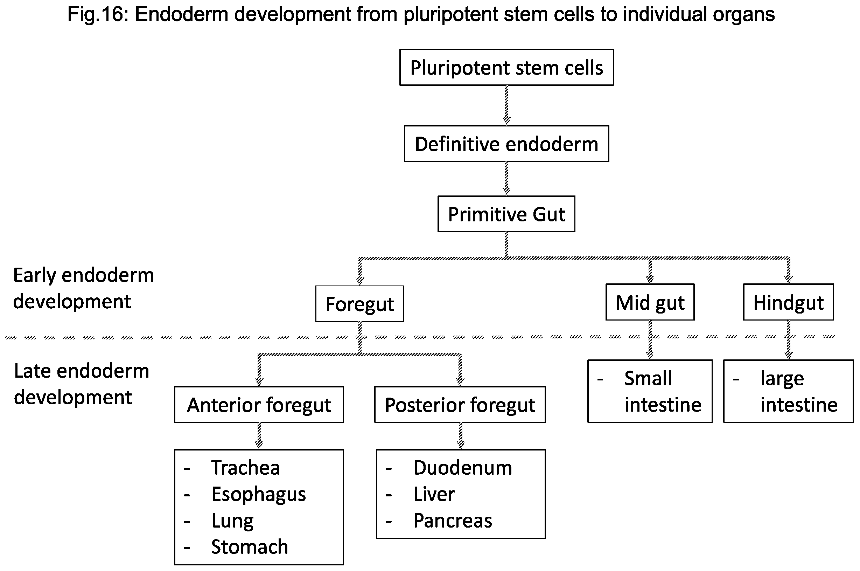

Figure 16. Endoderm development from pluripotent stem cells to individual organs. During development, the pluripotent stem cells first give rise to the definitive endoderm (DE) cells as the cells commit to the endoderm lineage. The DE cells give rise to the primitive gut where the anterior to the posterior regions would give rise to different organs. During late endoderm development, the different parts of the guts start to express specific regulation factors (master transcription factors) and is committed to form the respective organs, from the trachea in the anterior region to the large intestine in the hindgut. MESP represents late endoderm progenitor that is committed to form the liver, pancreas and duodenum. The key advantage of a late endoderm progenitor is the reduced cell contaminations when the progenitor is differentiated to specific tissue types. Early endoderm progenitors require more differentiation steps to give rise to target tissues. As these cells are less committed, they generate tissues of other organs during differentiation, generating contaminating cell types.

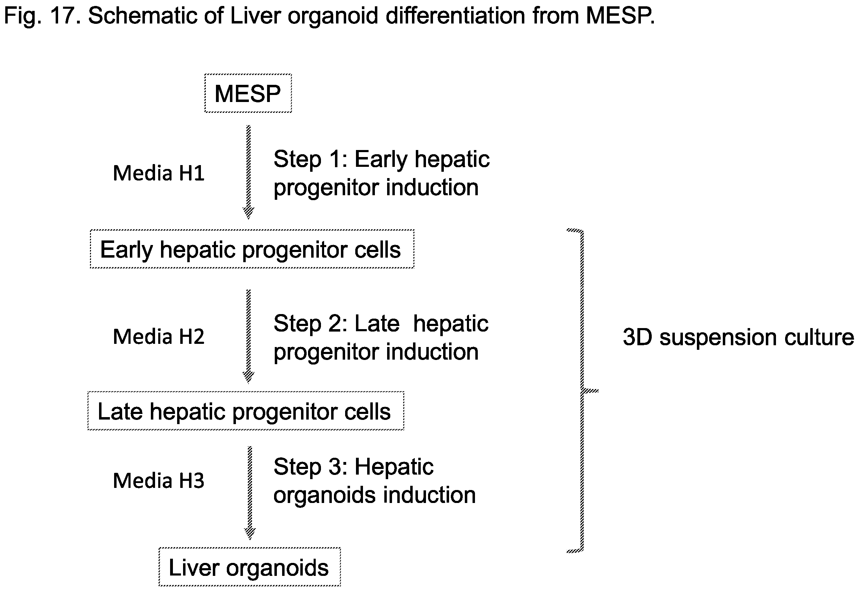

Figure 17. Schematic of Liver organoid differentiation from MESP. MESP are seeded as single cells in the H1 media in suspension. The cells aggregate to form a spheroid and expresses early hepatic progenitor markers such as AFP. The spheroids are subsequently treated with H2 induction media to induce late hepatic progenitor formation which express ALB. Lastly, the late hepatic progenitor spheroids are differentiated into hepatic organoids using H3 media

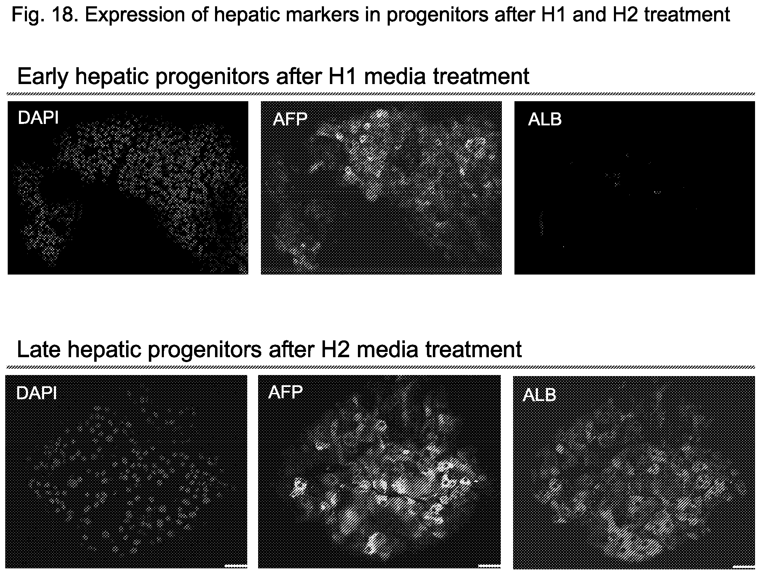

Figure 18. Expression of key hepatic markers in progenitors after H1 and H2 treatment. After H1 media treatment, the differentiated MESP (early hepatic progenitor) starts to express AFP. This marks the commitment of the cells into the hepatic lineage. After H2 media treatment, the cells express ALB, suggesting that the cells are late hepatic progenitors. The stepwise induction of the hepatic markers after each media treatment shows that the system is highly controlled and can be utilize for modeling liver development in vitro.

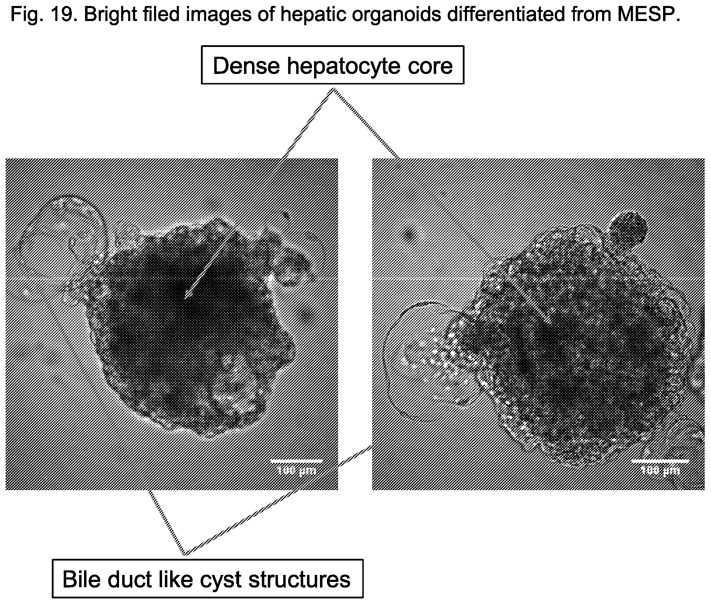

Figure 19. Bright field images of hepatic organoids differentiated from MESP. The organoids contain a dense core of hepatocyte cells reflect by the opaque center. To the peripheral, the cells are less dense and bile duct-like cyst structures can be observed.

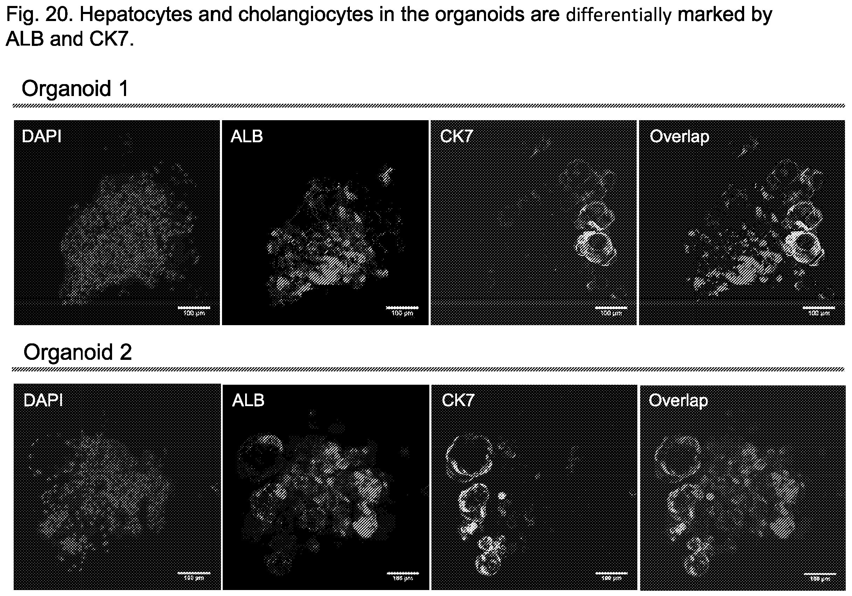

Figure 20. Hepatocytes and cholangiocytes in the organoids are differentially marked by ALB and CK7 respectively. In the organoids, the ALB positive cells are largely hepatocytes and cholangiocytes expresses CK7. The CK7 cells forms cyst like structures in the periphery of the organoid with a dense hepatocyte core. The cells are spatially organized and non-randomly distributed in the organoids.

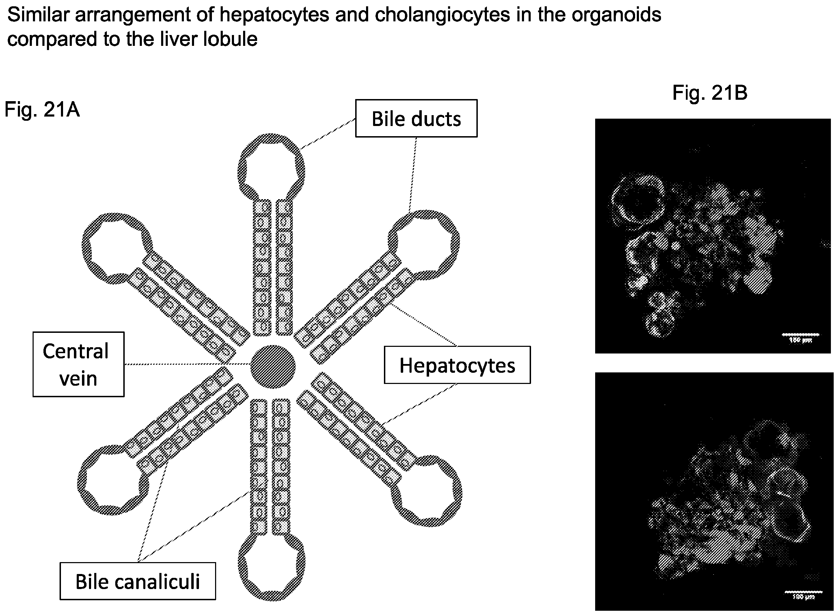

Figure 21A and 21B. Similar arrangement of hepatocytes and cholangiocytes in the organoids compared to the liver lobule. The organoids resemble the basic structural unit of the liver lobule (A), where the hepatocytes are located in the core of the liver lobule surrounding a portal vein, and the cholangiocytes similarly form bile duct-like cyst structures at the periphery of the hepatocyte core (B).

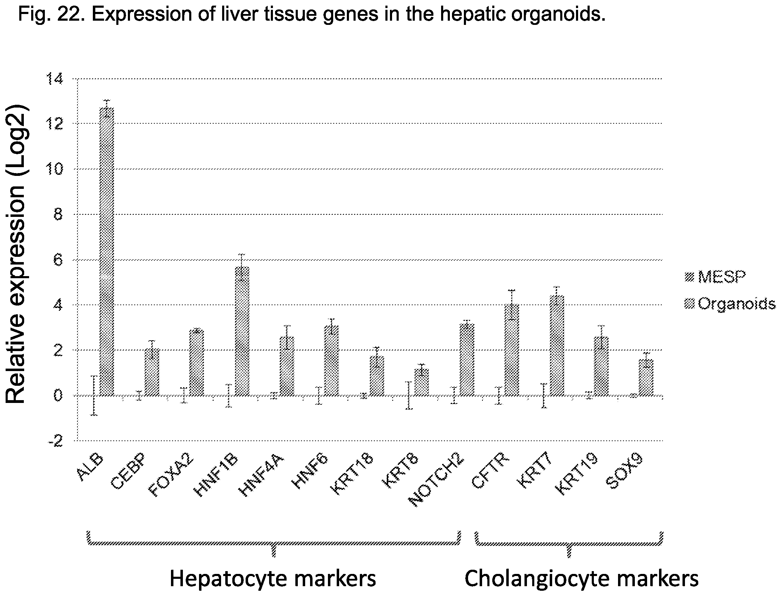

Figure 22. Expression of liver tissue genes in the hepatic organoids. Both hepatocyte specific genes and cholangiocyte specific genes are expressed in the liver organoids compared to undifferentiated MESP. Result supports the existence of both cell types in the organoids.

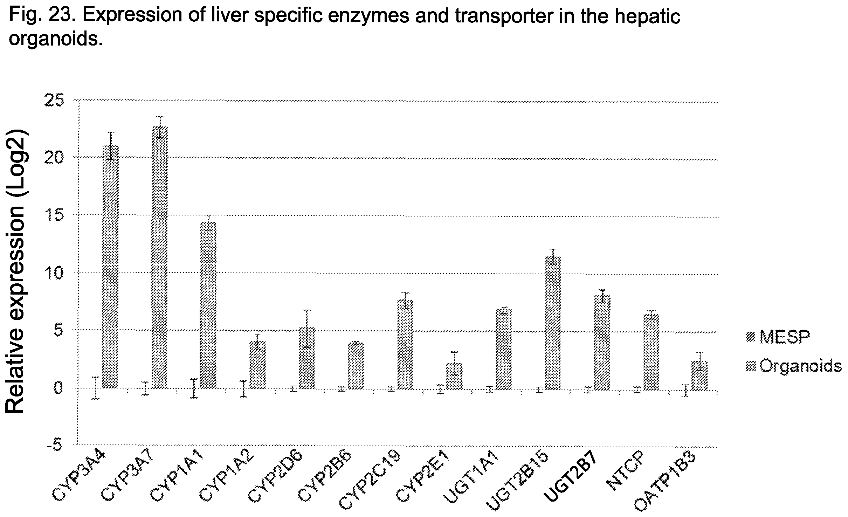

Figure 23. Expression of liver specific enzymes and transporter in the hepatic organoids. The liver organoids expresses all the major cytochrome P450 enzymes and also UDP-glucuronosyltransferase enzymes. These enzymes are important for all the metabolic and detoxification functions of the liver. Results supports that the organoids can perform metabolic activities of the liver organ. The organoids also expresses bile acid transporter NTCP and OATPIB3. Expression of these metabolic enzymes and transporters suggest that the organoids have detoxification and bile secretion function similar to the liver organ.

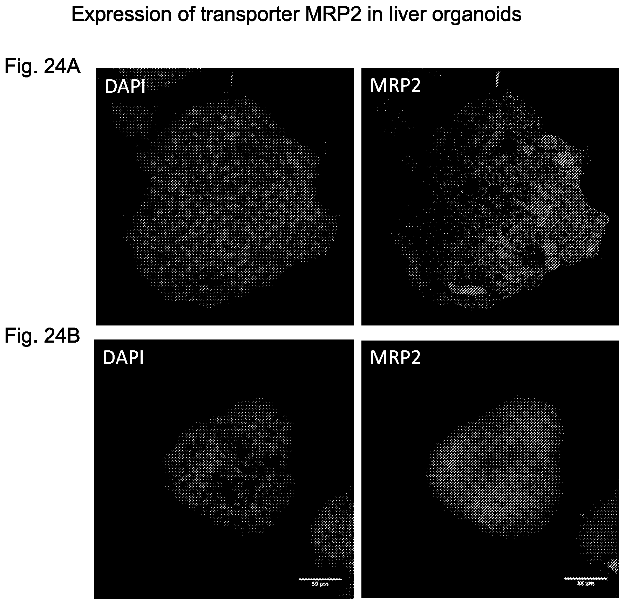

Figure 24. Expression of Multi-drug resistance-associated protein 2 (MRP2) in liver organoids. Staining of liver organoids with antibody specific for the transporter MRP2. The hepatocytes in the organoid expresses MRP2 and the staining shows networks in the hepatic organoid that is similar to bile canaliculi in liver tissue. MRP2 is localized to the apical region of hepatocytes and is responsible for hepatocyte bile secretion into the bile canaliculi. Expression of this protein in the organoids suggest the existence of a bile canaliculi network in the organoids.

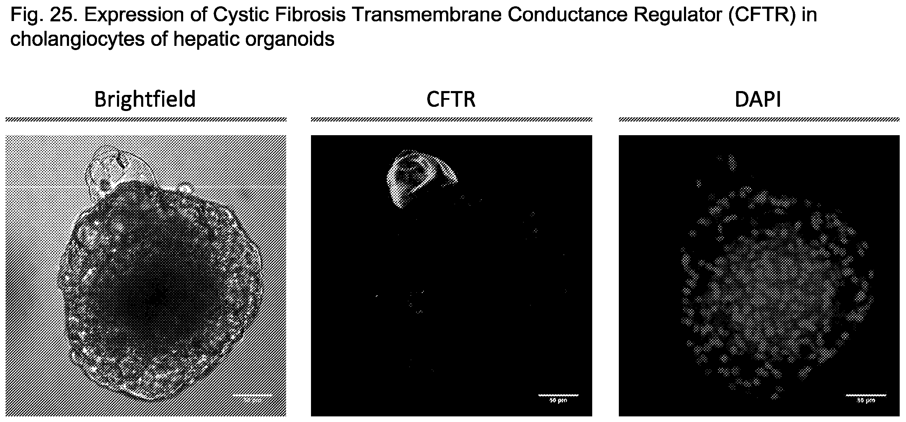

Figure 25. Expression of Cystic Fibrosis Transmembrane Conductance Regulator (CFTR) in cholangiocytes of hepatic organoids. Staining of liver organoids with antibody specific for the transporter CFTR. CFTR is exclusively expressed in the cholangiocytes in the bile duct like structures, similar to previous reports. CFTR is an important membrane transporter and mutation of this gene results in cystic fibrosis which is a common genetic disease.

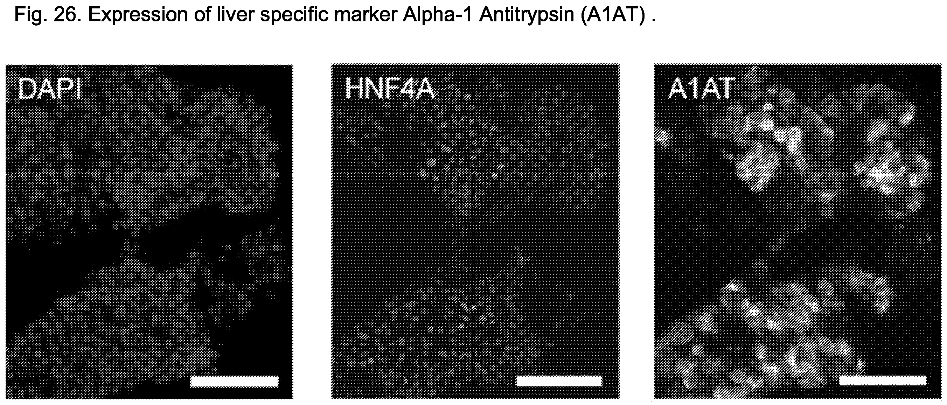

Figure 26. Expression of liver specific marker Alpha-1 Antitrypsin (A1AT). Staining of liver organoids with antibody specific for the A1AT. A1AT is a protease inhibitor produce and secreted by the liver and is important for the inhibition of enzymes such as elastase, secreted by the neutrophils. Deficiency in A1AT is a common genetic disease which results in lung disorders such as emphysema and chronic obstructive pulmonary disease (COPD).

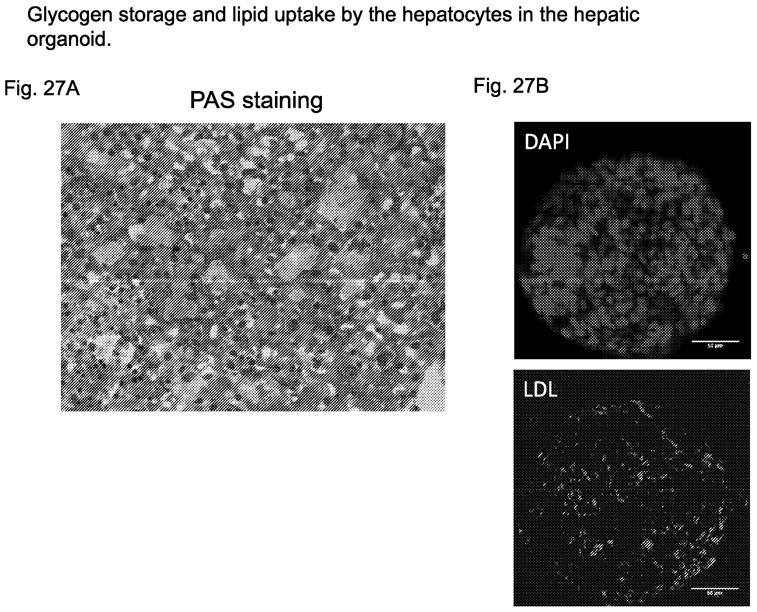

Figure 27A. Glycogen storage and lipid uptake by the hepatocytes in the hepatic organoid. PAS staining shows accumulation of the glycogen in the hepatocytes of the liver organoids. Results supports that the organoids have glucose regulation function similar to the liver.
Figure 27B. Glycogen storage and lipid uptake by the hepatocytes in the hepatic organoid. Organoids are treated with fluorophore labeled low density lipoprotein (LDL). Accumulation of the LDL can be observed in the cells of the organoids, suggesting that liver organoids can uptake lipids, similar to the liver organ.

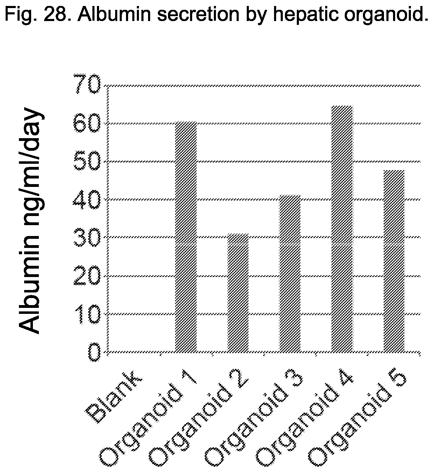

Figure 28. Albumin secretion by hepatic organoids. The culture media of the organoids is collected 24hrs after the media is changed. The amount of albumin in the media is quantitated by ELISA, with recombinant albumin as standards. Results confirm that the liver organoids can secrete albumin.

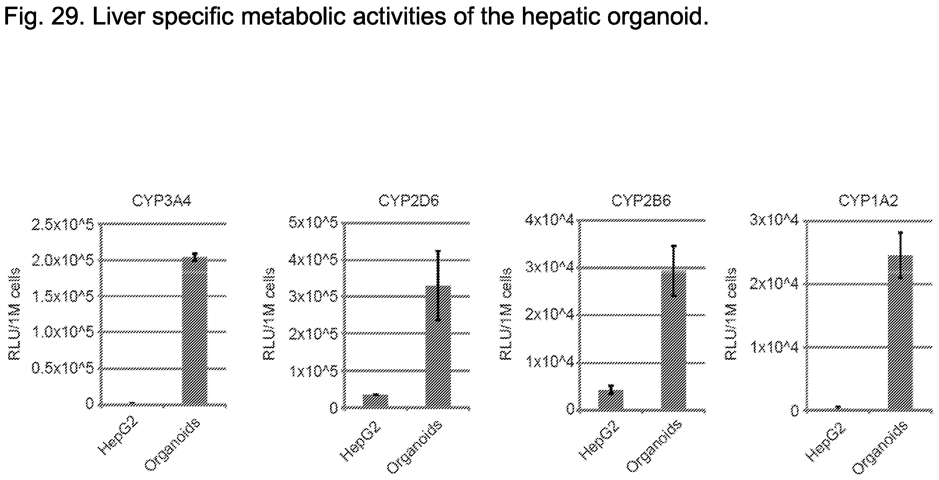

Figure 29. Liver specific metabolic activities of the hepatic organoid. Activities of individual cytochrome 450 enzymes profiled using luciferase-based assays. The relative luciferase unit is normalized to the total number of cells in the liver organoids. Shown are the relative luciferase unit for a million cells. The level of CYP metabolic activities of the liver organoids are compared to hepG2 cell lines commonly used in the industry. The liver organoids have much higher levels CYP1A2, CYP2B6 and CYP3A4 activities compared to HepG2. These 3 major cytochrome enzymes account for 80% of CYP activities in the liver. Thus, the liver organoids described herein have similar metabolic functions as the intact liver.

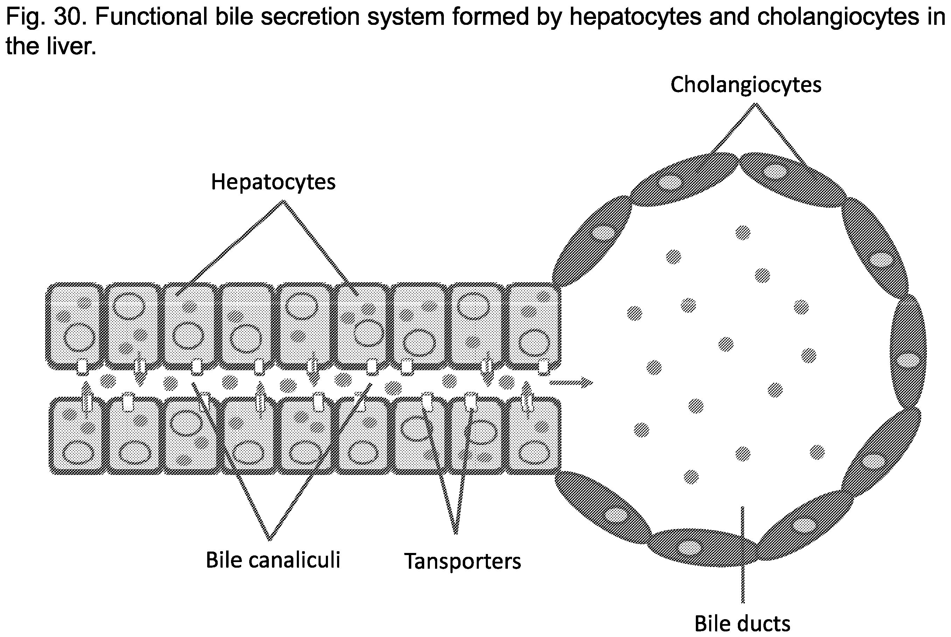

Figure 30. Functional bile secretion system formed by hepatocytes and cholangiocytes in the liver. Illustration of the organ level functions performed by the hepatocytes and cholangiocytes. Molecules generated by the liver are actively transported into the bile canaliculi which are intercellular channels formed between the hepatocytes. These molecules are transported along the bile canaliculi towards the bile duct formed by the cholangiocyte and transported out of the liver to the small intestines.

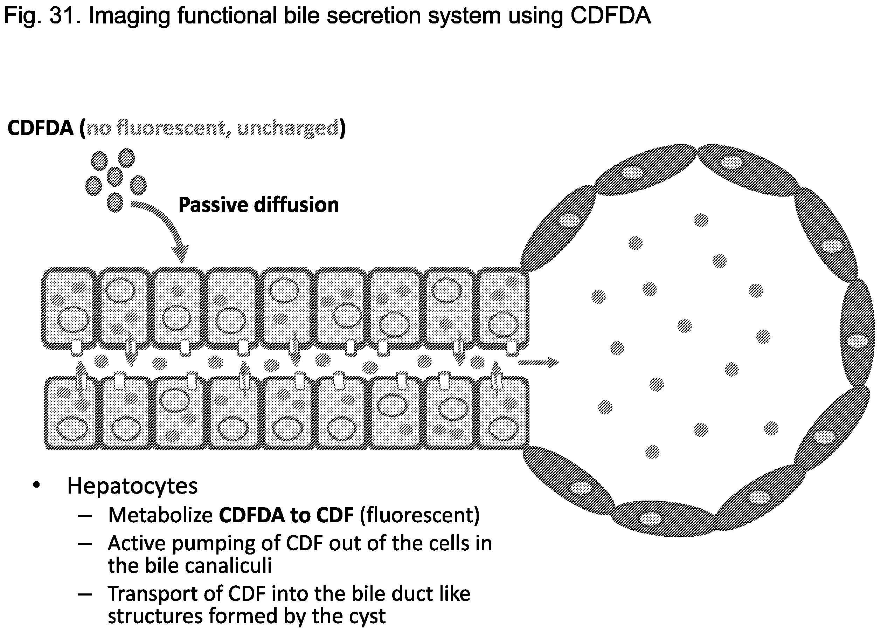

Figure 31. Imaging functional bile secretion system using CDFDA. Schematic diagram to illustrate how the functional bile secretion system is imaged in the liver organoids. CDFDA is an uncharged and non-fluorophetic molecule. This molecule passively diffuses into the cells. Hepatocytes which express de-esterification enzymes are able to convert CDFDA to CDF by removing the ester groups on the molecule. Unlike CDFDA, CDF is a charged fluorophore. CDF is actively pumped out of the hepatocyte through the MRP2/3 transporter into the bile canaliculi. CDF is transported along the bile canaliculi towards the bile duct formed by the cholangiocytes. In a chronological order, CDF would first accumulate in the hepatocytes (fluorescent in the hepatocytes), CDF would be actively pumped into the bile canaliculi and transported to the bile duct like structure. Unlike the hepatocytes, the cholangiocytes do not convert the CDFDA into the CDF. The CDF accumulated in the bile duct like structures are not produced by the cholangiocytes.

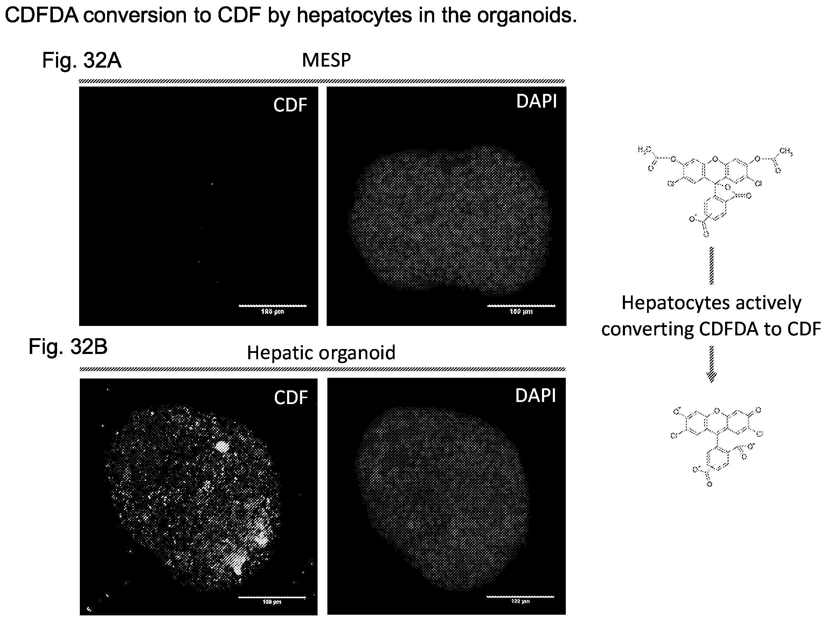

Figure 32A. CDFDA conversion to CDF by hepatocytes in the organoids. Live confocal images of undifferentiated MESP treated with CDFDA for 30 minutes. MESP cells do not have enzyme to convert CDFDA into CDF. No CDF is detected after 30 minutes of treatment.
Figure 32B. CDFDA conversion to CDF by hepatocytes in the organoids. Live confocal images of hepatic organoid treated with CDFDA for 30 minutes. CDF is produce in the hepatocytes and begin to accumulate in the organoids over 30 minutes.

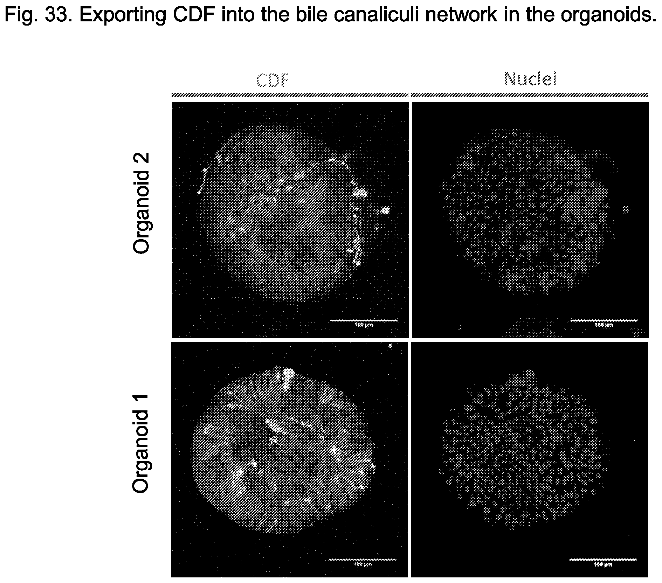

Figure 33. Exporting CDF into the bile canaliculi network in the organoids. Live confocal images of 2 organoids that have been treated with CDFDA for 3 hours. The CDF are no longer accumulated in the cells but exported into the bile canaliculi. The network of bile canaliculi is illuminated by the CDF. Both organoids show clear network of bile canaliculi formed within the organoids.

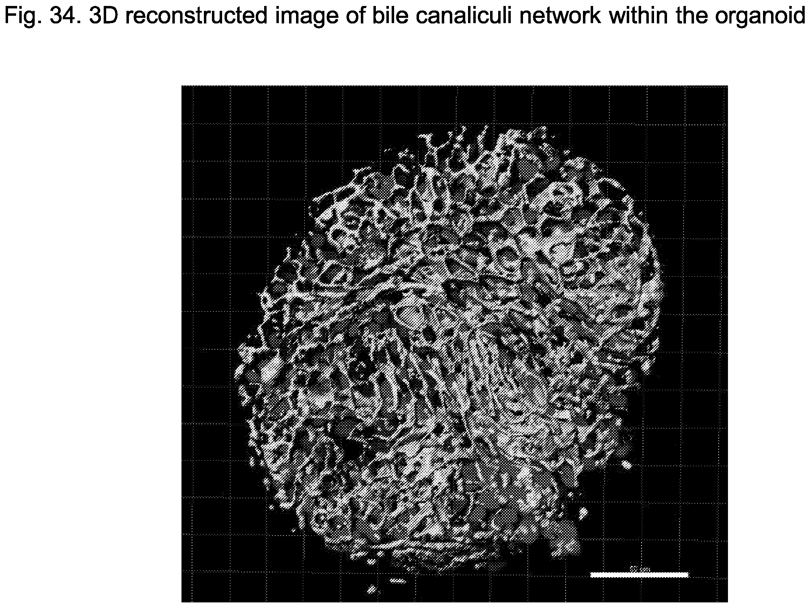

Figure 34. 3D reconstructed image of bile canaliculi network within the organoid. The Network of bile canaliculi envelopes each round nuclei which demarcates the cells. Image shows an intricate network of bile canaliculi channels formed within the organoid.

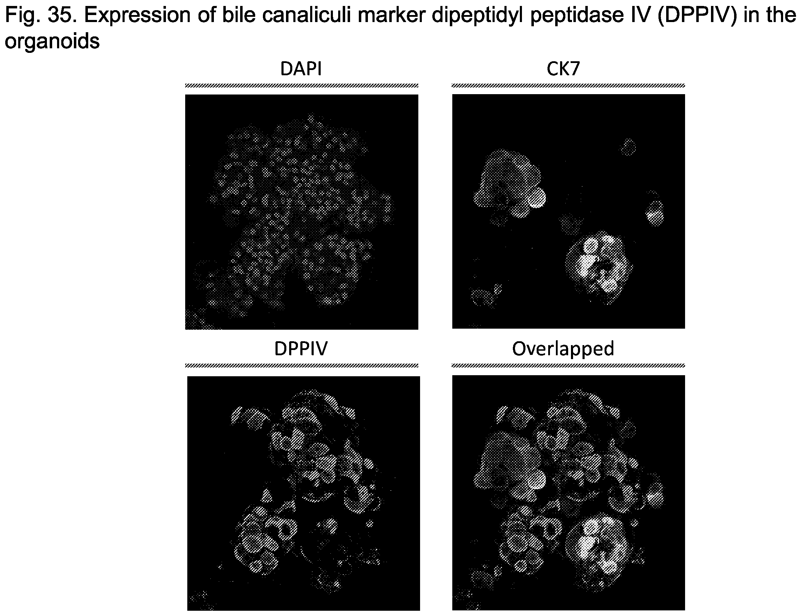

Figure 35. Expression of bile canaliculi marker dipeptidyl peptidase IV (DPPIV) in the organoids. CK7 expression marks the cholangiocytes in the organoids. The DPPIV is expressed on the surface of the hepatocytes that forms the bile canaliculi. The exclusive staining of DPPIV in the hepatocytes (no overlapping with the cholangiocytes) supports the existence of the bile canaliculi network in the hepatocyte core.

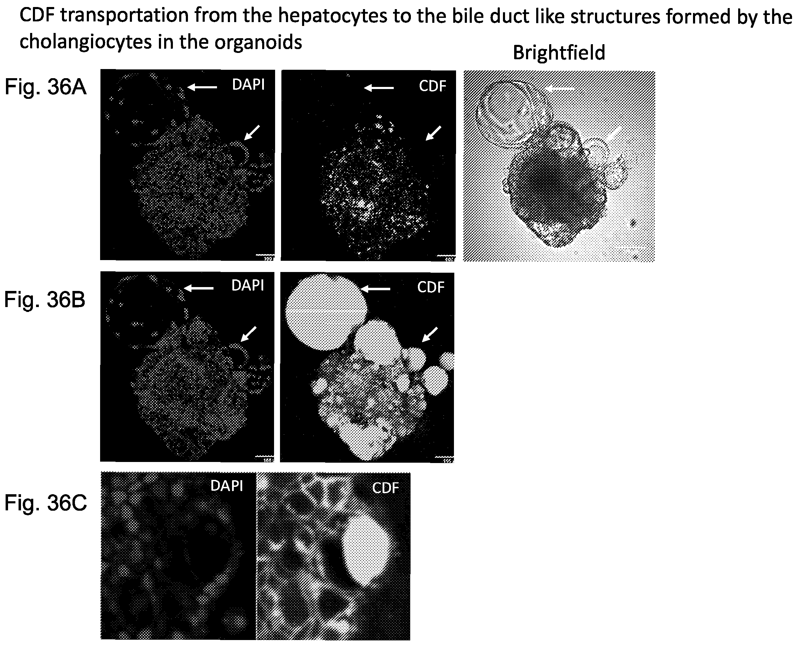

Figure 36A. CDF transportation from the hepatocytes to the bile duct-like cyst structures formed by the cholangiocytes in the organoids. Bright field image (far right) shows that the cholangiocyte forms large cyst structures surrounding the organoids. Organoids are treated with CDFDA for 30mins. Imaging of the CDF shows that the CDF is only produce in the hepatocyte cores. No CDF is produced by the cholangiocytes that forms the bile duct-like cyst structures (arrows).
Figure 36B. CDF transportation from the hepatocytes to the bile duct-like cyst structures formed by the cholangiocytes in the organoids. Organoids treated with CDFDA for 1hr. Network of bile canaliculi is formed in the core of the hepatocytes. The CDF is transported in the bile canaliculi to the bile duct like structure which can be seen clearly from the CDF filled cyst. Results show that the bile canaliculi network is functional in the organoids. The cholangiocytes forming the cyst do not produce CDF. CDF fills the network of bile canaliculi as more are produced after 1 hour treatment. The CDF is subsequently transported and fills the bile duct-like cyst structures.
Figure 36C. CDF transportation from the hepatocytes to the bile duct-like cyst structures formed by the cholangiocytes in the organoids. Enlarged image of the bile canaliculi network connection with the bile duct like structure. The bile canaliculi network formed in the hepatocyte core connects to the bile duct-like cyst structure in the periphery.

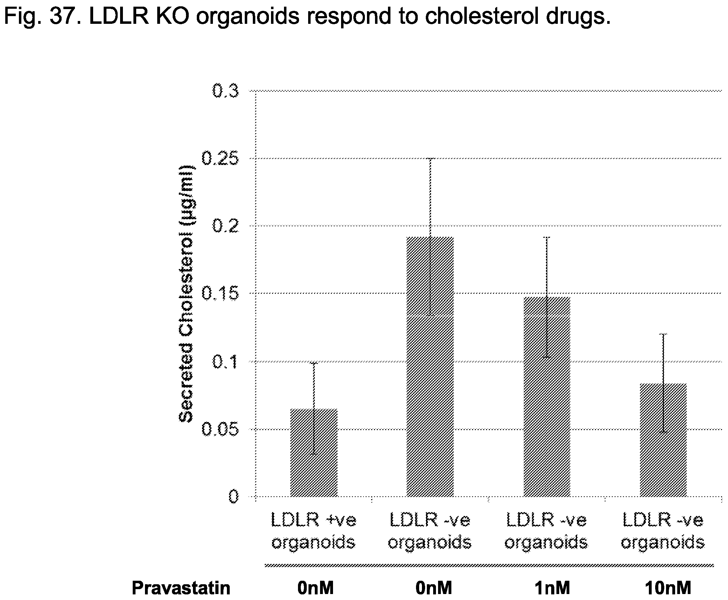

Figure 37. LDLR KO organoids respond to cholesterol drugs. LDLR deficient organoids are generated from the LDLR KO MESP. The LDLR deficient organoids secretes a higher level of cholesterol compared to normal liver organoids. Remarkably, this elevated secretion of cholesterol can be lowered by increasing levels of the statin treatment (10nM of pravastatin treatment lowered cholesterol secretion level to similar levels of wild type LDLR+ organoids).

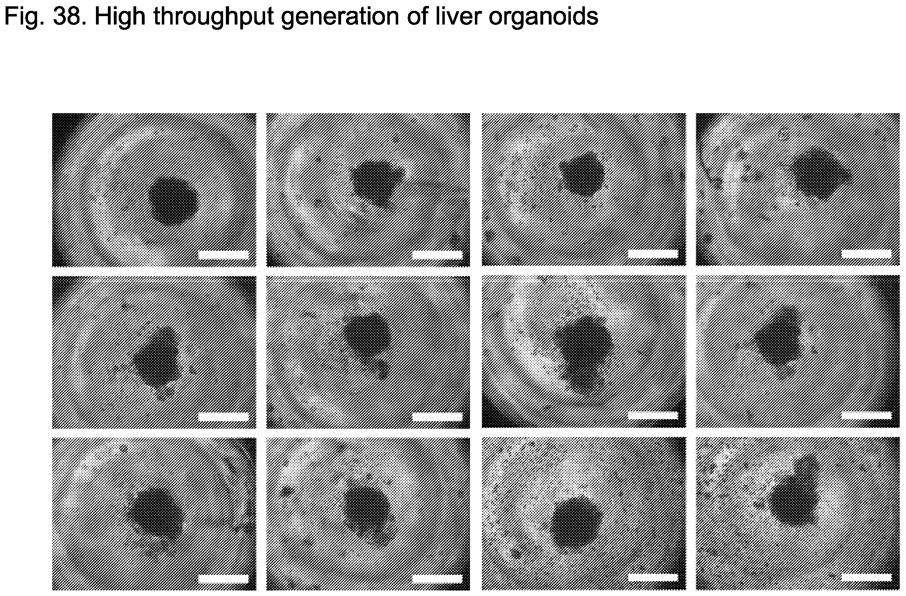

Figure 38. High throughput generation of liver organoids. Liver organoids can be produce in 96 well formats. Each well contains a single organoid of similar size and structure (scale bar =500µm). This allows the liver organoids to be used in screening platforms for drug screenings and toxicological testing.

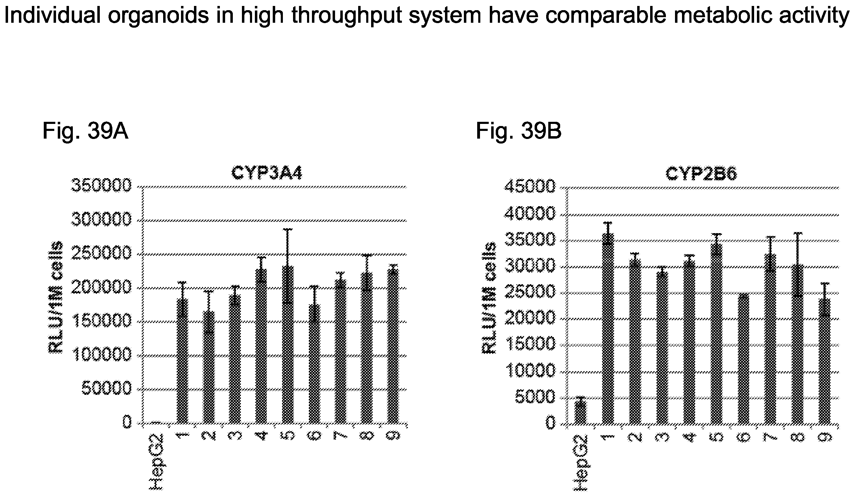

Figure 39A. Individual organoids in high throughput system have comparable metabolic activity. 9 organoids in different wells are tested for CYP3A4 activity. All the organoids show similar level of CYP3A4 activity. This result shows that the high throughput system generates organoids of similar metabolic function capacity.
Figure 39B. Individual organoids in high throughput system have comparable metabolic activity. 9 organoids in different wells are tested for CYP2B6 activity. All the organoids show similar level of CYP2B6 activity. This result shows that the high throughput system generates organoids of similar metabolic function capacity.

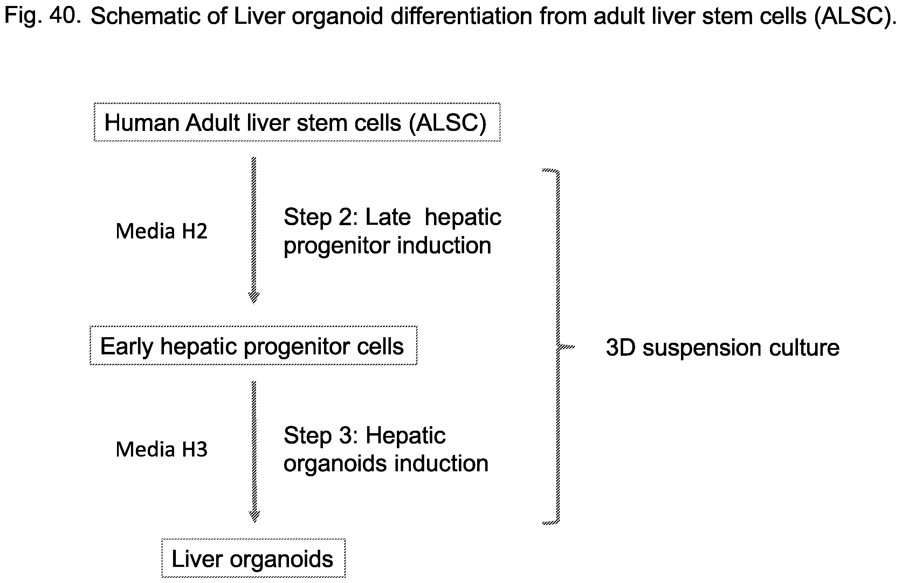

Figure 40. Schematic of Liver organoid differentiation from adult liver stem cells. Human liver adult stem cells are seeded as single cells in the H2 media in suspension. The cells aggregate to form a spheroid and expresses hepatic progenitor markers such as ALB. The spheroids are subsequently treated with H3 induction media to induce organoid formation.

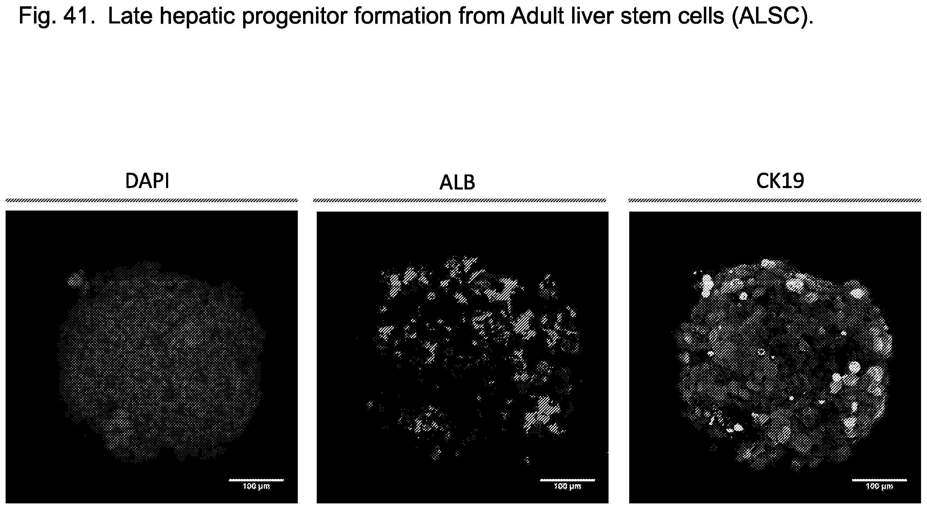

Figure 41. Late hepatic progenitor formation from adult liver stem cells. Human liver adult stem cells are dissociated into single cells and seeded in suspension in H2 media. The liver adult stem cells aggregate to form a spheroid structure and cells commit to form later hepatic stem cells that expresses CK19 and ALB. Results support that H2 media can be used for liver stem cells as well for inducing late hepatic progenitor formation.

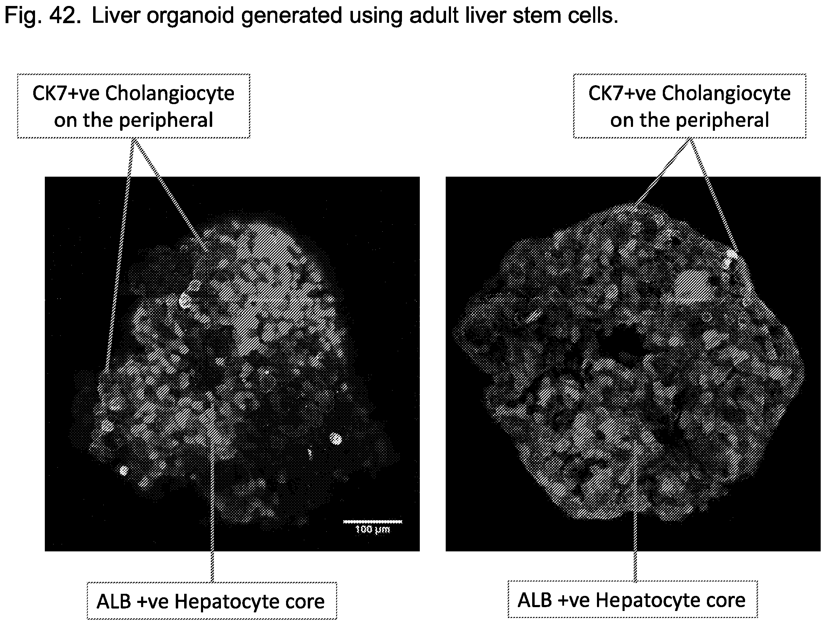

Figure 42. Liver organoid generated using adult liver stem cells. After H2 treatment, the hepatic progenitor spheroids are treated with H3 media for 14 days to induce liver organoid formation. The hepatic spheroids form liver organoids which are similar to the MESP derived organoids, consisting of a hepatocyte (ALB+ve) core and cholangiocytes (CK7+ve) forming ductal like structures in the periphery.

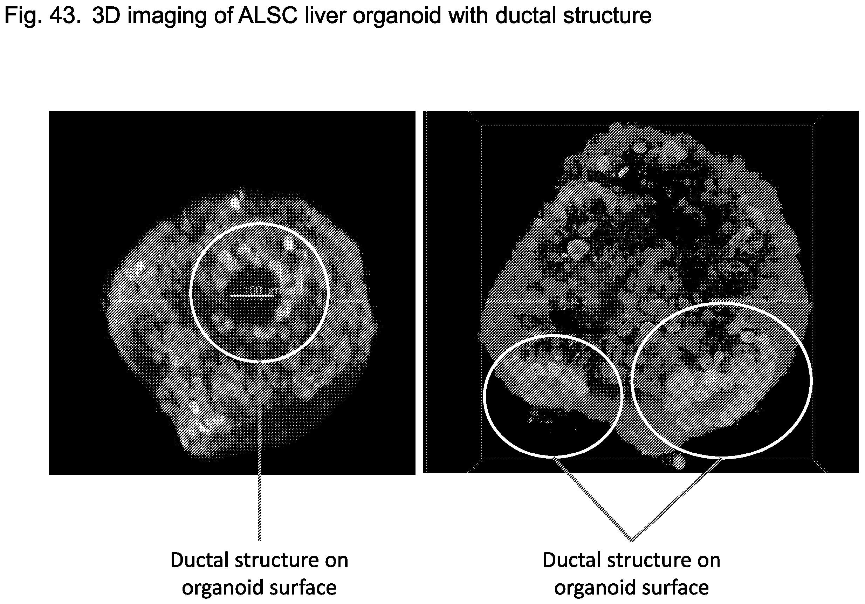

Figure 43. 3D imaging of liver organoids derived from adult liver stem cells with ductal structure. 3D imaging of the liver organoids derived from adult liver stem cells with lightsheet microscopy shows that the cholangiocytes at the periphery of the organoids form a ductal like structure with a lumen in the center (left image). Multiple of this ductal like structure form by the cholangiocyte can be observed on the surface of the organoids (Right image).

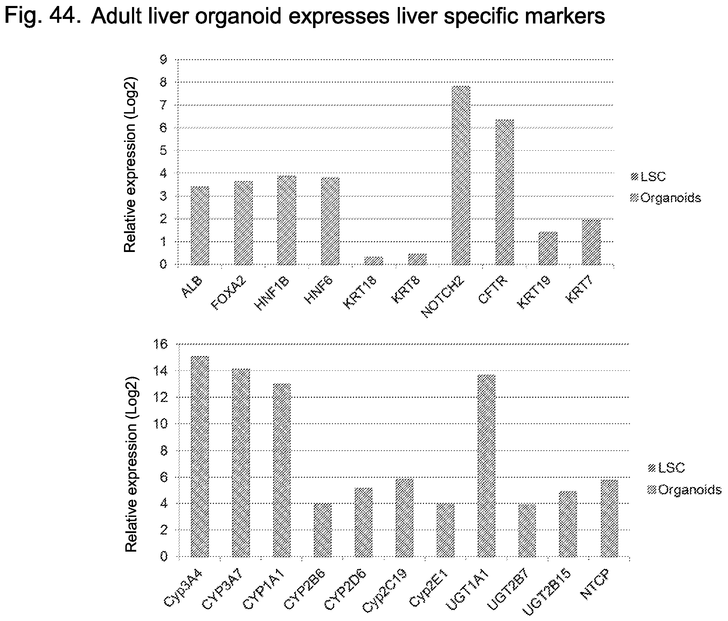

Figure 44. Organoids generated from adult liver stem cells express liver specific markers. Both hepatocyte specific genes and cholangiocyte specific genes are expressed in the liver organoids compared to undifferentiated adult stem cells. Result supports the existence of both cell types in the organoids. Similar to liver organoids derived from MESP, the adult liver organoids also expresses all the major cytochrome P450 enzymes and also UDP-glucuronosyltransferase enzymes and bile acid transporter NTCP.

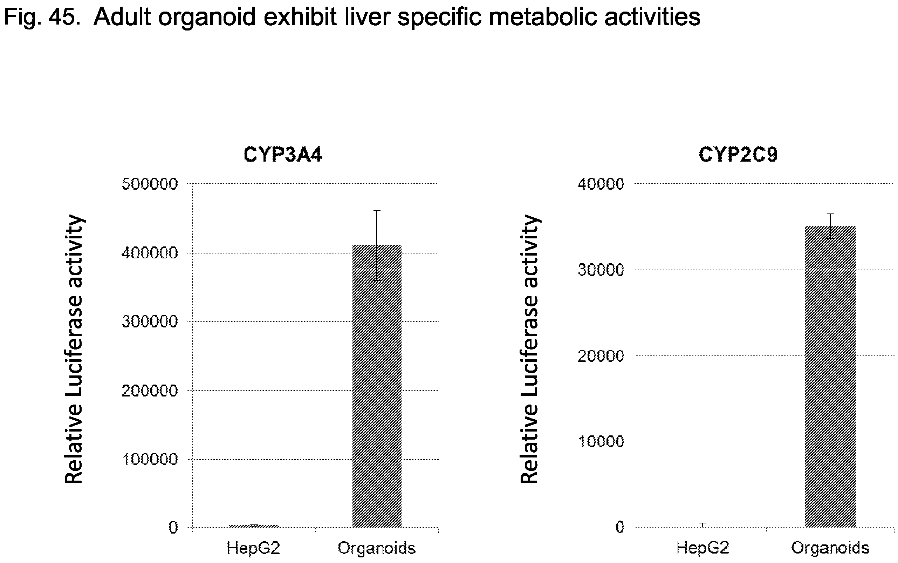

Figure 45. Organoids generated from adult liver stem cells exhibit liver specific metabolic activities. Organoids derived from adult liver stem cells were assayed for CYP activity using luciferase assay kits (Promega). The adult organoids exhibit strong CYP3A4 and CYP2C9 activities compared to the HepG2. Results show that adult organoid exhibit liver metabolic activities and CYP2C9 activity which is specific to adult hepatocytes is also detected in the adult liver organoids.

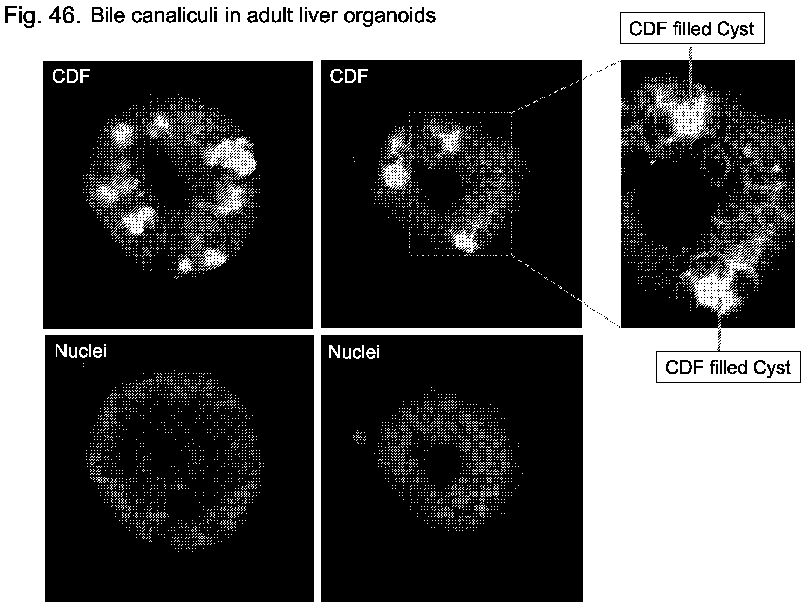

Figure 46. Bile canaliculi in liver organoids. CDF imaging of bile canaliculi network in the liver organoids derived from adult stem cells. Similar to the MESP derived organoids, the adult stem cell derived liver organoids contain a network of bile canaliculi that functionally transports CDF secreted by the hepatocytes. The CDF is similarly accumulated in the ductal-like structures in the organoids.
**Reference** Figure 47. Schematic of pancreatic spheroid production from MESP. MESP is treated with media P1 to induce pancreatic lineage commitment by the MESP. After P1 media treatment, the early pancreatic progenitors are retrieved from the matrigel and seeded in suspension culture with P2 media and subsequently P3 media to obtain Pancreatic spheroids.
**Reference** Figure 48. Pancreatic spheroid generated from MESP. The pancreatic spheroid generated using MESP and stained with pancreatic markers PDX1 and NKX6.1. The pancreatic spheroid contains of a lumen and the cells stains positive for both PDX1 and NKX6.1. The 2 markers are present in the earliest pancreatic progenitor cell during development and are also expressed in terminally specialized pancreatic cells such as the Beta-islet cells.
**Reference** Figure 49. Polarized E-cadherin expression in MESP. Staining of SOX9 and E-cadherin in MESP. E-Cadherin is a key transmembrane adhesion molecule which localize to the apical and lateral membranes of epithelial cell types. This is observed in MESP suggesting polarity in MESP.

### DETAILED DESCRIPTION OF THE INVENTION

### 1. Multipotent Endoderm Spheroid Progenitor (MESP) Cells Described herein for Reference Only

Described herein but not claimed is a method to derive spheroid progenitor cells of the endoderm lineage from pluripotent stem cells (PSCs). The progenitor cells can be stably propagated and expanded in culture. These spheroid progenitor cells exhibit potential to generate multiple organ cell types of the endoderm lineage, including the intestine, liver and pancreas. Hence, this spheroid progenitor is hereby described as Multipotent Endoderm Spheroid Progenitor (MESP). The MESPs can be stably propagated, do not exhibit signs of senescence, and maintain homogenous expression of stem cell markers. The cells also maintain a normal karyotype of 23 pairs of chromosome without major chromosomal mutations even after long term culture. This stable progenitor culture system is scalable and more cells can be generated with larger culture vessels, making these cells suitable for large scale production of downstream organ cell types for various applications including regenerative therapy and industrial applications.

The MESP represents a different endoderm progenitor stem cell state that differs from endoderm progenitor cells reported by Cheng et al. (Cell Stem Cell, 2012) and Hannan et al. (Stem Cell Reports, 2013). Cheng et al (Cell Stem Cell, 2012) report a progenitor stem cell that resembles early definitive endoderm stem cells, which is the earliest stem cell stage of the endoderm lineage development. Hannan et al (Stem Cell Reports, 2013) describe a progenitor stem cell that resembles the foregut progenitor during endoderm development. The culture conditions and stem cell markers of these two reports are highly similar. On the other hand, the culture conditions, media and stem cell markers of MESP are different from those described in the above references (see Table 1). MESP cells express markers similar to those expressed by cells of the posterior foregut during late endoderm lineage development (Figure 16).

As described herein, the pluripotent stem cells are embryonic stem cells (hESCs).

As described herein, the pluripotent stem cells are induced pluripotent stem cells (iPSCs). The iPSCs can be generated from any human adult tissues using the iPSCs reprogramming technology into cells with pluripotent capacity. MESP can then be generated from these pluripotent stem cells.

As described herein, MESP can also be generated from early endoderm progenitors reflecting early endoderm lineage development. The current methods generate MESP via stepwise differentiation along the endoderm lineage, where the PSCs first become definitive endoderm cells and subsequently differentiate into primitive gut endoderm cells.

As described herein, the pluripotent stem cells can be genetically modified by genome editing tools such as the Clustered regularly interspaced short palindromic repeats (CRISPR)/Cas system. These pluripotent stem cells maintain their pluripotential capacity and MESP can be generated from these genetically modified PSCs.

As described herein, the pluripotent stem cells can be induced pluripotent stem cells (iPSCs) from human tissues with specific genetic diseases. The disease-specific human iPSCs maintains their pluripotential capacity to give rise to endoderm lineage tissues. MESP can be generated from these disease-specific iPSCs.

### MESP culture system described herein for reference only

The Multi Endodermal Spheroid Progenitor (MESP) culture system described herein comprises a plurality of soluble agents in a stem cell culture media and a cellular support capable of providing structural and nutritional support. The cellular support maintains the progenitor

cells in a 3D structure such as a spheroid or organoid. The cellular support provides both structural support and cytokines that plays a part in maintaining liver stem cells in the undifferentiated state. As described hereinAs described herein, the plurality of soluble agents comprises one or more growth factors, an enhancer of the (canonical) WNT pathway, and a stem cell differentiation inhibitor.

As described herein, a method for producing a multipotent spheroid progenitor (MESP) cell is provided, the method comprising:
i) culturing an endoderm progenitor cell in a first medium on a cellular support under conditions suitable to differentiate the endoderm progenitor cell into a definitive endoderm (DE) cell;
ii) culturing the definitive endoderm cell in a second medium under conditions suitable to differentiate the definitive endoderm cell into a primitive gut cell; and
iii) culturing the primitive gut cell in a third medium on a cellular support under conditions suitable to differentiate the primitive gut cell into a MESP cell.

As described herein, the conditions suitable to differentiate the endoderm progenitor cell into a definitive endoderm (DE) cell, and/or the conditions suitable to differentiate the definitive endoderm cell into a primitive gut cell comprise two-dimensional or monolayer culture. As described herein, the conditions suitable to differentiate the endoderm progenitor cell into a definitive endoderm (DE) cell, and/or the conditions suitable to differentiate the definitive endoderm cell into a primitive gut cell comprise three-dimensional culture. As described herein, the conditions suitable to differentiate the primitive gut (GUT) cell into a MESP cell comprise three-dimensional culture. As described herein, the endoderm progenitor cell can be cultured in the first or second medium to differentiate the endoderm progenitor cell into a DE cell and a primitive gut cell, which can subsequently be cultured in the third medium to generate MESP cells.

As described herein, the cellular support comprises a material selected from the group consisting of matrigel, gelatine, methylcellulose, collagen, alginate, alginate beads, agarose, fibrin, fibrin glue, fibrinogen, blood plasma fibrin beads, whole plasma or components thereof, laminins, fibronectins, protecogylcans, HSP, chitosan, heparin, and synthetic polymers or polymer scaffolds.

### Culture Media described herein for reference only:

As described herein, the first culture medium comprises an activator of the TGF-β signaling pathway, such as Activin (e.g., Activin A, B or AB) or TGF-β. As described herein, the first culture medium futher comprises an activator of the BMP signaling pathway and an activator of the FGF signaling pathway.

As described herein, the second medium comprises an activator of the BMP signaling pathway and an activator of the FGF signaling pathway.

As described herein, the third medium comprises an inhibitor of the TGF-β signaling pathway, an activator of the WNT signaling pathway, and an activator of the Notch signaling pathway. As described herein, the third culture medium futher comprises a steroid, an activator of cAMP-dependent pathways, such as an activator of Protein Kinase A signaling pathway, an activator of the AKT/PI3K signaling pathway, and an inhibitor of histone deacetylase (HDAC), as described herein.

As described herein, the culture medium for deriving and maintaining endoderm spheroid progenitor cells comprises or consists of at least one, two, three, four, five, six, seven, eight or all of the following:
a) a TGF-β inhibitor and/or SMAD2/3 inhibitor;
b) a WNT-signaling activator and/or a Glycogen synthase kinase 3 (GSK3) inhibitor.
c) a steroid
d) an activator of AKT/PI3K signaling pathway and/or MAPK signaling pathway
e) an activator of STAT3, GAB1 mediated cell adhesion and AKT/PI3K signaling pathway
f) an activator of cAMP-dependent pathways, such as an activator of Protein Kinase A signaling pathway
g) an activator of the Notch receptor
h) a repressor of NFκB activity and activator of mitogen-activated protein (MAP) kinase ERK, p38 and JNK, or
i) an inhibitor of histone deacetylase (HDACs).

As described herein, the stem cell differentiation inhibitor is a TGF-beta signaling inhibitor, wherein the TGF-β inhibitor is characterized by any one of the following:
a) inhibition of TGF-β type I receptor ALK5 kinase;
b) inhibition of type I Activin/nodal receptor ALK4;
c) inhibition of type I nodal receptor ALK7;
d) inhibition of SMAD2/3 phosphorylation; and/or
e) inhibition of the Activin/TGF β /SMAD signaling pathway.

The TGF-beta inhibitor can block activation of the TGF-beta pathway, which induces stem cell differentiation, whereas inactivation of the TGF-beta pathway can maintain proliferation of endodermal stem cells. As described herein, the TGF-beta inhibitor is selected from the group consisting of:
i. A83-01 3-(6-Methyl-2-pyridinyl)-N-phenyl-4-(4-quinolinyl)-1H-pyrazole-1-carbothioamide,
ii. A 77-01 4-(3-(6-methylpyridin-2-yl)-1H-pyrazol-4-yl)quinoline,
iii. SD-208 2-(5-chloro-2-fluorophenyl)-N-pyridin-4-ylpteridin-4-amine, LY2157299 4-[2-(6-methylpyridin-2-yl)-5,6-dihydro-4H-pyrrolo[1,2-b]pyrazol-3-yl]quinoline-6-carboxamide ,
iv. SB 431542 4-[4-(1,3-benzodioxol-5-yl)-5-pyridin-2-yl-1H-imidazol-2-yl]benzamide, GW788388 N-(oxan-4-yl)-4-[4-(5-pyridin-2-yl-1H-pyrazol-4-yl)pyridin-2-yl]benzamide,
v. SB505124 2-[4-(1,3-benzodioxol-5-yl)-2-tert-butyl-1H-imidazol-5-yl]-6-methylpyridine,
vi. SB525334 6-[2-tert-butyl-5-(6-methylpyridin-2-yl)-1H-imidazol-4-yl]quinoxaline, IN 1130 2-[3-[(4-amino-2-methylpyrimidin-5-yl)methyl]-4-methyl-1,3-thiazol-3-ium-5-yl]ethanol,
vii. ITD 1 (6,6-dimethyl-5,7-dihydroimidazo[2,1-b][1,3]thiazol-4-ium-3-yl)methyl N,N'-dicyclohexylcarbamimidothioate,
viii. LY2109761 4-[2-[4-(2-pyridin-2-yl-5,6-dihydro-4H-pyrrolo[1,2-b]pyrazol-3-yl)quinolin-7-yl]oxyethyl]morpholine,
ix. K02288 3-[6-amino-5-(3,4,5-trimethoxyphenyl)pyridin-3-yl]phenol,
x. TGF-β RI kinase inhibitor [3-(Pyridin-2-yl)-4-(4-quinonyl)]-1H-pyrazole],
and derivatives thereof.

As described herein, the TGF-beta inhibitor is used at a concentration of between about 0.5 nM to 20 µM, or 100 nM to 10 µM, or 250 nM to 5 µM, or 400 nM to 2.5 µM, or 0.5 nM to 1 µM, or 0.5 nM to 0.5 µM, or between about 1.5 nM to 0.4 µM, or between about 10 nM to 0.3 µM, or between about 30 nM to 0.2 µM, or between about 40 nM to 0.1 µM, or between about 50 nM to 85 nM, or about 1, 5, 15, 25, 30, 35, 45, 50, 65, 75, 130, 150, 170, 250, 350 or 450 nM; or about 0.5, 0.7, 0.9, 1, 1.5, 2, 2.5, 3, 3.5, 4, 4.5, 5, 5.5, 6, 8, 9, 10, 12, 14, 16, 18, or 20 µM. As described herein, the TGF-beta inhibitor selected from the group consisting of (i) to (x) above is used at a concentration of between about 0.5 nM to 20 µM, or 100 nM to 10 µM, or 250 nM to 5 µM, or 400 nM to 2.5 µM, or 0.5 nM to 1 µM, or 0.5 nM to 0.5 µM, or between about 1.5 nM to 0.4 µM, or between about 10 nM to 0.3 µM, or between about 30 nM to 0.2 µM, or between about 40 nM to 0.1 µM, or between about 50 nM to 85 nM, or about 1, 5, 15, 25, 30, 35, 45, 50, 65, 75, 130, 150, 170, 250, 350 or 450 nM; or about 0.5, 0.7, 0.9, 1, 1.5, 2, 2.5, 3, 3.5, 4, 4.5, 5, 5.5, 6, 8, 9, 10, 12, 14, 16, 18, or 20 µM.

As described herein, the steroid is capable of inhibiting the NF-κB pathway, activating the PI3K/AKT/mTOR pathway, inhibiting the TGF-β signaling pathway and/or inhibiting the IGF signaling pathway. As described herein, the steroid is a corticosteroid such as a glucocorticoid or an anti-inflammatory glucocorticoid which improves maintenance of endodermal stem cells.

As described herein, the glucocorticoid is selected from the group consisting of:
Dexamethasone (8S,9R,10S,118,13S,14S,16R,17R)-9-Fluoro-11,17-dihydroxy-17-(2-hydroxyacetyl)-10,13,16-trimethyl-6,7,8,9,10,11,12,13,14,15,16,17-dodecahydro-3H-cyclopenta[a]phenanthren-3-one,
Cortisol (11β)-11,17,21-trihydroxypregn-4-ene-3,20-dione,
Cortisone (8S,9S,10R,13S,14S,17R)-17-Hydroxy-17-(2-hydroxyacetyl)-10,13-dimethyl-1,2,6,7,8,9,12,14,15,16-decahydrocyclopenta [a]phenanthrene-3,11-dione,
Prednisone 17,21-dihydroxypregna-1,4-diene-3,11,20-trione,
Prednisolone (11β)-11,17,21-trihydroxypregna-1,4-diene-3,20-dione, Methylprednisolone (1*S*,2*R*,8*S*,10*S*,11*S*,14*R*,15*S*,17*S*)-14,17-dihydroxy-14-(2-hydroxyacetyl)-2,8,15-trimethyltetracyclo[8.7.0.0^{2,7}.0^{11,15}]heptadeca-3,6-dien-5-one, Betamethasone (8*S*,9*R*,10*S*,11*S*,13*S*,14*S*,16*S*,17*R*)-9-fluoro- 11,17-dihydroxy-17-(2-hydroxyacetyl)-10,13,16-trimethyl- 6,7,8,9,10,11,12,13,14,15,16,17-dodecahydro- 3*H-*cyclopenta[*a*]phenanthren-3-one,
Triamcinolone (11β,16^{α})-9-Fluoro-11,16,17,21-tetrahydroxypregna-1,4-diene-3,20-dione, Beclometasone (8*S*,9*R*,10*S*,11*S*,13*S*,14*S*,16*S*,17*R*)-9-chloro-11-hydroxy-10,13,16-trimethyl-3-oxo-17-[2-(propionyloxy)acetyl]-6,7,8,9,10,11,12,13,14,15,16,17-dodecahydro-3*H*-cyclopenta[*a*]phenanthren-17-yl propionate,
Fludrocortisone acetate 9-fluoro-11,17-dihydroxy-17- (2-hydroxyacetyl)- 10,13-dimethyl- 1,2,6,7,8,9,10,11,12, 13,14,15,16,17- tetradecahydrocyclopenta[a]phenanthren-3-one,
Aldosterone 11β,21-Dihydroxy-3,20-dioxopregn-4-en-18-al,
and derivatives thereof.

As described herein, the steroid, corticosteroid or glucocorticoid (such as dexamethasone) is used at a concentration of between about 0.5 µM to 200 µM, or between about 1.5 µM to 150 µM, or between about 5 µM to 100 µM, or between about 10 µM to 90 µM, or between about 20 µM to 80 µM, or between about 30 µM to 70 µM, or between about 40 µM to 60 µM, or about 2, 8, 15, 25, 30, 35, 45, 65, 75, 110, 130, 140, 160, 170 or 190 µM.

As described herein, the WNT-signaling activator is a Glycogen synthase kinase 3 (GSK3) inhibitor.

As described herein, the GSK3 inhibitor is selected from the group consisting of:
CHIR-99021 6-[2-[[4-(2,4-dichlorophenyl)-5-(5-methyl-1H-imidazol-2-yl)pyrimidin-2-yl]amino]ethylamino]pyridine-3-carbonitrile, BIO 6-bromoindirubin-3'-oxime,
SB 216763 3-(2,4-dichlorophenyl)-4-(1-methylindol-3-yl)pyrrole-2,5-dione, CHIR-98014 6-N-[2-[[4-(2,4-dichlorophenyl)-5-imidazol-1-ylpyrimidin-2-yl]amino]ethyl]-3-nitropyridine-2,6-diamine,
TWS119 3-[[6-(3-aminophenyl)-7H-pyrrolo[2,3-d]pyrimidin-4-yl]oxy]phenol, IM-12 3-[2-(4-fluorophenyl)ethylamino]-1-methyl-4-(2-methyl-1H-indol-3-yl)pyrrole-2,5-dione, 1-Azakenpaullone 9-bromo-7,12-dihydropyrido[3',2':2,3]azepino[4,5-b]indol-6(5H)-one, AR-A014418 1-[(4-methoxyphenyl)methyl]-3-(5-nitro-1,3-thiazol-2-yl)urea, SB415286 3-(3-chloro-4-hydroxyanilino)-4-(2-nitrophenyl)pyrrole-2,5-dione, AZD1080 (3E)-3-[5-(morpholin-4-ylmethyl)-1H-pyridin-2-ylidene]-2-oxo-1H-indole-5-carbonitrile, AZD2858 3-amino-6-[4-(4-methylpiperazin-1-yl)sulfonylphenyl]-N-pyridin-3-ylpyrazine-2-carboxamide,
Indirubin (3E)-3-(3-oxo-1H-indol-2-ylidene)-1H-indol-2-one,
and derivatives thereof.

As described herein, the WNT-signaling activator is used at a concentration of between about 0.1 µM to 10 µM, or between about 0.5 µM to 8 µM, or between about 1 µM to 7 µM, or between about 2 µM to 6 µM, or between about 3 µM to 5 µM, or about 0.2, 0.3, 0.4,3, 4, 4.5, 5, 6, 7.5, 8.5, 9, 9.5 or 10 µM.

As described herein, the GSK3 inhibitor (e.g., CHIR-09921) is used at a concentration of between about 0.1 µM to 10 µM, or between about 0.5 µM to 8 µM, or between about 1 µM to 7 µM, or between about 2 µM to 6 µM, or between about 3 µM to 5 µM, or about 0.2, 0.3, 0.4,3, 4, 4.5, 5, 6, 7.5, 8.5, 9, 9.5 or 10 µM.

As described herein, the medium further comprises an activator of the AKT/PI3K signaling pathway and/or MAPK signaling pathway; such as but not limited to a compound selected from the group consisting of an epidermal growth factor (EGF), amphiregulin (AR), epigen (EPG), transforming growth factor alpha (TGFα), betacellulin (BTC), epiregulin (EPR), heparin-binding EGF-like growth factor (HB-EGF), and Neuregulin (NRG). As described herein, the activator of the AKT/PI3K signaling pathway and/or MAPK signaling pathway is used at a concentration of between about 5 ng/ml to 5 µg/ml, or between about 20 ng/ml to 4 µg/ml, or between about 30 ng/ml to 3 µg/ml, or between about 40 ng/ml to 2 µg/ml, or between about 45 ng/ml to 500 ng/ml, or between about 50 ng/ml to 300 ng/ml, or about 35, 40, 45, 50, 60, 70, 90, 100, 150, 200, 250, 300, 400, 450, 600, 700 or 800 ng/ml. As described herein, the epidermal growth factor (EGF), amphiregulin (AR), epigen (EPG), transforming growth factor alpha (TGFα), betacellulin (BTC), epiregulin (EPR), heparin-binding EGF-like growth factor (HB-EGF), and/or Neuregulin (NRG) is used at a concentration of between about 5 ng/ml to 5 µg/ml, or between about 20 ng/ml to 4 µg/ml, or between about 30 ng/ml to 3 µg/ml, or between about 40 ng/ml to 2 µg/ml, or between about 45 ng/ml to 500 ng/ml, or between about 50 ng/ml to 300 ng/ml, or about 35, 40, 45, 50, 60, 70, 90, 100, 150, 200, 250, 300, 400, 450, 600, 700 or 800 ng/ml.

As described herein, the medium further comprises an activator of STAT3, an activator of GAB1 mediated cell adhesion and/or an activator of the AKT/PI3K signaling pathway. As described herein, the activator of STAT3, activator of GAB1 mediated cell adhesion and/or activator of the AKT/PI3K signaling pathway is used at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, or 70 ng/ml, or about 1, 2.5, 3.5 or 4.5 µg/ml. As described herein, the activator of the AKT/PI3K signaling pathway is a hepatocyte growth factor (HGF). As described herein, HGF is used at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, or 70 ng/ml, or about 1, 2.5, 3.5 or 4.5 µg/ml.

As described herein, the medium further comprises an activator of cAMP-dependent pathways, such as an activator of the Protein Kinase A signaling pathway, which induces proliferation of epithelial cell types. As described herein, the activator of the cAMP-dependent pathway is a compound selected from the group consisting of dibutyryl-cAMP(dbCAMP), forskolin ((3R,4aR,5S,6S,6aS,10S,10aR,10bS)-6,10,10b-trihydroxy-3,4a,7,7,10a-pentamethyl-1-oxo-3-vinyldodecahydro-1H-benzo[f]chromen-5-yl acetate), caffeine, theophylline, cholera toxin and pertussis toxin. As described herein, the activator of cAMP-dependent pathways is used at a concentration of between about 20 ng/ml to 1 µg/ml, or between about 10 ng/ml to 0.8 µg/ml, or between about 15 ng/ml to 0.6 µg/ml, or between about 20 ng/ml to 2 µg/ml, or about 5, 18, 20, 25, 28, 30, 50, 60, or 70 ng/ml, or about 1, 2.5, 3.5 or 4.5 µg/ml. As described herein, a compound selected from the group consisting of dibutyryl-cAMP(dbCAMP), forskolin ((3R,4aR,58,68,6aS,10S,10aR,10bS)-6,10,10b-trihydroxy-3,4a,7,7,10a-pentamethyl-1-oxo-3-vinyldodecahydro-1H-benzo[f]chromen-5-yl acetate), caffeine, theophylline, cholera toxin and pertussis toxin is used at a concentration of between about 20 ng/ml to 1 µg/ml, or between about 10 ng/ml to 0.8 µg/ml, or between about 15 ng/ml to 0.6 µg/ml, or between about 20 ng/ml to 2 µg/ml, or about 5, 18, 20, 25, 28, 30, 50, 60, or 70 ng/ml, or about 1, 2.5, 3.5 or 4.5 µg/ml.

In some cases, the medium further comprises an activator of the Notch receptor. As described herein, the activator of the Notch receptor is used at a concentration of between about 10 nM to 100 µM, or between about 50 nM to 80 µM, or between about 100 nM to 60 µM, or between about 500 nM to 40 µM, or between about 800 nM to 20 µM, or between about 900 nM to 10 µM, or about 20, 40, 60, or 80 nM or about 1, 1.5, 15, 30, 50, 60, 90 or 100 µM. As described herein, the activator of the Notch receptor is a compound selected from the group consisting of Jagged1 protein (homo sapiens, also known as AGS; AHD; AWS; HJ1; CD339; JAGL1; JAG1), Jagged2 (NCBI 3714), Delta-like1 (NCBI 28514), Delta-like3 (NCBI 10683), and Delta-like4 (NCBI 54567). As described herein, the Jagged1 protein (homo sapiens, also known as AGS; AHD; AWS; HJ1; CD339; JAGL1; JAG1), Jagged2 (NCBI 3714), Delta-like1 (NCBI 28514), Delta-like3 (NCBI 10683), and/or Delta-like4 (NCBI 54567) is used at a concentration of between about 10 nM to 100 µM, or between about 50 nM to 80 µM, or between about 100 nM to 60 µM, or between about 500 nM to 40 µM, or between about 800 nM to 20 µM, or between about 900 nM to 10 µM, or about 20, 40, 60, or 80 nM or about 1, 1.5, 15, 30, 50, 60, 90 or 100 µM.

As described herein, the medium further comprises a molecule which is a repressor of NFκB activity and/or activator of mitogen-activated protein (MAP) kinase ERK, p38 and JNK. As described herein, the molecule which is a repressor of NFκB activity and/or activator of mitogen-activated protein (MAP) kinase ERK, p38 and JNK is used at a concentration of between about 0.1 mM to 1 M, or between about 2 mM to 0.8 M, or between about 4 mM to 0.6 M, or between about 6 mM to 0.4 M, or between about 8 mM to 0.2 M, or between about 10 mM to 800 mM, or between about 50 mM to 500 mM, or about 3, 5, 9, 15, 20, 30, 50, 80, 100, 120, 150, 200, 250, 300, 350, 400 or 450 mM. As described herein, the medium further comprises a compound selected from the group consisting of nicotinamide, nicotinic acid, 5-fluoronicotinamide, isonicotinic acid hydrazide, and nikethamide. As described herein, nicotinamide, nicotinic acid, 5-fluoronicotinamide, isonicotinic acid hydrazide, and/or nikethamide is used at a concentration of between about 0.1 mM to 1 M, or between about 2 mM to 0.8 M, or between about 4 mM to 0.6 M, or between about 6 mM to 0.4 M, or between about 8 mM to 0.2 M, or between about 10 mM to 800 mM, or between about 50 mM to 500 mM, or about 3, 5, 9, 15, 20, 30, 50, 80, 100, 120, 150, 200, 250, 300, 350, 400 or 450 mM.

As described herein, the medium further comprises an inhibitor of histone deacetylase (HDACs). As described herein, the inhibitor of histone deacetylase is a compound selected from the group consisting of valporic acid (VPA) (2-propylpentanoic acid), sodium butyrate (sodium;4-hydroxybutanoate), vorinotstat (N'-hydroxy-N-phenyloctanediamide), panobinostat ((E)-N-hydroxy-3-[4-[[2-(2-methyl-1H-indol-3-yl)ethylamino]methyl]phenyl]prop-2-enamide), trichostatin A ((2E,4E,6R)-7-[4-(dimethylamino)phenyl]-N-hydroxy-4,6-dimethyl-7-oxohepta-2,4-dienamide), mocetinostat (N-(2-aminophenyl)-4-[[(4-pyridin-3-ylpyrimidin-2-yl)amino]methyl]benzamide), BG45 (N-(2-aminophenyl)-2-pyrazinecarboxamide), 4SC-202 ((E)-N-(2-aminophenyl)-3-(1-((4-(1-methyl-1H-pyrazol-4-yl)phenyl)sulfonyl)-1H-pyrrol-3-yl)acrylamide), belinostat, scriptaid (6-(1,3-Dioxo-1H-benzo[de]isoquinolin-2(3H)-yl)-N-hydroxyhexanamide), M344 (4-(dimethylamino)-N-[7-(hydroxyamino)-7-oxoheptyl]benzamide), dacinostat ((E)-N-hydroxy-3-[4-[[2-hydroxyethyl-[2-(1H-indol-3-yl)ethyl]amino]methyl]phenyl]prop-2-enamide), abexinostat, CUDC-101 (7-(4-(3-ethynylphenylamino)-7-methoxyquinazolin-6-yloxy)-N-hydroxyheptanamide), CUDC-907 (N-hydroxy-2-(((2-(6-methoxypyridin-3-yl)-4-morpholinothieno[3,2-d]pyrimidin-6-yl)methyl)(methyl)amino)pyrimidine-5-carboxamide), and AR-42 ((S)-N-hydroxy-4-(3-methyl-2-phenylbutanamido)benzamide). As described herein, the inhibitor of histone deacetylase (including but not limited to the compounds listed above) is used at a concentration of between about 0.1 µM to 5 mM, or between about 0.3 µM to 4 mM, or between about 0.6 µM to 3 mM, or between about 0.8 µM to 2 mM, or between about 1 mM to 1.5 mM, or about 0.2, 0.4, 0.7, 0.9, 5, 10, 20, 50, 70 or 90 µM, or about 1.5, 2.5, 3.5 mM.

### Extracellular matrix:

As described herein, stem cells are cultured in a microenvironment that mimics at least in part a cellular niche in which said stem cells naturally reside. This cellular niche may be mimicked by culturing said stem cells in the presence of biomaterials, such as matrices, scaffolds, and culture substrates that represent key regulatory signals controlling stem cell fate. Such biomaterials comprise natural, semi-synthetic and synthetic biomaterials, and/or mixtures thereof. A scaffold provides a two-dimensional or three dimensional network. Suitable synthetic materials for such a scaffold comprise polymers selected from porous solids, nanofibers, and hydrogels such as, for example, peptides including self-assembling peptides, hydrogels composed of polyethylene glycol phosphate, polyethylene glycol fumarate, polyacrylamide, polyhydroxyethyl methacrylate, polycellulose acetate, and/or co-polymers thereof (see, for example, Saha et al., 2007. Curr Opin Chem. Biol. 11(4): 381-387; Saha et al., 2008. Biophysical Journal 95: 4426-4438; Little et al., 2008. Chem. Rev 108, 1787-1796). As is known to a skilled person, the mechanical properties such as, for example, the elasticity of the scaffold influences proliferation, differentiation and migration of stem cells. As described herein, the scaffold comprises biodegradable (co)polymers that are replaced by natural occurring components after transplantation in a subject, for example to promote tissue regeneration and/or wound healing. As described herein, said scaffold does not substantially induce an immunogenic response after transplantation in a subject. Said scaffold is supplemented with natural, semi-synthetic or synthetic ligands, which provide the signals that are required for proliferation and/or differentiation, and/or migration of stem cells. In one embodiment, said ligands comprise defined amino acid fragments. Examples of said synthetic polymers comprise Pluronic^{®} F127 block copolymer surfactant (BASF), and Ethisorb (Johnson and Johnson).

A cellular niche is in part determined by the stem cells and surrounding cells, and the extracellular matrix (ECM) that is produced by the cells in said niche. As described herein, MESP are attached to an ECM. ECM is composed of a variety of polysaccharides, water, elastin, and glycoproteins, wherein the glycoproteins comprise collagen, entactin (nidogen), fibronectin, and laminin. ECM is secreted by connective tissue cells. Different types of ECM are known, comprising different compositions including different types of glycoproteins and/or different combination of glycoproteins. Said ECM can be provided by culturing ECM-producing cells, such as for example fibroblast cells, in a receptacle, prior to the removal of these cells and the addition of isolated liver fragments or isolated biliary duct or isolated epithelial stem cells. Examples of extracellular matrix-producing cells are chondrocytes, producing mainly collagen and proteoglycans, fibroblast cells, producing mainly type IV collagen, laminin, interstitial procollagens, and fibronectin, and colonic myofibroblasts producing mainly collagens (type I, III, and V), chondroitin sulfate proteoglycan, hyaluronic acid, fibronectin, and tenascin-C. Alternatively, said ECM is commercially provided. Examples of commercially available extracellular matrices are extracellular matrix proteins (Invitrogen) and basement membrane preparations from Engelbreth-Holm-Swarm (EHS) mouse sarcoma cells (e.g. Matrigel^{™} (BD Biosciences)). A synthetic extracellular matrix material, such as ProNectin (Sigma Z378666) may be used. Mixtures of extracellular matrix materials may be used, if desired. The use of an ECM for culturing stem cells enhanced long-term survival of the stem cells and the continued presence of undifferentiated stem cells. In the absence of an ECM, stem cell cultures could not be cultured for longer periods and no continued presence of undifferentiated stem cells was observed. In addition, the presence of an ECM allowed culturing of three-dimensional tissue organoids, which could not be cultured in the absence of an ECM. The extracellular matrix material will normally be coated onto a cell culture vessel, but may (in addition or alternatively) be supplied in solution. A fibronectin solution of about 1 mg/ml may be used to coat a cell culture vessel, or between about 1 µg/cm² to about 250 µg/cm², or at about 1 µg/cm² to about 150 µg/cm². As described herein, a cell culture vessel is coated with fibronectin at between 8 µg/cm² and 125 µg/cm². As described herein, the ECM comprises at least two distinct glycoproteins, such as two different types of collagen or a collagen and laminin. The ECM can be a synthetic hydrogel extracellular matrix or a naturally occurring ECM. AnotherECM is provided by Matrigel^{™} (BD Biosciences), which comprises laminin, entactin, and collagen IV.

### Characterization of MESP described herein for reference only:

The endoderm spheroid progenitor cells described herein are characterized by expression of any one or more, or at least two, or at least three, or at least four, or at least five, or at least six, or 1 or 2 or 3 or 4 or 5 or 6 or 7 or all of the following markers: HNF4A (NCBI Gene: 3172), PDX1 (NCBI Gene: 3651), CDX2 (NCBI Gene: 1045), SOX9 (NCBI Gene: 6662), KRT19 (NCBI Gene: 3880), AFP (NCBI Gene: 174), ONECUT2 (NCBI Gene: 9480), LGR5 (NCBI Gene: 8549), EPHB2 (NCBI Gene: 2048), LGR4 (NCBI Gene: 55366), NR5A2 (NCBI Gene: 2494), CDH1 (NCBI Gene: 999), KRT7 (NCBI Gene: 3855), ZNF503 (NCBI Gene: 84858), MSX2 (NCBI Gene: 4488), TRPS1 (NCBI Gene: 7227), ASCL2 (NCBI Gene: 430), IRF8 (NCBI Gene: 3394), HNF4G (NCBI Gene: 3174), ID2 (NCBI Gene: 3398), CD44 (NCBI Gene: 960), EPCAM (NCBI Gene: 4072), MET (NCBI Gene: 4233), IHH (NCBI Gene: 3549) and CLDN3 (NCBI Gene: 1365).

The endoderm spheroid progenitor cells do not express or essentially do not express any one or two or three or four or five or all of the following markers SOX2 (NCBI Gene: 6657), CER1 (NCBI Gene: 9350), GATA4 (NCBI Gene: 2626), SOX17 (NCBI Gene: 64321), FOXA2 (NCBI Gene: 3170) and CXCR4 (NCBI Gene: 7852).

The endoderm spheroid progenitor cells display polarity (herein, polarity of cells refers to the unique expression of proteins in specific membrane regions of the cells that is in contact with different environment. Polarity in endoderm spheroid progenitor cells is evident from the uneven distribution of E-cadherin protein on the cells. Regions enriched with E-Cadherin marks the apical and lateral membrane of the cells (Figure 49).

### Multipotent Endoderm Spheriod Progenitor (MESP) described herein for reference only

MESP is a unique stem cell that expresses many markers of the posterior foregut, including the HNF4A, PDX1 and CDX2 (Figure 4 and 13). Developmentally, the posterior foregut gives rise to three essential organs of human adult body, namely the liver, pancreas and duodenum. Cells forming these 3 organs express the respective regulatory factors HNF4A (in the liver), PDX1 (in the pancreas) and CDX2 (in the small intestine). MESP express all 3 factors and similarly has the ability to generate cells of the liver (Figure 17-20) , pancreas (Figure 45) and intestine (Figure 11 and 12).

Unlike reported endoderm progenitor stem cells, MESP is cultured in 3D and forms a spheroid structure compared to the 2D monolayer cells (Table 1). Cells cultured in spheroid are arranged spatially to generate a lumen within. The 2 surface of the cells are thus exposed to 2 different environments, adding to the complexity of cell state regulation in the spheroid. The uneven distribution of the adhesion molecule E-cadherin further supports that the cells expose to 2 different environments exhibit polarity. The endoderm stem cell state maintained in the MESPs is different from the other endoderm stem cell reported. This difference is also evident in the signaling requirement of the MESP. TGFβ signaling plays a role in maintaining early endoderm stem cell state (Table 1) and has been widely used in many PSCs differentiation protocol to induce early endoderm development (Figure 16) (Basma et al., 2009; D'Amour et al., 2006; Spence., et al 2011; Cheng., et al 2012; Gieseck., et al 2014; Hannan., et al 2013; Si-Tayeb., et al 2010a; Schwartz., et al 2014 ). In contrast, the TGFβ signaling pathway is inhibited in the maintenance of MESP (Table 1) and Wnt signaling pathway is activated. Corresponding to its late endoderm progenitor state (figure 16), MESP do not express many of the early endoderm progenitor markers such as SOX17, CXCR4, FOXA2, SOX2 and CER1 (Figure 9). Instead, MESP expresses many specific stem cell markers found in adult and fetal stem cells derive from the pancreas, liver, small intestine such as NR5A2, ASCL2, HNF4A, KRT7, SOX9, KRT19, PDX1, LGR4, LGR5 and ONECUT2 (Figure 10) (Si-Tayeb et al., 2010b; Dan et al., 2006; Schmelzer et al., 2007). In line with its posterior foregut identity, MESP do not express stem cell markers found in the colon stem cell and prostate progenitor (Figure 10).

As a stem cell, MESP can be propagated for 19 passages (Figure 3A) and more, and display the ability to form the entire spheroid from a single cell (Figure 3B). Cells in the entire spheroid stain homogenously for the stem cell markers HNF4A, PDX1, CDX2, CK19 and SOX9 (Figure 5A and 5B). Stem cell culture is a powerful resource that enables large scale expansion of cells for applications such as regenerative therapy, and large scale genomics and proteomics studies. MESP culture can be linearly scaled up in larger culture vessels to obtain large number of cells (Figure 6). As the cells are cultured in 3D, more cells are obtained when using a culture vessel of similar dimension compared to cells culture in 2D. MESP also display similar proliferation capacity over long term passages (Figure 7A). Importantly, the cells are able to maintain a normal karyotype in culture for more than 140 days (Figure 7B).

As MESP can be efficiently derived by pluripotent stem cells including iPSCs (Figure 13), MESP is potentially an invaluable cell resource for modeling diseases (Figure 14). This can be achieve by genome editing of PSCs including both hESCs and iPSCs or reprogramming somatic cells from patients with specific genetic disease back to the pluripotent stem cells states. These PSCs can be further used to the generate MESP with disease genetic background. These disease-specific MESP can be subsequently be used to the generate models of liver, pancreas and intestine diseases (Figure 14, 15 and 37). Thus, MESP is a power tool to generate models of diseased liver, pancreas and intestine for research.

### Derviation and Maintenance of MESP:

Exemplary methods for deriving and maintaining MESP from endoderm cells are described in the Examples. The steps are illustrated in Figure 1.

### 2. Liver organoids

Described herein is a method to generate liver organoids from progenitors and stem cells. The liver organoid fulfills key criteria's of a mini-organ which contain multiple cell types of the organ, spatially organized into structures that resembles organ tissues and performing organ specific functions. The liver organoid described herein contain at least the two major cells types of the liver, the hepatocytes and the cholangiocytes. The core of the organoids is formed by the hepatocytes and the cholangiocytes form bile duct-like structures around the core of hepatocytes. The hepatocytes form a network of bile canaliculi which connect to the cholangiocyte bile duct-like structures at the periphery, resembling the hepatocytes arrangement in the liver lobule which similarly connects to the bile duct via the bile canaliculi network (Figure 22). The liver organoids exhibit multiple liver specific functions including the liver specific metabolic activities, storage of glycogen, uptake of lipids, and secretion of albumin. Importantly, organoids also exhibit similar bile secretion functions where the hepatocytes secretions are transported via the network of bile canaliculi to the bile duct structures formed by the cholangiocytes. This is the first demonstration in vitro where the hepatocytes and cholangiocytes are functionally connected by a network of bile canaliculi. The transport of the hepatocyte exports through the canaliculi to the cholangiocyte formed cyst structures mimics the *in vivo* bile secretion events in the liver. The liver organoid described herein thereby fulfills the criteria of an organoid which mimics the *in vivo* organ. The versatility of the method employ is demonstrated by the use both MESP and adult liver stem cell to generate the liver organoids. The 2 distinct stem cell types generated organoids of similar composition and structure, supporting that the current method could be apply on different late endoderm stem cells that has the capacity to form liver cell types.

One advantage of generating liver organoids from stem cells is scalability and an amendable system for modeling diseases. The stem cells can be expanded in large scale to allow production of large number of organoids. The self-renewing ability of the stem cells allows continuous generation of liver organoids from the stem cell population. Genetic modifications using genome editing tools such as CRISPR/Cas system or iPSC reprogramming would facilitate the generation disease-specific stem cells and liver organoids that exhibit various disease phenotypes. These disease specific organoids would be highly useful for modeling disease in vitro (Figure 16) and identification of disease mechanism. The methods described herein also demonstrated the adaptability of the organoid generation method in high throughput manner where single organoids are generated in 96 well dishes. The individual organoids exhibited similar morphology and metabolic functions.

Liver organoid-like structures have also been reported; however, they do not consist of somatic liver cell types and do not perform liver specific functions. A liver epithelial organoid has been derived from the adult liver (PCT/IB11/02167). The 'organoid' describe in the PCT/IB11/02167 application consists largely of liver epithelial stem cells. These stem cells are used to generate either hepatocytes or cholangiocytes. These epithelial organoid stem cells do not contain multiple liver cell types, nor structures that resemble human liver tissue, and do not exhibit liver functions. A liver bud consisting of multiple cells types including liver hepatoblast have been generated (WO2013047639 A1). The liver bud consists of mesenchymal cells, endothelial cells and liver hepatoblast which are aggregated on a gel. While the three cell types aggregate to form a mass on a dish, this liver bud does not form organized structures and does not exhibit liver specific functions. The liver bud has to be transplanted into a host for further maturation to functional liver tissues. In contrast, we describe herein the first human liver organoid comprising multiple, functional liver cell types, which has liver tissue organization and performs organ level functions such as bile secretion and transport (Table 2 and Table 3).

Liver tissues have also been engineered in vitro using 3D printing technology (US 2014/0287960 A1). In contrast with 3D printing technology, the instant methods employ the self-organizing capacity of stem cells during differentiation. 3D printed liver tissues employ extracellular matrices as gels to adhere cells in layers at precise locations. The resulting liver tissue structure is predetermined and cells are printed to desired configurations. In constrast, the stem cell derived organoids described herein provide conditions for the cells to interact and self-organize into structures resembling the liver tissue. The cells in the organoid interact and adhere without the need for addition of extracellular matrix. For example, the organoids described herein comprise endogenous extracellular matrix adhesion molecules produced by the cells in the organoid, compared to previous methods that use an exogenous matrigel or other extracellular matrix to adhere the cells together in the structure. Table 4 summarizes important differences between the liver organoids described herein and 3D printed liver tissues. For example, in the liver organoids described herein, the parenchymal and non-parachymal cells are derived from primary stem cells, whereas previous methods (e.g., 3D printing and cell aggregation methods) use parenchymal and non-parachymal cell types from different stem cell origins or immortalized cell lines. Further, the liver organoids described herein comprise functional bile canaliculi, which were not produced using previous methods.

In certain embodiments, the stem cells are the MESP.

In certain embodiments, the stem cells are adult liver stem cells.

In certain embodiments, the stem cells can be endoderm lineage progenitors that have the potential to give rise to liver tissue cell types.

Thus, in some embodiments, the method of producing a liver organoid comprises culturing an endoderm stem cell in a first cell culture medium to obtain an early hepatic progenitor. In some cases, the endoderm stem cell is an early endoderm progenitor cell, a pluripotent stem cell, an induced pluripotent stem cell, a human embryonic stem cell, an MESP, or an adult liver stem cell.

In certain embodiments, the organoids can consist of hepatocytes and cholangiocytes with at least one other liver cell types including stellate cells, Kupffer cells, hepatic progenitor cells and liver endothelial sinusoidal stem cells.

In some embodiments, the organoids do not comprise genetically engineered cells, such as recombinantly modified cells. In some embodiments, the organoids do not comprise cells that are genetically engineered to express gene products such as RNA and/or proteins that regulate the proliferation of the cells.

As shown in Table 5, the liver organoids described herein differ in certain aspects from primary liver tissue. For example, the hepatocytes in primary liver tissue are larger in size, comprise a double nucleus and exhibit polyploid chromosome number, whereas the hepatocytes in the liver organoids are about half the size of hepatocytes in primary liver, and comprise a single nucleus containing diploid chromosome number. In addition, primary hepatocytes show a rapid decline in CYP functions after 24 hours in culture, whereas CYP function in the organoid hepatocytes is stable and maintained for weeks in culture. Primary cholangiocytes form long branching tubular structures and proliferate in culture, whereas organoid cholangiocytes form large cysts in culture, and do not proliferate.

### Derivation of hepatic organoids from MESP

The Hepatic organoid culture system described herein comprises a plurality of soluble agents in three different hepatic culture media, a cellular support and suspension culture system. The cellular support provides culture conditions suitable for differentiation of MESP to early hepatic progenitors, and the suspension culture system provides culture conditions suitable for formation of late hepatic progenitors and subsequently organoids. In some embodiments, the plurality of soluble agents comprises one or more growth factors, an enhancer of the (canonical) WNT pathway, an inhibitor of TGF-β signaling, and an inhibitor of Notch signaling.

### Media H1, H2 and H3

### Media components:

### H1 media

In some embodiments, H1 media comprises:
a. an activator of STAT3, GAB1 mediated cell adhesion and/or AKT/PI3K signaling pathway;
b. a molecule which is an repressor of NFκB activity and activator of mitogen-activated protein (MAP) kinase ERK, p38 and JNK;
c. a TGF-β inhibitor and/or SMAD2/3 inhibitor;
d. a WNT-signaling activator;
e. a steroid;
f. at least one molecule(s) inducing phosphorylation of SMAD1, SMADS and SMAD8 and activating MAPK signaling; and
g. a molecule activating FGF and MAPK pathway.

In some embodiments, molecule for inducing hepatic specification is a TGF-beta signaling inhibitor, wherein the TGF-β inhibitor is characterized by any one of the following:
a) inhibition of TGF-β type I receptor ALK5 kinase;
b) inhibition of type I Activin/nodal receptor ALK4;
c) inhibition of type I nodal receptor ALK7;
d) inhibition of SMAD2/3 phosphorylation; and/or
e) inhibition of the Activin/TGF β /SMAD signaling pathway.

The TGF-beta inhibitor can block activation of TGF-beta pathway, inducing hepatic lineage specification. In some embodiments, the TGF-beta inhibitor is selected from the group consisting of:
i. A83-01 3-(6-Methyl-2-pyridinyl)-N-phenyl-4-(4-quinolinyl)-1H-pyrazole-1-carbothioamide,
ii. A 77-01 4-(3-(6-methylpyridin-2-yl)-1H-pyrazol-4-yl)quinoline,
iii. SD-208 2-(5-chloro-2-fluorophenyl)-N-pyridin-4-ylpteridin-4-amine, LY2157299 4-[2-(6-methylpyridin-2-yl)-5,6-dihydro-4H-pyrrolo[1,2-b]pyrazol-3-yl]quinoline-6-carboxamide ,
iv. SB 431542 4-[4-(1,3-benzodioxol-5-yl)-5-pyridin-2-yl-1H-imidazol-2-yl]benzamide, GW788388 N-(oxan-4-yl)-4-[4-(5-pyridin-2-yl-1H-pyrazol-4-yl)pyridin-2-yl]benzamide,
v. SB505124 2-[4-(1,3-benzodioxol-5-yl)-2-tert-butyl-1H-imidazol-5-yl]-6-methylpyridine,
vi. SB525334 6-[2-tert-butyl-5-(6-methylpyridin-2-yl)-1H-imidazol-4-yl]quinoxaline, IN 1130 2-[3-[(4-amino-2-methylpyrimidin-5-yl)methyl]-4-methyl-1,3-thiazol-3-ium-5-yl]ethanol,
vii. ITD 1 (6,6-dimethyl-5,7-dihydroimidazo[2,1-b][1,3]thiazol-4-ium-3-yl)methyl N,N'-dicyclohexylcarbamimidothioate,
viii. LY2109761 4-[2-[4-(2-pyridin-2-yl-5,6-dihydro-4H-pyrrolo[1,2-b]pyrazol-3-yl)quinolin-7-yl]oxyethyl]morpholine,
ix. K02288 3-[6-amino-5-(3,4,5-trimethoxyphenyl)pyridin-3-yl]phenol,
x. TGF-β RI kinase inhibitor [3-(Pyridin-2-yl)-4-(4-quinonyl)]-1H-pyrazole] and derivatives thereof.

In some embodiments, the TGF-beta inhibitor is used at a concentration of between about 0.5 nM to 20 µM, or 100 nM to 10 µM, or 250 nM to 5 µM, or 400 nM to 2.5 µM, or 0.5 nM to 1 µM, or 0.5 nM to 0.5 µM, or between about 1.5 nM to 0.4 µM, or between about 10 nM to 0.3 µM, or between about 30 nM to 0.2 µM, or between about 40 nM to 0.1 µM, or between about 50 nM to 85 nM, or about 1, 5, 15, 25, 30, 35, 45, 50, 65, 75, 130, 150, 170, 250, 350 or 450 nM; or about 0.5, 0.7, 0.9, 1, 1.5, 2, 2.5, 3, 3.5, 4, 4.5, 5, 5.5, 6, 8, 9, 10, 12, 14, 16, 18, or 20 µM. In some embodiments, the TGF-beta inhibitor selected from the group consisting of (i) to (x) above is used at a concentration of between about 0.5 nM to 20 µM, or 100 nM to 10 µM, or 250 nM to 5 µM, or 400 nM to 2.5 µM, or 0.5 nM to 1 µM, or 0.5 nM to 0.5 µM, or between about 1.5 nM to 0.4 µM, or between about 10 nM to 0.3 µM, or between about 30 nM to 0.2 µM, or between about 40 nM to 0.1 µM, or between about 50 nM to 85 nM, or about 1, 5, 15, 25, 30, 35, 45, 50, 65, 75, 130, 150, 170, 250, 350 or 450 nM; or about 0.5, 0.7, 0.9, 1, 1.5, 2, 2.5, 3, 3.5, 4, 4.5, 5, 5.5, 6, 8, 9, 10, 12, 14, 16, 18, or 20 µM.

In some embodiments, the steroid is capable of inhibiting the NF-κB pathway, activating the PI3K/AKT/mTOR pathway, inhibiting the TGF-β signaling pathway and/or inhibiting the IGF signaling pathway. In some embodiments, the steroid is a corticosteroid such as a glucocorticoid or an anti-inflammatory glucocorticoid which improves maintenance of endodermal stem cells.

In some embodiments, the glucocorticoid is selected from the group consisting of:
Dexamethasone (8S,9R,10S,11S,13S,14S,16R,17R)-9-Fluoro-11,17-dihydroxy-17-(2-hydroxyacetyl)-10,13,16-trimethyl-6,7,8,9,10,11,12,13,14,15,16,17-dodecahydro-3H-cyclopenta[a]phenanthren-3-one,
Cortisol (11β)-11,17,21-trihydroxypregn-4-ene-3,20-dione,
Cortisone (8S,9S,10R,13S,14S,17R)-17-Hydroxy-17-(2-hydroxyacetyl)-10,13-dimethyl-1,2,6,7,8,9,12,14,15,16-decahydrocyclopenta[a]phenanthrene-3,11-dione,
Prednisone 17,21-dihydroxypregna-1,4-diene-3,11,20-trione,
Prednisolone (11β)-11,17,21-trihydroxypregna-1,4-diene-3,20-dione,
Methylprednisolone (1*S*,2*R*,8*S*,10*S*,11*S*,14*R*,15*S*,17*S*)-14,17-dihydroxy-14-(2-hydroxyacetyl)-2,8,15-trimethyltetracyclo[8.7.0.0^{2,7}.0^{11,15}]heptadeca-3,6-dien-5-one, Betamethasone (8*S*,9*R*,10*S*,11*S*,13*S*,14*S*,16*S*,17*R*)-9-fluoro- 11,17-dihydroxy-17-(2-hydroxyacetyl)-10,13,16-trimethyl- 6,7,8,9,10,11,12,13,14,15,16,17-dodecahydro- 3*H-*cyclopenta[*a*]phenanthren-3-one,
Triamcinolone (11β,16^{α})-9-Fluoro-11,16,17,21-tetrahydroxypregna-1,4-diene-3,20-dione, Beclometasone (8*S*,9*R*,10*S*,11*S*,13*S*,14*S*,16*S*,17*R*)-9-chloro-11-hydroxy-10,13,16-trimethyl-3-oxo-17-[2-(propionyloxy)acetyl]-6,7,8,9,10,11,12,13,14,15,16,17-dodecahydro-3*H*-cyclopenta[*a*]phenanthren-17-yl propionate,
Fludrocortisone acetate 9-fluoro-11,17-dihydroxy-17- (2-hydroxyacetyl)- 10,13-dimethyl- 1,2,6,7,8,9,10,11,12, 13,14,15,16,17- tetradecahydrocyclopenta[a]phenanthren-3-one,
Aldosterone 11β,21-Dihydroxy-3,20-dioxopregn-4-en-18-al,
and derivatives thereof.

In some embodiments, the steroid, corticosteroid or glucocorticoid (such as dexamethasone) is used at a concentration of between about 0.5 µM to 200 µM, or between about 1.5 µM to 150 µM, or between about 5 µM to 100 µM, or between about 10 µM to 90 µM, or between about 20 µM to 80 µM, or between about 30 µM to 70 µM, or between about 40 µM to 60 µM, or about 2, 8, 15, 25, 30, 35, 45, 65, 75, 110, 130, 140, 160, 170 or 190 µM.

In some embodiments, the WNT-signaling activator is a Glycogen synthase kinase 3 (GSK3) inhibitor. In some embodiments, the GSK3 inhibitor is selected from the group consisting of:
CHIR-99021 6-[2-[[4-(2,4-dichlorophenyl)-5-(5-methyl-1H-imidazol-2-yl)pyrimidin-2-yl]amino]ethylamino]pyridine-3-carbonitrile, BIO 6-bromoindirubin-3'-oxime,
SB 216763 3-(2,4-dichlorophenyl)-4-(1-methylindol-3-yl)pyrrole-2,5-dione, CHIR-98014 6-N-[2-[[4-(2,4-dichlorophenyl)-5-imidazol-1-ylpyrimidin-2-yl]amino]ethyl]-3-nitropyridine-2,6-diamine,
TWS119 3-[[6-(3-aminophenyl)-7H-pyrrolo[2,3-d]pyrimidin-4-yl]oxy]phenol, IM-12 3-[2-(4-fluorophenyl)ethylamino]-1-methyl-4-(2-methyl-1H-indol-3-yl)pyrrole-2,5-dione, 1-Azakenpaullone 9-bromo-7,12-dihydropyrido[3',2':2,3]azepino[4,5-b]indol-6(5H)-one, AR-A014418 1-[(4-methoxyphenyl)methyl]-3-(5-nitro-1,3-thiazol-2-yl)urea, SB415286 3-(3-chloro-4-hydroxyanilino)-4-(2-nitrophenyl)pyrrole-2,5-dione, AZD1080 (3E)-3-[5-(morpholin-4-ylmethyl)-1H-pyridin-2-ylidene]-2-oxo-1H-indole-5-carbonitrile, AZD2858 3-amino-6-[4-(4-methylpiperazin-1-yl)sulfonylphenyl]-N-pyridin-3-ylpyrazine-2-carboxamide,
Indirubin (3E)-3-(3-oxo-1H-indol-2-ylidene)-1H-indol-2-one,
and derivatives thereof.

In some embodiments, the WNT-signaling activator is used at a concentration of between about 0.1 µM to 10 µM, or between about 0.5 µM to 8 µM, or between about 1 µM to 7 µM, or between about 2 µM to 6 µM, or between about 3 µM to 5 µM, or about 0.2, 0.3, 0.4,3, 4, 4.5, 5, 6, 7.5, 8.5, 9, 9.5 or 10 µM.

In some embodiments, the GSK3 inhibitor (e.g., CHIR-09921) is used at a concentration of between about 0.1 µM to 10 µM, or between about 0.5 µM to 8 µM, or between about 1 µM to 7 µM, or between about 2 µM to 6 µM, or between about 3 µM to 5 µM, or about 0.2, 0.3, 0.4,3, 4, 4.5, 5, 6, 7.5, 8.5, 9, 9.5 or 10 µM.

In some embodiments, the medium further comprises an activator of the AKT/PI3K signaling pathway and/or MAPK signaling pathway; such as but not limited to a compound selected from the group consisting of an epidermal growth factor (EGF), amphiregulin (AR), epigen (EPG), transforming growth factor alpha (TGFα), betacellulin (BTC), epiregulin (EPR), heparin-binding EGF-like growth factor (HB-EGF), and Neuregulin (NRG). In some embodiments, the activator of the AKT/PI3K signaling pathway and/or MAPK signaling pathway is used at a concentration of between about 5 ng/ml to 5 µg/ml, or between about 20 ng/ml to 4 µg/ml, or between about 30 ng/ml to 3 µg/ml, or between about 40 ng/ml to 2 µg/ml, or between about 45 ng/ml to 500 ng/ml, or between about 50 ng/ml to 300 ng/ml, or about 35, 40, 45, 50, 60, 70, 90, 100, 150, 200, 250, 300, 400, 450, 600, 700 or 800 ng/ml. In some embodiments, the epidermal growth factor (EGF), amphiregulin (AR), epigen (EPG), transforming growth factor alpha (TGFα), betacellulin (BTC), epiregulin (EPR), heparin-binding EGF-like growth factor (HB-EGF), and/or Neuregulin (NRG) is used at a concentration of between about 5 ng/ml to 5 µg/ml, or between about 20 ng/ml to 4 µg/ml, or between about 30 ng/ml to 3 µg/ml, or between about 40 ng/ml to 2 µg/ml, or between about 45 ng/ml to 500 ng/ml, or between about 50 ng/ml to 300 ng/ml, or about 35, 40, 45, 50, 60, 70, 90, 100, 150, 200, 250, 300, 400, 450, 600, 700 or 800 ng/ml.

In some embodiments, the medium further comprises an activator of STAT3, an activator of GAB1 mediated cell adhesion and/or an activator of the AKT/PI3K signaling pathway. In some embodiments, the activator of STAT3, activator of GAB1 mediated cell adhesion and/or activator of the AKT/PI3K signaling pathway is used at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, or 70 ng/ml, or about 1, 2.5, 3.5 or 4.5 µg/ml. In some embodiments, the activator of the AKT/PI3K signaling pathway is a hepatocyte growth factor (HGF). In some embodiments, HGF is used at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, or 70 ng/ml, or about 1, 2.5, 3.5 or 4.5 µg/ml.

In some embodiments, the medium further comprises a molecule which is a repressor of NFκB activity and/or activator of mitogen-activated protein (MAP) kinase ERK, p38 and JNK. In some embodiments, the molecule which is a repressor of NFκB activity and/or activator of mitogen-activated protein (MAP) kinase ERK, p38 and JNK is used at a concentration of between about 0.1 mM to 1 M, or between about 2 mM to 0.8 M, or between about 4 mM to 0.6 M, or between about 6 mM to 0.4 M, or between about 8 mM to 0.2 M, or between about 10 mM to 800 mM, or between about 50 mM to 500 mM, or about 3, 5, 9, 15, 20, 30, 50, 80, 100, 120, 150, 200, 250, 300, 350, 400 or 450 mM. In some embodiments, the medium further comprises a compound selected from the group consisting of nicotinamide, nicotinic acid, 5-fluoronicotinamide, isonicotinic acid hydrazide, and nikethamide. In some embodiments, nicotinamide, nicotinic acid, 5-fluoronicotinamide, isonicotinic acid hydrazide, and/or nikethamide is used at a concentration of between about 0.1 mM to 1 M, or between about 2 mM to 0.8 M, or between about 4 mM to 0.6 M, or between about 6 mM to 0.4 M, or between about 8 mM to 0.2 M, or between about 10 mM to 800 mM, or between about 50 mM to 500 mM, or about 3, 5, 9, 15, 20, 30, 50, 80, 100, 120, 150, 200, 250, 300, 350, 400 or 450 mM.

In some embodiments, the medium further comprises at least one, at least two, or at least three molecules inducing phosphorylation of SMAD1, SMADS and SMAD8 and activating MAPK signaling. In some embodiments, the molecule(s) inducing phosphorylation of SMAD1, SMADS and SMAD8 and activating MAPK signaling is used at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, 70 ng/ml, or about 1, 2.5, 3.5 or 4.5 µg/ml. In some embodiments, the medium further comprises a molecule selected from the group consisting of BMP4, BMP2, BMP3, BMP5, BMP6, and BMP7. In some embodiments, the BMP family molecule(s) is/are used at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, 70 ng/ml, or about 1, 2.5, 3.5 or 4.5 µg/ml.

In some embodiments, the medium further comprises a molecule which is an activator of the FGF and MAPK pathway. In some embodiments, the medium further comprises a molecule selected from the group consisting of FGF7, FGF1, FGF3, FGF10, and FGF22. In some embodiments, the activator of the FGF and MAPK pathway, or FGF family molecule is used at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, 70 ng/ml, or about 1, 2.5, 3.5 or 4.5 µg/ml.

Additional components found in some embodiments:

In some embodiments, the medium further comprises an activator of cAMP-dependent pathways, such as an activator of the Protein Kinase A signaling pathway, which induces proliferation of epithelial cell types. In some embodiments, the activator of the cAMP-dependent pathway is a compound selected from the group consisting of dibutyryl-cAMP(dbCAMP), forskolin ((3R,4aR,5S,6S,6aS,10S,10aR,10bS)-6,10,10b-trihydroxy-3,4a,7,7,10a-pentamethyl-1-oxo-3-vinyldodecahydro-1H-benzo[f]chromen-5-yl acetate), caffeine, theophylline, cholera toxin and pertussis toxin. In some embodiments, the activator of cAMP-dependent pathways is used at a concentration of between about 20 ng/ml to 1 µg/ml, or between about 10 ng/ml to 0.8 µg/ml, or between about 15 ng/ml to 0.6 µg/ml, or between about 20 ng/ml to 2 µg/ml, or about 5, 18, 20, 25, 28, 30, 50, 60, or 70 ng/ml, or about 1, 2.5, 3.5 or 4.5 µg/ml. In some embodiments, a compound selected from the group consisting of dibutyryl-cAMP(dbCAMP), forskolin ((3R,4aR,5S,6S,6aS,10S,10aR,10bS)-6,10,10b-trihydroxy-3,4a,7,7,10a-pentamethyl-1-oxo-3-vinyldodecahydro-1H-benzo[f]chromen-5-yl acetate), caffeine, theophylline, cholera toxin and pertussis toxin is used at a concentration of between about 20 ng/ml to 1 µg/ml, or between about 10 ng/ml to 0.8 µg/ml, or between about 15 ng/ml to 0.6 µg/ml, or between about 20 ng/ml to 2 µg/ml, or about 5, 18, 20, 25, 28, 30, 50, 60, or 70 ng/ml, or about 1, 2.5, 3.5 or 4.5 µg/ml.

In some cases, the medium further comprises an activator of the Notch receptor. In some embodiments, the activator of the Notch receptor is used at a concentration of between about 10 nM to 100 µM, or between about 50 nM to 80 µM, or between about 100 nM to 60 µM, or between about 500 nM to 40 µM, or between about 800 nM to 20 µM, or between about 900 nM to 10 µM, or about 20, 40, 60, or 80 nM or about 1, 1.5, 15, 30, 50, 60, 90 or 100 µM.In some embodiments, the activator of the Notch receptor is a compound selected from the group consisting of Jagged1 protein (homo sapiens, also known as AGS; AHD; AWS; HJ1; CD339; JAGL1; JAG1), Jagged2 (NCBI 3714), Delta-like1 (NCBI 28514), Delta-like3 (NCBI 10683), and Delta-like4 (NCBI 54567). In some embodiments, the Jagged1 protein (homo sapiens, also known as AGS; AHD; AWS; HJ1; CD339; JAGL1; JAG1), Jagged2 (NCBI 3714), Delta-like1 (NCBI 28514), Delta-like3 (NCBI 10683), and/or Delta-like4 (NCBI 54567) is used at a concentration of between about 10 nM to 100 µM, or between about 50 nM to 80 µM, or between about 100 nM to 60 µM, or between about 500 nM to 40 µM, or between about 800 nM to 20 µM, or between about 900 nM to 10 µM, or about 20, 40, 60, or 80 nM or about 1, 1.5, 15, 30, 50, 60, 90 or 100 µM.

In some embodiments, the medium further comprises an inhibitor of histone deacetylase (HDACs). In some embodiments, the inhibitor of histone deacetylase is a compound selected from the group consisting of valporic acid (VPA) (2-propylpentanoic acid), sodium butyrate (sodium;4-hydroxybutanoate), vorinotstat (N'-hydroxy-N-phenyloctanediamide), panobinostat ((E)-N-hydroxy-3-[4-[[2-(2-methyl-1H-indol-3-yl)ethylamino]methyl]phenyl]prop-2-enamide), trichostatin A ((2E,4E,6R)-7-[4-(dimethylamino)phenyl]-N-hydroxy-4,6-dimethyl-7-oxohepta-2,4-dienamide), mocetinostat (N-(2-aminophenyl)-4-[[(4-pyridin-3-ylpyrimidin-2-yl)amino]methyl]benzamide), BG45 (N-(2-aminophenyl)-2-pyrazinecarboxamide), 4SC-202 ((E)-N-(2-aminophenyl)-3-(1-((4-(1-methyl-1H-pyrazol-4-yl)phenyl)sulfonyl)-1H-pyrrol-3-yl)acrylamide), belinostat, scriptaid (6-(1,3-Dioxo-1H-benzo[de]isoquinolin-2(3H)-yl)-N-hydroxyhexanamide), M344 (4-(dimethylamino)-N-[7-(hydroxyamino)-7-oxoheptyl]benzamide), dacinostat ((E)-N-hydroxy-3-[4-[[2-hydroxyethyl-[2-(1H-indol-3-yl)ethyl]amino]methyl]phenyl]prop-2-enamide), abexinostat, CUDC-101 (7-(4-(3-ethynylphenylamino)-7-methoxyquinazolin-6-yloxy)-N-hydroxyheptanamide), CUDC-907 (N-hydroxy-2-(((2-(6-methoxypyridin-3-yl)-4-morpholinothieno[3,2-d]pyrimidin-6-yl)methyl)(methyl)amino)pyrimidine-5-carboxamide), and AR-42 ((S)-N-hydroxy-4-(3-methyl-2-phenylbutanamido)benzamide). In some embodiments, the inhibitor of histone deacetylase (including but not limited to the compounds listed above) is used at a concentration of between about 0.1 µM to 5 mM, or between about 0.3 µM to 4 mM, or between about 0.6 µM to 3 mM, or between about 0.8 µM to 2 mM, or between about 1 mM to 1.5 mM, or about 0.2, 0.4, 0.7, 0.9, 5, 10, 20, 50, 70 or 90 µM, or about 1.5, 2.5, or 3.5 mM.

In some embodiments, culture using the first media H1 is for 1 to 10 days or 1 to 8 days or 1 to 6 days.

In some embodiments, culture using second media H2 is for 6 to 12 days or 4 to 10 days or 6 to 8 days.

In some embodiments, culture using third media H3 is for 18 to 26 days or 20 to 24 days or 19 to 22 days.

### H2 media

In some embodiments, H2 medium comprises:
a) an activator of STAT3, GAB1 mediated cell adhesion and/or AKT/PI3K signaling pathway;
b) a molecule which is an repressor of NFκBactivity and activator of mitogen-activated protein (MAP) kinase ERK, p38 and JNK;
c) a TGF-β inhibitor and/or SMAD2/3 inhibitor;
d) a steroid;
e) a molecule inducing phosphorylation of SMAD1 and SMADS and activating MAPK signaling; and
f) a molecule that regulates bile acid synthesis and activates FGF and MAPK pathway.

In some embodiments, the TGF-beta signaling inhibitor is as described and used at the concentrations described in media H1.

In some embodiments, the steroid and concentrations are as described above for media H1.

In some embodiments, the medium further comprises an activator of AKT/PI3K signaling pathway and MAPK signaling pathway as described and used at the concentrations described in media H1. In some embodiments, the medium further comprises a compound selected from the group consisting of an epidermal growth factor (EGF), amphiregulin (AR), epigen (EPG), transforming growth factor alpha (TGFα), betacellulin (BTC), epiregulin (EPR), heparin-binding EGF-like growth factor (HB-EGF), and Neuregulin (NRG). In some embodiments, the compound (e.g., EGF) is used at a concentration of between about 5 ng/ml to 5 µg/ml, or between about 20 ng/ml to 4 µg/ml, or between about 30 ng/ml to 3 µg/ml, or between about 40 ng/ml to 2 µg/ml, or between about 45 ng/ml to 500 ng/ml, or between about 50 ng/ml to 300 ng/ml, or about 35, 40, 45, 50, 60, 70, 90, 100, 150, 200, 250, 300, 400, 450, 600, 700 or 800 ng/ml.

In some embodiments, the medium further comprises an activator of STAT3, GAB1 mediated cell adhesion and AKT/PI3K signaling pathway as described and used at the concentrations described in media H1. In some embodiments, the medium further comprises a hepatocyte growth factor (HGF). In some embodiments, HGF is used at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, 70 ng/ml, or about 1, 2.5, 3.5 or 4.5 µg/ml.

In some embodiments, the medium further comprises a molecule which is an repressor of NFκB activity and activator of mitogen-activated protein (MAP) kinase ERK, p38 and JNK as described and used at the concentrations described in media H1. In some embodiments, the medium further comprises a compound selected from the group consisting of nicotinamide, nicotinic acid, 5-fluoronicotinamide, isonicotinic acid hydrazide, and nikethamide. In some embodiments, the compound (e.g., Nicotinamide) is used at a concentration of between about 0.1 mM to 1 M, or between about 2 mM to 0.8 M, or between about 4 mM to 0.6 M, or between about 6 mM to 0.4 M, or between about 8 mM to 0.2 M, or between about 10 mM to 800 mM, or between about 50 mM to 500 mM, or about 3, 5, 9, 15, 20, 30, 50, 80, 100, 120, 150, 200, 250, 300, 350, 400 or 450 mM.

In some embodiments, the medium further comprises at least one, at least two, or at least three molecule(s) inducing phosphorylation of SMAD1, SMADS and SMAD8 and activating MAPK signaling as described and used at the concentrations described in media H1.

In some embodiments, the medium further comprises a molecule regulating bile acid synthesis and activates FGF and MAPK pathway as described and used at the concentrations described in media H1. In some embodiments, the molecule is selected from the group consisting of FGF19, FGF1, FGF2, FGF4, FGF6, FGF8, FGF9, FGF16, FGF17, FGF18, FGF20, and FGF23. In some embodiments, the FGF family member is used at a concentration of 5 ng/ml to 0.8 µg/ml, or between about 10 ng/ml to 0.6 µg/ml, or between about 50 ng/ml to 0.5 µg/ml, or between about 150 ng/ml to 1 µg/ml, or about 5, 20, 50, 100, 200, 250, 300, 400, 500 ng/ml, or about 1, 0.8, 0.7 or 0.9 µg/ml.

H2 media can further comprises a component for inducing late hepatic progenitor differentiation, wherein the component is any one or two of the following components:
An inhibitor of γ-secretase; and
a YAP inhibitor.

In some embodiments, the inhibitor of γ-secretase is selected from the group consisting of Compound E (C-E) (2S)-2-[[2-(3,5-difluorophenyl)acetyl]amino]-N-[(3S)-1-methyl-2-oxo-5-phenyl-3H-1,4-benzodiazepin-3-yl]propanamide, Dibenzazepine (DBZ): (2S)-2-[[2-(3,5-difluorophenyl)acetyl]amino]-N-[(7S)-5-methyl-6-oxo-7H-benzo[d][1]benzazepin-7-yl]propanamide, DAPT:tert-butyl (2S)-2-[[(2S)-2-[[2-(3,5-difluorophenyl)acetyl]amino]propanoyl]amino]-2-phenylacetate, Begacestat: 5-chloro-N-[(2S)-4,4,4-trifluoro-1-hydroxy-3-(trifluoromethyl)butan-2-yl]thiophene-2-sulfonamide, and Flurizan: (2R)-2-(3-fluoro-4-phenylphenyl)propanoic acid. In some embodiments, the inhibitor of γ-secretase is used at a concentration of between about 10 nM to 5 µM, or between about 100 nM to 4 µM, or between about 200 nM to 3.5 µM, or between about 300 nM to 3 µM, or between about 400 nM to 2.5 µM, or between about 450 nM to 2 µM, or between about 500 nM to 1.5 µM, or about 50, 90, 150, 250, 350, 450, 480, 500, 650, or 700 nM.

In some embodiments, the YAP inhibitor is selected from the group consisting of:
Verteporfin 3-[(23*S*,24*R*)-14-ethenyl-5-(3-methoxy-3-oxopropyl)-22,23-*bis*(methoxycarbonyl)-4,10,15,24-tetramethyl-25,26,27,28-tetraazahexacyclo[16.6.1.1^{3,6}.1^{8,11}.1^{13,16}.0^{19,24}]octacosa-1,3,5,7,9,11(27),12,14,16,18(25),19,21-dodecaen-9-yl]propanoic acid,
LPA [(2R)-2-hydroxy-3-phosphonooxypropyl] (Z)-octadec-9-enoate,
S1P [(E,2S,3R)-2-amino-3-hydroxyoctadec-4-enyl] dihydrogen phosphate, Thrombin (2S)-2-[[(2S)-1-[(2S)-5-amino-2-[[2-[[(2S)-6-amino-2-[[2-[[(2S)-2-[[(2S)-4-amino-2-[[(2S)-2-[[(2S,3R)-2-[[(2S)-2-[[(2S,3R)-2-amino-3-hydroxybutanoyl]amino]-3-(1H-indol-3-yl)propanoyl]amino]-3-hydroxybutanoyl]amino]propanoyl]amino]-4-oxobutanoyl]amino]-3-methylbutanoyl]amino]acetyl]amino]hexanoyl]amino]acetyl]amino]-5-oxopentanoyl]pyrrolidine-2-carbonyl]amino]-3-hydroxypropanoic acid, Epinephrine 4-[(1R)-1-hydroxy-2-(methylamino)ethyl]benzene-1,2-diol, Glucagon (2S)-2-[[(2S,3R)-2-[[2-[[(2S)-5-amino-2-[[(2S)-2-[[(2S)-2-amino-3-(1H-imidazol-5-yl)propanoyl]amino]-3-hydroxypropanoyl]amino]-5-oxopentanoyl]amino]acetyl]amino]-3-hydroxybutanoyl]amino]-3-phenylpropanoic acid,
Dihydrexidine(6aR,12bS)-5,6,6a,7,8,12b-hexahydrobenzo[a]phenanthridine-10,11-diol, Dobutamine4-[2-[4-(4-hydroxyphenyl)butan-2-ylamino]ethyl]benzene-1,2-diol, DasatinibN-(2-chloro-6-methylphenyl)-2-[[6-[4-(2-hydroxyethyl)piperazin-1-yl]-2-methylpyrimidin-4-yl]amino]-1,3-thiazole-5-carboxamide,
Latrunculin A (4R)-4-[(1R,4S,5Z,7E,11Z,15R,17R)-17-hydroxy-4,11-dimethyl-13-oxo-14,18-dioxabicyclo[13.3.1]nonadeca-5,7,11-trien-17-yl]-1,3-thiazolidin-2-one, Latrunculin B (4R)-4-[(1R,4S,5Z,9Z,13R,15R)-15-hydroxy-4,9-dimethyl-11-oxo-12,16-dioxabicyclo[11.3.1]heptadeca-5,9-dien-15-yl]-1,3-thiazolidin-2-one,
Cytochalasin D(3S,3aR,4S,6S,6aR,7E,10S,12R,13E,15R,15aR)-3-Benzyl-6,12-dihydroxy-4,10,12-trimethyl-5-methylene-1,11-dioxo-2,3,3a,4,5,6,6a,9,10,11,12,15-dodecahydro-1H-cycloundeca[d]isoindol-15-yl acetate
Blebbistatin 3a-hydroxy-6-methyl-1-phenyl-2,3-dihydropyrrolo[2,3-b]quinolin-4-one, ML7 1-(5-iodonaphthalen-1-yl)sulfonyl-1,4-diazepane,
Botulinum C3 4-N-(3-chloro-7-methoxyacridin-1-yl)-1-N,1-N-diethylpentane-1,4-diamine; dihydrochloride,
Y27632 4-[(1R)-1-aminoethyl]-N-pyridin-4-ylcyclohexane-1-carboxamide, and
derivatives thereof. In some embodiments, the YAP inhibitor (e.g.,Verteporfin) is used at a concentration of between about 1 nM to 10 µM, or between about 50 nM to 8 µM, or between about 200 nM to 6 µM, or between about 500 nM to 4 µM, or between about 700 nM to 2 µM, or between about 1 µM to 2 µM, or about 1, 10, 30, 70, 130, 180, 250, 350, 400, 600, 800 or 900 nM or about 9, 7, 5, 3, 2.5, 1.5 or 1 µM.

### H3 Media

In some embodiments, H3 medium comprises:
- a TGF-β inhibitor and/or SMAD2/3 inhibitor;
- a pleiotropic cytokine that belongs to the interleukin 6 group of cytokines;
- an inhibitor of γ-secretase; and
- a steroid.

The pleiotropic cytokine that belongs to the interleukin 6 group of cytokines is capable of activating JAK-STAT, MAPK and AKT/PI3K signaling. In some embodiments, the pleiotropic cytokine is oncostatin M (OSM) or leukemia inhibitory factor (LIF; NCBI: 3976), or Cardiotrophin-1/CT-1 (NCBI: 1489), or ciliary neurotrophic factor receptor (CNTF; NCBI: 1271), IL-11 or IL-31. In some embodiments, the pleiotropic cytokine (e.g., OSM) is used at a concentration of between about 0.1 ng/ml to 1 µg/ml, or between about 10 ng/ml to 0.8 µg/ml, or between about 15 ng/ml to 0.6 µg/ml, or between about 20 ng/ml to 2 µg/ml, or about 5, 18, 20, 25, 28, 30, 50, 60, 70 ng/ml, or about 1, 2.5, 3.5 or 4.5 µg/ml.

In some embodiments, the inhibitor of γ-secretase is selected from the group consisting of Compound E (C-E) (2S)-2-[[2-(3,5-difluorophenyl)acetyl]amino]-N-[(3S)-1-methyl-2-oxo-5-phenyl-3H-1,4-benzodiazepin-3-yl]propanamide, Dibenzazepine (DBZ): (2S)-2-[[2-(3,5-difluorophenyl)acetyl]amino]-N-[(7S)-5-methyl-6-oxo-7H-benzo[d][1]benzazepin-7-yl]propanamide, DAPT:tert-butyl (2S)-2-[[(2S)-2-[[2-(3,5-difluorophenyl)acetyl]amino]propanoyl]amino]-2-phenylacetate, Begacestat: 5-chloro-N-[(2S)-4,4,4-trifluoro-1-hydroxy-3-(trifluoromethyl)butan-2-yl]thiophene-2-sulfonamide, and Flurizan: (2R)-2-(3-fluoro-4-phenylphenyl)propanoic acid. In some embodiments, the inhibitor of γ-secretase (e.g., Compound E (C-E)) is used at a concentration of between about 10 nM to 5 µM, or between about 100 nM to 4 µM, or between about 200 nM to 3.5 µM, or between about 300 nM to 3 µM, or between about 400 nM to 2.5 µM, or between about 450 nM to 2 µM, or between about 500 nM to 1.5 µM, or about 50, 90, 150, 250, 350, 450, 480, 500, 650, or 700 nM.

In some embodiments, the TGF-beta signaling inhibitor is as described and is used at the concentrations described above for media H1.

In some embodiments, the steroid is as described and is used at the concentrations described above for media H1.

H3 medium can further comprises at least one or two or three or four or five or six component(s) promoting maturation of hepatic organoid and/or at least one or two or three component(s) promoting survival of hepatic organoids.

The component(s) promoting maturation of the hepatic organoid is selected from the group consisting of:
a compound inducing phosphorylation of SMAD1, SMADS and SMAD8 and activating MAPK signaling;
an interleukin that acts as both a pro-inflammatory cytokine and an anti-inflammatory myokine;
a compound that regulates bile acid synthesis and activates FGF and MAPK pathway; activator of cAMP-dependent pathways;
a YAP inhibitor; and
a compound with biliary acid potency.

In some embodiments, the above components that promote maturation of the hepatic organoid are as described and are used at the concentrations described above for media H1 and H2.

The interleukin that acts as both a pro-inflammatory cytokine and an anti-inflammatory myokine activates JAK-STAT, MAPK and AKT/PI3K signaling. In some embodiments, the interleukin is IL-6. In some embodiments, the interleukin is at a concentration of between about 0.1 ng/ml to 1 µg/ml, or between about 5 ng/ml to 0.5 µg/ml or between about 10 ng/ml to 0.8 µg/ml, or between about 15 ng/ml to 0.6 µg/ml, or between about 20 ng/ml to 2 µg/ml, or about 5, 18, 20, 25, 28, 30, 50, 60, 70 ng/ml, or about 1, 2.5, 3.5 or 4.5 µg/ml.

In some embodiments, the compound with biliary acid potency is characterized by any one or more or all of the following capabilities: activating the nuclear farnesoid X receptor, increasing cAMP and thus activating the PKC signaling pathway. In some embodiments, the compound with biliary acid potency is selected from the group consisting of:
Taurocholic acid 2-[[(4R)-4-[(3R,5S,7R,8R,9S,10S,12S,13R,14S,17R)-3,7,12-trihydroxy-10,13-dimethyl-2,3,4,5,6,7,8,9,11,12,14,15,16,17-tetradecahydro-1H-cyclopenta[a]phenanthren-17-yl]pentanoyl]amino]ethanesulfonic acid,
Cholic acid (4R)-4-[(3R,5S,7R,8R,9S,10S,12S,13R,14S,17R)-3,7,12-trihydroxy-10,13-dimethyl-2,3,4,5,6,7,8,9,11,12,14,15,16,17-tetradecahydro-1H-cyclopenta[a]phenanthren-17-yl]pentanoic acid,
Chenodeoxycholic acid (4R)-4-[(3R,5S,7R,8R,9S,10S,13R,14S,17R)-3,7-dihydroxy-10,13-dimethyl-2,3,4,5,6,7,8,9,11,12,14,15,16,17-tetradecahydro-1H-cyclopenta[a]phenanthren-17-yl]pentanoic acid,
Glycocholic acid 2-[[(4R)-4-[(3R,5S,7R,8R,9S,10S,12S,13R,14S,17R)-3,7,12-trihydroxy-10,13-dimethyl-2,3,4,5,6,7,8,9,11,12,14,15,16,17-tetradecahydro-1H-cyclopenta[a]phenanthren-17-yl]pentanoyl]amino]acetic acid,
Deoxycholic acid (4R)-4-[(3R,5R,8R,9S,10S,12S,13R,14S,17R)-3,12-dihydroxy-10,13-dimethyl-2,3,4,5,6,7,8,9,11,12,14,15,16,17-tetradecahydro-1H-cyclopenta[a]phenanthren-17-yl]pentanoic acid,
Glycochenodeoxycholic acid 2-[[(4R)-4-[(3R,5S,7R,8R,9S,10S,13R,14S,17R)-3,7-dihydroxy-10,13-dimethyl-2,3,4,5,6,7,8,9,11,12,14,15,16,17-tetradecahydro-1H-cyclopenta[a]phenanthren-17-yl]pentanoyl]amino]acetic acid,
Taurochenodeoxycholic acid 2-[[(4R)-4-[(3R,5S,7R,8R,9S,10S,13R,14S,17R)-3,7-dihydroxy-10,13-dimethyl-2,3,4,5,6,7,8,9,11,12,14,15,16,17-tetradecahydro-1H-cyclopenta[a]phenanthren-17-yl]pentanoyl]amino]ethanesulfonic acid,
Lithocholic acid (4R)-4-[(3R,5R,8R,9S,108,13R,14S,17R)-3-hydroxy-10,13-dimethyl-2,3,4,5,6,7,8,9,11,12,14,15,16,17-tetradecahydro-1H-cyclopenta[a]phenanthren-17-yl]pentanoic acid, and derivatives thereof.

In some embodiments, the compound with biliary acid potency is used at a concentration of between about 1 µM to 1 mM, or about 10 µM to 0.8 mM, or about 50 µM to 0.6 mM, or about 100 µM to 0.4 mM, or about 150 µM to 0.2 mM, or about 5, 15, 20, 40, 60, 80, 90, 100, 150, 250, 350, 450, 550, 650, 750, or 850 µM.

In some embodiments, the component(s) promoting survivability of the hepatic organoid is selected from the group consisting of:
an activator of STAT3, GAB1 mediated cell adhesion and/or AKT/PI3K signaling pathway;
a glycosaminoglycan; and
an activator of AKT/PI3K signaling pathway and MAPK signaling pathway.

In some embodiments, the activator of the STAT3, GAB1 mediated cell adhesion and/or AKT/PI3K signaling pathway, and the activator of AKT/PI3K signaling pathway and MAPK signaling pathway are as described and are used at the concentrations described above for media H1 and H2.

In some embodiments, the glycosaminoglycan is used at a concentration of between about 100 ng/ml to 1 mg/ml, or between about 100 ng/ml to 1 mg/ml, or between about 500 ng/ml to 0.5 mg/ml, , or between about 1 µg/ml to 0.1 mg/ml, or between about 5 µg/ml to 500 µg/ml, or about 300, 500, 700 or 800 ng/ml, or about 1, 2, 3, 10, 20, 40, 50, 60, 100, 500, 700 µg/ml. In some embodiments, the glycosaminoglycan is heparin, and is used at a concentration of between about 100 ng/ml to 1 mg/ml, or between about 100 ng/ml to 1 mg/ml, or between about 500 ng/ml to 0.5 mg/ml, , or between about 1 µg/ml to 0.1 mg/ml, or between about 5 µg/ml to 500 µg/ml, or about 300, 500, 700 or 800 ng/ml, or about 1, 2, 3, 10, 20, 40, 50, 60, 100, 500, 700 µg/ml

### Extracellular matrix and suspension culture

### Extracellular matrix:

In some embodiments, Stem cells are cultured in a microenvironment that mimics at least in part a cellular niche in which said stem cells naturally reside. This cellular niche may be mimicked by culturing said stem cells in the presence of biomaterials, such as matrices, scaffolds, and culture substrates that represent key regulatory signals controlling stem cell fate. Such biomaterials comprise natural, semi-synthetic and synthetic biomaterials, and/or mixtures thereof. A scaffold provides a two-dimensional or three dimensional network. Suitable synthetic materials for such a scaffold comprise polymers selected from porous solids, nanofibers, and hydrogels such as, for example, peptides including self-assembling peptides, hydrogels composed of polyethylene glycol phosphate, polyethylene glycol fumarate, polyacrylamide, polyhydroxyethyl methacrylate, polycellulose acetate, and/or co-polymers thereof (see, for example, Saha et al., 2007. Curr Opin Chem. Biol. 11(4): 381-387; Saha et al., 2008. Biophysical Journal 95: 4426-4438; Little et al., 2008. Chem. Rev 108, 1787-1796). As is known to a skilled person, the mechanical properties such as, for example, the elasticity of the scaffold influences proliferation, differentiation and migration of stem cells. In some embodiments, the scaffold comprises biodegradable (co)polymers that are replaced by natural occurring components after transplantation in a subject, for example to promote tissue regeneration and/or wound healing. In some embodiments, said scaffold does not substantially induce an immunogenic response after transplantation in a subject. Said scaffold is supplemented with natural, semi-synthetic or synthetic ligands, which provide the signals that are required for proliferation and/or differentiation, and/or migration of stem cells. In one embodiment, said ligands comprise defined amino acid fragments. Examples of said synthetic polymers comprise Pluronic^{®} F127 block copolymer surfactant (BASF), and Ethisorb (Johnson and Johnson). A cellular niche is in part determined by the stem cells and surrounding cells, and the extracellular matrix (ECM) that is produced by the cells in said niche. In some embodiments, MESP are attached to an ECM. ECM is composed of a variety of polysaccharides, water, elastin, and glycoproteins, wherein the glycoproteins comprise collagen, entactin (nidogen), fibronectin, and laminin. ECM is secreted by connective tissue cells. Different types of ECM are known, comprising different compositions including different types of glycoproteins and/or different combination of glycoproteins. Said ECM can be provided by culturing ECM-producing cells, such as for example fibroblast cells, in a receptacle, prior to the removal of these cells and the addition of isolated liver fragments or isolated biliary duct or isolated epithelial stem cells. Examples of extracellular matrix-producing cells are chondrocytes, producing mainly collagen and proteoglycans, fibroblast cells, producing mainly type IV collagen, laminin, interstitial procollagens, and fibronectin, and colonic myofibroblasts producing mainly collagens (type I, III, and V), chondroitin sulfate proteoglycan, hyaluronic acid, fibronectin, and tenascin-C. Alternatively, said ECM is commercially provided. Examples of commercially available extracellular matrices are extracellular matrix proteins (Invitrogen) and basement membrane preparations from Engelbreth-Holm-Swarm (EHS) mouse sarcoma cells (e.g. Matrigel^{™} (BD Biosciences)). A synthetic extracellular matrix material, such as ProNectin (Sigma Z378666) may be used. Mixtures of extracellular matrix materials may be used, if desired. The use of an ECM for culturing stem cells enhanced long-term survival of the stem cells and the continued presence of undifferentiated stem cells. In the absence of an ECM, stem cell cultures could not be cultured for longer periods and no continued presence of undifferentiated stem cells was observed. In addition, the presence of an ECM allowed culturing of three-dimensional tissue organoids, which could not be cultured in the absence of an ECM. The extracellular matrix material will normally be coated onto a cell culture vessel, but may (in addition or alternatively) be supplied in solution. A fibronectin solution of about 1 mg/ml may be used to coat a cell culture vessel, or between about 1 µg/cm2 to about 250 µg/cm2, or at about 1 µg/cm2 to about 150 µg/cm2. In some embodiments, a cell culture vessel is coated with fibronectin at between 8 µg/cm2 and 125 µg/cm2. One ECM for use in the methods described herein comprises at least two distinct glycoproteins, such as two different types of collagen or a collagen and laminin. The ECM can be a synthetic hydrogel extracellular matrix or a naturally occurring ECM. Another ECM is provided by Matrigel^{™} (BD Biosciences), which comprises laminin, entactin, and collagen IV.

The suspension culture system refers to any culture system, in which the cells are not embedded in a solid or semi-solid matrix in the culture and are free floating in the culture apparatus without resting on the bottom of the apparatus.

### Characterization of early hepatic progenitors, late hepatic progenitors and hepatic organoids:

As described herein, the early hepatic progenitor is characterized by any one or more or at least two, or at least three, or at least four, or at least five, or at least six, or between 1 or 2 or 3 to 4 or 5 or 6 or 7 or all, or all of the following markers: SOX9 (NCBI: 6662), CK19 (NCBI: 3880), CK18 (NCBI: 3875), HNF4a (NCBI: 3172), PROX1 (NCBI: 5629), ONECUT1 (NCBI: 3175), AFP (NCBI: 174), TBX3 (NCBI:6926).

As described herein, the late hepatic progenitor is characterized by any one or more or at least two, or at least three, or at least four, or at least five, or at least six, or between 1 or 2 or 3 to 4 or 5 or 6 or 7 or all, or all of the following markers: CK19 (NCBI: 3880), CK18 (NCBI: 3875), HNF4a (NCBI: 3172), PROX1 (NCBI: 5629), ONECUT1 (NCBI: 3175), AFP (NCBI: 174), TBX3 (NCBI:6926), ALB (NCBI: 213).

In some embodiments, the hepatic (liver) organoids comprise more than one liver specific cell type selected from the group consisting of hepatocytes, and cholangiocytes, and optinally liver specific endothelial cells (LSEC), stellate cells, hepatic myofibroblast and hepatoblasts.

The hepatocytes are characterized by their expression of albumin (ALB) and not cholangiocytes marker, such as Cytokeratin 7 (CK7). The hepatocytes express all of the following hepatocyte markers: HNF4a (NCBI: 3172), FAH (NCBI: 2184), TAT (NCBI: 6898), GCK (NCBI: 2645), TTR (NCBI: 7276), MET (NCBI: 4233), GLU1/MGAM (NCBI: 8972), FAHD2A (NCBI: 51011), HNF1B (NCBI: 6928), HNF1A (NCBI: 6927), CYP3A4 (NCBI: 1576), CYP2C9 (NCBI: 1559), CYP2C19 (NCBI: 1557), CYP1A2 (NCBI: 1544), CYP2E1 (NCBI: 1571), CYP2D6 (NCBI: 1565), CYP3A7 (NCBI: 1551), CYP1A1 (NCBI: 1543), CYP3A5 (NCBI: 1577), CYP27A1 (NCBI: 1593) and CYP2B6 (NCBI: 1555).

In some embodiments, the cholangiocytes are characterized by their expression of CK7 but not albumin (ALB) and optionally by their expression of other cholangiocytes markers, such as CK19 (NCBI: 3880), HNF1B (NCBI: 6928) and SOX9 (NCBI: 6662).

In some embodiments, the hepatoblasts are characterized by expression of any one or more markers selected from the group consisting of SOX9 (NCBI: 6662), CK19 (NCBI: 3880), CK18 (NCBI: 3875), HNF4a (NCBI: 3172), PROX1 (NCBI: 5629), ONECUT1 (NCBI: 3175), AFP (NCBI: 174), and ALB (NCBI: 213).

In some embodiments, the liver specific endothelial cells (LSEC) are characterized by expression of any one or more markers selected from the group consisting of CD45, CD80, CD86, CD11c, VAP1, STAB1 and CD31 that is mainly expressed in the cytoplasm and not on the cell surface.

In some embodiments, the stellate cells are characterized by expression of any one or more markers selected from the group consisting of GFAP, VIM, LHX2, LRAT, PDGFRb, HAND2, ICAM-1, VCAM-1, and N-CAM

In some embodiments, the hepatic myofibroblast are characterized by expression of any one or more markers selected from the group consisting of COL1A1 and α-SMA.

The hepatic (liver) organoids described herein are capable of performing liver functions and exhibit a structural composition observed in liver.

In some embodiments, the liver functions are selected from the group consisting of albumin secretion, cytochrome enzymatic activities, glycogen storage, low density lipo-protein uptake, bile acid production and drug metabolism.

The structural composition observed in liver that is found in the hepatic (liver) organoid described herein is characterized by the non-random distribution of the different liver cell types of which the liver is composed.

### Liver organoids

Liver organoids can be generated from the MESP via a step wise induction method where the stem cells first commit to early hepatic progenitors expressing AFP but not ALB (Figure 17 and 18). The cells are subsequently induced to form late hepatic progenitors expressing both AFP and ALB (Figure 18). As hepatic progenitor, the cells give rise to multiple liver cell types as the spheroid is induced to form the organoid containing terminally differentiated hepatocytes that only express ALB but not CK7 and cholangiocytes that only expresses CK7 and not ALB (Figure 20). The cholangiocytes form a bile duct-like cyst structures that locates in the periphery of the organoids, surrounding a dense cluster of hepatocytes (Figure 19 and 20). This arrangement of the cholangiocyte cyst structures around the core of hepatocytes closely resembles the arrangement of the hepatocytes and the bile ducts in the liver tissue, supporting that the organoid consist of multiple cell types that are spatially arranged in structures similar to that found in the liver organ (Figure 21). The gene expression analysis also shows that the organoids expresses hepatocytes markers such as ALB, CEBP, FOXA2, HNF1B, HNF4A, HNF6, KRT18, KRT8 and NOTCH, and also cholangiocyte markers such as CFTR, KRT7, KRT19 and SOX9 (Figure 22). The specific expression of the markers by individual cell type is validated by the specific staining of markers such as ALB in the hepatocytes (Figure 21), and CK7 and CFTR in the cholangiocytes (Figure 21 and 25).

The liver organoids generated from MESP also expresses many of the functional metabolic enzymes found in the liver (Figure 23). This includes the major cytochrome P450 enzymes and the uridine diphosphate glucuronosyltransferase enzymes. The organoids also expresses major bile secretion transporter such as NTCP and OATP1B3. Expression of these enzymes and transporter strongly suggest that the liver organoids perform major functions of the liver. Indeed, the liver organoids was shown to perform many liver specific functions such glucose metabolism demonstrated by the glycogen storage capacity of the hepatocytes in the organoids (Figure 27A) and also the lipid metabolism, as shown by the lipid uptake in the hepatocyte (Figure 27B). The organoids were also shown to be able to secrete albumin (Figure 28). Alpha-1 Antitrypsin (A1AT) is important enzyme produced and secreted by the liver. A1AT deficiency in humans results in lung disorders such as COPD. The liver organoids is also capable of producing this important enzyme (Figure 26). CYP450 enzymatic activity is important for the detoxification role of the liver. The liver organoids exhibited much stronger CYP3A4, CYP2D6, CYP2B6 and CYP1A2 activity compared to HepG2 cell lines (Figure 29). These major CYPs account for close to 70% of drugs metabolized by the liver (Zanger and Schwab, 2013).

The liver tissue structures form by multiple cell types observed in the liver organoid would allow us to model liver organ function which is not possible using pure 2D and 3D hepatocyte cultures. Herein, the liver organ is shown to exhibit liver specific organ-level functions such as the bile secretion and transport to the bile duct. Bile secretion is an important unique function of the liver (Boyer et al, 2013). The hepatocytes secrete bile which contains many important components such as bile salts, cholesterol and metabolized exogenous drugs, xenobiotics and toxins. Bile plays important physiological functions such as the removal of the harmful lipophilic substances, digestion and absorption of fats in the small intestine, elimination of cholesterols and regulation of many hormones and pheromones which aids in the development of the intestine. Bile secretion and transport in the liver cannot be modeled with 2D and 3D and hepatocytes as there are no functional network of bile canaliculi that connects to the bile duct. In contrast, the liver organoid described herein contains an extensive network of bile canaliculi in the core of hepatocytes which connects to the bile duct-like cyst (Figure 30- 36). The live imaging with CDFDA treatment and staining of bile canaliculi marker DPP IV shows that the hepatocytes forms reticulating bile canaliculi which extends from the center of the organoid to the periphery. The bile canaliculi is also functional as the hepatocyte secreted molecules (CDF) is shown to be transported from the bile canaliculi into bile duct-like cyst structure formed by the cholangiocytes (Figure 36). Thus, the liver organoid described herein exhibits organ level functions which could only be achieved by functional interaction of multiple cell types to form tissue structures. Such organ level functions differentiates the liver organoids described herein from 1) Liver epithelial organoids which largely contain stem cells (Huch et al, 2015) and 2) Liver bud formed by the aggregation of multiple cell types which is non-functional *in vitro* and only matures to form liver tissue when transplanted into animals (Takaebe et al, 2013). The Liver organoid generated in this application now allow us to investigate organ level functions in a dish which would be more reflective of human specific liver diseases.

The successful generation of liver organoids from MESP enables the modeling of genetic diseases of the liver. The LDLR^{-/-} MESP can be differentiated to form liver organoids. These liver organoids produces an elevated level of cholesterol which reflects the pathological conditions of Familial hypercholesterolemia (Figure 37). Familial hypercholesterolemia is a genetic disease where patients have mutations in the low-density lipoprotein receptor (LDLR) gene, resulting in the deficiency of the LDLR protein in the liver. This deficiency results in higher level of cholesterol secreted by the liver and into the bloodstream. The elevated blood cholesterol levels eventually results in early onset of cardiovascular disease and patients usually undergo statin regime to lower blood cholesterol levels. Remarkably, similar dosage dependent treatment of the LDLR^{-/-} liver organoids with statin results in the decrease in cholesterol secretion levels similar to that organoids expressing LDLR (Figure 37). These results show that the genetically modified MESP and its derived liver organoids recapitulate pathophysiological conditions of the genetic diseases and responses to known drug treatments. Thus, this system (Figure 14) is a powerful tool that enables the potential modeling of liver, pancreas and intestine diseases.

While organoids that mimic different human organs have been generated, a key hurdle is to produce homogenous organoids in a high throughput manner to allow large scale drug screening (Spence et al 2011, Lancaster et al 2013, Takasato et al 2016). The complex culture conditions and reliance on the self-organizing capacity of stem cells for organoid generation make it hard to generate a dish of organoids of similar size, structure and function. This is a challenging hurdle to overcome, towards the use of organoids in industrial applications. As described herein, the organoids are optimized for generation in a high throughput manner where each single 96 well contains a single organoid of similar size and structure (Figure 38). In addition to the morphology, the organoids generated using this high throughput manner exhibits similar metabolic activity for cytochrome enzymes CYP3A4 and CYPB6. This generation of near homogenous liver organoids in a high throughput manner makes it suitable for the current methods to be employed in large scale lab or industrial settings.

### Derivation of hepatic organoids from MESP

Exemplary methods for producing hepatic organoids from MESP are described in the Examples and illustrated schematically in Figure 17.

### Adult liver organoids

The successful step wise generation of the liver organoids from the posterior foregut-like MESP suggests that this methodology can also be used to generate liver organoids from stem cells of the endoderm lineage that is developmentally in line with liver organ development. This would include early hepatic progenitor stem cells arising from the MESP or stem cells existing in the adult liver stem cells. Using stem cells derived from human adult liver (as described in PCT/SG2016/050270) the organoid generation methodology described herein can similarly be employed to generate liver organoids from the adult liver stem cells (ALSC) (Figures 41-44). The organoids that develop from ALSC are referred to herein as Adult Liver Organoids. In comparison to the MESP, the ALSC are developmentally committed to form tissues of the liver lineage. Thus, in some embodiments, initial treatment with H1 media for hepatic lineage specification is not required. In some embodiments, liver organoids can be generated by culturing ALSC in media H2 and H3 (Figure 40). The adult liver stem cells treated with H2 media differentiate into late hepatic progenitors that expresses ALB and CK19 (Figure 41). The late progenitor subsequently differentiates in the H3 media to form liver organoids (Figures 42 and 43). The cholangiocytes are localized to the periphery of the organoids and the hepatocytes form the core of the organoids. The cholangiocytes organized itself into the ductal-like structure with a lumen in the center (Figure 43). Similar to the MESP derived organoids, the ALSC derived organoids expresses markers of both hepatocytes and cholangiocytes, as well as liver enriched and specific metabolic enzymes and transporters (Figure 44). Importantly, the adult liver organoid also exhibit liver specific metabolic activities (Figure 45). CYP2C9 enzyme is highly active only in adult hepatocytes. The high level of CYP2C9 activity exhibited by the adult liver organoid underlines that the adult organoid shows metabolic activities similar to the adult liver. In addition to the liver functions, the adult liver organoids also possess similar bile canaliculi structures as MESP derived organoids (Figure 46).

Both MESP and ALSC generated similar organoids consisting of a hepatocyte core with cholangiocyte forming ductal-like structures in the periphery of organoids. Structural differences can be observed in the ductal structure formed by the cholangiocytes in both organoids. The cholangiocytes of the MESP derived organoid forms a spherical cyst structure whereas the cholangiocytes of the ALSC derived organoids arrange into a ring with a lumen in the center. Such differences can be expected as the stem cells are of different developmental potential. MESP derived from embryonic stem cells are fetal in nature and similarly the liver organoids derived MESP reflects fetal liver tissue. On the other hand, the liver organoids derived from ALSC reflect the adult liver tissue. In light of the differences in the developmental stages of both organoids, the overall structure and cell organization of the organoids are similar; Hepatocytes are found in the core of the organoids and cholangiocytes are in the periphery of the organoids. More remarkably, the multi-step methodology described herein can be differentially employed according to the initial endoderm stem cell state to generate liver organoids. As such, it is obvious that any early endoderm progenitors such as definitive endoderm or foregut can be first differentiated to MESP and generating organoids subsequently using methods described herein. Similarly, protocol can be employed by someone skilled in the art on stem cells or progenitors that arise during the development of posterior foregut to the adult liver.

### Derivation of hepatic organoids from adult liver stem cells

The Hepatic organoid culture system described herein comprises a plurality of soluble agents in two different hepatic culture media and suspension culture system. The suspension culture system provides conditions for formation of late hepatic progenitors and subsequently organoids. In some embodiments, the plurality of soluble agents comprises one or more growth factors, an enhancer of the (canonical) WNT pathway, a TGF-β inducer and an inhibitor of Notch signaling.

### Characterization of adult late hepatic progenitors and adult hepatic organoids

As described herein, the late hepatic progenitor is characterized by any one or more or at least two, or at least three, or at least four, or at least five, or at least six, or 1 or 2 or 3 or 4 or 5 or 6 or all, or all of the following markers: CK19 (NCBI: 3880), CK18 (NCBI: 3875), HNF4a (NCBI: 3172), ALB (NCBI: 213), HNF1B (NCBI: 6928) and SOX9 (NCBI).

In some embodiments, the hepatic (liver) organoids comprise more than one liver specific cell type selected from the group consisting of hepatocytes, cholangiocytes, liver specific endothelial cells (LSEC), stellate cells, hepatic myofibroblast and hepatoblasts.

In some embodiments, the hepatocytes are characterized by
a. their expression of albumin (ALB) and not cholangiocyte markers, such as Cytokeratin 7 (CK7); and
b. expression of all of the following hepatocyte markers: HNF4a (NCBI: 3172), FAH (NCBI: 2184), TAT (NCBI: 6898), GCK (NCBI: 2645), TTR (NCBI: 7276), MET (NCBI: 4233), GLUl/MGAM (NCBI: 8972), FAHD2A (NCBI: 51011), HNF1B (NCBI: 6928), HNF1A (NCBI: 6927), CYP3A4 (NCBI: 1576), CYP2C9 (NCBI: 1559), CYP2C19 (NCBI: 1557), CYP1A2 (NCBI: 1544), CYP2E1 (NCBI: 1571), CYP2D6 (NCBI: 1565), CYP3A7 (NCBI: 1551), CYP1A1 (NCBI: 1543), CYP3A5 (NCBI: 1577), CYP27A1 (NCBI: 1593) and CYP2B6 (NCBI: 1555).

In some embodiments, the cholangiocytes are characterized by their expression of CK7 but not albumin (ALB) and optionally by their expression of other cholangiocytes markers, such as CK19 (NCBI: 3880), HNF1B (NCBI: 6928) and SOX9 (NCBI: 6662).

In some embodiments, the liver specific endothelial cells (LSEC) are characterized by expression of any one or more markers selected from the group consisting of CD45, CD80, CD86, CD11c, VAP1, STAB1 and CD31, wherein the CD31 is predominantly expressed in the cytoplasm and not on the cell surface.

In some embodiments, the stellate cells are characterized by expression of any one or more markers selected from the group consisting of GFAP, VIM, LHX2, LRAT, PDGFRb, HAND2, ICAM-1, VCAM-1, and N-CAM

In some embodiments, the hepatic myofibroblast are characterized by expression of any one or more markers selected from the group consisting of COL1A1 and α-SMA.

In some embodiments, the hepatoblasts are characterized by expression of any one or more markers selected from the group consisting of SOX9 (NCBI: 6662), CK19 (NCBI: 3880), CK18 (NCBI: 3875), HNF4a (NCBI: 3172), HNF1B (NCBI: 6928) and ALB (NCBI: 213).

The hepatic (liver) organoids derived from ALSC are capable of performing liver functions and exhibit a structural composition observed in liver.

The liver functions are selected from the group consisting of albumin secretion, cytochrome enzymatic activities, glycogen storage, low density lipo-protein uptake, bile acid production and drug metabolism.

The structural composition observed in liver that is found in the hepatic (liver) organoid is characterized by the non-random distribution of the different liver cell types of which the liver is composed.

An exemplary method for generating hepatic organoids from adult liver stem cells is described in the Examples and illustrated schematically in Figure 41.

### EXAMPLES

### Materials and methods

### Gene expression analysis with Quantitative PCR (qPCR)

Total RNA from the cells was isolated using the TRIzol reagent (thermos scientific) according to manufacturer's protocol. Briefly, 1ml of trizol was used for not more than 1.5 million cells. Trizol was added to the cells directly after media was removed. The samples were incubated for 15-30 min to completely lyse the cells. 200µl of the 100% chloroform was added and samples were vigorously mixed and left to stand for 5min at room temperature. The samples were centrifuge for 15 min at 13,000 RPM in 4°C and top aqueous layer was retrieve into a new 1.5ml appendorf tube. Equal volume of 100% Isopropanol was added to the aqueous solution to precipitate the Total DNA and RNA. The samples were left to stand for 10mins and centrifuge for 10 min at 13,000 RPM in 4°C. The total DNA and RNA pelleted are washed once with 70% ethanol and centrifuge for 5 min at 5,000 RPM at room temperature. The total DNA and RNA is reconstituted with DEPC water. DNA contaminations is removed via DNASE I treatment (Thermo Scientific). The total RNA is clean up using RNA purification kit (PureLink, Invitrogen) according to the manufacturer's protocol. 500ng of total RNA was input for the reverse transcription process using the SuperScript II reverse transcriptase reagents (Invitrogen) according to the manufacturer's protocol. The cDNA was quantitated using the SYBR FAST qPCR Master Mix (KAPA) reagents and read with the Real-Time PCR System (Applied Biosystem).

### Cell staining using immunofluorescence

For immunofluorescence of 3D suspension cultures, the organoids were washed three times with PBS before fixing with 4% PFA for 30mins at room temperature. The samples were permeabilized with 0.5% Triton X-100 and blocked with 0.5% Triton X-100 + 5% BSA respectively for 1hr. The samples are incubated with the primary antibody diluted in 0.1% Tween-20 containing 5% BSA overnight at 4°C. After 16-24hr, the samples were washed three times with 0.1% Tween-20 for 15mins during each wash. The organoids are incubated with secondary antibody diluted in 0.1% Tween-20 containing 5% BSA for 3hrs at room temperature and subsequently washed three times with 0.1% Tween-20. Hoechst 33342 was added during the last wash. For immunofluorescence of 3D matrigel cultures, the Matrigel containing the cells was mechanically dissociated, transferred to an eppendorff tube and kept on ice. A combination of dispase and low temperature was used to liquefy the Matrigel. After 15mins, the samples were centrifuged at 1,000 r.p.m for 5mins and the supernatant was aspirated. The pellet was washed once with cold PBS to remove remaining Matrigel contaminants. The samples were subsequently fixed and stained as described above. All immunofluorescence images of 3D samples were acquired using a confocal microscopy (Olympus FV1000 inverted). ImageJ 1.48k software (Wayne Rasband, NIHR, USA, http://imagej.nih.gov/ij) was used for image processing. Changes in brightness or contrast during processing were applied equally across the entire image.

### REFERENCE EXAMPLE 1

### Derivation of MESP from human embryonic stem cells (hESC)

H1 human embryonic stem cells were purchased from Wicell. The H1 hESC was used to generate MESP. H1 hESC were culture in 6 well dishes (Falcon) using mTeSR1 media (STEMCELL Technologies). 2mls of media was provided for each 6 well and media was refreshed daily. hESC were routinely passage every 5-7 days upon confluency. Briefly, 6 well dish was thinly coated with 30X diluted matrigel (200µl per well) and incubator for 1hr before use. To passage the cells, the media is aspirated and cells were washed once with 1.5 to 2ml of 1X PBS (Gibco). After aspirating the 1X PBS, 0.5-1ml of 1X Dispase (Gibco) was added to each well of the hESC and cells were incubated for 5-7mins at 37°C. The dispase was removed and cells were washed once with 2ml of 1X PBS and 1 ml of mTeSR1 media was added to the well. The cells were lifted from the plate with a cell scraper and the hESC colonies were dissociated into cell clumps of 50-100 cells and seeded at a ratio of 1:12 into well pre-coated with matrigel.

To seed hESC for generating MESP (Figure 1), cells were instead dissociated with TryPLE (Gibco). 500µl of TryPLE was added to each well and cells were incubated for 1min at 37°C. The cells were dissociated to single cells and seeded in similar matrigel pre-coated dishes at 80-90% confluency. To differentiate hESC to Definitive endoderm cells, the hESCs were culture in RPMI (Gibco) containing 1x B27 (Gibco), 1x penicillin/streptomycin (Gibco), 100ng/ml Activin-A (R&D), 20ng/ml BMP4 (R&D) and 10ng/ml bFGF (R&D) for 2 days before being cultured in RPMI + B27 media containing 100ng/ml Activin-A for 4 days. To differentiate DE to primitive GUT cells, cells were culture with RPMI (Gibco) containing 1x B27 (Gibco), 1x penicillin/streptomycin (Gibco), 20ng/ml BMP4 (R&D) and 10ng/ml bFGF (R&D) for 2 days. All cells were incubated at 37°C with 5% CO₂.

The differentiation process can be monitored with qPCR of marker genes expression. During differentiation, levels of the pluripotent stem cell markers OCT4 and NANOG would start to decrease in the DE and GUT (Figure 2), indicating the exit of pluripotency. Commitment to the endoderm lineage is supported by the upregulation of the Definitive endoderm (DE) markers SOX17, CER1, HHEX1, CXCR4 and FOXA2 (Loh., et al 2014). During the differentiation of DE to GUT, some of these early endoderm markers SOX17, CER1, HHEX1 and CXCR4 begin to decrease whereas GUT markers HNF4A, FOXA2 and HNF1B are up-regulated (Figure 2).

To differentiate GUT into MESP (Figure 1), the cells were seeded on Matrigel (BD Biosciences) containing B27 supplement (Invitrogen). MESP were cultured in Advanced Dulbecco's modified Eagle's medium (DMEM)/F12 (Gibco) containing N2 supplement (Gibco), B27 supplement (Gibco), penicillin/streptomycin (Gibco), A83-01 (Stemgent), Dexamethasone (Stemgent), ChIR99021 (Tocris), Valproic Acid (VPA) (Stemgent), human HGF (R&D), human EGF (R&D), Jagged-1 (Anaspec), N6,2'-O-Dibutyryladenosine 3',5'-cyclic monophosphate sodium salt (dbCAMP) (Sigma-Aldrich), Nicotinamide (Sigma-Aldrich). Cells were seeded at a density of 8,000-10,000 cells/cm2 and media was changed every 2 days. Spheroids would appear from single cells seeded after 14-16 days (Figure 3).

### EXAMPLE 2

### Long term stable culture of MESP

The MESP were seeded on Matrigel (BD Biosciences) containing B27 supplement (Invitrogen). MESP were cultured in Advanced Dulbecco's modified Eagle's medium (DMEM)/F12 (Gibco) containing N2 supplement (Gibco), B27 supplement (Gibco), penicillin/streptomycin (Gibco), A83-01 (Stemgent), Dexamethasone (Stemgent), ChIR99021 (Tocris), Valproic Acid (VPA) (Stemgent), human HGF (R&D), human EGF (R&D), Jagged-1 (Anaspec), N6,2'-O-Dibutyryladenosine 3',5'-cyclic monophosphate sodium salt (dbCAMP) (Sigma-Aldrich), Nicotinamide (Sigma-Aldrich). MESP were passaged every 14-16 days using Dispase and TryLE. Matrigel was mechanically dissociated, transferred to an eppendorff tube and kept on ice to allow a combination of dispase and low temperature to liquefy the Matrigel. After 5mins, the samples were centrifuged at 1,000 r.p.m for 5mins and the supernatant was aspirated. Spheroids were then incubated in TryLE at 37°C for 5mins before being dissociated into single cells. Cells were centrifuged at 1,000 r.p.m for 5mins. Cells were washed once with MESP media and then resuspended in MESP media. Cells were seeded at a density of 12,000 cells/cm2. Media was changed every 2 days.

To identify the developmental identity of the MESP along the endoderm lineage, markers targeting different regions of the GUT tube were selected and detected via gene expression analysis with qPCR (Figure 4). MESP do not express key marker genes found in the anterior foregut, midgut and hindgut but marker genes found in the posterior foregut such as PDX1, CDX2 and HNF4A. These makers are also present at the protein level from the cell staining results using antibodies specific to the protein of each marker (Figure 5). MESP also expresses stem cell markers CK19 and SOX9 (Figure 5 and 10) commonly expressed in different fetal and adult progenitor and stem cells. This stem cell characteristic of MESP is reflected in the stable proliferation capacity of the cells (Figure 7A).

This proliferation capacity of MESP is valuable for the large scale production of cells which is required for regenerative therapy, and in proteomics and genomics studies. To scale up the production of MESP, the cells are seeded in larger vessels of 24 well and 12 well dishes. MESP were seeded at similar density of 12,000 cells/cm2 and the volume of matrigel used was increase proportionally to the volume of the culture chamber. The media was similarly refreshed every 2 days. By increasing the size of the chamber, the number of MESP retrieved also proportionally increased (Figure 6). Over a million cells can be generated from a small surface area of 3.7cm², demonstrating the scalability of the system to produce large number of MESP for various downstream applications.

To further characterize the unique stem cell state of MESP, the transcriptome is profiled using whole genome microarrays. Briefly, the total RNA from MESP, hESCs, DE and GUT cells were extracted using Trizol reagent, DNASE treated and purified using Purelink RNA kit, using similar approach for gene expression analysis with qPCR. For the microarray, 500ng DNase-treated total RNA was amplified into biotin labeled cRNA with Illumina Total Prep RNA Amplification Kit (Ambion) according to manufacturer's protocol. Subsequently, 750ng of cRNA was hybridized, washed and labelled with Cy3-streptavidin onto the array (Illumina HumanHT-12_v4_BeadChips) according to manufacturer's Protocol. Reading of the hybridized chips was done using Illumina HiScan Platform. The data was processed using Genome Studio (Illumina). Samples were subjected to Quantile Normalization. The normalized data was exported into GeneSpring format and to Microsoft Excel for subsequent analysis. Briefly, data was filtered to remove probes which do not have any signal. Generation of the Principal component analysis plots, statistical analysis to generate genes was done with GeneSpring (GeneSpring). Generation of heatmaps and clustering was done using Gene-E (Broard Institute). The cluster analysis shows that the transcriptome of MESP is highly different from hESCs, DE and GUT cells. MESP expresses a unique expression signature as a stem cell state. Many of the early endoderm specific markers such as SOX2, CER1, GATA4, SOX17, CXCR4, FOXA2 and CD34 are not expressed in MESP (Figure 9). MESP expresses a list of unique genes expressed in late endoderm progenitors and stem cells isolated from fetal or adult liver, pancreas and small intestine (Figure 10). Expression of these markers supports the MESP is a late endoderm stem cells that is distinct from other early endoderm stem cells reported (Cheng., et al 2012; Hannan., et al 2013).

### EXAMPLE 3

### Derivation of MESP from induced pluripotent stem cells (iPSCs)

Pluripotent stem cells encompass both embryonic stem cells and induced pluripotent stem cells. iPSCs is generated by the nobel winning method by Takahashi and Yamanaka (Takahashi and Yamanaka, 2006) where terminally differentiated somatic cells are converted back into a pluripotent cells state. The iPSC technology has vast application potentials and one of the key breakthroughs includes the modeling of genetic diseases. The disease patient somatic cells such as blood or skin fibroblast can be reverted back to a pluripotent cell state. This disease patient pluripotent stem cell can be used to generate the cell type of interest which harbors the disease phenotype. Thus, this technology potentially allows the modeling of any genetic disease in a dish. It is thus important to show that iPSC can similarly be used to generate MESP for modeling diseases.

iPSCs are generated and characterized as previously described (Chia., et al, nature 2010). Briefly, human MRC5 fibroblast (ATCC) culture in DMEM (Gibco) supplement with 15% fetal bovine serum (Hyclone) were infected with retroviruses harboring the overexpression cassettes for genes OCT4, SOX2, KLF4, CMYC and PRDM14. After 3-5 days infection, the fibroblast was plated on the Mitomycin C inactivated CF-1 feeders. After 24-48hrs post infection, the cells were culture in DMEM/F12 containing 20% Knockout serum replacement (Gibco), 1 mM L-glutamine, 1% non-essential amino acids, 0.1 mM 2-mercaptoethanol and supplemented with 4-8 ng/ml basic fibroblast growth factor (Invitrogen). The media was refreshed every 2 days. Human iPSC colonies will appear and ready to manually picked 3 weeks post seeding. The pluripotent cell state of the iPSC clones picked was validated by gene expression of pluripotent stem cell markers and ability to form teratomas in the SCID mice (Chia., et al, nature 2010).

iPSCs were differentiated to MESP using the same protocol described above for derivation from hESC. The MESP derived from iPSC is morphologically similar to those derived from hESC and expresses similar key MESP markers HNF4A, CDX2, PDX1, CK19 and SOX9 (Figure 13). Hence, the technology described herein can be applied to both hESC and iPSCs. This supports that MESP coupled with iPSC technology would be useful for modeling diseases in a dish.

### EXAMPLE 4

### Generation of LDLR knockout MESP from genome edited hESCs

A dual expression vector (PX330-2AmCherry) encoding for SpCas9 linked to a mCherry reporter cassette via a T2A peptide, and a single guided RNA (sgRNA), was utilized for the generation of LDLR knockout hESC lines. sgRNAs were designed to target the first protein coding exons for both genes respectively, with additional nucleotide sequences appended to the 5' and 3' ends of the oligos intended for BbsI cloning. All sgRNA cloned PX330 vectors were validated via Sanger Sequencing using the following U6 promoter primer sequence (5'-GAGGGCCTATTTCCCATGAT-3'; SEQ ID NO:94), amplify using Stbl3 cells and purified using FavorPrep^{™} Plasmid DNA Extraction Mini Kit (FAVORGEN Biotech Corp). Nucleofection of hESC was performed using the P3 Primary Cell 4D-Nucleofector^{®} X Kit L (Lonza, #V4XP-3012) following manufacturer's protocol. Briefly, hESCs were grown to 80% confluency in a well of a 6 well dish and harvested as single cells with TrypLE^{™} (ThermoFisher Scientific). A tube containing a total of 1 X 10⁶ single cell hESCs was resuspended into 50µl of the P30 nucleofector solution and mixed with another 50µl of the P30 nucleofector solution containing 5µg of plasmid. The final hESC and DNA mixture was transferred into a Nucleocuvette^{™} and nucleofected with a 4D-Nucleofector^{™} System using the CM-113 experimental parameter setting. Following nucleofection, hESCs was transferred to a matrigel coated well of a 6 well plate and recovered in mTESR containing 0. 5 µM Rock Inhibitor Thiazovivin (STEMGENT). After 48h, mCherry positive hESCs were sorted using FACS and cells were plated as single cells in a 10 cm dish and cultured in mTESR containing Rock Inhibitor for 5-7 days. Upon confluency, single colonies were then picked and expanded individually in mTESR. Each clonal line was later split at a ratio of 1:2, with half of each expanded clonal line retained for maintenance and another half lysed in QuickExtract solution for genomic DNA extraction. gDNA of each clonal line was subsequently used as a PCR template together with specific PCR primers designed to amplify sgRNA targeted regions of approximately 200bp in size. To identify clones with potential frameshift mutations, PCR products from clones and WT hESC were analysed via gel electrophoresis (3% Agarose; 150V for 5h) and distinct shifts in band size (arrows) were observed in clones with successful gene targeting (Figure 15A). Targeting efficacy with hESC for LDLR locus was 16.7% (Figure 15A). Selected products were purified and sequenced for further analysis of insertion or deletion mutations at the gene loci targeted. Figure 15B shows an example of a sequencing result where 2 copies of the LDLR gene has a 31bp deletion (highlighted). This deletion would result in a frameshift mutation in the LDLR transcript which affects its proper translation. A non-functional truncated LDLR would result in a loss of function mutation. This LDLR KO hESC can be used to generate LDLR KO MESP.

The LDLR KO hESCs is subjected to similar MESP differentiation protocol (Figure 1) as the wildtype hESC described above. LDLR KO MESP expresses all the key markers of HNF4A, CDX2, PDX1, CK19 and SOX9. Results show that MESP system can be used to generate genetic disease models for downstream studies.

### EXAMPLE 5

### Generation of intestinal organoids from MESP

MESP gene expression profile suggests that this stem cell closely resembles the posterior foregut which has the developmental potential to generate the liver pancreas and intestine. We tested if MESP is able to generated intestinal organoids adopting and modifying differentiation strategies reported by others generating the organoids from hESC (Spence., et al 2011). MESP were cultured in in a media comprised of Advanced Dulbecco's modified Eagle's medium (DMEM)/F12 (Gibco) containing N2 supplement (Gibco), B27 supplement (Gibco), 1x penicillin/streptomycin (Gibco), A83-01 (Stemgent), SB202190 (Tocris), human EGF (R&D), Nicotinamide (Sigma-Aldrich), Noggin (R&D), Wnt3A (R&D), R-spondin1 (R&D), N-acetyl cysteine (Sigma) and FGF4 (R&D) for 8 days to induce specification towards the intestinal lineage. After which, spheroids were removed from matri-gel using Dispase and dissociated into single cells using TryLE. Briefly Matrigel was mechanically dissociated, transferred to an eppendorff tube and kept on ice to allow a combination of dispase and low temperature to liquefy the Matrigel. After 5mins, the samples were centrifuged at 1,000 r.p.m for 5mins and the supernatant was aspirated. Spheroids were then incubated in TryLE at 37°C for 5mins before being dissociated into single cells. Cells were centrifuged at 1,000 r.p.m for 5mins. Cells were washed once with I2 media which comprises of Advanced Dulbecco's modified Eagle's medium (DMEM)/F12 (Gibco) containing N2 supplement (Gibco), B27 supplement (Gibco), 1x penicillin/streptomycin (Gibco), A83-01 (Stemgent), SB202190 (Tocris), human EGF (R&D), Nicotinamide (Sigma-Aldrich), Noggin (R&D), Wnt3A (R&D), R-spondin1 (R&D), N-acetyl cysteine (Sigma) and FGF4 (R&D) Cells were subsequently seeded at a density of 5,000 cells/per well (96 well) and cultured in I2 as suspension culture for 30 days in a 96 well ultra-low attachment plate to derive intestinal organoids.

After 3 weeks of differentiation, coiled-coil structures resembling the small intestine can be observed in dish (Figure 11). The intestinal-like structures are embedded in a mesh of fibroblast like tissue which resembles mesenchyme tissues. These organoids forms similar structure that are morphological similar to the previously reported intestinal organoids (Spence., et al 2011). Indeed, these MESP generated intestinal organoids that expressed key intestinal markers Villin and CDX2. The cells self-organized and envelope a lumen similar to that of the small intestine. The cells are polarized as evident from the asymmetrical distribution of Villin. The results reflect the developmental potential of MESP to form liver, pancreas and intestinal tissue.

### EXAMPLE 6

### Generation of liver organoids from MESPs

The posterior foregut forms the liver organ in the human body. Thus, MESP spheroid has the potential to generate liver organoids. Herein we developed a stepwise induction protocol to the generate human liver organoids from MESPs (Figure 17). Briefly, MESP were cultured in Advanced Dulbecco's modified Eagle's medium (DMEM)/F12 (Gibco) containing N2 supplement (Gibco), B27 supplement (Gibco), 1x penicillin/streptomycin (Gibco), A83-01 (Stemgent), ChIR99021 (Tocris), human HGF (R&D), human EGF (R&D), Jagged-1 (Anaspec), N⁶,2'-O-Dibutyryladenosine 3',5'-cyclic monophosphate sodium salt (dbCAMP) (Sigma-Aldrich), Nicotinamide (Sigma-Aldrich), BMP4, BMP7 (R&D) and FGF7/KGF (R&D) for 8 days to first induce specification towards the hepatic lineage. At this stage, the MESP starts to express Alpha-fetal protein (AFP) (Figure 18, top panel) which is not expressed in MESP (Figure 5, 13 and 15). This marks the commitment of the spheroids into the hepatic lineage as early hepatic progenitor.

The early hepatic progenitors were subsequently removed from matri-gel using Dispase and dissociated into single cells using TryLE. Briefly, Matrigel was mechanically dissociated, transferred to an eppendorff tube and kept on ice to allow a combination of dispase and low temperature to liquefy the Matrigel. After 5mins, the samples were centrifuged at 1,000 r.p.m for 5mins and the supernatant was aspirated. The early hepatic progenitors were then incubated in TryLE at 37°C for 5mins before being dissociated into single cells. Cells were centrifuged at 1,000 r.p.m for 5mins. Cells were washed once with H2 media and then resuspended in H2 media. Cells were seeded at a density of 5,000 cells/per well (96 well) and cultured in Advanced Dulbecco's modified Eagle's medium (DMEM)/F12 (Gibco) containing N2 supplement (Gibco), B27 supplement (Gibco), 1x penicillin/streptomycin (Gibco), A83-01 (Stemgent), ChIR99021 (Tocris), human HGF (R&D), human EGF (R&D), , N⁶,2'-O-Dibutyryladenosine 3',5'-cyclic monophosphate sodium salt (dbCAMP) (Sigma-Aldrich), (Sigma-Aldrich), BMP7 (R&D) and FGF7/KGF (R&D) as suspension culture for 15 days in a 96 well ultra-low attachment plate to derive late hepatic progenitors (Figure 17). In addition to AFP, the late hepatic progenitors start to express Albumin (ALB) which is a definitive marker for liver cells.

To derived hepatic organoids, hepatic progenitors were cultured in Clonetics^{™} HCM^{™} Hepatocyte Culture Medium (Lonza) containing A83-01, Dexamethasone, Compound-E (EMD Millipore), HGF, BMP7, FGF19, Oncostatin-M (R&D) for at least 3 weeks (Figure 17). During differentiation, the spheroids become more and more compact in size and the center of the spheroid becomes dense and more opaque. After 3 weeks of differentiation, the organoid will consist of a dense core of cells and cyst structures begin to form at the periphery. After formation, the organoids is stable in culture for more than 2 weeks.

### EXAMPLE 7

### Multiple cell types exist in liver organoids

The liver is largely consisting of hepatocyte which is the major metabolic cell type of the organ. Other than the hepatocytes, the other liver parenchyma cell type is the cholangiocytes. The cholangiocytes form the bile ducts in the liver that export the bile secretions from the hepatocytes out of the liver and into the small intestine. To investigate if the liver organoids contain both parenchyma cell type of the liver, the organoids are co-stained with antibodies specific for ALB (specifically expressed in hepatocytes) and cytokeratin 7 (CK7) (specifically expressed in cholangiocytes). From the staining results (Figure 20), CK7 positive cells are only found in the cyst structures and not in the core of liver organoids and ALB positive cells are only found in the core of the organoids and not in the cyst. Results show that both cholangiocytes and hepatocytes are present in the organoids derived from MESP. The existence of both parenchyme cell types in the liver organoid is also validated by the gene expression analysis of markers genes expressed in both hepatocytes and cholangiocytes. The liver organoids expresses many of the hepatocyte enriched markers such as ALB, CEBP, FOXA2, HNF1B, HNF4A, HNF6, KRT18, KRT8 and NOTCH2 compared to the MESP. Cholangiocyte enriched markers such as CFTR, KRT7, KRT19 and SOX9 are also much highly expressed in the liver organoids compared to MESP (Figure 22). Staining with CFTR specific antibodies also shows that the cyst structures are made up of the cholangiocytes (Figure 25).

Not only are both parenchyma liver cells found in the liver organoids, the cells are arranged in a specific manner that mimics the *in vivo* liver tissue (Figure 21). In the liver lobule (basic unit that makes the entire liver) the hepatocytes are compacted circularly around the central vein. The cholangiocytes forms the bile ducts that are located in the periphery of the lobule (Figure 21A). Similarly in the MESP derived liver organoids, the hepatocytes are densely packed in the core and the cholangiocytes forms cyst structures that mimic the bile ducts (Figure 21B). The results supports that the liver organoids contains both the major parenchyma cell type of the liver organ and the cells are organized similarly to the cells found in the liver tissues.

### EXAMPLE 8

### Liver organoids exhibit multiple liver specific functions

To determine if the liver organoids are functional, the expression of various metabolic enzymes and transported were detect by qPCR. The liver organoids expresses most of the cytochrome P450 enzymes (CYPs) including CYP3A4, CYP3A7, CYP1A1, CYP2D6, CYP2B6, CYP2C19 and CYP2E1 compared to MESP. In addition, the liver organoids also expresses major UDP-glucuronosyltransferase enzymes UGT1A1, UGT2B15 and UGT2B7 compared to MESP. These enzymes are important for the different phase of detoxification functions of the liver. The CYPs essentially metabolized almost 75% of the drugs in the human body. In the first phase of detoxification in the liver, these enzymes introduce reactive subgroups to the substrates to increase water solubility of the molecules for removal. In the second phase, the UDP-glucuronosyltransferase enzymes conjugate these reactive metabolites from the CYP enzymes with charge groups such as glucuronic acid to increase the mass of this substrate and reducing its reactivity. In the last phase of detoxification, bile transporter such as NTCP and OATP1B3 expressed in the organoids actively transport the detoxified products out of the hepatocytes into the bile canaliculi and towards the bile duct for removal. Cell staining with another important liver transport MRP2 shows that liver organoids expresses the essential transport essential for liver detoxification functions. The expression analysis suggests that the organoids expresses most detoxification enzymes require for all phases of detoxification processes in the liver.

To further validate the enzymatic activity of the CYPs expressed in the organoids, the P450-Glo^{™} CYP450 Assays (Promega) was used to assay for the activity of various CYPs. Specific assay kits for the each CYP enzyme were used according to manufacturer's protocol. Independent organoids were used for each assay kits specific for detecting CYP3A4, CYP2D6, CYP2B6 and CYP1A2. The total luciferase reading taken from the luminometer is normalized to the total cell number in the organoids. The enzymatic assays showed that the organoids have highly active CYPs enzymatic activity compared HepG2 cell lines commonly employed in the industry for liver studies.

The organoids are also assay for specific liver functions such as albumin secretion. The media from individual organoids are collected after 24hrs. The amount of albumin in the media was detected using ELISA with a human albumin specific antibody and a spectrophotometer. The exact amount of albumin was determined using a standard control consisting of different concentration of recombinant albumin. The readings from the recombinant albumin of various concentrations generate a standard curve. The standard curve is used to extrapolate the amount of albumin in the media based on its readings in the ELISA. The results shows that liver organoids secretes 30-60ng/ml/day of albumin compared to media control.

Another major function of the liver is the storage of excess glucose as glycogen in the hepatocytes. Glycogen serves as an important form of energy storage and dysregulation of this process in the liver lead to diseases such as diabetes. The liver organoids were stained with Periodic acid-Schiff (PAS) which detects polysaccharides such as glycogen. The PAS staining shows that the hepatocytes (stained purpled, arrows) in the organoids are capable of the storing glycogen (Figure 27A). The hepatocytes in the liver not only regulates glucose homeostasis, the cells also actively uptakes lipid to modulate lipid homeostasis in the human body. To assay for the lipid uptake functions of the liver organoids, the organoids are treated with fluorescent tagged Low density lipoprotein. These LDL can be image and visualized in the organs. The results show that the hepatocytes in the liver organoids are capable of taking up LDL (Figure 27B).

### EXAMPLE 9

### Liver organoids contain functional bile canaliculi network

In the liver lobule, the hepatocytes and cholangiocytes are connected by a channel known as the bile canaliculi (Figure 30). This canal functions to transport bile secretions from the hepatocytes containing important bile salts for fat digestion and absorption, cholesterol and metabolized exogenous lipophilic substances from removal. This channel is the important functional connection canal between the hepatocytes and cholangiocytes. Hepatocytes actively pump solutes into the bile canaliculi which are subsequently transported to the bile ducts form by the cholangiocytes (arrows direction of hepatocyte secretion and transport to bile canaliculi) (Figure 30). The hepatocytes of the liver organoids express the key bile canaliculi marker DPP IV which marks the surface of the hepatocytes in contact with the bile canaliculi (Figure 35). To image the bile canaliculi, the organoids are treated with the molecule 5-(and-6)-carboxy-2',7'-dichlorofluorescein diacetate (CDFDA) (Figure 31). CDFDA is a large inert molecule that can be passively uptake by the hepatocytes and other cells (Zamek-Gliszczynski et al., 2003). The CDFDA is converted in the hepatocytes to 5-(and-6)-carboxy-2',7'-dichlorofluorescein (CDF) by esterases present in the hepatocytes but not the cholangiocytes. Compared to CDFDA, CDF is a fluorophore which can be detected by imaging techniques. The CDF generated in the hepatocytes are actively pumped out by the hepatocytes through MRP2 transporter into the bile canaliculi. The bile canaliculi subsequently transport the CDF to the bile ducts form by the cholangiocytes.

To detect the functional bile canaliculi network in the liver organoids, live organoids were treated with CDFDA. Briefly, media was removed and the organoids were first washed three times with PBS containing calcium and magnesium. Next, the organoids were incubated with 5µg/ml CDFDA (Molecular Probes) and 1µg/ml Hoechst 33342 for stipulated time at 37°C. Organoids were subsequently washed three times and imaging was performed on a confocal microscopy (Olympus FV1000 inverted). ImageJ 1.48k software (Wayne Rasband, NIHR, USA, http://imagej.nih.gov/ij) was used for image processing. Changes in brightness or contrast during processing were applied equally across the entire image.

Within 30 minutes of treatment with CDFDA, the hepatocytes in the organoids accumulated CDF (Figure 32B) compared to the control undifferentiated MESP (Figure 32A). Results show that the hepatocytes actively generate CDF from the CDFDA in the media. After prolong treatment of the organoids for up to 3hrs, the level of CDF is reduced in the hepatocytes and can be clearly seen in the outline of the cells (Figure 33). The CDF is transported out of the hepatocytes and located in the network of bile canaliculi formed in the organoids. A 3D reconstructed image of the confocal sections from the liver imaging of the organoids shows that the bile canaliculi network are highly connected and forms an intricate web of channels between the hepatocytes. To further show that the secretions from the hepatocytes are functionally transported to the cholangiocytes, organoids with large bile duct-like cyst structures were treated with CDFDA (Figure 36). Similarly after 30 mins of the CDFDA treatment, the hepatocytes starts to accumulate CDF in the cytoplasm and CDF is not detected in the cholangiocytes (arrow) forming the cyst structures (Figure 36A). After 1hr of CDFDA treatment, again the network of bile canaliculi can be observed in the core of the hepatocytes (Figure 36B). Remarkably, the cyst structures (arrows) are also filled up with CDF (Figure 36B). This CDF is likely to be generated and secreted from the hepatocytes and transport through the bile canaliculi to the cyst as the cholangiocytes do not generated CDF. A close up view of the organoids shows that the CDF filled network of bile canaliculi indeed connects to the cyst structures (Figure 36C). The CDFDA treatment and imaging assay shows that the hepatocytes and cholangiocytes in the organoids indeed form the important bile canaliculi network that connects the 2 cell types. This important functional structure of the liver organoids will be invaluable for the study of bile secretion processes in vitro. Such studies have only been conducted in mouse and rat livers as no bile canaliculi structures have been reported in vitro. This drastically slows down efforts to identify factors that disrupt this important process. Disruption of the bile secretion process results in cholestasis which accounts for many cases of drug induced liver injury (DILI) (Kaplowitz, 2004). DILI accounts for almost half of the cases of liver failure. Drugs inducing cholestasis have been difficult to assay due to the lack of *in vitro* models to allow real time monitoring of the event, which is highly challenging in animal models. Hence, the liver organoids described herein would be highly valuable for assaying drugs potentially induces cholestasis.

### EXAMPLE 10

### Modeling disease with liver organoids

The successful generation of the genetically modified MESP (example 4) highlighted the potential of modeling diseases of organs that can be generated from MESP. Herein, the LDLR KO MESP was used to generate liver organoids using similar approach with the wild type MESP (example 6). In patient deficient of the LDLR, the liver secretes high levels of the cholesterols in the human body, resulting in hypercholesterolemia. The elevated in levels of cholesterol in the blood stream results in cardiovascular diseases and patients undergo statin treatment to control blood cholesterol levels. To investigate if the LDLR deficient liver organoids mimics the liver organ of a hypercholesterolemia patient deficient in LDLR, we assay for the level of cholesterol secreted by the liver organoids. The media incubated with organoids after 24 hours are collected and the amount of cholesterol in the media is determine using the Amplex^{®} Red Cholesterol Assay Kit (Thermo fisher scientific) according to the manufacturer's protocol. Briefly, media from each organoids is incubated with the reagents provided in the kit and incubated for 30mins at 37°C. The fluorescence generated is detected and quantify using a fluorescence plate reader. The total amount of cholesterol for 1ml of the media is tabulated. To test the response of the LDLR deficient organoids to statin treatments, the organoids are incubated with the different concentrations of Pravastatin (Sigma) added into the media. The LDLR deficient liver organoids secreted higher levels of cholesterol compared to the LDLR expressing liver organoids (Figure 37). Importantly, the elevated level of cholesterol secreted was suppressed by increasing concentrations of statin treatment. The results supports that the genetically modified organoids can be used to model liver disease in vitro.

### EXAMPLE 11

### High throughput generation of liver organoids

To generate organoids in large numbers, the protocol was adapted to generate organoids of comparable size and function in a 96 well dish. Briefly, MESP were cultured in Advanced Dulbecco's modified Eagle's medium (DMEM)/F12 (Gibco) containing N2 supplement (Gibco), B27 supplement (Gibco), 1x penicillin/streptomycin (Gibco), 500nM A83-01 (Stemgent), 2µM ChIR99021 (Tocris), 20ng/ml human HGF (R&D), 50ng/ml human EGF (R&D), 1uM Jagged-1 (Anaspec), 300ng/ml N6,2'-O-Dibutyryladenosine 3',5'-cyclic monophosphate sodium salt (dbCAMP) (Sigma-Aldrich), 10mM Nicotinamide (Sigma-Aldrich), 20ng/ml BMP4, 20ng/ml BMP7 (R&D) and 25ng/ml FGF7/KGF (R&D) for 8 days to induce specification towards the hepatic lineage. After which, spheroids were removed from matri-gel, dissociated to single cells using TryPLE and seeded in a 96 well plate in Advanced Dulbecco's modified Eagle's medium (DMEM)/F12 (Gibco) containing N2 supplement (Gibco), B27 supplement (Gibco), 1x penicillin/streptomycin (Gibco), 2.5mM A83-01 (Stemgent), 2µM ChIR99021 (Tocris), 20ng/ml human HGF (R&D), 50ng/ml human EGF (R&D), 300ng/ml N6,2'-O-Dibutyryladenosine 3',5'-cyclic monophosphate sodium salt (dbCAMP) (Sigma-Aldrich), 10mM Nicotinamide (Sigma-Aldrich), 20ng/ml BMP4, 20ng/ml BMP7 (R&D) and 25ng/ml FGF7/KGF (R&D) as suspension culture for 2 weeks. The cells were subsequently cultured in Clonetics^{™} HCM^{™} Hepatocyte Culture Medium (Lonza) containing 500nM A83-01, 30µM Dexamethasone, 500nM Compound-E (EMD Millipore), 25ng/ml HGF, 25ng/ml BMP7, 25ng/ml FGF19, 20ng/ml Oncostatin-M (R&D) for another 3-4 weeks to derive hepatic organoids. One organoid was generated for each well of the 96 well format plates (Figure 39). The individual organoids are similar in size (400-500µM). The individual organoids are assay for functional CYP3A4 and CYP2B6 activity using the P450-Glo^{™} CYP450 Assays (Promega) described previously (example 8). Remarkably, each organoid exhibits very similar CYP3A4 and CYP2B6 activities. Hence, the methods provided herein are highly applicable for large scale industrial applications.

### EXAMPLE 12

### Generation of liver organoids from adult liver stem cells

The step wise generation of liver progenitors during the derivation of liver organoids from the MESP (example 6) suggests that the method could be applied to other stem cells of hepatic lineage. Correspondingly, the method was successfully adapted for deriving liver organoids from liver stem cells (PCT/SG2016/050270) derived from the adult liver tissue (Figure 40). Briefly, the adult liver stem cells were washed once with PBS and incubated with TryLE. After 5mins, TryLE is removed and cells were washed once with PBS. If the adult stem cells were grown on feeders, it is important to separate all feeders from adult liver stem cells. Samples were centrifuged at 1,000 r.p.m for 5mins and the supernatant was aspirated. The cells were washed once with H2 media consisting of Advanced Dulbecco's modified Eagle's medium (DMEM)/F12 (Gibco) containing N2 supplement (Gibco), B27 supplement (Gibco), 1x penicillin/streptomycin (Gibco), A83-01 (Stemgent), ChIR99021 (Tocris), human HGF (R&D), human EGF (R&D), N⁶,2'-O-Dibutyryladenosine 3',5'-cyclic monophosphate sodium salt (dbCAMP) (Sigma-Aldrich), BMP7 (R&D) and FGF7/KGF (R&D). The cells were seeded at a density of 20,000 to 40,000 cells/per well and cultured as suspension culture for 2 weeks in a 96 well ultra-low attachment plate to derive late ALB and CK19 expressing late hepatic progenitors (Figure 41). As the adult stem cells are already committed to the hepatic lineage unlike MESP, the cells did not have to undergo H1 media treatment.

The late hepatic progenitors were lastly cultured in Clonetics^{™} HCM^{™} Hepatocyte Culture Medium (Lonza) containing A83-01, Dexamethasone, Compound-E (EMD Millipore), HGF, BMP7, FGF19, Oncostatin-M (R&D) for another 3-4 weeks to derive liver organoids. The organoids were co-stained with antibodies specific for ALB and CK7 to check if both parenchyma liver cells (hepatocytes and cholangiocytes) are present in the organoids. The organoids derived from the adult liver stem cells similarly consist of ALB expressing hepatocytes and CK7 expressing cholangiocytes (Figure 42). Similarly, the cholangiocytes are found on the periphery of the organoids around the hepatocyte core. The hepatic organoids are imaged with lightsheet microscopy (Leica) to unravel the structure organization of the cholangiocytes in the periphery. The whole organoid 3D image captured by the lightsheet microscope (Figure 43) shows that the cholangiocytes at the surface of the organoids arranges to form ductal structures with a central lumen that is distinct from the cyst structures observed in the MESP derived organoids. This distinct ductal structure observed in the adult organoids may reflect the subtle differences between the developmental stages of the liver organoids; MESP from PSC generates liver organoids similar to the fetal liver and the adult liver stem cells generate liver organoids similar to the adult human liver. Nonetheless, both adult liver stem cell and MESP derived organoids have a similar structural organization as the liver lobule (describe in example 6). Gene expression analysis of the adult liver organoids using qPCR showed that the ALSC derived organoids also express hepatocyte and cholangiocyte enriched genes including metabolic enzymes and transporters (Figure 44). The adult liver organoids were assayed for CYP3A4 and CYP2C9 activity using the P450-Glo^{™} CYP450 Assays (Promega). The adult liver stem cell derived organoids not only exhibited strong CYP3A4 activity, but also has strong CYP2C9 enzymatic activity (Figure 45). CYP2C9 is a CYP expressed and highly active in adult liver tissue. This activity corresponds with the adult nature of the hepatic organoids generated from the adult liver stem cells. Using using live imaging with CDFDA (describe in example 6), the liver organoids are shown to similarly possess bile canaliculi network. The result supports that the protocol described herein is applicable to diverse endoderm stem cells or progenitor cells that exist during the development of posterior foregut to the liver stem cells. The MESP and liver adult stem cells represent distinct developmental stages in the human liver development. However, both stem cells can be used to generate similar liver organoids using the method describe herein.

### Reference EXAMPLE 13

### Generation of Pancreatic spheroids from MESP

PDX1 expression in MESP suggests that these spheroid stem cells similar to the posterior foregut have the ability to generate pancreatic tissues. Herein, we employed a step-wise protocol to differentiate the MESP into pancreatic spheroids (Figure 47). Briefly, MESP were cultured in P1 media consisting of Advanced Dulbecco's modified Eagle's medium (DMEM)/F12 (Gibco) containing N2 supplement (Gibco), B27 supplement (Gibco), 1x penicillin/streptomycin (Gibco), A83-01 (Stemgent), ChIR99021 (Tocris), human EGF (R&D), Nicotinamide (Sigma-Aldrich), Dexamethasone (Stemgent) and FGF7/KGF (R&D) for a week to induce specification towards the pancreatic lineage. The spheroids were subsequently retrieved from the matri-gel using Dispase and dissociated into single cells using TryLE. Briefly Matrigel was mechanically dissociated, transferred to an eppendorff tube and kept on ice to allow a combination of dispase and low temperature to liquefy the Matrigel. After 5mins, the samples were centrifuged at 1,000 r.p.m for 5mins and the supernatant was aspirated. Spheroids were then incubated in TryLE at 37°C for 5mins before dissociated into single cells. Cells were centrifuged at 1,000 r.p.m for 5mins. Cells were washed once with P2 media consisting of Advanced Dulbecco's modified Eagle's medium (DMEM)/F12 (Gibco) containing N2 supplement (Gibco), B27 supplement (Gibco), 1x penicillin/streptomycin (Gibco), KAAD-cyclopamine (Stemgent), DAPT (Stemgent), human Noggin(R&D), human EGF (R&D), FGF7/KGF (R&D) and incubated in P2 media. Cells were seeded at a density of 5,000 cells/per well in P2 media as suspension culture for 2 weeks in a 96 well ultra-low attachment plate. Lastly, the pancreatic progenitors were cultured in P3 media consisting of Advanced Dulbecco's modified Eagle's medium (DMEM)/F12 (Gibco) containing N2 supplement (Gibco), B27 supplement (Gibco), 1x penicillin/streptomycin (Gibco), KAAD-cyclopamine (Stemgent), DAPT (Stemgent), human Noggin (R&D), human EGF (R&D), FGF7/KGF (R&D), ALKii (EMD Millipore) and T3 (Sigma) for 3-4 weeks to derive pancreatic spheroids (Figure 48). The pancreatic spheroids were stained with antibodies specific for PDX1 and NKX6.1 which are expressed in almost all pancreatic cell type. All the cells in the pancreatic spheroid stained positively for the 2 pancreatic markers, supporting that the spheroids are indeed of pancreatic cell fate. The successful generation of pancreatic spheroid supports the multipotent capacity of MESP.

### REFERENCES

Basma, H., Soto-Gutierrez, A., Yannam, G.R., Liu, L., Ito, R., Yamamoto, T., Ellis, E., Carson, S.D., Sato, S., Chen, Y., et al. (2009). Differentiation and transplantation of human embryonic stem cell-derived hepatocytes. Gastroenterology 136, 990-999.
Cheng, X., Ying, L., Lu, L., Galvao, A.M., Mills, J.A., Lin, H.C., Kotton, D.N., Shen, S.S., Nostro, M.C., Choi, J.K., et al. (2012). Self-renewing endodermal progenitor lines generated from human pluripotent stem cells. Cell Stem Cell 10, 371-384.
D'Amour, K.A., Bang, A.G., Eliazer, S., Kelly, O.G., Agulnick, A.D., Smart, N.G., Moorman, M.A., Kroon, E., Carpenter, M.K., and Baetge, E.E. (2006). Production of pancreatic hormone-expressing endocrine cells from human embryonic stem cells. Nat Biotechnol 24, 1392-1401.
Fisher, R.A., and Strom, S.C. (2006). Human hepatocyte transplantation: worldwide results. Transplantation 82, 441-449.
Gieseck, R.L., 3rd, Hannan, N.R., Bort, R., Hanley, N.A., Drake, R.A., Cameron, G.W., Wynn, T.A., and Vallier, L. (2014). Maturation of induced pluripotent stem cell derived hepatocytes by 3D-culture. PLoS One 9, e86372.
Grompe, M., and Strom, S. (2013). Mice with human livers. Gastroenterology 145, 1209-1214.
Hannan, N.R., Fordham, R.P., Syed, Y.A., Moignard, V., Berry, A., Bautista, R., Hanley, N.A., Jensen, K.B., and Vallier, L. (2013). Generation of multipotent foregut stem cells from human pluripotent stem cells. Stem Cell Reports 1, 293-306.
Hentze, H., Soong, P.L., Wang, S.T., Phillips, B.W., Putti, T.C., and Dunn, N.R. (2009). Teratoma formation by human embryonic stem cells: evaluation of essential parameters for future safety studies. Stem Cell Res 2, 198-210.
Huch, M., Gehart, H., van Boxtel, R., Hamer, K., Blokzijl, F., Verstegen, M.M., Ellis, E., van Wenum, M., Fuchs, S.A., de Ligt, J., et al. (2015). Long-term culture of genome-stable bipotent stem cells from adult human liver. Cell 160, 299-312.
Lancaster, M.A., and Knoblich, J.A. (2014). Organogenesis in a dish: modeling development and disease using organoid technologies. Science 345, 1247125.
Matano, M., Date, S., Shimokawa, M., Takano, A., Fujii, M., Ohta, Y., Watanabe, T., Kanai, T., and Sato, T. (2015). Modeling colorectal cancer using CRISPR-Cas9-mediated engineering of human intestinal organoids. Nat Med 21, 256-262.
Mitaka, T. (1998). The current status of primary hepatocyte culture. Int J Exp Pathol 79, 393-409.
Murry, C.E., and Keller, G. (2008). Differentiation of embryonic stem cells to clinically relevant populations: lessons from embryonic development. Cell 132, 661-680.
Seok, J., Warren, H.S., Cuenca, A.G., Mindrinos, M.N., Baker, H.V., Xu, W., Richards, D.R., McDonald-Smith, G.P., Gao, H., Hennessy, L., et al. (2013). Genomic responses in mouse models poorly mimic human inflammatory diseases. Proc Natl Acad Sci U S A 110, 3507-3512.
Shan, J., Schwartz, R.E., Ross, N.T., Logan, D.J., Thomas, D., Duncan, S.A., North, T.E., Goessling, W., Carpenter, A.E., and Bhatia, S.N. (2013). Identification of small molecules for human hepatocyte expansion and iPS differentiation. Nat Chem Biol 9, 514-520.
Si-Tayeb, K., Noto, F.K., Nagaoka, M., Li, J., Battle, M.A., Duris, C., North, P.E., Dalton, S., and Duncan, S.A. (2010a). Highly efficient generation of human hepatocyte-like cells from induced pluripotent stem cells. Hepatology 51, 297-305.
Spence, J.R., Mayhew, C.N., Rankin, S.A., Kuhar, M.F., Vallance, J.E., Tolle, K., Hoskins, E.E., Kalinichenko, V.V., Wells, S.I., Zorn, A.M., et al. (2011). Directed differentiation of human pluripotent stem cells into intestinal tissue in vitro. Nature 470, 105-109.
Takebe, T., Sekine, K., Enomura, M., Koike, H., Kimura, M., Ogaeri, T., Zhang, R.R., Ueno, Y., Zheng, Y.W., Koike, N., et al. (2013). Vascularized and functional human liver from an iPSC-derived organ bud transplant. Nature.
van de Wetering, M., Francies, H.E., Francis, J.M., Bounova, G., Iorio, F., Pronk, A., van Houdt, W., van Gorp, J., Taylor-Weiner, A., Kester, L., et al. (2015). Prospective derivation of a living organoid biobank of colorectal cancer patients. Cell 161, 933-945.
van der Worp, H.B., Howells, D.W., Sena, E.S., Porritt, M.J., Rewell, S., O'Collins, V., and Macleod, M.R. (2010). Can animal models of disease reliably inform human studies? PLoS Med 7, e1000245.
Vilarinho, S., and Lifton, R.P. (2012). Liver transplantation: from inception to clinical practice. Cell 150, 1096-1099.
Zanger UM, Schwab M. Cytochrome P450 enzymes in drug metabolism: regulation of gene expression, enzyme activities, and impact of genetic variation. Pharmacol Ther. 2013 Apr;138(1):103-41. doi: 10.1016/j.pharmthera.2012.12.007. Epub 2013 Jan 16. Review.
Boyer JL. Bile formation and secretion. Compr Physiol. 2013 Jul;3(3):1035-78. doi: 10.1002/cphy.c120027.
Huch M, Gehart H, van Boxtel R, Hamer K, Blokzijl F, Verstegen MM, Ellis E, van Wenum M, Fuchs SA, de Ligt J, van de Wetering M, Sasaki N, Boers SJ, Kemperman H, de Jonge J, Ijzermans JN, Nieuwenhuis EE, Hoekstra R, Strom S, Vries RR, van der Laan LJ, Cuppen E, Clevers H. Long-term culture of genome-stable bipotent stem cells from adult human liver. Cell. 2015 Jan 15;160(1-2):299-312. doi: 10.1016/j.cell.2014.11.050. Epub 2014 Dec 18.
Takebe T, Sekine K, Enomura M, Koike H, Kimura M, Ogaeri T, Zhang RR, Ueno Y, Zheng YW, Koike N, Aoyama S, Adachi Y, Taniguchi H. Vascularized and functional human liver from an iPSC-derived organ bud transplant. Nature. 2013 Jul 25;499(7459):481-4. doi: 10.1038/nature12271. Epub 2013 Jul 3.
Spence JR, Mayhew CN, Rankin SA, Kuhar MF, Vallance JE, Tolle K, Hoskins EE, Kalinichenko VV, Wells SI, Zorn AM, Shroyer NF, Wells JM. Directed differentiation of human pluripotent stem cells into intestinal tissue in vitro. Nature. 2011 Feb 3;470(7332):105-9. doi: 10.1038/nature09691. Epub 2010 Dec 12.
Lancaster MA, Renner M, Martin CA, Wenzel D, Bicknell LS, Hurles ME, Homfray T, Penninger JM, Jackson AP, Knoblich JA. Cerebral organoids model human brain development and microcephaly. Nature. 2013 Sep 19;501(7467):373-9. doi: 10.1038/nature12517. Epub 2013 Aug 28.
Takasato M, Er PX, Chiu HS, Maier B, Baillie GJ, Ferguson C, Parton RG, Wolvetang EJ, Roost MS, Chuva de Sousa Lopes SM, Little MH. Kidney organoids from human iPS cells contain multiple lineages and model human nephrogenesis. Nature. 2015 Oct 22;526(7574):564-8. doi: 10.1038/nature15695. Epub 2015 Oct 7.
Hawkins MT, Lewis JH. Latest advances in predicting DILI in human subjects: focus on biomarkers. Expert Opin Drug Metab Toxicol. 2012 Dec;8(12):1521-30. doi: 10.1517/17425255.2012.724060. Epub 2012 Sep 24.
Dan, Y.Y., Riehle, K.J., Lazaro, C., Teoh, N., Haque, J., Campbell, J.S., and Fausto, N. (2006). Isolation of multipotent progenitor cells from human fetal liver capable of differentiating into liver and mesenchymal lineages. Proc Natl Acad Sci U S A 103, 9912-9917.
Si-Tayeb, K., Lemaigre, F.P., and Duncan, S.A. (2010b). Organogenesis and development of the liver. Dev Cell 18, 175-189.
Schmelzer, E., Zhang, L., Bruce, A., Wauthier, E., Ludlow, J., Yao, H.L., Moss, N., Melhem, A., McClelland, R., Turner, W., et al. (2007). Human hepatic stem cells from fetal and postnatal donors. J Exp Med 204, 1973-1987.
Schwartz, R.E., Fleming, H.E., Khetani, S.R., and Bhatia, S.N. (2014). Pluripotent stem cell-derived hepatocyte-like cells. Biotechnol Adv 32, 504-513.
Rezania, A., Bruin,J.E., Xu, J., Narayan,K., Fox, J.K., O'Neil, J.J., and Kieffer, T.J. (2013). Enrichment of human embryonic stem cell-derived NKX6.1-expressing pancreatic progenitor cells accelerates the maturation of insulin-secreting cells in vivo. Stem Cells 31, 2432-2442.
Burlison, J.S., Long, Q., Fujitani, Y., Wright, C.V., and Magnuson, M.A. (2008). Pdx-1 and Ptfla concurrently determine fate specifi-cation of pancreatic multipotent progenitor cells. Dev. Biol. 316,74-86.
Nostro MC, Sarangi F, Yang C, Holland A, Elefanty AG, Stanley EG, Greiner DL, Keller G. Efficient generation of NKX6-1+ pancreatic progenitors from multiple human pluripotent stem cell lines. Stem Cell Reports. 2015 Apr 14;4(4):591-604. doi: 10.1016/j.stemcr.2015.02.017. Epub 2015 Apr 2.
Dye BR, Hill DR, Ferguson MA, Tsai YH, Nagy MS, Dyal R, Wells JM, Mayhew CN, Nattiv R, Klein OD, White ES, Deutsch GH, Spence JR. In vitro generation of human pluripotent stem cell derived lung organoids. Elife. 2015 Mar 24;4. doi: 10.7554/eLife.05098.
Mustata RC, Vasile G, Fernandez-Vallone V, Strollo S, Lefort A, Libert F, Monteyne D, Pérez-Morga D, Vassart G, Garcia MI. Identification of Lgr5-independent spheroid-generating progenitors of the mouse fetal intestinal epithelium. Cell Rep. 2013 Oct 31;5(2):421-32. doi: 10.1016/j.celrep.2013.09.005. Epub 2013 Oct 17.
Loh, K.M., Ang, L.T., Zhang, J., Kumar, V., Ang, J., Auyeong, J.Q., Lee, K.L., Choo, S.H., Lim, C.Y., Nichane, M., et al. (2014). Efficient endoderm induction from human pluripotent stem cells by logically directing signals controlling lineage bifurcations. Cell Stem Cell 14, 237-252.
Takahashi K and Yamanaka S. Induction of pluripotent stem cells from mouse embryonic and adult fibroblast cultures by defined factors. Cell. 2006 Aug 25;126(4):663-76. Epub 2006 Aug 10.
Chia NY, Chan YS, Feng B, Lu X, Orlov YL, Moreau D, Kumar P, Yang L, Jiang J, Lau MS, Huss M, Soh BS, Kraus P, Li P, Lufkin T, Lim B, Clarke ND, Bard F, Ng HH. (2010). A genome-wide RNAi screen reveals determinants of human ES cell identity. Nature 468(7321):316-20.
Michael Karin & Hans Clevers Reparative inflammation takes charge of tissue regeneration. Nature 529, 307-315
Zamek-Gliszczynski, M.J., Xiong, H., Patel, N.J., Turncliff, R.Z., Pollack, G.M., and Brouwer, K.L. (2003). Pharmacokinetics of 5 (and 6)-carboxy-2',7'-dichlorofluorescein and its diacetate promoiety in the liver. J Pharmacol Exp Ther 304, 801-809.
Neil Kaplowitz. Drug-Induced Liver Injury. Clinical Infectious Diseases 2004; 38(Suppl 2):S44-8

**Table 1: Characteristics of endoderm progenitor cells described herein.**

| | **MESP** |
|---|---|
| Culture system | 3D culture system (cells embedded in matrigel) |
| Similarity to in vivo developmental state | Posterior Foregut |
| Signaling pathways in culture media | • Inhibition of TGF-B |
| | • Activation of Wnt signalling |
| | • Activation of Notch signalling |
| | • |
| Endoderm Markers | • SOX17 (do not express) |
| | • CXCR4 (do not express) |
| | • FOXA2 (do not express) |
| | • SOX2 (do not express) |
| | • PDX1, |
| | • HNF4A, |
| | • CDX2, SOX9, KRT19, |
| In-vitro differentiation potential | • Liver organoids |
| | • Intestinal organoids |
| | • Pancreatic Spheriods |

**Table 2. Organoid comparison chart**

| | **Hepatic Organiods described herein** | **(PCT/IB11/02167)** | **Method for producing tissue and organ. Taniguchi et al** WO2013047639 A1**,** US 2014/0289877 A1 |
|---|---|---|---|
| Cell types | Differentiated cells | • Epithelial stem cells | Stem cells |
| | • Hepatocytes | | • Hepatic endoderm cells (early hepatic progenitor cells derived from iPSCs) |
| | • Cholangiocytes | | |
| | | | • Human Mesenchymal stem cells |
| | | | • Cell lines |
| | | | • HUVEC endothelial cells |
| Function | • Glucose storage: PAS staining positive | • No Liver functions displayed | • No Liver functions displayed in vitro |
| | | • Generate hepatocytes or Cholangiocytes after differentiation | • Only mature into functional liver tissue when transplanted into mice. |
| | • Lipid uptake (LDL uptake) function | | |
| | • Four Cyps function | | |
| | • Albumin secretion | | |
| | • Functional bile canaliculi network connecting hepatocytes and cholangiocytes | | |
| Structure | • Non random distribution of cells | • No reported liver tissue structure. | • No liver tissue structure observed in the liver bud formed by the aggregates of the 3 cell types. |
| | • Bile canaliculi structure within organoids. | • Random distribution of cells. | |
| | • Cholangiocyte form cyst structures with lumen | | |

**Table 3. Liver Organoid Culture Media**

| | **Hepatic organoid described herein (3 media)** | | |
|---|---|---|---|
| | Media H1 | Media H2 | Media H3 |
| Media | • HGF | • HGF | • A8301 |
| | • Nicotinami de | • Nicotinamide | • Compound E |
| | | • A83-01 | • Dexamethasone |
| | • A83-01 | • Dexamethaso ne | • Oncostatin M Optional (Differentiation) |
| | • CHIR9902 1 | | |
| | | • BMP7 | |
| | • Dexametha sone | • FGF19 Optional (survival) | • IL6 |
| | | | • BMP7 |
| | • BMP4 | | • FGF19 |
| | • BMP7 | • EGF | • dbcAMP |
| | • FGF7 | • dbCAMP | • Verteporfin |
| | Optional | • Jagged1 | • Taurocholate Optional (Survival) |
| | • EGF | • VPA | |
| | • dbcAMP | • CHIR99021 Optional (differentiation) | |
| | • VPA | | • EGF |
| | • Jagged1 | | • Heparin |
| | | • Compound E | • HGF |
| | | • Verteporfin | |
| Culture | Matrigel t | Suspension (no matrices) | Suspension (no matrices) |

**Table 4. Organoid culture method comparison with 3D printed liver tissues.**

| | **Hepatic Organoid described herein** | US 2014/0287960 A1 |
|---|---|---|
| Approach | • Organ-like tissue structures formed by the self organizing properties of stem cells giving rise to different cells types found in the organ. | • Bioprinted Liver tissue constructs generated by engineering methodology where different cell types are deposited with a bioink to localize cells to achieve specific spatial organization. |
| Cell composition | • Parenchymal and non parenchymal cells derived from similar source of primary stem cells. | • HepRG and HepG2 immortalized hepatocyte cell lines. |
| | | • Cell lines for other liver specific cell types. |
| Functions | • Glucose storage: PAS staining positive | |
| | • Lipid uptake (LDL uptake) function | |
| | • Four different CYP function | |
| | • Albumin secretion | |
| | • Functional bile canaliculi network transport | |
| Structure | • Self organizing and non-directed architecture. | • Directed Architecture |
| | • Architecture resembling liver tissue formed by cellular interactions between differentiated cells generated by the stem cells | • Architecture predetermined by the engineering methods. |
| | | • No evidence of bile canaliculi network or other structural features formed by 2 liver cell types. |
| | • Functional bile canaliculi network formed among hepatocytes | |
| | • Functional link between bile canaliculi network in hepatocytes to bile duct like structures form by the cholangiocytes | |

**Table 5. Liver organoid comparison with primary liver tissues.**

| | **Hepatic organoid described herein** | **Primary liver tissue** |
|---|---|---|
| Hepatocytes | • Smaller in size (Half the size of the hepatocytes in the primary liver) | • Larger in Size |
| | | • Double nucleus and Polyploid in chromosome numbers |
| | • Single nucleus and diploid in chromosome numbers | • Rapid loss of CYP function after 24hrs in culture. |
| | • CYP function maintained for weeks in culture. | |
| Cholangiocytes | • Forms large cyst in culture | • Forms long branching tubular structure |
| | • Non-proliferative | |
| | | • Proliferative |

**Table 6. Characteristics of Pancreatic Spheroids Described Herein.**

| | **Pancreatic spheroid** |
|---|---|
| Marker expression | PDX1+/NKX6.1+ Pancreatic Progenitors |
| Source | Human pluripotent stem cell derived |

**Table 7. Pancreatic Spheroid Media.**

| | Media P1 | Media P2 | Media P3 |
|---|---|---|---|
| Media | • EGF | • Retinoic Acid | • Retinoic Acid |
| | • Nicotinamide | • KAAD-cyclopamine | • ALKii |
| | • A83-01 | | • KAAD-cyclopamine |
| | • CHIR99021 | • FGF7 | • FGF7 |
| | • Dexamethasone | • DAPT | • DAPT |
| | • FGF7 Optional | • Noggin Optional | • Noggin |
| | | | • T3 Optional (Survival) |
| | • dbcAMP | • EGF | |
| | • VPA | • dbCAMP | • Heparin |
| | • Jagged1 | • Nicotinamide | • EGF |
| | • HGF | • Verteporfin | • Nicotinamide |
| Culture | Suspension (no matrices) | Suspension (no matrices) | Suspension (no matrices) |

### LIST OF ABBREVIATIONS

PSC: Pluripotent Stem Cells; ESC: embryonic stem cell; MESP: Multipotent Endodermal Spheroid Progenitors; ECM; Extracellular Matrix; CYP: Cytochrome p450 e.g. CYP3A4: Cytochrome P450, Family 3, Subfamily A, Polypeptide 4 ; LGR5: Leucine-rich repeat-containing G-protein coupled receptor 5; KRT: Cyto-keratin e.g KRT19: Cyto-keratin 19; AFP: Alpha-Fetoprotein; HNF: Hepatocyte Nuclear Factor e.g. HNF4a: Hepatocyte Nuclear Factor 4 Alpha; IF: Immunofluorescence; E-CAD: E-Cadherin; KI67: Antigen KI-67; SOX: SRY (Sex Determining Region Y)-Box e.g. SOX9: SRY (Sex Determining Region Y)-Box 9;; PROM1: Prominin 1; FOXA: Forkhead Box Protein e.g. FOXA2: Forkhead Box Protein A2; ALB: Albumin; PROX1: Prospero Homeobox 1; qPCR: Quantitative polymerase chain reaction; FACS: Fluorescence-activated cell sorting; 2D: 2 dimensional; 3D: 3 dimensional; PAS: Periodic acid Schiff; LDL: Low-density lipoprotein; cAMP: cyclic adenosine monophosphate; BMP: Bone Morphogenetic Protein; HGF: Hepatocyte Growth Factor; FGF: Fibroblast Growth Factor; EGF: Epidermal Growth Factor; TGF-β: Transforming growth factor beta; MAPK; Mitogen-activated protein kinases; extracellular signal-regulated kinases; JNK; c-Jun N-terminal kinases; FGF; Fibroblast Growth Factor; STAT3: Signal transducer and activator of transcription 3; GAB1: GRB2-associated-binding protein 1; AKT/PI3K/mTOR: Protein kinase B/ Phosphatidylinositol-4,5-bisphosphate 3-kinase/ mechanistic target of rapamycin; NF-κB: nuclear factor kappa-light-chain-enhancer of activated B cells; YAP: Yes-associated protein; IGF; Insulin-like growth factor, IL: Interleukin e.g. IL-6: Interleukin-6, OSM: Oncostatin-M

### Deposit Statement

Biologically pure cultures of the endoderm spheroid progenitor cells described herein were deposited _, 2016, under terms of the Budapest Treaty with the American Type Culture Collection (ATCC^{®}) (10801 University Boulevard, Manassas, VA 20110 USA), and given the patent deposit designation number(s) _, respectively. The microorganism deposit was made under the provisions of the "Budapest Treaty on the International Recognition of the Deposit of Microorganisms for the Purposes of Patent Procedure." All restrictions on the availability to the public of these deposited microorganisms will be irrevocably removed upon issuance of a patent based on this application. For the purposes of this disclosure, any isolate having the identifying characteristics of the deposited cells, including subcultures and variants thereof having the identifying characteristics and activity as described herein, are included.

### Informal Sequence Listing

### Protein name, accession numbers, gene name and amino acid sequence of Markers

**SEQ ID NO:1**
   **Hepatocyte nuclear factor 4, alpha (HNF4A), accession number: AAI37540; Gene: HNF4A** 
MRLSKTLVDMDMADYSAALDPAYTTLEFENVQVLTMGNDTSPSEGTNLNAPNSLGVS
ALCAICGDRATGKHYGASSCDGCKGFFRRSVRKNHMYSCRFSRQCVVDKDKRNQCRY
CRLKKCFRAGMKKEAVQNERDRISTRRSSYEDSSLPSINALLQAEVLSRQITSPVSGINGD
IRAKKIASIADVCESMKEQLLVLVEWAKYIPAFCELPLDDQVALLRAHAGEHLLLGATK
RSMVFKDVLLLGNDYIVPRHCPELAEMSRVSIRILDELVLPFQELQIDDNEYAYLKAIIFF
DPDAKGLSDPGKIKRLRSQVQVSLEDYINDRQYDSRGRFGELLLLLPTLQSITWQMIEQI
QFIKLFGMAKIDNLLQEMLLGGPCQAQEGRGWSGDSPGDRPHTVSSPLSSLASPLCRFG
QVA
**SEQ ID NO:2**
   **pancreas/duodenum homeobox protein 1 (PDX1), accession number: NP_000200; Gene: PDX1**
**SEQ ID NO:3**
   **homeobox protein CDX-2, accession number: NP_001256; Gene: CDX2** 
MYVSYLLDKDVSMYPSSVRHSGGLNLAPQNFVSPPQYPDYGGYHVAAAAAAAANLDS
AQSPGPSWPAAYGAPLREDWNGYAPGGAAAAANAVAHGLNGGSPAAAMGYSSPADY
HPHHHPHHHPHHPAAAPSCASGLLQTLNPGPPGPAATAAAEQLSPGGQRRNLCEWMRK
PAQQSLGSQVKTRTKDKYRVVYTDHQRLELEKEFHYSRYITIRRKAELAATLGLSERQV
KIWFQNRRAKERKINKKKLQQQQQQPPQPPPPPPQPPQPQPGPLRSVPEPLSPVSSLQAS
VSGSVPGVLGPTGGVLNPTVTQ
**SEQ ID NO:4**
   **SOX9, accession number: CAA86598; Gene: SOX9**
**SEQ ID NO:5**
   **keratin, type I cytoskeletal 19 (KRT19), accession number: NP_002267; Gene: KRT19** 
MTSYSYRQSSATSSFGGLGGGSVRFGPGVAFRAPSIHGGSGGRGVSVSSARFVSSSSSGA
YGGGYGGVLTASDGLLAGNEKLTMQNLNDRLASYLDKVRALEAANGELEVKIRDWYQ
KQGPGPSRDYSHYYTTIQDLRDKILGATIENSRIVLQIDNARLAADDFRTKFETEQALRM
SVEADINGLRRVLDELTLARTDLEMQIEGLKEELAYLKKNHEEEISTLRGQVGGQVSVE
VDSAPGTDLAKILSDMRSQYEVMAEQNRKDAEAWFTSRTEELNREVAGHTEQLQMSRS
EVTDLRRTLQGLEIELQSQLSMKAALEDTLAETEARFGAQLAHIQALISGIEAQLGDVRA
DSERQNQEYQRLMDIKSRLEQEIATYRSLLEGQEDHYNNLSASKVL
**SEQ ID NO:6**
   **Alpha-fetoprotein (AFP), accession number: AAH27881; Gene: AFP**
**SEQ ID NO:7**
   **ONECUT-2 transcription factor (OC-2), accession number: CAB38253; Gene: OC-2** 
MNPELTMESLGTLHGARGGGSGGGGGGGGGGGGGPGHEQELLASPSPHHARRGPRG
SLRGPPPPPTAHQELGTAAAAAAASRSAMVTSMASILDGGDYRPELSIPLHHAMSMSC
DSSPPGMGMSNTYTTLTPLQPLPPISTVSDKFHHPHPHHHPHHHHHHHQRLSGNVSGS
FTLMRDERGLPAMNNLYSPYKEMPGMSQSLSPLAATPLGNGLGGLHNAQQSLPNYGPP
GHDKMLSPNFDAHHTAMLTRGEQHLSRGLGTPPAAMMSHLNGLHHPGHTQSHGPVLA
PSRERPPSSSSGSQVATSGQLEEINTKEVAQRITAELKRYSIPQAIFAQRVLCRSQGTLSDL
LRNPKPWSKLKSGRETFRRMWKWLQEPEFQRMSALRLAACKRKEQEPNKDRNNSQKK
SRLVFTDLQRRTLFAIFKENKRPSKEMQITISQQLGLELTTVSNFFMNARRRSLEKWQDD
LSTGGSSSTSSTCTKA
**SEQ ID NO:8**
   **G protein-coupled receptor LGR5, accession number: AAC77911; Gene: LGR5**
**SEQ ID NO:9**
   **EPHB2, accession number: AAH67861; Gene: EPHB2** 
MALRRLGAALLLLPLLAAVEETLMDSTTATAELGWMVHPPSGWEEVSGYDENMNTIRT
YQVCNVFESSQNNWLRTKFIRRRGAHRIHVEMKFSVRDCSSIPSVPGSCKETFNLYYYE
ADFDSATKTFPNWMENPWVKVDTIAADESFSQVDLGGRVMKINTEVRSFGPVSRSGFY
LAFQDYGGCMSLIAVRVFYRKCPRIIQNGAIFQETLSGAESTSLVAARGSCIANAEEVDV
PIKLYCNGDGEWLVPIGRCMCKAGFEAVENGTVCRGCPSGTFKANQGDEACTHCPINSR
TTSEGATNCVCRNGYYRADLDPLDMPCTTIPSAPQAVISSVNETSLMLEWTPPRDSGGR
EDLVYNIICKSCGSGRGACTRCGDNVQYAPRQLGLTEPRIYISDLLAHTQYTFEIQAVNG
VTDQSPFSPQFASVNITTNQAAPSAVSIMHQVSRTVDSITLSWSQPDQPNGVILDYELQY
YEKMKTQRS
**SEQ ID NO:10**
   **LGR4, accession number: AAH33039; Gene: LGR4** 
MPGPLGLLCFLALGLLGSAGPGGAAPPLCAAPCSCDGDRRVDCSGKGLTAVPEGLSAFT
QALQLAGNDLSFIHPKALSGLKELKVLTLQNNQLKTVPSEAIRGLSALQSLRLDANHITS
VPEDSFEGLVQLRHLWLDDNSLTEVPVHHLSNLPTLQALTLALNKISSIPDFAFTNLSSLV
VLHLHNNKIRSLSQHCFDGLDNLETLDLNYNNLGEFPQAIKALPSLKELGFHSNSISVIPD
GAFDGNPLLRTIHLYDNPLSFVGNSAFHNLSDLHSLVIRGASMVQQFPNLTGTVHLESLT
LTGTKISSIPNNLCQEQKMLRTLDLSYNNIRDLPSFNGCHALEEISLQRNQIYQIKEGTFQ
GLISLRILDLSRNLIHEIHSRAFATLGPITNLDVSFNELTSFPTEGLNGLNQLKLVGNFKLK
EALAAKDFVNLRSLSVPYAYQCCAFWGCDSYANLNTEDNSLQDHSVAQEKGTADAAN
 
VTSTLENEEHSQIIIHCTPSTGAFKPCEYLLGSWMIRLTVWFIFLVALFFNLLVILTTFASC
TSLPSSKLFIGLISVSNLFMGIYTGILTFLDAVSWGRFAEFGIWWETGSGCKVAGFLAVFS
SESAIFLLMLATVERSLSAKDIMKNGKSNHLKQFRVAALLAFLGATVAGCFPIFHRGEYS
ASPLCLPFPTGETPSLGFTVTLVLLNSLAFLLMAVIYTKLYCNLEKEDLSENSQSSMIKHV
AWLIFTNCIFFCPVAFFSFAPLITAISISPEIMKSVTLIFFPLPACLNPVLYVFFNPKFKEDW
KLLKRRVTKKSGSVSVSISSQGGCLEQDFYYDCGMYSHLQGNLTVCDCCESFLLTKPVS
CKHLIKSHSCPALAVASCQRPEGYWSDCGTQSAHSDYADEEDSFVSDSSDQVQACGRA
CFYQSRGFPLVRYAYNLPRVKD
**SEQ ID NO:11**
   **nuclear receptor subfamily 5 group A member 2 isoform 1 (NR5A2), accession number: NP_995582; Gene: NR5A2** 
MSSNSDTGDLQESLKHGLTPIGAGLPDRHGSPIPARGRLVMLPKVETEALGLARSHGEQ
GQMPENMQVSQFKMVNYSYDEDLEELCPVCGDKVSGYHYGLLTCESCKGFFKRTVQN
NKRYTCIENQNCQIDKTQRKRCPYCRFQKCLSVGMKLEAVRADRMRGGRNKFGPMYK
RDRALKQQKKALIRANGLKLEAMSQVIQAMPSDLTISSAIQNIHSASKGLPLNHAALPPT
DYDRSPFVTSPISMTMPPHGSLQGYQTYGHFPSRAIKSEYPDPYTSSPESIMGYSYMDSY
QTSSPASIPHLILELLKCEPDEPQVQAKIMAYLQQEQANRSKHEKLSTFGLMCKMADQT
LFSIVEWARSSIFFRELKVDDQMKLLQNCWSELLILDHIYRQVVHGKEGSIFLVTGQQVD
YSIIASQAGATLNNLMSHAQELVAKLRSLQFDQREFVCLKFLVLFSLDVKNLENFQLVE
GVQEQVNAALLDYTMCNYPQQTEKFGQLLLRLPEIRAISMQAEEYLYYKHLNGDVPYN
NLLIEMLHAKRA
**SEQ ID NO:12**
   **CDH1, accession number: AAI46663; Gene: CDH1** 
MGPWSRSLSALLLLLQVSSWLCQEPEPCHPGFDAESYTFTVPRRHLERGRVLGRVNFED
CTGRQRTAYFSLDTRFKVGTDGVITVKRPLRFHNPQIHFLVYAWDSTYRKFSTKVTLNT
VGHHHRPPPHQASVSGIQAELLTFPNSSPGLRRQKRDWVIPPISCPENEKGPFPKNLVQIK
SNKDKEGKVFYSITGQGADTPPVGVFIIERETGWLKVTEPLDRERIATYTLFSHAVSSNG
NAVEDPMEILITVTDQNDNKPEFTQEVFKGSVMEGALPGTSVMEVTATDADDDVNTYN
AAIAYTILSQDPELPDKNMFTINRNTGVISVVTTGLDRESFPAYTLVVQAADLQGEGLST
TATAVITVTDTNDNPPIFNPTTYKGQVPENEANVVITTLKVTDADAPNTPWEAVYTILND
DGGQFVVTTNPVNNDGILKTAKGLDFEAKQQYILHVAVTNVVPFEVSLTTSTATVTVDV
LDVNEAPIFVPPEKRVEVSEDFGVGQEITSYTAQEPDTFMEQKITYRIWRDTANWLEINP
DTGAISTRAELDREDFEHVKNSTYTALIIATDNGSPVATGTGTLLLILSDVNDNAPIPEPR
TIFFCERNPKPQVINIIDADLPPILASQSIGITDMSHCTCPAPQLPAIF
**SEQ ID NO:13**
   **keratin, type II cytoskeletal 7 (KRT7), accession number: NP_005547; Gene: KRT7** 
MSIHFSSPVFTSRSAAFSGRGAQVRLSSARPGGLGSSSLYGLGASRPRVAVRSAYGGPVG
AGIREVTINQSLLAPLRLDADPSLQRVRQEESEQIKTLNNKFASFIDKVRFLEQQNKLLET
 
KWTLLQEQKSAKSSRLPDIFEAQIAGLRGQLEALQVDGGRLEAELRSMQDVVEDFKNK
YEDEINHRTAAENEFVVLKKDVDAAYMSKVELEAKVDALNDEINFLRTLNETELTELQS
QISDTSVVLSMDNSRSLDLDGIIAEVKAQYEEMAKCSRAEAEAWYQTKFETLQAQAGK
HGDDLRNTRNEISEMNRAIQRLQAEIDNIKNQRAKLEAAIAEAEERGELALKDARAKQE
ELEAALQRGKQDMARQLREYQELMSVKLALDIEIATYRKLLEGEESRLAGDGVGAVNIS
VMNSTGGSSSGGGIGLTLGGTMGSNALSFSSSAGPGLLKAYSIRTASASRRSARD
**SEQ ID NO:14**
   **zinc finger protein 503 (ZNF503), accession number: NP_116161; Gene: ZNF503** 
MSTAPSLSALRSSKHSGGGGGGGGGGGADPAWTSALSGNSSGPGPGSSPAGSTKPFVHA
VPPSDPLRQANRLPIKVLKMLTARTGHILHPEYLQPLPSTPVSPIELDAKKSPLALLAQTC
SQIGKPDPSPSSKLSSVASNGGGAGGAGGGAAGDKDTKSGPLKLSDIGVEDKSSFKPYS
KPGSDKKEPGGGGGGGGGGGGGGGGVSSEKSGFRVPSATCQPFTPRTGSPSSSASACSP
GGMLSSAGGAPEGKDDKKDTDVGGGGKGTGGASAEGGPTGLAHGRISCGGGINVDVN
QHPDGGPGGKALGSDCGGSSGSSSGSGPSAPTSSSVLGSGLVAPVSPYKPGQTVFPLPPA
GMTYPGSLAGAYAGYPPQFLPHGVALDPTKPGSLVGAQLAAAAAGSLGCSKPAGSSPL
AGASPPSVMTASLCRDPYCLSYHCASHLAGAAAASASCAHDPAAAAAALKSGYPLVYP
THPLHGVHSSLTAAAAAGATPPSLAGHPLYPYGFMLPNDPLPHICNWVSANGPCDKRFA
TSEELLSHLRTHTAFPGTDKLLSGYPSSSLASAAAAAMACHMHIPTSGAPGSPGTLALR
SPHHALGLSSRYHPYSKSPLPTPGAPVPVPAATGPYYSPYALYGQRLTTASALGYQ
**SEQ ID NO:15**
   **homeobox protein MSX-2, accession number: NP_002440; Gene: MSX2** 
MASPSKGNDLFSPDEEGPAVVAGPGPGGAEGAAEERRVKVSSLPFSVEALMSDKKPP
KEASPLPAESASAGATLRPLLLSGHGAREAHSPGPLVKPFETASVKSENSEDGAAWMQE
PGRYSPPPRHMSPTTCTLRKHKTNRKPRTPFTTSQLLALERKFRQKQYLSIAERAEFSSSL
NLTETQVKIWFQNRRAKAKRLQEAELEKLKMAAKPMLPSSFSLPFPISSPLQAASIYGAS
YPFHRPVLPIPPVGLYATPVGYGMYHLS
**SEQ ID NO:16**
   **zinc finger transcription factor Trps1, accession number: NP_054831; Gene: TRPS1**
**SEQ ID NO:17**
   **achaete-scute homolog 2 (ASCL2), accession number: NP_005161; Gene: ASCL2** 
MDGGTLPRSAPPAPPVPVGCAARRRPASPELLRCSRRRRPATAETGGGAAAVARRNERE
RNRVKLVNLGFQALRQHVPHGGASKKLSKVETLRSAVEYIRALQRLLAEHDAVRNALA
GGLRPQAVRPSAPRGPPGTTPVAASPSRASSSPGRGGSSEPGSPRSAYSSDDSGCEGALSP
AERELLDFSSWLGGY
**SEQ ID NO:18**
   **interferon regulatory factor 8 (IRF8), accession number: NP_002154; Gene: IRF8** 
MCDRNGGRRLRQWLIEQIDSSMYPGLIWENEEKSMFRIPWKHAGKQDYNQEVDASIFK
AWAVFKGKFKEGDKAEPATWKTRLRCALNKSPDFEEVTDRSQLDISEPYKVYRIVPEEE
QKCKLGVATAGCVNEVTEMECGRSEIDELIKEPSVDDYMGMIKRSPSPPEACRSQLLPD
WWAQQPSTGVPLVTGYTTYDAHHSAFSQMVISFYYGGKLVGQATTTCPEGCRLSLSQP
GLPGTKLYGPEGLELVRFPPADAIPSERQRQVTRKLFGHLERGVLLHSSRQGVFVKRLC
QGRVFCSGNAVVCKGRPNKLERDEVVQVFDTSQFFRELQQFYNSQGRLPDGRVVLCFG
EEFPDMAPLRSKLILVQIEQLYVRQLAEEAGKSCGAGSVMQAPEEPPPDQVFRMFPDICA
SHQRSFFRENQQITV
**SEQ ID NO:19**
   **hepatocyte nuclear factor 4-gamma (HNF4G), accession number: NP_004124; Gene: HNF4G** 
MDMANYSEVLDPTYTTLEFETMQILYNSSDSSAPETSMNTTDNGVNCLCAICGDRATG
KHYGASSCDGCKGFFRRSIRKSHVYSCRFSRQCVVDKDKRNQCRYCRLRKCFRAGMKK
EAVQNERDRISTRRSTFDGSNIPSINTLAQAEVRSRQISVSSPGSSTDINVKKIASIGDVCES
MKQQLLVLVEWAKYIPAFCELPLDDQVALLRAHAGEHLLLGATKRSMMYKDILLLGN
NYVIHRNSCEVEISRVANRVLDELVRPFQEIQIDDNEYACLKAIVFFDPDAKGLSDPVKIK
NMRFQVQIGLEDYINDRQYDSRGRFGELLLLLPTLQSITWQMIEQIQFVKLFGMVKIDNL
 
LQEMLLGGASNDGSHLHHPMHPHLSQDPLTGQTILLGPMSTLVHADQISTPETPLPSPPQ
GSGQEQYKIAANQASVISHQHLSKQKQL
**SEQ ID NO:20**
   **DNA-binding protein inhibitor ID-2, accession number: NP_002157; Gene: ID-2** 
MKAFSPVRSVRKNSLSDHSLGISRSKTPVDDPMSLLYNMNDCYSKLKELVPSIPQNKKV
SKMEILQHVIDYILDLQIALDSHPTIVSLHHQRPGQNQASRTPLTTLNTDISILSLQASEFPS
ELMSNDSKALCG
**SEQ ID NO:21**
   **CD44, accession number: ACI46596; Gene: CD44** 
MDKFWWHAAWGLCLVPLSLAQIDLNITCRFAGVFHVEKNGRYSISRTEAADLCKAFNS
TLPTMAQMEKALSIGFETCRYGFIEGHVVIPRIHPNSICAANNTGVYILTSNTSQYDTYCF
NASAPPEEDCTSVTDLPNAFDGPITITIVNRDGTRYVQKGEYRTNPEDIYPSNPTDDDVSS
GSSSERSSTSGGYIFYTFSTVHPIPDEDSPWITDSTDRIPATRHSHGSQEGGANTTSGPIRTP
QIPEWLIILASLLALALILAVCIAVNSRRRCGQKKKLVINSGNGAVEDRKPSGLNGEASKS
QEMVHLVNKESSETPDQFMTADETRNLQNVDMKIGV
**SEQ ID NO:22**
   **Epithelial cell adhesion molecule (EPCAM), accession number: AAH14785; Gene: EPCAM** 
MAPPQVLAFGLLLAAATATFAAAQEECVCENYKLAVNCFVNNNRQCQCTSVGAQNTV
ICSKLAAKCLVMKAEMNGSKLGRRAKPEGALQNNDGLYDPDCDESGLFKAKQCNGTS
TCWCVNTAGVRRTDKDTEITCSERVRTYWIIIELKHKAREKPYDSKSLRTALQKEITTRY
QLDPKFITSILYENNVITIDLVQNSSQKTQNDVDIADVAYYFEKDVKGESLFHSKKMDLT
VNGEQLDLDPGQTLIYYVDEKAPEFSMQGLKAGVIAVIVVVVIAVVAGIVVLVISRKKR
MAKYEKAEIKEMGEMHRELNA
**SEQ ID NO:23**
   **MET proto-oncogene protein (MET), accession number: AAA59591; Gene: MET** 
MKAPAVLAPGILVLLFTLVQRSNGECKEALAKSEMNVNMKYQLPNFTAETPIQNVILHE
HHIFLGATNYIYVLNEEDLQKVAEYKTGPVLEHPDCFPCQDCSSKANLSGGVWKDNIN
MALVVDTYYDDQLISCGSVNRGTCQRHVFPHNHTADIQSEVHCIFSPQIEEPSQCPDCVV
SALGAKVLSSVKDRFINFFVGNTINSSYFPDHPLHSISVRRLKETKDGFMFLTDQSYIDVL
PEFRDSYPIKYVHAFESNNFIYFLTVQRETLDAQTFHTRIIRFCSINSGLHSYMEMPLECIL
TEKRKKRSTKKEVFNILQAAYVSKPGAQLARQIGASLNDDILFGVFAQSKPDSAEPMDR
SAMCAFPIKYVNDFFNKIVNKNNVRCLQHFYGPNHEHCFNRTLLRNSSGCEARRDEYRT
EFTTALQRVDLFMGQFSEVLLTSISTFIKGDLTIANLGTSEGRFMQVVVSRSGPSTPHVNF
LLDSHPVSPEVIVEHTLNQNGYTLVITGKKITKIPLNGLGCRHFQSCSQCLSAPPFVQCG
WCHDKCVRSEECLSGTWTQQICLPAIYKVFPNSAPLEGGTRLTICGWDFGFRRNNKFDL
 
KKTRVLLGNESCTLTLSESTMNTLKCTVGPAMNKHFNMSIIISNGHGTTQYSTFSYVDPV
ITSISPKYGPMAGGTLLTLTGNYLNSGNSRHISIGGKTCTLKSVSNSILECYTPAQTISTEF
AVKLKIDLANRETSIFSYREDPIVYEIHPTKSFISTWWKEPLNIVSFLFCFASGGSTITGVG
KNLNSVSVPRMVINVHEAGRNFTVACQHRSNSEIICCTTPSLQQLNLQLPLKTKAFFMLD
GILSKYFDLIYVHNPVFKPFEKPVMISMGNENVLEIKGNDIDPEAVKGEVLKVGNKSCEN
IHLHSEAVLCTVPNDLLKLNSELNIEWKQAISSTVLGKVIVQPDQNFTGLIAGVVSISTAL
LLLLGFFLWLKKRKQIKDLGSELVRYDARVHTPHLDRLVSARSVSPTTEMVSNESVDYR
ATFPEDQFPNSSQNGSCRQVQYPLTDMSPILTSGDSDISSPLLQNTVHIDLSALNPELVQA
VQHVVIGPSSLIVHFNEVIGRGHFGCVYHGTLLDNDGKKIHCAVKSLNRITDIGEVSQFL
TEGIIMKDFSHPNVLSLLGICLRSEGSPLVVLPYMKHGDLRNFIRNETHNPTVKDLIGFGL
QVAKAMKYLASKKFVHRDLAARNCMLDEKFTVKVADFGLARDMYDKEYYSVHNKT
GAKLPVKWMALESLQTQKFTTKSDVWSFGVVLWELMTRGAPPYPDVNTFDITVYLLQ
GRRLLQPEYCPDPLYEVMLKCWHPKAEMRPSFSELVSRISAIFSTFIGEHYVHVNATYVN
VKCVAPYPSLLSSEDNADDEVDTRPASFWETS
**SEQ ID NO:24**
   **indian hedgehog protein preproprotein (IHH), accession number: NP_002172; Gene: IHH**
**SEQ ID NO:25**
   **claudin-3 (CLDN3), accession number: NP_001297; Gene: CLDN3** 
MSMGLEITGTALAVLGWLGTIVCCALPMWRVSAFIGSNIITSQNIWEGLWMNCVVQSTG
QMQCKVYDSLLALPQDLQAARALIVVAILLAAFGLLVALVGAQCTNCVQDDTAKAKIT
IVAGVLFLLAALLTLVPVSWSANTIIRDFYNPVVPEAQKREMGAGLYVGWAAAALQLL
GGALLCCSCPPREKKYTATKVVYSAPRSTGPGASLGTGYDRKDYV
**SEQ ID NO:26**
   **SOX2, accession number: NP_003097; Gene: SOX2**
**SEQ ID NO:27**
   **Cerberus 1 (CER1), accession number: AAH69491; Gene: CER1**
**SEQ ID NO:28**
   **GATA-4, accession number: NP_001295022; Gene: GATA4** 
MYQSLAMAANHGPPPGAYEAGGPGAFMHGAGAASSPVYVPTPRVPSSVLGLSYLQGG
GAGSASGGASGGSSGGAASGAGPGTQQGSPGWSQAGADGAAYTPPPVSPRFSFPGTTG
SLAAAAAAAAREAAAYSSGGGAAGAGLAGREQYGRAGFAGSYSSPYPAYMADVGA
SWAAAAAASAGPFDSPVLHSLPGRANPAARHPNLVDMFDDFSEGRECVNCGAMSTPL
WRRDGTGHYLCNACGLYHKMNGINRPLIKPQRRLSASRRVGLSCANCQTTTTTLWRRN
AEGEPVCNACGLYMKLHGVPRPLAMRKEGIQTRKRKPKNLNKSKTPAAPSGSESLPPAS
GASSNSSNATTSSSEEMRPIKTEPGLSSHYGHSSSVSQTFSVSAMSGHGPSIHPVLSALKL
SPQGYASPVSQSPQTSSKQDSWNSLVLADSHGDIITA
**SEQ ID NO:29**
   **SOX17, accession number: NP_071899; Gene: SOX17** 
MYQSLAMAANHGPPPGAYEAGGPGAFMHGAGAASSPVYVPTPRVPSSVLGLSYLQGG
GAGSASGGASGGSSGGAASGAGPGTQQGSPGWSQAGADGAAYTPPPVSPRFSFPGTTG
SLAAAAAAAAREAAAYSSGGGAAGAGLAGREQYGRAGFAGSYSSPYPAYMADVGA
SWAAAAAASAGPFDSPVLHSLPGRANPAARHPNLVDMFDDFSEGRECVNCGAMSTPL
WRRDGTGHYLCNACGLYHKMNGINRPLIKPQRRLSASRRVGLSCANCQTTTTTLWRRN
AEGEPVCNACGLYMKLHGVPRPLAMRKEGIQTRKRKPKNLNKSKTPAAPSGSESLPPAS
GASSNSSNATTSSSEEMRPIKTEPGLSSHYGHSSSVSQTFSVSAMSGHGPSIHPVLSALKL
SPQGYASPVSQSPQTSSKQDSWNSLVLADSHGDIITA
**SEQ ID NO:30**
   **hepatocyte nuclear factor 3-beta (HNF3B), accession number: NP_068556; Gene: FOXA2**
**SEQ ID NO:31**
   **C-X-C chemokine receptor type 4 (CXCR4), accession number: NP_001008540; Gene: CXCR4** 
MSIPLPLLQIYTSDNYTEEMGSGDYDSMKEPCFREENANFNKIFLPTIYSIIFLTGIVGNGL
VILVMGYQKKLRSMTDKYRLHLSVADLLFVITLPFWAVDAVANWYFGNFLCKAVHVI
YTVNLYSSVLILAFISLDRYLAIVHATNSQRPRKLLAEKVVYVGVWIPALLLTIPDFIFAN
VSEADDRYICDRFYPNDLWVVVFQFQHIMVGLILPGIVILSCYCIIISKLSHSKGHQKRKA
LKTTVILILAFFACWLPYYIGISIDSFILLEIIKQGCEFENTVHKWISITEALAFFHCCLNPIL
YAFLGAKFKTSAQHALTSVSRGSSLKILSKGKRGGHSSVSTESES
SSFHSS
**SEQ ID NO:32**
   **Fumarylacetoacetase (FAH), accession number: NP_000128; Gene: FAH**
**SEQ ID NO:33**
   **tyrosine aminotransferase (TAT), accession number: NP_000344; Gene: TAT**
**SEQ ID NO:34**
   **Glucokinase (GCK), accession number: NP_000153; Gene: GCK**
**SEQ ID NO:35**
   **Transthyretin (TTR), accession number: AAH20791; Gene: TTR**
**SEQ ID NO:36**
   **maltase-glucoamylase (MGAM), accession number: NP_004659; Gene: GLUI** 
MARKKLKKFTTLEIVLSVLLLVLFIISIVLIVLLAKESLKSTAPDPGTTGTPDPGTTGTPDP
GTTGTTHARTTGPPDPGTTGTTPVSAECPVVNELERINCIPDQPPTKATCDQRGCCWNPQ
GAVSVPWCYYSKNHSYHVEGNLVNTNAGFTARLKNLPSSPVFGSNVDNVLLTAEYQTS
NRFHFKLTDQTNNRFEVPHEHVQSFSGNAAASLTYQVEISRQPFSIKVTRRSNNRVLFDS
SIGPLLFADQFLQLSTRLPSTNVYGLGEHVHQQYRHDMNWKTWPIFNRDTTPNGNGTN
LYGAQTFFLCLEDASGLSFGVFLMNSNAMEVVLQPAPAITYRTIGGILDFYVFLGNTPEQ
VVQEYLELIGRPALPSYWALGFHLSRYEYGTLDNMREVVERNRAAQLPYDVQHADIDY
MDERRDFTYDSVDFKGFPEFVNELHNNGQKLVIIVDPAISNNSSSKPYGPYDRGSDMKI
WVNSSDGVTPLIGEVWPGQTVFPDYTNPNCAVWWTKEFELFHNQVEFDGIWIDMNEVS
NFVDGSVSGCSTNNLNNPPFTPRILDGYLFCKTLCMDAVQHWGKQYDIHNLYGYSMAV
ATAEAAKTVFPNKRSFILTRSTFAGSGKFAAHWLGDNTATWDDLRWSIPGVLEFNLFGI
PMVGPDICGFALDTPEELCRRWMQLGAFYPFSRNHNGQGYKDQDPASFGADSLLLNSS
RHYLNIRYTLLPYLYTLFFRAHSRGDTVARPLLHEFYEDNSTWDVHQQFLWGPGLLITP
VLDEGAEKVMAYVPDAVWYDYETGSQVRWRKQKVEMELPGDKIGLHLRGGYIFPTQQ
PNTTTLASRKNPLGLIIALDENKEAKGELFWDNGETKDTVANKVYLLCEFSVTQNRLEV
NISQSTYKDPNNLAFNEIKILGTEEP
SNVTVKHNGVPSQTSPTVTYDSNLKVAIITDIDLLLGEAYTVEWSIKIRDEEKIDCYPDEN
GASAENCTARGCIWEASNSSGVPFCYFVNDLYSVSDVQYNSHGATADISLKSSVYANAF
PSTPVNPLRLDVTYHKNEMLQFKIYDPNKNRYEVPVPLNIPSMPSSTPEGQLYDVLIKKN
PFGIEIRRKSTGTIIWDSQLLGFTFSDMFIRISTRLPSKYLYGFGETEHRSYRRDLEWHTW
GMFSRDQPPGYKKNSYGVHPYYMGLEEDGSAHGVLLLNSNAMDVTFQPLPALTYRTT
GGVLDFYVFLGPTPELVTQQYTELIGRPVMVPYWSLGFQLCRYGYQNDSEIASLYDEM
VAAQIPYDVQYSDIDYMERQLDFTLSPKFAGFPALINRMKADGMRVILILDPAISGNETQ
PYPAFTRGVEDDVFIKYPNDGDIVWGKVWPDFPDVVVNGSLDWDSQVELYRAYVAFP
 
DFFRNSTAKWWKREIEELYNNPQNPERSLKFDGMWIDMNEPSSFVNGAVSPGCRDASL
NHPPYMPHLESRDRGLSSKTLCMESQQILPDGSLVQHYNVHNLYGWSQTRPTYEAVQE
VTGQRGVVITRSTFPSSGRWAGHWLGDNTAAWDQLKKSIIGMMEFSLFGISYTGADICG
FFQDAEYEMCVRWMQLGAFYPFSRNHNTIGTRRQDPVSWDVAFVNISRTVLQTRYTLL
PYLYTLMHKAHTEGVTVVRPLLHEFVSDQVTWDIDSQFLLGPAFLVSPVLERNARNVT
AYFPRARWYDYYTGVDINARGEWKTLPAPLDHINLHVRGGYILPWQEPALNTHLSRQK
FMGFKIALDDEGTAGGWLFWDDGQSIDTYGKGLYYLASFSASQNTMQSHIIFNNYITGT
NPLKLGYIEIWGVGSVPVTSVSISVSGMVITPSFNNDPTTQVLSIDVTDRNISLHNFTSLT
WISTL
**SEQ ID NO:37**
   **Fumarylacetoacetate hydrolase domain containing 2A (FAHD2A), accession number: AAI10912; Gene: FAHD2A** 
MLVSGRRRLLTVLLQAQKWPFQPSRDMRLVQFRAPHLVGPHLGLETGNGGGVINLNAF
DPTLPKTMTQFLEQGEATLSVARRALAAQLPVLPRSEVTFLAPVTRPDKVVCVGMNYV
DHCKEQNVPVPKEPIIFSKFASSIVGPYDEVVLPPQSQEVDWEVELAVVIGKKGKHIKAT
DAMAHVAGFTVAHDVSARDWQMRRNGKQWLLGKTFDTFCPLGPALVTKDSVADPHN
LKICCRVNGEVVQSGNTNQMVFKTEDLIAWVSQFVTFYPGDVILTGTPPGVGVFRKPPV
FLKKGDEVQCEIEELGVIINKVV
**SEQ ID NO:38**
   **hepatocyte nuclear factor 1-beta (HNF1β), accession number: NP_000449; Gene: HNF1β**
**SEQ ID NO:39**
   **hepatocyte nuclear factor 1-alpha (HNF1A), accession number: NP_001293108; Gene: HNF1A** 
MVSKLSQLQTELLAALLESGLSKEALIQALGEPGPYLLAGEGPLDKGESCGGGRGELAE
LPNGLGETRGSEDETDDDGEDFTPPILKELENLSPEEAAHQKAVVETLLQEDPWRVAKM
VKSYLQQHNIPQREVVDTTGLNQSHLSQHLNKGTPMKTQKRAALYTWYVRKQREVAQ
 
QFTHAGQGGLIEEPTGDELPTKKGRRNRFKWGPASQQILFQAYERQKNPSKEERETLVE
ECNRAECIQRGVSPSQAQGLGSNLVTEVRVYNWFANRRKEEAFRHK
LAMDTYSGPPPGPGPGPALPAHSSPGLPPPALSPSKVHGVRYGQPATSETAEVPSSSGGP
LVTVSTPLHQVSPTGLEPSHSLLSTEAKLVSAAGGPLPPVSTLTALHSLEQTSPGLNQQPQ
NLIMASLPGVMTIGPGEPASLGPTFTNTGASTLVIGLASTQAQSVPVINSMGSSLTTLQPV
QFSQPLHPSYQQPLMPPVQSHVTQSPFMATMAQLQSPHALYSHKPEVAQYTHTGLLPQT
MLITDTTNLSALASLTPTKQEAALLPQVFTSDTEASSESGLHTPASQATTLHVPSQDPAGI
QHLQPAHRLSASPTVSSSSLVLYQSSDSSNGQSHLLPSNHSVIETFISTQMASSSQ
**SEQ ID NO:40**
   **cytochrome P450 family 3 subfamily A polypeptide 4 (CYP3A4), accession number: ABI96208; Gene: CYP3A4**
**SEQ ID NO:41**
   **cytochrome P450 family 2 subfamily C polypeptide 9 (CYP2C9), accession number: AGS09764; Gene: CYP2C9**
**SEQ ID NO:42**
   **cytochrome P450, family 2, subfamily C, polypeptide 19 (CYP2C19), accession number: AAV41877; Gene: CYP2C19**
**SEQ ID NO:43**
   **Cytochrome P450, family 1, subfamily A, polypeptide 2 (CYP1A2), accession number: AAH67428; Gene: CYP1A2** 
MALSQSVPFSATELLLASAIFCLVFWVLKGLRPRVPKGLKSPPEPWGWPLLGHVLTLGK
NPHLALSRMSQRYGDVLQIRIGSTPVLVLSRLDTIRQALVRQGDDFKGRPDLYTSTLITD
GQSLTFSTDSGPVWAARRRLAQNALNTFSIASDPASSSSCYLEEHVSKEAKALISRLQEL
MAGPGHFDPYNQVVVSVANVIGAMCFGQHFPESSDEMLSLVKNTHEFVETASSGNPLD
FFPILRYLPNPALQRFKAFNQRFLWFLQKTVQEHYQDFDKNSVRDITGALFKHSKKGPR
ASGNLIPQEKIVNLVNDIFGAGFDTVTTAISWSLMYLVTKPEIQRKIQKELDTVIGRERRP
RLSDRPQLPYLEAFILETFRHSSFLPFTIPHSTTRDTTLNGFYIPKKCVFVNQWQVNHDPE
LWEDPSEFRPERFLTADGTAINKPLSEKMMLFGMGKRRCIGEVLAKWEIFLFLAILLQQL
EFSVPPGVKVDLTPIYGLTMKHARCEHVQARLRFSIN
**SEQ ID NO:44**
   **Cytochrome P450, family 2, subfamily E, polypeptide 1 (CYP2E1), accession number: AAH67435; Gene: CYP2E1**
**SEQ ID NO:45**
   **Cytochrome P450, family 2, subfamily D, polypeptide 6 (CYP2D6), accession number: ABB01372; Gene: CYP2D6** 
MGLEALVPLAVIVAIFLLLVDLMHRRQRWAARYSPGPLPLPGLGNLLHVDFQNTPYCFD
QLRRRFGDVFSLQLAWTPVVVLNGLAAVREALVTHGEDTADRPPVPITQILGFGPRSQG
VFLARYGPAWREQRRFSVSTLRNLGLGKKSLEQWVTEEAACLCAAFANHSGRPFRPNG
LLDKAVSNVIASLTCGRRFEYDDPRFLRLLDLAQEGLKEESGFLREVLNAVPVLLHIPAL
 
AGKVLRFQKAFLTQLDELLTEHRMTWDPAQPPRDLTEAFLAEMEKAKGNPESSFNDEN
LRIVVADLFSAGMVTTSTTLAWGLLLMILHPDVQRRVQQEIDDVIGQVRRPEMGDQAH
MPYTTAVIHEVQRFGDIVPLGVTHMTSRDIEVQGFRIPKGTTLITNLSSVLKDEAVWEKP
FRFHPEHFLDAQGHFVKPEAFLPFSAGRRACLGEPLARMELFLFFTSLLQHFSFSVPTGQP
RPSHHGVFAFLVTPSPYELCAVPR
**SEQ ID NO:46**
   **Cytochrome P450, family 3, subfamily A, polypeptide 7 (CYP3A7), accession number: AAH67436; Gene: CYP3A7** 
MDLIPNLAVETWLLLAVSLILLYLYGTRTHGLFKKLGIPGPTPLPFLGNALSFRKGYWTF
DMECYKKYRKVWGIYDCQQPMLAITDPDMIKTVLVKECYSVFTNRRPFGPVGFMKNAI
SIAEDEEWKRIRSLLSPTFTSGKLKEMVPIIAQYGDVLVRNLRREAETGKPVTLKHVFGA
YSMDVITSTSFGVSIDSLNNPQDPFVENTKKLLRFNPLDPFVLSIKVFPFLTPILEALNITVF
PRKVISFLTKSVKQIKEGRLKETQKHRVDFLQLMIDSQNSKDSETHKALSDLELMAQSIIF
IFAGYETTSSVLSFIIYELATHPDVQQKVQKEIDTVLPNKAPPTYDTV
LQLEYLDMVVNETLRLFPVAMRLERVCKKDVEINGMFIPKGVVVMIPSYVLHHDPKYW
TEPEKFLPERFSKKNKDNIDPYIYTPFGSGPRNCIGMRFALVNMKLALVRVLQNFSFKPC
KETQIPLKLRFGGLLLTEKPIVLKAESRDETVSGA
**SEQ ID NO:47**
   **Cytochrome P450, family 1, subfamily A, polypeptide 1 (CYP1A1), accession number: AAH23019; Gene: CYP1A1**
**SEQ ID NO:48**
   **Cytochrome P450, family 3, subfamily A, polypeptide 5 (CYP3A5), accession number: NP_000768; Gene: CYP3A5** 
MDLIPNLAVETWLLLAVSLVLLYLYGTRTHGLFKRLGIPGPTPLPLLGNVLSYRQGLWK
FDTECYKKYGKMWGTYEGQLPVLAITDPDVIRTVLVKECYSVFTNRRSLGPVGFMKSAI
SLAEDEEWKRIRSLLSPTFTSGKLKEMFPIIAQYGDVLVRNLRREAEKGKPVTLKDIFGA
YSMDVITGTSFGVNIDSLNNPQDPFVESTKKFLKFGFLDPLFLSIILFPFLTPVFEALNVSLF
PKDTINFLSKSVNRMKKSRLNDKQKHRLDFLQLMIDSQNSKETESHKALSDLELAAQSII
 
FIFAGYETTSSVLSFTLYELATHPDVQQKLQKEIDAVLPNKAPPTYDAVVQMEYLDMVV
NETLRLFPVAIRLERTCKKDVEINGVFIPKGSMVVIPTYALHHDPKYWTEPEEFRPERFSK
KKDSIDPYIYTPFGTGPRNCIGMRFALMNMKLALIRVLQNFSFKPCKETQIPLKLDTQGL
LQPEKPIVLKVDSRDGTLSGE
**SEQ ID NO:49**
   **Cytochrome P450, family 27, subfamily A, polypeptide 1 (CYP27A1), accession number: AAH40430; Gene: CYP27A1**
**SEQ ID NO:50**
   **Cytochrome P450, family 2, subfamily B, polypeptide 6 (CYP2B6), accession number: NP_000758; Gene: CYP2B6** 
MELSVLLFLALLTGLLLLLVQRHPNTHDRLPPGPRPLPLLGNLLQMDRRGLLKSFLRFRE
KYGDVFTVHLGPRPVVMLCGVEAIREALVDKAEAFSGRGKIAMVDPFFRGYGVIFANG
NRWKVLRRFSVTTMRDFGMGKRSVEERIQEEAQCLIEELRKSKGALMDPTFLFQSITANI
ICSIVFGKRFHYQDQEFLKMLNLFYQTFSLISSVFGQLFELFSGFLKYFPGAHRQVYKNL
QEINAYIGHSVEKHRETLDPSAPKDLIDTYLLHMEKEKSNAHSEFSHQNLNLNTLSLFFA
GTETTSTTLRYGFLLMLKYPHVAERVYREIEQVIGPHRPPELHDRAKMPYTEAVIYEIQR
FSDLLPMGVPHIVTQHTSFRGYIIPKDTEVFLILSTALHDPHYFEKPDAFNPDHFLDANGA
LKKTEAFIPFSLGKRICLGEGIARAELFLFFTTILQNFSMASPVAPEDIDLTPQECGVGKIPP
TYQIRFLPR
**SEQ ID NO:51**
   **keratin, type I cytoskeletal 18 (KRT18), accession number: NP_954657; Gene: KRT18** 
MSFTTRSTFSTNYRSLGSVQAPSYGARPVSSAASVYAGAGGSGSRISVSRSTSFRGGMGS
GGLATGIAGGLAGMGGIQNEKETMQSLNDRLASYLDRVRSLETENRRLESKIREHLEKK
GPQVRDWSHYFKIIEDLRAQIFANTVDNARIVLQIDNARLAADDFRVKYETELAMRQSV
 
ENDIHGLRKVIDDTNITRLQLETEIEALKEELLFMKKNHEEEVKGLQAQIASSGLTVEVD
APKSQDLAKIMADIRAQYDELARKNREELDKYWSQQIEESTTVVTTQSAEVGAAETTLT
ELRRTVQSLEIDLDSMRNLKASLENSLREVEARYALQMEQLNGILLHLESELAQTRAEG
QRQAQEYEALLNIKVKLEAEIATYRRLLEDGEDFNLGDALDSSNSMQTIQKTTTRRIVDG
KVVSETNDTKVLRH
**SEQ ID NO:52**
   **prospero homeobox protein 1 (PROX1), accession number: NP_002754; Gene: PROX1** 
MPDHDSTALLSRQTKRRRVDIGVKRTVGTASAFFAKARATFFSAMNPQGSEQDVEYSV
VQHADGEKSNVLRKLLKRANSYEDAMMPFPGATIISQLLKNNMNKNGGTEPSFQASGL
SSTGSEVHQEDICSNSSRDSPPECLSPFGRPTMSQFDMDRLCDEHLRAKRARVENIIRGM
SHSPSVALRGNENEREMAPQSVSPRESYRENKRKQKLPQQQQSFQQLVSARKEQKRE
ERRQLKQQLEDMQKQLRQLQEKFYQIYDSTDSENDEDGNLSEDSMRSEILDARAQDSV
GRSDNEMCELDPGQFIDRARALIREQEMAENKPKREGNNKERDHGPNSLQPEGKHLAE
TLKQELNTAMSQVVDTVVKVFSAKPSRQVPQVFPPLQIPQARFAVNGENHNFHTANQR
LQCFGDVIIPNPLDTFGNVQMASSTDQTEALPLVVRKNSSDQSASGPAAGGHHQPLHQS
PLSATTGFTTSTFRHPFPLPLMAYPFQSPLGAPSGSFSGKDRASPESLDLTRDTTSLRTKM
SSHHLSHHPCSPAHPPSTAEGLSLSLIKSECGDLQDMSEISPYSGSAMQEGLSPNHLKKA
KLMFFYTRYPSSNMLKTYFSDVKFNRCITSQLIKWFSNFREFYYIQMEKYARQAINDGV
TSTEELSITRDCELYRALNMHYNKANDFEVPERFLEVAQITLREFFNAIIAGKDVDPSWK
KAIYKVICKLDSEVPEIFKSPNCLQELLHE
**SEQ ID NO:53**
   **hepatocyte nuclear factor 6, accession number: NP_004489; Gene: HNF6** 
MNAQLTMEAIGELHGVSHEPVPAPADLLGGSPHARSSVAHRGSHLPPAHPRSMGMASL
LDGGSGGDYHHHHRAPEHSLAGPLHPTMTMACETPPGMSMPTTYTTLTPLQPLPPIST
VSDKFPHHHHHHHHHHPHHHQRLAGNVSGSFTLMRDERGLASMNNLYTPYHKDVA
GMGQSLSPLSSSGLGSIHNSQQGLPHYAHPGAAMPTDKMLTPNGFEAHHPAMLGRHGE
QHLTPTSAGMVPINGLPPHHPHAHLNAQGHGQLLGTAREPNPSVTGAQV
SNGSNSGQMEEINTKEVAQRITTELKRYSIPQAIFAQRVLCRSQGTLSDLLRNPKPWSKL
KSGRETFRRMWKWLQEPEFQRMSALRLAACKRKEQEHGKDRGNTPKKPRLVFTDVQR
RTLHAIFKENKRPSKELQITISQQLGLELSTVSNFFMNARRRSLDKWQDEGSSNSGNSSSS
SSTCTKA
**SEQ ID NO:54**
   **Albumin, accession number: AAH36003; Gene: ALB**
**SEQ ID NO:55**
   **receptor-type tyrosine-protein phosphatase C (CD45), accession number: NP_002829; Gene: CD45**
**SEQ ID NO:56**
   **T-lymphocyte activation antigen CD80, accession number: NP_005182; Gene: CD80** 
MGHTRRQGTSPSKCPYLNFFQLLVLAGLSHFCSGVIHVTKEVKEVATLSCGHNVSVEEL
AQTRIYWQKEKKMVLTMMSGDMNIWPEYKNRTIFDITNNLSIVILALRPSDEGTYECVV
LKYEKDAFKREHLAEVTLSVKADFPTPSISDFEIPTSNIRRIICSTSGGFPEPHLSWLENGE
ELNAINTTVSQDPETELYAVSSKLDFNMTTNHSFMCLIKYGHLRVNQTFNWNTTKQEHF
PDNLLPSWAITLISVNGIFVICCLTYCFAPRCRERRRNERLRRESVRPV
**SEQ ID NO:57**
   **T-lymphocyte activation antigen CD86, accession number: NP_787058; Gene: CD86** 
MDPQCTMGLSNILFVMAFLLSGAAPLKIQAYFNETADLPCQFANSQNQSLSELVVFWQD
QENLVLNEVYLGKEKFDSVHSKYMGRTSFDSDSWTLRLHNLQIKDKGLYQCIIHHKKPT
GMIRIHQMNSELSVLANFSQPEIVPISNITENVYINLTCSSIHGYPEPKKMSVLLRTKNSTI
EYDGIMQKSQDNVTELYDVSISLSVSFPDVTSNMTIFCILETDKTRLLSSPFSIELEDPQPP
PDHIPWITAVLPTVIICVMVFCLILWKWKKKKRPRNSYKCGTNTMEREESEQTKKREKIH
IPERSDEAQRVFKSSKTSSCDKSDTCF
**SEQ ID NO:58**
   **integrin alpha-X (CD11c), accession number: NP_001273304; Gene: CD11c** 
MTRTRAALLLFTALATSLGFNLDTEELTAFRVDSAGFGDSVVQYANSWVVVGAPQKIT
AANQTGGLYQCGYSTGACEPIGLQVPPEAVNMSLGLSLASTTSPSQLLACGPTVHHECG
RNMYLTGLCFLLGPTQLTQRLPVSRQECPRQEQDIVFLIDGSGSISSRNFATMMNFVRAV
ISQFQRPSTQFSLMQFSNKFQTHFTFEEFRRSSNPLSLLASVHQLQGFTYTATAIQNVVHR
LFHASYGARRDAAKILIVITDGKKEGDSLDYKDVIPMADAAGIIRYAIGVGLAFQNRNS
WKELNDIASKPSQEHIFKVEDFDALKDIQNQLKEKIFAIEGTETTSSSSFELEMAQEGFSA
VFTPDGPVLGAVGSFTWSGGAFLYPPNMSPTFINMSQENVDMRDSYLGYSTELALWKG
VQSLVLGAPRYQHTGKAVIFTQVSRQWRMKAEVTGTQIGSYFGASLCSVDVDSDGSTD
LVLIGAPHYYEQTRGGQVSVCPLPRGWRRWWCDAVLYGEQGHPWGRFGAALTVLGD
VNGDKLTDVVIGAPGEEENRGAVYLFHGVLGPSISPSHSQRIAGSQLSSRLQYFGQALSG
GQDLTQDGLVDLAVGARGQVLLLRTRPVLWVGVSMQFIPAEIPRSAFECREQVVSEQTL
VQSNICLYIDKRSKNLLGSRDLQSSVTLDLALDPGRLSPRATFQETKNRSLSRVRVLGLK
AHCENFNLLLPSCVEDSVTPITLRLNFTLVGKPLLAFRNLRPMLAADAQRYFTASLPFEK
NCGADHICQDNLGISFSFPGLKSLLVGSNLELNAEVMVWNDGEDSYGTTITFSHPAGLS
YRYVAEGQKQGQLRSLHLTCDSAPVGSQGTWSTSCRINHLIFRGGAQITFLATFDVSPK
AVLGDRLLLTANVSSENNTPRTSKTTFQLELPVKYAVYTVVSSHEQFTKYLNFSESEEKE
SHVAMHRYQVNNLGQRDLPVSINFWVPVELNQEAVWMDVEVSHPQNPSLRCSSEKIAP
PASDFLAHIQKNPVLDCSIAGCLRFRCDVPSFSVQEELDFTLKGNLSFGWVRQILQKKVS
VVSVAEITFDTSVYSQLPGQEAFMRAQTTTVLEKYKVHNPTPLIVGSSIGGLLLLALITAV
LYKVGFFKRQYKEMMEEANGQIAPENGTQTPSPPTPHYPQDNV
**SEQ ID NO:59**
   **membrane primary amine oxidase (VAP1), accession number: NP_003725; Gene: AOC** 
MNQKTILVLLILAVITIFALVCVLLVGRGGDGGEPSQLPHCPSVSPSAQPWTHPGQSLF
ADLSREELTAVMRFLTQRLGPGLVDAAQARPSDNCVFSVELQLPPKAALAHLDRGSPP
PAREALAIVFFGRQPQPNVSELVVGPLPHPSYMRDVTVERHGGPLPYHRRPVLFQEYLDI
DQMIFNRELPQASGLLHHCCFYKHRGRNLVTMTTAPRGLQSGDRATWFGLYYNISGAG
FFLHHVGLELLVNHKALDPARWTIQKVFYQGRYYDSLAQLEAQFEAGLVNVVLIPDNG
TGGSWSLKSPVPPGPAPPLQFYPQGPRFSVQGSRVASSLWTFSFGLGAFSGPRIFDVRFQ
GERLVYEISLQEALAIYGGNSPAAMTTRYVDGGFGMGKYTTPLTRGVDCPYLATYVDW
HFLLESQAPKTIRDAFCVFEQNQGLPLRRHHSDLYSHYFGGLAETVLVVRSMSTLLNYD
 
YVWDTVFHPSGAIEIRFYATGYISSAFLFGATGKYGNQVSEHTLGTVHTHSAHFKVDLD
VAGLENWVWAEDMVFVPMAVPWSPEHQLQRLQVTRKLLEMEEQAAFLVGSATPRYL
YLASNHSNKWGHPRGYRIQMLSFAGEPLPQNSSMARGFSWERYQLAVTQRKEEEPSSSS
VFNQNDPWAPTVDFSDFINNETIAGKDLVAWVTAGFLHIPHAEDIPNTVTVGNGVGFFL
RPYNFFDEDPSFYSADSIYFRGDQDAGACEVNPLACLPQAAACAPDLPAFSHGGFSHN
**SEQ ID NO:60**
   **stabilin-1 (STAB1), accession number: NP_055951; Gene: STAB1** 
MAGPRGLLPLCLLAFCLAGFSFVRGQVLFKGCDVKTTFVTHVPCTSCAAIKKQTCPSGW
LRELPDQITQDCRYEVQLGGSMVSMSGCRRKCRKQVVQKACCPGYWGSRCHECPGGA
ETPCNGHGTCLDGMDRNGTCVCQENFRGSACQECQDPNRFGPDCQSVCSCVHGVCNH
GPRGDGSCLCFAGYTGPHCDQELPVCQELRCPQNTQCSAEAPSCRCLPGYTQQGSECRA
PNPCWPSPCSLLAQCSVSPKGQAQCHCPENYHGDGMVCLPKDPCTDNLGGCPSNSTLC
VYQKPGQAFCTCRPGLVSINSNASAGCFAFCSPFSCDRSATCQVTADGKTSCVCRESEV
GDGRACYGHLLHEVQKATQTGRVFLQLRVAVAMMDQGCREILTTAGPFTVLVPSVSSF
SSRTMNASLAQQLCRQHIIAGQHILEDTRTQQTRRWWTLAGQEITVTFNQFTKYSYKYK
DQPQQTFNIYKANNIAANGVFHVVTGLRWQAPSGTPGDPKRTIGQILASTEAFSRFETIL
ENCGLPSILDGPGPFTVFAPSNEAVDSLRDGRLIYLFTAGLSKLQELVRYHIYNHGQLTV
EKLISKGRILTMANQVLAVNISEEGRILLGPEGVPLQRVDVMAANGVIHMLDGILLPPTIL
PILPKHCSEEQHKIVAGSCVDCQALNTSTCPPNSVKLDIFPKECVYIHDPTGLNVLKKGC
ASYCNQTIMEQGCCKGFFGPDCTQCPGGFSNPCYGKGNCSDGIQGNGACLCFPDYKGIA
CHICSNPNKHGEQCQEDCGCVHGLCDNRPGSGGVCQQGTCAPGFSGRFCNESMGDCGP
TGLAQHCHLHARCVSQEGVARCRCLDGFEGDGFSCTPSNPCSHPDRGGCSENAECVPGS
LGTHHCTCHKGWSGDGRVCVAIDECELDMRGGCHTDALCSYVGPGQSRCTCKLGFAG
DGYQCSPIDPCRAGNGGCHGLATCRAVGGGQRVCTCPPGFGGDGFSCYGDIFRELEAN
AHFSIFYQWLKSAGITLPADRRVTALVPSEAAVRQLSPEDRAFWLQPRTLPNLVRAHFL
QGALFEEELARLGGQEVATLNPTTRWEIRNISGRVWVQNASVDVADLLATNGVLHILSQ
VLLPPRGDVPGGQGLLQQLDLVPAFSLFRELLQHHGLVPQIEAATAYTIFVPTNRSLEAQ
GNSSHLDADTVRHHVVLGEALSMETLRKGGHRNSLLGPAHWIVFYNHSGQPEVNHVPL
EGPMLEAPGRSLIGLSGVLTVGSSRCLHSHAEALREKCVNCTRRFRCTQGFQLQDTPRK
SCVYRSGFSFSRGCSYTCAKKIQVPDCCPGFFGTLCEPCPGGLGGVCSGHGQCQDRFLGS
GECHCHEGFHGTACEVCELGRYGPNCTGVCDCAHGLCQEGLQGDGSCVCNVGWQGL
RCDQKITSPQCPRKCDPNANCVQDSAGASTCACAAGYSGNGIFCSEVDPCAHGHGGCSP
HA
NCTKVAPGQRTCTCQDGYMGDGELCQEINSCLIHHGGCHIHAECIPTGPQQVSCSCREG
YSGDGIRTCELLDPCSKNNGGCSPYATCKSTGDGQRTCTCDTAHTVGDGLTCRARVGL
ELLRDKHASFFSLRLLEYKELKGDGPFTIFVPHADLMSNLSQDELARIRAHRQLVFRYHV
VGCRRLRSEDLLEQGYATALSGHPLRFSEREGSIYLNDFARVVSSDHEAVNGILHFIDRV
LLPPEALHWEPDDAPIPRRNVTAAAQGFGYKIFSGLLKVAGLLPLLREASHRPFTMLWP
TDAAFRALPPDRQAWLYHEDHRDKLAAILRGHMIRNVEALASDLPNLGPLRTMHGTPIS
FSCSRTRAGELMVGEDDARIVQRHLPFEGGLAYGIDQLLEPPGLGARCDHFETRPLRLNT
CSICGLEPPCPEGSQEQGSPEACWRFYPKFWTSPPLHSLGLRSVWVHPSLWGRPQGLGR
GCHRNCVTTTWKPSCCPGHYGSECQACPGGPSSPCSDRGVCMDGMSGSGQCLCRSGFA
GTACELCAPGAFGPHCQACRCTVHGRCDEGLGGSGSCFCDEGWTGPRCEVQLELQPVC
TPPCAPEAVCRAGNSCECSLGYEGDGRVCTVADLCQDGHGGCSEHANCSQVGTMVTC
 
TCLPDYEGDGWSCRARNPCTDGHRGGCSEHANCLSTGLNTRRCECHAGYVGDGLQCL
EESEPPVDRCLGQPPPCHSDAMCTDLHFQEKRAGVFHLQATSGPYGLNFSEAEAACEAQ
GAVLASFPQLSAAQQLGFHLCLMGWLANGSTAHPVVFPVADCGNGRVGIVSLGARKN
LSERWDAYCFRVQDVACRCRNGFVGDGISTCNGKLLDVLAATANFSTFYGMLLGYAN
ATQRGLDFLDFLDDELTYKTLFVPVNEGFVDNMTLSGPDLELHASNATLLSANASQGKL
LPAHSGLSLIISDAGPDNSSWAPVAPGTVVVSRIIVWDIMAFNGIIHALASPLLAPPQPQA
VLAPEAPPVAAGVGAVLAAGALLGLVAGALYLRARGKPMGFGFSAFQAEDDADDDFS
PWQEGTNPTLVSVPNPVFGSDTFCEPFDDSLLEEDFPDTQRILTVK
**SEQ ID NO:61**
   **platelet endothelial cell adhesion molecule (CD31), accession number; Gene: NP_000433, CD31** 
MQPRWAQGATMWLGVLLTLLLCSSLEGQENSFTINSVDMKSLPDWTVQNGKNLTLQC
FADVSTTSHVKPQHQMLFYKDDVLFYNISSMKSTESYFIPEVRIYDSGTYKCTVIVNNKE
KTTAEYQVLVEGVPSPRVTLDKKEAIQGGIVRVNCSVPEEKAPIHFTIEKLELNEKMVKL
KREKNSRDQNFVILEFPVEEQDRVLSFRCQARIISGIHMQTSESTKSELVTVTESFSTPKFH
ISPTGMIMEGAQLHIKCTIQVTHLAQEFPEIIIQKDKAIVAHNRHGNKAVYSVMAMVEHS
GNYTCKVESSRISKVSSIVVNITELFSKPELESSFTHLDQGERLNLSCSIPGAPPANFTIQKE
DTIVSQTQDFTKIASKSDSGTYICTAGIDKVVKKSNTVQIVVCEMLSQPRISYDAQFEVIK
GQTIEVRCESISGTLPISYQLLKTSKVLENSTKNSNDPAVFKDNPTEDVEYQCVADNCHS
HAKMLSEVLRVKVIAPVDEVQISILSSKVVESGEDIVLQCAVNEGSGPITYKFYREKEGK
PFYQMTSNATQAFWTKQKASKEQEGEYYCTAFNRANHASSVPRSKILTVRVILAPWKK
GLIAVVIIGVIIALLIIAAKCYFLRKAKAKQMPVEMSRPAVPLLNSNNEKMSDPNMEANS
HYGHNDDVRNHAMKPINDNKEPLNSDVQYEVQVSSAESHKDLGKKDTETVYSEVRKA
VPDAVESRYSRTEGSLDGT
**SEQ ID NO:62**
   **glial fibrillary acidic protein (GFAP), accession number: AAB22581; Gene: GFAP** 
MERRRITSAARRSYVSSGEMMVGGLAPGRRLGPGTRLSLARMPPPLPTRVDFSLAGALN
AGFKETRASERAEMMELNDRFASYIEKVRFLEQQNKALAAELNQLRAKEPTKLADVYQ
AELRELRLRLDQLTANSARLEVERDNLAQDLATVRQKLQDETNLRLEAENNLAAYRQE
ADEATLARLDLERKIESLEEEIRFLRKIHEEEVRELQEQLARQQVHVELDVAKPDLTAAL
KEIRTQYEAMASSNMHEAEEWYRSKFADLTDAAARNAELLRQAKHEANDYRRQLQSL
TCDLESLRGTNESLERQMREQEERHVREAASYQEALARLEEEGQSKDEMARHLQEYQD
LLNVKLALDIEIATYRKLLEGEENRITIPVQTFSNLQIRETSLDTKSVSEGHLKRNIVVKTV
EMRDGEVIKESKQEHKDVM
**SEQ ID NO:63**
   **Vimentin (VIM), accession number: AAH66956; Gene: VIM** 
MSTRSVSSSSYRRMFGGPGTASRPSSSRSYVTTSTRTYSLGSALRPSTSRSLYASSPGGVY
ATRSSAVRLRSSVPGVRLLQDSVDFSLADAINTEFKNTRTNEKVELQELNDRFANYIDK
 
VRFLEQQNKILLAELEQLKGQGKSRLGDLYEEEMRELRRQVDQLTNDKARVEVERDNL
AEDIMRLREKLQEEMLQREEAENTLQSFRQDVDNASLARLDLERKVESLQEEIAFLKKL
HEEEIQELQAQIQEQHVQIDVDVSKPDLTAALRDVRQQYESVAAKNLQEAEEWYKSKF
ADLSEAANRNNDALRQAKQESTEYRRQVQSLTCEVDALKGTNESLERQMREMEENFA
VEAANYQDTIGRLQDEIQNMKEEMARHLREYQDLLNVKMALDIEIATYRKLLEGEESRI
SLPLPNFSSLNLRETNLDSLPLVDTHSKRTLLIKTVETRDGQVINETSQHHDDLE
**SEQ ID NO:64**
   **LIM/homeobox protein Lhx2, accession number: NP_004780; Gene: LHX2** 
MLFHSLSGPEVHGVIDEMDRRAKSEAPAISSAIDRGDTETTMPSISSDRAALCAGCGGKI
SDRYYLLAVDKQWHMRCLKCCECKLNLESELTCFSKDGSIYCKEDYYRRFSVQRCARC
HLGISASEMVMRARDLVYHLNCFTCTTCNKMLTTGDHFGMKDSLVYCRLHFEALLQG
EYPAHFNHADVAAAAAAAAAAKSAGLGAAGANPLGLPYYNGVGTVQKGRPRKRKSP
GPGADLAAYNAALSCNENDAEHLDRDQPYPSSQKTKRMRTSFKHHQLRTMKSYFAINH
NPDAKDLKQLAQKTGLTKRVLQVWFQNARAKFRRNLLRQENTGVDKSTDAALQTGTP
SGPASELSNASLSPSSTPTTLTDLTSPTLPTVTSVLTSVPGNLEGHEPHSPSQTTLTNLF
**SEQ ID NO:65**
   **lecithin retinol acyltransferase (LRAT), accession number: AAD13529; Gene: LRAT** 
MKNPMLEVVSLLLEKLLLISNFTLFSSGAAGKDKGRNSFYETSSFHRGDVLEVPRTHLTH
YGIYLGDNRVAHMMPDILLALTDDMGRTQKVVSNKRLILGVIVKVASIRVDTVEDFAY
GANILVNHLDESLQKKALLNEEVARRAEKLLGFTPYSLLWNNCEHFVTYCRYGTPISPQ
SDKFCETVKIIIRDQRSVLASAVLGLASIVCTGLVSYTTLPAIFIPFFLWMAG
**SEQ ID NO:66**
   **platelet-derived growth factor receptor beta (PDGFRβ), accession number: NP _002600; Gene: PDGFRβ**
**SEQ ID NO:67**
   **Heart and neural crest derivatives-expressed protein 2 (HAND2), accession number: NP_068808, HAND2** 
MSLVGGFPHHPVVHHEGYPFAAAAAAAAAAAASRCSHEENPYFHGWLIGHPEMSPPDY
SMALSYSPEYASGAAGLDHSHYGGVPPGAGPPGLGGPRPVKRRGTANRKERRRTQSINS
AFAELRECIPNVPADTKLSKIKTLRLATSYIAYLMDLLAKDDQNGEAEAFKAEIKKTDVK
EEKRKKELNEILKSTVSSNDKKTKGRTGWPQHVWALELKQ
**SEQ ID NO:68**
   **intercellular adhesion molecule 1 (ICAM1), accession number: NP_000192; Gene: ICAM1** 
MAPSSPRPALPALLVLLGALFPGPGNAQTSVSPSKVILPRGGSVLVTCSTSCDQPKLLGIE
TPLPKKELLLPGNNRKVYELSNVQEDSQPMCYSNCPDGQSTAKTFLTVYWTPERVELAP
LPSWQPVGKNLTLRCQVEGGAPRANLTVVLLRGEKELKREPAVGEPAEVTTTVLVRRD
HHGANFSCRTELDLRPQGLELFENTSAPYQLQTFVLPATPPQLVSPRVLEVDTQGTVVCS
LDGLFPVSEAQVHLALGDQRLNPTVTYGNDSFSAKASVSVTAEDEGTQRLTCAVILGNQ
SQETLQTVTIYSFPAPNVILTKPEVSEGTEVTVKCEAHPRAKVTLNGVPAQPLGPRAQLL
LKATPEDNGRSFSCSATLEVAGQLIHKNQTRELRVLYGPRLDERDCPGNWTWPENSQQ
TPMCQAWGNPLPELKCLKDGTFPLPIGESVTVTRDLEGTYLCRARSTQGEVTRKVTVNV
LSPRYEIVIITVVAAAVIMGTAGLSTYLYNRQRKIKKYRLQQAQKGTPMKPNTQATPP
**SEQ ID NO:69**
   **vascular cell adhesion protein 1 (VCAM1), accession number: NP_001069; Gene: VCAM1** 
MPGKMVVILGASNILWIMFAASQAFKIETTPESRYLAQIGDSVSLTCSTTGCESPFFSWRT
QIDSPLNGKVTNEGTTSTLTMNPVSFGNEHSYLCTATCESRKLEKGIQVEIYSFPKDPEIH
LSGPLEAGKPITVKCSVADVYPFDRLEIDLLKGDHLMKSQEFLEDADRKSLETKSLEVTF
TPVIEDIGKVLVCRAKLHIDEMDSVPTVRQAVKELQVVYISPKNTVISVNPSTKLQEGGSV
TMTCSSEGLPAPEIFWSKKLDNGNLQHLSGNATLTLIAMRMEDSGIYVCEGVNLIGKNR
KEVELIVQEKPFTVEISPGPRIAAQIGDSVMLTCSVMGCESPSFSWRTQIDSPLSGKVRSE
GTNSTLTLSPVSFENEHSYLCTVTCGHKKLEKGIQVELYSFPRDPEIEMSGGLVNGSSVT
 
VSCKVPSVYPLDRLEIELLKGETILENIEFLEDTDMKSLENKSLEMTFIPTIEDTGKALVCQ
AKLHIDDMEFEPKQRQSTQTLYVNVAPRDTTVLVSPSSILEEGSSVNMTCLSQGFPAPKI
LWSRQLPNGELQPLSENATLTLISTKMEDSGVYLCEGINQAGRSRKEVELIIQVTPKDIKL
TAFPSESVKEGDTVIISCTCGNVPETWIILKKKAETGDTVLKSIDGAYTIRKAQLKDAGV
YECESKNKVGSQLRSLTLDVQGRENNKDYFSPELLVLYFASSLIIPAIGMIIYFARKANM
KGSYSLVEAQKSKV
**SEQ ID NO:70**
   **neural cell adhesion molecule 1 (NCAM1), accession number: NP_000606; Gene: NCAM1** 
MLQTKDLIWTLFFLGTAVSLQVDIVPSQGEISVGESKFFLCQVAGDAKDKDISWFSPNGE
KLTPNQQRISVVWNDDSSSTLTIYNANIDDAGIYKCVVTGEDGSESEATVNVKIFQKLMF
KNAPTPQEFREGEDAVIVCDVVSSLPPTIIWKHKGRDVILKKDVRFIVLSNNYLQIRGIKK
TDEGTYRCEGRILARGEINFKDIQVIVNVPPTIQARQNIVNATANLGQSVTLVCDAEGFPE
PTMSWTKDGEQIEQEEDDEKYIFSDDSSQLTIKKVDKNDEAEYICIAENKAGEQDATIHL
KVFAKPKITYVENQTAMELEEQVTLTCEASGDPIPSITWRTSTRNISS
EEKTLDGHMVVRSHARVSSLTLKSIQYTDAGEYICTASNTIGQDSQSMYLEVQYAPKLQ
GPVAVYTWEGNQVNITCEVFAYPSATISWFRDGQLLPSSNYSNIKIYNTPSASYLEVTPD
SENDFGNYNCTAVNRIGQESLEFILVQADTPSSPSIDQVEPYSSTAQVQFDEPEATGGVPI
LKYKAEWRAVGEEVWHSKWYDAKEASMEGIVTIVGLKPETTYAVRLAALNGKGLGEI
SAASEFKTQPVQGEPSAPKLEGQMGEDGNSIKVNLIKQDDGGSPIRHYLVRYRALSSEW
KPEIRLPSGSDHVMLKSLDWNAEYEVYVVAENQQGKSKAAHFVFRTSAQPTAIPANGSP
TSGLSTGAIVGILIVIFVLLLVVVDITCYFLNKCGLFMCIAVNLCGKAGPGAKGKDMEEG
KAAFSKDESKEPIVEVRTEEERTPNHDGGKHTEPNETTPLTEPEKGPVEAKPECQETETK
PAPAEVKTVPNDATQTKENESKA
**SEQ ID NO:71**
   **collagen alpha-1(I) chain protein (COL1A1), accession number: NP_000079; Gene: COL1A1**
**SEQ ID NO:72**
   **actin, aortic smooth muscle (α-SMA), accession number: NP_001307784; Gene: ACTA2** 
MCEEEDSTALVCDNGSGLCKAGFAGDDAPRAVFPSIVGRPRHQGVMVGMGQKDSYVG
DEAQSKRGILTLKYPIEHGIITNWDDMEKIWHHSFYNELRVAPEEHPTLLTEAPLNPKAN
REKMTQIMFETFNVPAMYVAIQAVLSLYASGRTTGIVLDSGDGVTHNVPIYEGYALPHA
IMRLDLAGRDLTDYLMKILTERGYSFVTTAEREIVRDIKEKLCYVALDFENEMATAASSS
SLEKSYELPDGQVITIGNERFRCPETLFQPSFIGMESAGIHETTYNSIMKCDIDIRKDLYAN
NVLSGGTTMYPGIADRMQKEITALAPSTMKIKIIAPPERKYSVWIGGSILASLSTFQQMWI
SKQEYDEAGPSIVHRKCF
**SEQ ID NO:73**
   **Glucagon (GCG), accession number: AAH05278; Gene: GCG** 
MKSIYFVAGLFVMLVQGSWQRSLQDTEEKSRSFSASQADPLSDPDQMNEDKRHSQGTF
TSDYSKYLDSRRAQDFVQWLMNTKRNRNNIAKRHDEFERHAEGTFTSDVSSYLEGQAA
KEFIAWLVKGRGRRDFPEEVAIVEELGRRHADGSFSDEMNTILDNLAARDFINWLIQTKI
TDRK
**SEQ ID NO:74**
   **Insulin, accession number: AAA59172; Gene: INS**
**SEQ ID NO:75**
   **Somatostatin, accession number: AAH32625; Gene: SST** 
MLSCRLQCALAALSIVLALGCVTGAPSDPRLRQFLQKSLAAAAGKQELAKYFLAELLSE
PNQTENDALEPEDLSQAAEQDEMRLELQRSANSNPAMAPRERKAGCKNFFWKTFTSC
**SEQ ID NO:76**
   **homeobox protein Nkx-6.1, accession number: NP_006159; Gene: NKX6.1** 
MLAVGAMEGTRQSAFLLSSPPLAALHSMAEMKTPLYPAAYPPLPAGPPSSSSSSSSSSSPS
PPLGTHNPGGLKPPATGGLSSLGSPPQQLSAATPHGINDILSRPSMPVASGAALPSASPSG
SSSSSSSSASASSASAAAAAAAAAAAAASSPAGLLAGLPRFSSLSPPPPPPGLYFSPSAAA
VAAVGRYPKPLAELPGRTPIFWPGVMQSPPWRDARLACTPHQGSILLDKDGKRKHTRPT
FSGQQIFALEKTFEQTKYLAGPERARLAYSLGMTESQVKVWFQNRRTKWRKKHAAEM
ATAKKKQDSETERLKGASENEEEDDDYNKPLDPNSDDEKITQLLKKHKSSSGGGGGLLL
HASEPESSS
**SEQ ID NO:77**
   **homeobox protein Nkx-2.2, accession number: NP_002500; Gene: NKX2.2** 
MSLTNTKTGFSVKDILDLPDTNDEEGSVAEGPEEENEGPEPAKRAGPLGQGALDAVQSL
PLKNPFYDSSDNPYTRWLASTEGLQYSLHGLAAGAPPQDSSSKSPEPSADESPDNDKETP
GGGGDAGKKRKRRVLFSKAQTYELERRFRQQRYLSAPEREHLASLIRLTPTQVKIWFQN
HRYKMKRARAEKGMEVTPLPSPRRVAVPVLVRDGKPCHALKAQDLAAATFQAGIPFSA
YSAQSLQHMQYNAQYSSASTPQYPTAHPLVQAQQWTW
**SEQ ID NO:78**
   **neurogenic differentiation factor 1 (NEUROD1), accession number: NP_002491; Gene: NEUROD1** 
MTKSYSESGLMGEPQPQGPPSWTDECLSSQDEEHEADKKEDDLEAMNAEEDSLRNGGE
EEDEDEDLEEEEEEEEEEDDQKPKRRGPKKKKMTKARLERFKLRRMKANARERNRMH
GLNAALDNLRKVVPCYSKTQKLSKIETLRLAKNYIWALSEILRSGKSPDLVSFVQTLCKG
LSQPTTNLVAGCLQLNPRTFLPEQNQDMPPHLPTASASFPVHPYSYQSPGLPSPPYGTMD
SSHVFHVKPPPHAYSAALEPFFESPLTDCTSPSFDGPLSPPLSINGNFSFKHEPSAEFEKNY
AFTMHYPAATLAGAQSHGSIFSGTAAPRCEIPIDNIMSFDSHSHHERVMSAQLNAIFHD
**SEQ ID NO:79**
   **transcription factor MafA (MAFA), accession number: NP_963883; Gene: MAFA** 
MAAELAMGAELPSSPLAIEYVNDFDLMKFEVKKEPPEAERFCHRLPPGSLSSTPLSTPCS
SVPSSPSFCAPSPGTGGGGGAGGGGGSSQAGGAPGPPSGGPGAVGGTSGKPALEDLYW
MSGYQHHLNPEALNLTPEDAVEALIGSGHHGAHHGAHHPAAAAAYEAFRGPGFAGGG
GADDMGAGHHHGAHHAAHHHHAAHHHHHHHHHGGAGHGGGAGHHVRLEERFSD
DQLVSMSVRELNRQLRGFSKEEVIRLKQKRRTLKNRGYAQSCRFKRVQQRHILESEKCQ
LQSQVEQLKLEVGRLAKERDLYKEKYEKLAGRGGPGSAGGAGFPREPSPPQAGPGGAK
GTADFFL
**SEQ ID NO:80**
   **trypsin-1, accession number: NP_002760; Gene: PRSS**
**SEQ ID NO:81**
   **carboxypeptidase A1 (CPA1), accession number: NP_001859; Gene: CPA1**
**SEQ ID NO:82**
   **alpha-amylase, accession number: AAA52279; Gene: AMY1** 
MKLFWLLFTIGFCWAQYSSNTQQGRTSIVHLFEWRWVDIALECERYLAPKGFGGVQVS
PPNENVAIHNPFRPWWERYQPVSYKLCTRSGNEDEFRNMVTRCNNVGVRIYVDAVINH
MCGNAVSAGTSSTCGSYFNPGSRDFPAVPYSGWDFNDGKCKTGSGDIENYNDATQVRD
CRLSGLLDLALGKDYVRSKIAEYMNHLIDIGVAGFRIDASKHMWPGDIKAILDKLHNLN
SNWFPEGSKPFIYQEVIDLGGEPIKSSDYFGNGRVTEFKYGAKLGTVIRKWNGEKMSYL
KNWGEGWGFMPSDRALVFVDNHDNQRGHGAGGASILTFWDARLYKMAVGFMLAHP
YGFTRVMSSYRWPRYFENGXDVNDWVGPPNDNGVTKEVTINPDTTCGNDWVCEHRW
RQIRNMVNFRNVVDGQPFTNWYDNGSNQVAFGRGNRGFIVFNNDDWTFSLTLQTGLP
AGTYCDVISGDKINGNCTGIKIYVSDDGKAHFSISNSAEDPFIAIHAESKL
**SEQ ID NO:83**
   **chymotrypsin-C, accession number; Gene: NP_009203,**
**SEQ ID NO:84**
   **villin-1, accession number: NP_009058; Gene: Villin-1** 
MTKLSAQVKGSLNITTPGLQIWRIEAMQMVPVPSSTFGSFFDGDCYIILAIHKTASSLSYD
IHYWIGQDSSLDEQGAAAIYTTQMDDFLKGRAVQHREVQGNESEAFRGYFKQGLVIRK
GGVASGMKHVETNSYDVQRLLHVKGKRNVVAGEVEMSWKSFNRGDVFLLDLGKLIIQ
WNGPESTRMERLRGMTLAKEIRDQERGGRTYVGVVDGENELASPKLMEVMNHVLGKR
RELKAAVPDTVVEPALKAALKLYHVSDSEGNLVVREVATRPLTQDLL
SHEDCYILDQGGLKIYVWKGKKANEQEKKGAMSHALNFIKAKQYPPSTQVEVQNDGA
ESAVFQQLFQKWTASNRTSGLGKTHTVGSVAKVEQVKFDATSMHVKPQVAAQQKMV
DDGSGEVQVWRIENLELVPVDSKWLGHFYGGDCYLLLYTYLIGEKQHYLLYVWQGSQ
ASQDEITASAYQAVILDQKYNGEPVQIRVPMGKEPPHLMSIFKGRMVVYQGGTSRTNNL
ETGPSTRLFQVQGTGANNTKAFEVPARANFLNSNDVFVLKTQSCCYLWCGKGCSGDER
EMAKMVADTISRTEKQVVVEGQEPANFWMALGGKAPYANTKRLQEENLVITPRLFECS
NKTGRFLATEIPDFNQDDLEEDDVFLLDVWDQVFFWIGKHANEEEKKAAATTAQEYLK
THPSGRDPETPIIVVKQGHEPPTFTGWFLAWDPFKWSNTKSYEDLKAELGNSRDWSQIT
AEVTSPKVDVFNANSNLSSGPLPIFPLEQLVNKPVEELPEGVDPSRKEEHLSIEDFTQAFG
MTPAAFSALPRWKQQNLKKEKGLF
**SEQ ID NO:85**
   **CCAAT/enhancer-binding protein, accession number: NP_001274353; Gene: CEBP** 
MRGRGRAGSPGGRRRRPAQAGGRRGSPCRENSNSPMESADFYEAEPRPPMSSHLQSPPH
APSSAAFGFPRGAGPAQPPAPPAAPEPLGGICEHETSIDISAYIDPAAFNDEFLADLFQHSR
QQEKAKAAVGPTGGGGGGDFDYPGAPAGPGGAVMPGGAHGPPPGYGCAAAGYLDGR
LEPLYERVGAPALRPLVIKQEPREEDEAKQLALAGLFPYQPPPPPPPSHPHPHPPPAHLAA
PHLQFQIAHCGQTTMHLQPGHPTPPPTPVPSPHPAPALGAAGLPGPGSALKGLGAAHPD
LRASGGSGAGKAKKSVDKNSNEYRVRRERNNIAVRKSRDKAKQRNVETQQKVLELTS
DNDRLRKRVEQLSRELDTLRGIFRQLPESSLVKAMGNCA
**SEQ ID NO:86**
   **keratin, type II cytoskeletal 8 (KRT8), accession number: NP_001243211; Gene: KRT8** 
MNGVSWSQDLQEGISAWFGPPASTPASTMSIRVTQKSYKVSTSGPRAFSSRSYTSGPGSR
ISSSSFSRVGSSNFRGGLGGGYGGASGMGGITAVTVNQSLLSPLVLEVDPNIQAVRTQEK
EQIKTLNNKFASFIDKVRFLEQQNKMLETKWSLLQQQKTARSNMDNMFESYINNLRRQ
LETLGQEKLKLEAELGNMQGLVEDFKNKYEDEINKRTEMENEFVLIKKDVDEAYMNKV
ELESRLEGLTDEINFLRQLYEEEIRELQSQISDTSVVLSMDNSRSLDMDSIIAEVKAQYEDI
ANRSRAEAESMYQIKYEELQSLAGKHGDDLRRTKTEISEMNRNISRLQAEIEGLKGQRA
SLEAAIADAEQRGELAIKDANAKLSELEAALQRAKQDMARQLREYQELMNVKLALDIEI
ATYRKLLEGEESRLESGMQNMSIHTKTTSGYAGGLSSAYGGLTSPGLSYSLGSSFGSGA
GSSSFSRTSSSRAVVVKKIETRDGKLVSESSDVLPK
**SEQ ID NO:87**
   **NOTCH2, accession number: AAG37073; Gene: NOTCH2** 
MPALRPALLWALLALWLCCATPAHALQCRDGYEPCVNEGMCVTYHNGTGYCKCPEGF
LGEYCQHRDPCEKNRCQNGGTCVAQAMLGKATCRCASGFTGEDCQYSTSHPCFVSRPC
LNGGTCHMLSRDTYECTCQVGFTGKECQWTDACLSHPCANGSTCTTVANQFSCKCLTG
FTGQKCETDVNECDIPGHCQHGGTCLNLPGSYQCQCLQGFTGQYCDSLYVPCAPSPCVN
GGTCRQTGDFTFECNCLPGFEGSTCERNIDDCPNHRCQNGGVCVDGVNTYNCRCPPQW
TGQFCTEDVDECLLQPNACQNGGTCANRNGGYGCVCVNGWSGDDCSENIDDCAFASC
TPGSTCIDRVASFSCMCPEGKAGLLCHLDDACISNPCHKGALCDTNPLNGQYICTCPQG
YKGADCTEDVDECAMANSNPCEHAGKCVNTDGAFHCECLKGYAGPRCEMDINECHSD
PCQNDATCLDKIGGFTCLCMPGFKGVHCELEINECQSNPCVNNGQCVDKVNRFQCLCPP
GFTGPVCQIDIDDCSSTPCLNGAKCIDHPNGYECQCAT
GFTGVLCEENIDNCDPDPCHHGQCQDGIDSYTCICNPGYMGAICSDQIDECYSSPCLNDG
RCIDLVNGYQCNCQPGTSGVNCEINFDDCASNPCIHGICMDGINRYSCVCSPGFTGQRCN
IDIDECASNPCRKGATCINGVNGFRCICPEGPHHPSCYSQVNECLSNPCIHGNCTGGLSGY
KCLCDAGWVGINCEVDKNECLSNPCQNGGTCDNLVNGYRCTCKKGFKGYNCQVNIDE
CASNPCLNQGTCFDDISGYTCHCVLPYTGKNCQTVLAPCSPNPCENAAVCKESPNFESY
TCLCAPGWQGQRCTIDIDECISKPCMNHGLCHNTQGSYMCECPPGFSGMDCEEDIDDCL
ANPCQNGGSCMDGVNTFSCLCLPGFTGDKCQTDMNECLSEPCKNGGTCSDYVNSYTCK
CQAGFDGVHCENNINECTESSCFNGGTCVDGINSFSCLCPVGFTGSFCLHEINECSSHPCL
NEGTCVDGLGTYRCSCPLGYTGKNCQTLVNLCSRSPCKNKGTCVQKKAESQCLCPSGW
AGAYCDVPNVSCDIAASRRGVLVEHLCQHSGVCINAGNTHYCQCPLGYTGSYCEEQLD
ECASNPCQHGATCSDFIGGYRCECVPGYQGVNCEYEVDECQNQPCQNGGTCIDLVNHF
KCSCPPGTRGLLCEENIDDCARGPHCLNGGQCMDRIGGYSCRCLPGFAGERCEGDINEC
LSNPCSSEGSLDCIQLTNDYLCVCRSAFTGRHCETFVDVCPQMPCLNGGTCAVASNMPD
GFICRCPPGFSGARCQSSCGQVKCRKGEQCVHTASGPRCFCPSPRDCESGCASSPCQHGG
SCHPQRQPPYYSCQCAPPFSGSRCELYTAPPSTPPATCLSQYCADKARDGVCDEACNSH
ACQWDGGDCSLTMENPWANCSSPLPCWDYINNQCDELCNTVECLFDNFECQGNSKTC
KYDKYCADHFKDNHCDQGCNSEECGWDGLDCAADQPENLAEGTLVIVVLMPPEQLLQ
DARSFLRALGTLLHTNLRIKRDSQGELMVYPYYGEKSAAMKKQRMTRRSLPGEQEQEV
AGSKVFLEIDNRQCVQDSDHCFKNTDAAAALLASHAIQGTLSYPLVSVVSESLTPERTQL
LYLLAVAVVIILFIILLGVIMAKRKRKHGSLWLPEGFTLRRDASNHKRREPVGQDAVGLK
NLSVQVSEANLI
GTGTSEHWVDDEGPQPKKVKAEDEALLSEEDDPIDRRPWTQQHLEAADIRRTPSLALTP
PQAEQEVDVLDVNVRGPDGCTPLMLASLRGGSSDLSDEDEDAEDSSANIITDLVYQGAS
LQAQTDRTGEMALHLAARYSRADAAKRLLDAGADANAQDNMGRCPLHAAVAADAQ
GVFQILIRNRVTDLDARMNDGTTPLILAARLAVEGMVAELINCQADVNAVDDHGKSAL
HWAAAVNNVEATLLLLKNGANRDMQDNKEETPLFLAAREGSYEAAKILLDHFANRDIT
DHMDRLPRDVARDHMHHDIVRLLDEYNVTPSPPGTVLTSALSPVICGPNRSFLSLKHTP
MGKKSRRPSAKSTMPTSLPNLAKEAKDAKGSRRKKSLSEKVQLSESSVTLSPVDSLESP
HTYVSDTTSSPMITSPGILQASPNPMLATAAPPAPVHAQHALSFSNLHEMQPLAHGASTV
LPSVSQLLSHHHIVSPGSGSAGSLSRLHPVPVPADWMNRMEVNETQYNEMFGMVLAPA
EGTHPGIAPQSRPPEGKHITTPREPLPPIVTFQLIPKGSIAQPAGAPQPQSTCPPAVAGPLPT
MYQIPEMARLPSVAFPTAMMPQQDGQVAQTILPAYHPFPASVGKYPTPPSQHSYASSNA
AERTPSHSGHLQGEHPYLTPSPESPDQWSSSSPHSASDWSDVTTSPTPGGAGGGQRGPGT
HMSEPPHNNMQVYA
**SEQ ID NO:88**
   **cystic fibrosis transmembrane conductance regulator (CFTR), accession number: NP_000483; Gene: CFTR** 
MQRSPLEKASVVSKLFFSWTRPILRKGYRQRLELSDIYQIPSVDSADNLSEKLEREWDRE
LASKKNPKLINALRRCFFWRFMFYGIFLYLGEVTKAVQPLLLGRIIASYDPDNKEERSIAI
YLGIGLCLLFIVRTLLLHPAIFGLHHIGMQMRIAMFSLIYKKTLKLSSRVLDKISIGQLVSL
LSNNLNKFDEGLALAHFVWIAPLQVALLMGLIWELLQASAFCGLGFLIVLALFQAGLGR
MMMKYRDQRAGKISERLVITSEMIENIQSVKAYCWEEAMEKMIENLRQTELKLTRKAA
YVRYFNSSAFFFSGFFVVFLSVLPYALIKGIILRKIFTTISFCIVLRMAV
TRQFPWAVQTWYDSLGAINKIQDFLQKQEYKTLEYNLTTTEVVMENVTAFWEEGFGEL
FEKAKQNNNNRKTSNGDDSLFFSNFSLLGTPVLKDINFKIERGQLLAVAGSTGAGKTSLL
MVIMGELEPSEGKIKHSGRISFCSQFSWIMPGTIKENIIFGVSYDEYRYRSVIKACQLEEDI
SKFAEKDNIVLGEGGITLSGGQRARISLARAVYKDADLYLLDSPFGYLDVLTEKEIFESC
VCKLMANKTRILVTSKMEHLKKADKILILHEGSSYFYGTFSELQNLQPDFSSKLMGCDSF
DQFSAERRNSILTETLHRFSLEGDAPVSWTETKKQSFKQTGEFGEKRKNSILNPINSIRKF
SIVQKTPLQMNGIEEDSDEPLERRLSLVPDSEQGEAILPRISVISTGPTLQARRRQSVLNLM
THSVNQGQNIHRKTTASTRKVSLAPQANLTELDIYSRRLSQETGLEISEEINEEDLKECFF
DDMESIPAVTTWNTYLRYITVHKSLIFVLIWCLVIFLAEVAASLVVLWLLGNTPLQDKG
NSTHSRNNSYAVIITSTSSYYVFYIYVGVADTLLAMGFFGLPLVHTLITVSKILHHKMLHS
VLQAPMSTLNTLKAGGILNRFSKDIAILDDLLPLTIFDFIQLLLIVIGAIAVVAVLQPYIFVA
TVPVIVAFIMLRAYFLQTSQQLKQLESEGRSPIFTHLVTSLKGLWTLRAFGRQPYFETLFH
KALNLHTANWFLYLSTLRWFQMRIEMIFVIFFIAVTFISILTTGEGEGRVGIILTLAMNIMS
TLQWAVNSSIDVDSLMRSVSRVFKFIDMPTEGKPTKSTKPYKNGQLSKVMIIENSHVKK
DDIWPSGGQMTVKDLTAKYTEGGNAILENISFSISPGQRVGLLGRTGSGKSTLLSAFLRL
LNTEGEIQIDGVSWDSITLQQWRAFGVIPQKVFIFSGTFRKNLDPYEQWSDQEIWKVADE
VGLRSVIEQFPGKLDFVLVDGGCVLSHGHKQLMCLARSVLSKAKILLLDEPSAHLDPVT
YQIIRRTLKQAFADCTVILCEHRIEAMLECQQFLVIEENKVRQYDSIQKLLNERSLFRQAI
SPSDRVKLFPHRNSSKCKSKPQIAALKEETEEEVQDTRL
**SEQ ID NO:89**
   **UDP glucuronosyltransferase family 1 member A1 (UGT1A1), accession number: NP_000454; Gene: UGT1A1** 
MAVESQGGRPLVLGLLLCVLGPVVSHAGKILLIPVDGSHWLSMLGAIQQLQQRGHEIVV
LAPDASLYIRDGAFYTLKTYPVPFQREDVKESFVSLGHNVFENDSFLQRVIKTYKKIKKD
SAMLLSGCSHLLHNKELMASLAESSFDVMLTDPFLPCSPIVAQYLSLPTVFFLHALPCSL
EFEATQCPNPFSYVPRPLSSHSDHMTFLQRVKNMLIAFSQNFLCDVVYSPYATLASEFLQ
REVTVQDLLSSASVWLFRSDFVKDYPRPIMPNMVFVGGINCLHQNPLSQEFEAYINASG
EHGIVVFSLGSMVSEIPEKKAMAIADALGKIPQTVLWRYTGTRPSNLANNTILVKWLPQ
NDLLGHPMTRAFITHAGSHGVYESICNGVPMVMMPLFGDQMDNAKRMETKGAGVTLN
VLEMTSEDLENALKAVINDKSYKENIMRLSSLHKDRPVEPLDLAVFWVEFVMRHKGAP
HLRPAAHDLTWYQYHSLDVIGFLLAVVLTVAFITFKCCAYGYRKCLGKKGRVKKAHKS
KTH
**SEQ ID NO:90**
   **UDP glucuronosyltransferase family 2 member B15 (UGT2B15), accession number: NP_001067; Gene: UGT2B15** 
MSLKWTSVFLLIQLSCYFSSGSCGKVLVWPTEYSHWINMKTILEELVQRGHEVTVLTSS
ASTLVNASKSSAIKLEVYPTSLTKNYLEDSLLKILDRWIYGVSKNTFWSYFSQLQELCWE
YYDYSNKLCKDAVLNKKLMMKLQESKFDVILADALNPCGELLAELFNIPFLYSLRFSVG
YTFEKNGGGFLFPPSYVPVVMSELSDQMIFMERIKNMIHMLYFDFWFQIYDLKKWDQF
YSEVLGRPTTLFETMGKAEMWLIRTYWDFEFPRPFLPNVDFVGGLHCKPAKPLPKEMEE
FVQSSGENGIVVFSLGSMISNMSEESANMIASALAQIPQKVLWRFDGKKPNTLGSNTRLY
KWLPQNDLLGHPKTKAFITHGGTNGIYEAIYHGIPMVGIPLFADQHDNIAHMKAKGAAL
SVDIRTMSSRDLLNALKSVINDPVYKENVMKLSRIHHDQPMKPLDRAVFWIEFVMRHK
GAKHLRVAAHNLTWIQYHSLDVIAFLLACVATVIFIITKFCLFCFRKLAKKGKKKKRD
**SEQ ID NO:91**
   **UDP glucuronosyltransferase family 2 member B7 (UGT2B7), accession number: NP_001065; Gene: UGT2B7** 
MSVKWTSVILLIQLSFCFSSGNCGKVLVWAAEYSHWMNIKTILDELIQRGHEVTVLASS
ASILFDPNNSSALKIEIYPTSLTKTELENFIMQQIKRWSDLPKDTFWLYFSQVQEIMSIFGD
ITRKFCKDVVSNKKFMKKVQESRFDVIFADAIFPCSELLAELFNIPFVYSLSFSPGYTFEK
HSGGFIFPPSYVPVVMSELTDQMTFMERVKNMIYVLYFDFWFEIFDMKKWDQFYSEVL
GRPTTLSETMGKADVWLIRNSWNFQFPYPLLPNVDFVGGLHCKPAKPLPKEMEDFVQS
SGENGVVVFSLGSMVSNMTEERANVIASALAQIPQKVLWRFDGNKPDTLGLNTRLYKW
IPQNDLLGHPKTRAFITHGGANGIYEAIYHGIPMVGIPLFADQPDNIAHMKARGAAVRVD
FNTMSSTDLLNALKRVINDPSYKENVMKLSRIQHDQPVKPLDAVFWIEFVMRHKGAKH
LRVAAHDLTWFQYHSLDVIGFLLVCVATVIFIVTKCCLFCFWKFARKAKKGKND
**SEQ ID NO:92**
   **sodium/bile acid cotransporter (NTCP), accession number: NP_003040; Gene: NTCP** 
MEAHNASAPFNFTLPPNFGKRPTDLALSVILVFMLFFIMLSLGCTMEFSKIKAHLWKPKG
LAIALVAQYGIMPLTAFVLGKVFRLKNIEALAILVCGCSPGGNLSNVFSLAMKGDMNLSI
VMTTCSTFCALGMMPLLLYIYSRGIYDGDLKDKVPYKGIVISLVLVLIPCTIGIVLKSKRP
QYMRYVIKGGMIIILLCSVAVTVLSAINVGKSIMFAMTPLLIATSSLMPFIGFLLGYVLSA
LFCLNGRCRRTVSMETGCQNVQLCSTILNVAFPPEVIGPLFFFPLLYMIFQLGEGLLLIAIF
WCYEKFKTPKDKTKMIYTAATTEETIPGALGNGTYKGEDCSPCTA
**SEQ ID NO:93**
   **solute carrier organic anion transporter family member 1B3 (OATP1B3), accession number: NP_062818; Gene: OATP1B3**

MDQHQHLNKTAESASSEKKKTRRCNGFKMFLAALSFSYIAKALGGIIMKISITQIERRFDI
SSSLAGLIDGSFEIGNLLVIVFVSYFGSKLHRPKLIGIGCLLMGTGSILTSLPHFFMGYYRY

SKETHINPSENSTSSLSTCLINQTLSFNGTSPEIVEKDCVKESGHMWIYVFMGNMLRGIG
ETPIVPLGISYIDDFAKEGHSSLYLGSLNAIGMIGPVIGFALGSLFAKMYVDIGYVDLSTIR
ITPKDSRWVGAWWLGFLVSGLFSIISSIPFFFLPKNPNKPQKERKISLSLHVLKTNDDRNQ
TANLTNQGKNVTKNVTGFFQSLKSILTNPLYVIFLLLTLLQVSSFIGSFTYVFKYMEQQY
GQSASHANFLLGIITIPTVATGMFLGGFIIKKFKLSLVGIAKFSFLTSMISFLFQLLYFPLICE
SKSVAGLTLTYDGNNSVASHVDVPLSYCNSECNCDESQWEPVCGNNGITYLSPCLAGC
KSSSGIKKHTVFYNCSCVEVTGLQNRNYSAHLGECPRDNTCTRKFFIYVAIQVINSLFSA
TGGTTFILLTVKIVQPELKALAMGFQSMVIRTLGGILAPIYFGALIDKTCMKWSTNSCGA
QGACRIYNSVFFGRVYLGLSIALRFPALVLYIVFIFAMKKKFQGKDTKASDNERKVMDE
ANLEFLNNGEHFVPSAGTDSKTCNLDMQDNAAAN

## Claims

1. A liver organoid comprising at least two cell types selected from the group consisting of hepatocytes and cholangiocytes, wherein
the hepatocytes
a) express albumin (ALB);
b) express the hepatocyte markers HNF4a (NCBI: 3172), FAH (NCBI: 2184), TAT (NCBI: 6898), GCK (NCBI: 2645), TTR (NCBI: 7276), MET (NCBI: 4233), GLU1/MGAM (NCBI: 8972), FAHD2A (NCBI: 51011), HNF1B (NCBI: 6928), HNF1A (NCBI: 6927), CYP3A4 (NCBI: 1576), CYP2C9 (NCBI: 1559), CYP2C19 (NCBI: 1557), CYP1A2 (NCBI: 1544), CYP2E1 (NCBI: 1571), CYP2D6 (NCBI: 1565), CYP3A7 (NCBI: 1551), CYP1A1 (NCBI: 1543), CYP3A5 (NCBI: 1577), CYP27A1 (NCBI: 1593), MRP2 (NCBI:1244), NTCP (NCBI: 6554), OATP1B3 (NCBI: 28234), UGT2B7 (NCBI: 7364), UGT2B15 (NCBI: 7366), UGT1A1 (NCBI: 54658), CEBP (NCBI: 1050), KRT8 (NCBI: 3856), NOTCH2 (NCBI: 4853) and CYP2B6 (NCBI: 1555); and
c) do not express the cholangiocyte marker Cytokeratin 7 (CK7); and
d) the organoids are capable of performing liver functions and exhibit a spatially organized structure observed in liver.

2. The liver organoid of claim 1 further comprising at least one cell type selected from the group consisting of liver specific endothelial cells (LSEC), stellate cells, hepatic myofibroblast and hepatoblasts, wherein:
the cholangiocytes express CK7 but do not express albumin (ALB); or
the hepatoblasts express at least one marker selected from the group consisting of SOX9 (NCBI: 6662), CK19 (NCBI: 3880), CK18 (NCBI: 3875), HNF4a (NCBI: 3172), PROX1 (NCBI: 5629), ONECUT1 (NCBI: 3175), AFP (NCBI: 174), and ALB (NCBI: 213); or
the liver specific endothelial cells (LSEC) express at least one marker selected from the group consisting of CD45, CD80, CD86, CD11c, VAP1, STAB1 and CD31, wherein the CD31 expression is mainly in the cytoplasm and not on the cell surface; or
the stellate cells express at least one marker selected from the group consisting of GFAP, VIM, LHX2, LRAT, PDGFRb, HAND2, ICAM-1, VCAM-1, and N-CAM-1; or
the hepatic myofibroblast express a marker selected from the group consisting of COL1A1 and α-SMA; or
the parenchymal cell types originate from the same stem cell; or
the cells are cultured in suspension without the use of extracellular matrices.

3. A method of deriving and maintaining a hepatic (liver) organoid, wherein the method of deriving and maintaining a hepatic (liver) organoid comprises:
a) culturing an endoderm stem cell in a first cell culture medium to obtain early hepatic progenitor;
b) transferring and culturing the cells obtained under a) in a suspension culture system in a second cell culture medium to obtain late hepatic progenitor; and
c) culturing the late hepatic progenitors obtained under b) in a suspension culture system in a third cell culture medium to obtain hepatic (liver) organoid
wherein the first medium comprises:
an activator of STAT3, GAB1 mediated cell adhesion and AKT/PI3K signaling pathway;
a molecule which is an repressor of NFκB activity and activator of mitogen-activated protein (MAP) kinase ERK, p38 and JNK;
a TGF-β inhibitor and/or SMAD2/3 inhibitor;
a WNT-signaling activator;
a steroid;
at least one molecule(s) inducing phosphorylation of SMAD1, SMADS and SMAD8 and activating MAPK signaling; and
a molecule activating the FGF and MAPK pathway, wherein:
i) the activator of STAT3, GAB1 mediated cell adhesion and AKT/PI3K signaling pathway in the first medium is a hepatocyte growth factor (HGF) at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, 70 ng/ml, or about 1, 2.5, 3.5 or 4.5 µg/ml; and
ii) the molecule which is an repressor of NFκB activity and activator of mitogen-activated protein (MAP) kinase ERK, p38 and JNK in the first medium is a compound selected from the group consisting of nicotinamide, nicotinic acid, 5-fluoronicotinamide, isonicotinic acid hydrazide, and nikethamide; at a concentration of between about 0.1 mM to 1.0 M, or between about 2 mM to 0.8 M, or between about 4 mM to 0.6 M, or between about 6 mM to 0.4 M, or between about 8 mM to 0.2 M, or between about 10 mM to 800 mM, or between about 50 mM to 500 mM, or about 3, 5, 9, 15, 20, 30, 50, 80, 100, 120, 150, 200, 250, 300, 350, 400 or 450 mM; and
iii) the TGF-β inhibitor in the first medium is selected from the group consisting of
A83-01 3-(6-Methyl-2-pyridinyl)-N-phenyl-4-(4-quinolinyl)-1H-pyrazole-1-carbothioamide,
A 77-01 4-(3-(6-methylpyridin-2-yl)-1H-pyrazol-4-yl)quinoline, SD-208 2-(5-chloro-2-fluorophenyl)-N-pyridin-4-ylpteridin-4-amine, LY2157299 4-[2-(6-methylpyridin-2-yl)-5,6-dihydro-4H-pyrrolo[1,2-b]pyrazol-3-yl]quinoline-6-carboxamide,
SB 431542 4-[4-(1,3-benzodioxol-5-yl)-5-pyridin-2-yl-1H-imidazol-2-yl]benzamide, GW788388 N-(oxan-4-yl)-4-[4-(5-pyridin-2-yl-1H-pyrazol-4-yl)pyridin-2-yl]benzamide,
SB505124 2-[4-(1,3-benzodioxol-5-yl)-2-tert-butyl-1H-imidazol-5-yl]-6-methylpyridine,
SB525334 6-[2-tert-butyl-5-(6-methylpyridin-2-yl)-1H-imidazol-4-yl]quinoxaline, IN 1130 2-[3-[(4-amino-2-methylpyrimidin-5-yl)methyl]-4-methyl-1,3-thiazol-3-ium-5-yl]ethanol,
ITD 1 (6,6-dimethyl-5,7-dihydroimidazo[2,1-b][1,3]thiazol-4-ium-3-yl)methyl N,N'-dicyclohexylcarbamimidothioate,
LY2109761 4-[2-[4-(2-pyridin-2-yl-5,6-dihydro-4H-pyrrolo[1,2-b]pyrazol-3-yl)quinolin-7-yl]oxyethyl]morpholine,
K02288 3-[6-amino-5-(3,4,5-trimethoxyphenyl)pyridin-3-yl]phenol, TGF-β RI kinase inhibitor [3-(Pyridin-2-yl)-4-(4-quinonyl)]-1H-pyrazole] and derivatives thereof; and
iv) wherein the TGF-β inhibitor in the first medium is at a concentration of between about 0.5 nM to 20 µM, or 100 nM to 10 µM, or 250 nM to 5 µM, or 400 nM to 2.5 µM, or 0.5 nM to 1 µM, or 0.5 nM to 0.5 µM, or between about 1.5 nM to 0.4 µM, or between about 10 nM to 0.3 µM, or between about 30 nM to 0.2 µM, or between about 40 nM to 0.1 µM, or between about 50 nM to 85 nM, or about 1, 5, 15, 25, 30, 35, 45, 50, 65, 75, 130, 150, 170, 250, 350 or 450 nM; or about 0.5, 0.7, 0.9, 1, 1.5, 2, 2.5, 3, 3.5, 4, 4.5, 5, 5.5, 6, 8, 9, 10, 12, 14, 16, 18, or 20 µM; or
v) the SMAD2/3 inhibitor is selected from the group consisting of a Smad2/3 phosphorylation inhibitor and siRNA targeting the mRNA of SMAD2 and SMAD3 transcript; and
vi) the WNT-signaling activator in the first medium is a Glycogen synthase kinase 3 (GSK3) inhibitor; and the WNT-signaling activator in the first medium is at a concentration of between about 0.1 µM to 10 µM, or between about 0.5 µM to 8 µM, or between about 1 µM to 7 µM, or between about 2 µM to 6 µM, or between about 3 µM to 5 µM, or about 0.2, 0.3, 0.4,3, 4, 4.5, 5, 6, 7.5, 8.5, 9, 9.5 or 10 µM; and
vii) the steroid in the first medium is a corticosteroid; and the steroid is at a concentration of between about 0.5 µM to 200 µM, or between about 1.5 µM to 150 µM, or between about 5 µM to 100 µM, or between about 10 µM to 90 µM, or between about 20 µM to 80 µM, or between about 30 µM to 70 µM, or between about 40 µM to 60 µM, or about 2, 8, 15, 25, 30, 35, 45, 65, 75, 110, 130, 140, 160, 170 or 190 µM; or
viii) at least one molecule(s) in the first medium inducing phosphorylation of SMAD1, SMADS and SMAD8 and activating MAPK signaling is/are selected from the group consisting of BMP4, BMP2, BMP3, BMP5, BMP6, and BMP7; and the at least one molecule(s) in the first medium inducing phosphorylation of SMAD1, SMADS and SMAD8 and activating MAPK signaling is at a concentration of between about 2 ng/ml to 5 µg/ml, or between about 5 ng/ml to 5 µg/ml, or between about 10 ng/ml to 4 µg/ml, or between about 15 ng/ml to 3 µg/ml, or between about 20 ng/ml to 2 µg/ml, or about 5, 18, 20, 25, 28, 30, 50, 60, 70 ng/ml, or about 1, 2.5, 3.5 or 4.5 µg/ml; and
ix) the molecule in the first medium activating the FGF and MAPK pathway is selected from the group consisting of FGF7, FGF1, FGF3, FGF10, and FGF22.

4. A method of deriving and maintaining a hepatic (liver) organoid according to claim 3, wherein the first medium further comprises an activator of AKT/PI3K signaling pathway and MAPK signaling pathway, wherein the activator is a compound selected from the group consisting of an epidermal growth factor (EGF), amphiregulin (AR), epigen (EPG), transforming growth factor alpha (TGFα), betacellulin (BTC), epiregulin (EPR), heparin-binding EGF-like growth factor (HB-EGF), and Neuregulin (NRG); and the compound is at a concentration of between about 5 ng/ml to 5 µg/ml, or between about 20 ng/ml to 4 µg/ml, or between about 30 ng/ml to 3 µg/ml, or between about 40 ng/ml to 2 µg/ml, or between about 45 ng/ml to 500 ng/ml, or between about 50 ng/ml to 300 ng/ml, or about 35, 40, 45, 50, 60, 70, 90, 100, 150, 200, 250, 300, 400, 450, 600, 700 or 800 ng/ml.

5. A method of deriving and maintaining a hepatic (liver) organoid according to claim 3 or claim 4, wherein the first medium further comprises an activator of the cAMP-dependent pathways and/or Protein Kinase A signaling pathway; wherein the activator is a compound selected from the group consisting of dibutyryl-cAMP(dbCAMP), forskolin ((3R,4aR,5S,6S,6aS,10S,10aR,10bS)-6,10,10b-trihydroxy-3,4a,7,7,10a-pentamethyl-1-oxo-3-vinyldodecahydro-1H-benzo[f]chromen-5-yl acetate), caffeine, theophylline, cholera toxin and pertussis toxin; and the compound is at a concentration of between about 20 ng/ml to 1 µg/ml, or between about 10 ng/ml to 0.8 µg/ml, or between about 15 ng/ml to 0.6 µg/ml, or between about 20 ng/ml to 2 µg/ml, or about 5, 18, 20, 25, 28, 30, 50, 60, 70 ng/ml, or about 1, 2.5, 3.5 or 4.5 µg/ml.

6. A method of deriving and maintaining a hepatic (liver) organoid according to claim 3 to 5, wherein the first medium further comprises an inhibitor of histone deacetylase (HDACs); wherein the inhibitor is a compound selected from the group consisting of valporic acid (VPA), sodium butyrate, vorinotstat, panobinostat, trichostatin A, mocetinostat, BG45 (N-(2-aminophenyl)-2-pyrazinecarboxamide), 4SC-202 ((E)-N-(2-aminophenyl)-3-(1-((4-(1-methyl-1H-pyrazol-4-yl)phenyl)sulfonyl)-1H-pyrrol-3-yl)acrylamide), belinostat, scriptaid (6-(1,3-Dioxo-1H-benzo[de]isoquinolin-2(3H)-yl)-N-hydroxyhexanamide), M344 (4-(dimethylamino)-N-[7-(hydroxyamino)-7-oxoheptyl]benzamide), dacinostat ((E)-N-hydroxy-3-[4-[[2-hydroxyethyl-[2-(1H-indol-3-yl)ethyl]amino]methyl]phenyl]prop-2-enamide), abexinostat, CUDC-101 (7-(4-(3-ethynylphenylamino)-7-methoxyquinazolin-6-yloxy)-N-hydroxyheptanamide), CUDC-907 (N-hydroxy-2-(((2-(6-methoxypyridin-3-yl)-4-morpholinothieno[3,2-d]pyrimidin-6-yl)methyl)(methyl)amino)pyrimidine-5-carboxamide), and AR-42 ((S)-N-hydroxy-4-(3-methyl-2-phenylbutanamido)benzamide); and the compound is at a concentration of between about 0.1 µM to 5 mM, or between about 0.3 µM to 4 mM, or between about 0.6 µM to 3 mM, or between about 0.8 µM to 2 mM, or between about 1 mM to 1.5 mM, or about 0.2, 0.4, 0.7, 0.9, 5, 10, 20, 50, 70 or 90 µM, or about 1.5, 2.5, 3.5 mM.

7. A method of deriving and maintaining a hepatic (liver) organoid according to claim 3 to 6, wherein the first medium further comprises an activator of the Notch receptor; wherein the activator is a compound selected from the group consisting of Jagged1 protein (homo sapiens, also known as AGS; AHD; AWS; HJ1; CD339; JAGL1; JAG1), Jagged2 (NCBI 3714), Delta-like1 (NCBI 28514), Delta-like3 (NCBI 10683), and Delta-like4 (NCBI 54567); and the compound is at a concentration of between about 10 nM to 100 µM, or between about 50 nM to 80 µM, or between about 100 nM to 60 µM, or between about 500 nM to 40 µM, or between about 800 nM to 20 µM, or between about 900 nM to 10 µM, or about 20, 40, 60, 80 nM or about 1, 1.5, 15, 30, 50, 60, 90 or 100 µM.

8. The method of any one of claims 3 to 7, wherein the second cell culture medium comprises:
an activator of STAT3, GAB1 mediated cell adhesion and AKT/PI3K signaling pathway;
a molecule which is an repressor of NFκB activity and activator of mitogen-activated protein (MAP) kinase ERK, p38 and JNK;
a TGF-β inhibitor and/or SMAD2/3 inhibitor;
a steroid;
a molecule inducing phosphorylation of SMAD1 and SMAD5 and activating MAPK signaling; and
a molecule that regulates bile acid synthesis and activates a FGF and MAPK pathway, and
a component for inducing late hepatic progenitor differentiation, wherein the component is a YAP inhibitor:
i) wherein the activator of STAT3, GAB1 mediated cell adhesion and AKT/PI3K signaling pathway is as defined in claim 3 i); or
ii) wherein the molecule which is a repressor of NFκB activity and activator of mitogen-activated protein (MAP) kinase ERK, p38 and JNK is as defined in claim 3 ii); or
iii) wherein the TGF-β inhibitor and/or SMAD2/3 inhibitor is as defined in claims 3 iii) to 3 v); or
iv) wherein the steroid is as defined in claim 3 vii); or
v) wherein the molecule inducing phosphorylation of SMAD1, SMADS and SMAD8 and activating MAPK signaling is as defined in claim 3 viii); or
vi) wherein the molecule that regulates bile acid synthesis and activates the FGF and MAPK pathway is selected from the group consisting of FGF19, FGF1, FGF2, FGF4, FGF6, FGF8, FGF9, FGF16, FGF17, FGF18, FGF20, and FGF23;
and
wherein the molecule that regulates bile acid synthesis and activates the FGF and MAPK pathway is at a concentration of between about 1 ng/ml to 1 µg/ml, or between about 5 ng/ml to 0.8 µg/ml, or between about 10 ng/ml to 0.6 µg/ml, or between about 50 ng/ml to 0.5 µg/ml, or between about 150 ng/ml to 1 µg/ml, or about 5, 20, 50, 100, 200, 250, 300, 400, 500 ng/ml, or about 1, 0.8, 0.7 or 0.9 µg/ml.

9. The method of claim 8, wherein the second cell culture medium further comprises a component to promote survival of late hepatic progenitors, wherein the component is selected from one or two or three or all of the following components:
an activator of AKT/PI3K signaling pathway and MAPK signaling pathway, wherein the activator is a compound selected from the group consisting of an epidermal growth factor (EGF), amphiregulin (AR), epigen (EPG), transforming growth factor alpha (TGFα), betacellulin (BTC), epiregulin (EPR), heparin-binding EGF-like growth factor (HB-EGF), and Neuregulin (NRG); and the compound is at a concentration of between about 5 ng/ml to 5 µg/ml, or between about 20 ng/ml to 4 µg/ml, or between about 30 ng/ml to 3 µg/ml, or between about 40 ng/ml to 2 µg/ml, or between about 45 ng/ml to 500 ng/ml, or between about 50 ng/ml to 300 ng/ml, or about 35, 40, 45, 50, 60, 70, 90, 100, 150, 200, 250, 300, 400, 450, 600, 700 or 800 ng/ml;
an activator of cAMP-dependent pathways or an activator of Protein Kinase A signaling pathway, wherein the activator is a compound selected from the group consisting of dibutyryl-cAMP(dbCAMP), forskolin ((3R,4aR,5S,6S,6aS,10S,10aR,10bS)-6,10,10b-trihydroxy-3,4a,7,7,10a-pentamethyl-1-oxo-3-vinyldodecahydro-1H-benzo[f]chromen-5-yl acetate), caffeine, theophylline, cholera toxin and pertussis toxin; and the compound is at a concentration of between about 20 ng/ml to 1 µg/ml, or between about 10 ng/ml to 0.8 µg/ml, or between about 15 ng/ml to 0.6 µg/ml, or between about 20 ng/ml to 2 µg/ml, or about 5, 18, 20, 25, 28, 30, 50, 60, 70 ng/ml, or about 1, 2.5, 3.5 or 4.5 µg/ml;
an activator of the Notch receptor, wherein the activator is a compound selected from the group consisting of Jagged1 protein (homo sapiens, also known as AGS; AHD; AWS; HJ1; CD339; JAGL1; JAG1), Jagged2 (NCBI 3714), Delta-like1 (NCBI 28514), Delta-like3 (NCBI 10683), and Delta-like4 (NCBI 54567); and the compound is at a concentration of between about 10 nM to 100 µM, or between about 50 nM to 80 µM, or between about 100 nM to 60 µM, or between about 500 nM to 40 µM, or between about 800 nM to 20 µM, or between about 900 nM to 10 µM, or about 20, 40, 60, 80 nM or about 1, 1.5, 15, 30, 50, 60, 90 or 100 µM.; and
an inhibitor of histone deacetylase (HDACs), wherein the inhibitor is a compound selected from the group consisting of valporic acid (VPA), sodium butyrate, vorinotstat, panobinostat, trichostatin A, mocetinostat, BG45 (N-(2-aminophenyl)-2-pyrazinecarboxamide), 4SC-202 ((E)-N-(2-aminophenyl)-3-(1-((4-(1-methyl-1H-pyrazol-4-yl)phenyl)sulfonyl)-1H-pyrrol-3-yl)acrylamide), belinostat, scriptaid (6-(1,3-Dioxo-1H-benzo[de]isoquinolin-2(3H)-yl)-N-hydroxyhexanamide), M344 (4-(dimethylamino)-N-[7-(hydroxyamino)-7-oxoheptyl]benzamide), dacinostat ((E)-N-hydroxy-3-[4-[[2-hydroxyethyl-[2-(1H-indol-3-yl)ethyl]amino]methyl]phenyl]prop-2-enamide), abexinostat, CUDC-101 (7-(4-(3-ethynylphenylamino)-7-methoxyquinazolin-6-yloxy)-N-hydroxyheptanamide), CUDC-907 (N-hydroxy-2-(((2-(6-methoxypyridin-3-yl)-4-morpholinothieno[3,2-d]pyrimidin-6-yl)methyl)(methyl)amino)pyrimidine-5-carboxamide), and AR-42 ((S)-N-hydroxy-4-(3-methyl-2-phenylbutanamido)benzamide); and the compound is at a concentration of between about 0.1 µM to 5 mM, or between about 0.3 µM to 4 mM, or between about 0.6 µM to 3 mM, or between about 0.8 µM to 2 mM, or between about 1 mM to 1.5 mM, or about 0.2, 0.4, 0.7, 0.9, 5, 10, 20, 50, 70 or 90 µM, or about 1.5, 2.5, 3.5 mM.

10. The method of claim 8 or claim 9, wherein the second cell culture medium further comprises a component for inducing late hepatic progenitor formation, wherein the component is a WNT-signaling activator, wherein the WNT-signaling activator is defined in claims 3 vi).

11. The method of any one of claims 3 to 5, wherein the third cell culture medium comprises:
a TGF-β inhibitor and/or SMAD2/3 inhibitor as defined in claims 3 iii) to 3 v); a pleiotropic cytokine that belongs to the interleukin 6 group of cytokines, wherein the pleiotropic cytokine that belongs to the interleukin 6 group of cytokines signaling is selected from oncostatin M (OSM) or leukemia inhibitory factor (LIF; NCBI: 3976), or Cardiotrophin-1/CT-1 (NCBI: 1489), or ciliary neurotrophic factor receptor (CNTF; NCBI: 1271), IL-11 or IL-31, and wherein the pleiotropic cytokine is at a concentration of between about 0.1 ng/ml to 1 µg/ml, or between about 10 ng/ml to 0.8 µg/ml, or between about 15 ng/ml to 0.6 µg/ml, or between about 20 ng/ml to 2 µg/ml, or about 5, 18, 20, 25, 28, 30, 50, 60, 70 ng/ml, or about 1, 2.5, 3.5 or 4.5 µg/ml;
an inhibitor of γ-secretase; and
a steroid as defined in claim 3 vii),
wherein:
the inhibitor of γ-secretase is selected from the group consisting of Compound E (C-E) (2S)-2-[[2-(3,5-difluorophenyl)acetyl]amino]-N-[(3S)-1-methyl-2-oxo-5-phenyl-3H-1,4-benzodiazepin-3-yl]propanamide, Dibenzazepine (DBZ): (2S)-2-[[2-(3,5-difluorophenyl)acetyl]amino]-N-[(7S)-5-methyl-6-oxo-7H-benzo[d][1]benzazepin-7-yl]propanamide, DAPT:tert-butyl (2S)-2-[[(2S)-2-[[2-(3,5-difluorophenyl)acetyl]amino]propanoyl]amino]-2-phenylacetate, Begacestat: 5-chloro-N-[(2S)-4,4,4-trifluoro-1-hydroxy-3-(trifluoromethyl)butan-2-yl]thiophene-2-sulfonamide, and Flurizan: (2R)-2-(3-fluoro-4-phenylphenyl)propanoic acid; wherein the inhibitor of γ-secretase is at a concentration of between about 10 nM to 5 µM, or between about 100 nM to 4 µM, or between about 200 nM to 3.5 µM, or between about 300 nM to 3 µM, or between about 400 nM to 2.5 µM, or between about 450 nM to 2 µM, or between about 500 nM to 1.5 µM, or about 50, 90, 150, 250, 350, 450, 480, 500, 650, or 700 nM.

12. The method of any one of claims 3 to 5, wherein the third cell culture medium comprises at least one or two or three or four or five or six component(s) promoting maturation of hepatic organoid and/or at least one or two or three component(s) promoting survival of hepatic organoids:
i) wherein the component(s) promoting maturation of the hepatic organoid is selected from the group consisting of:
a compound inducing phosphorylation of SMAD1, SMADS and SMAD8 and activating MAPK signalling as defined in claim 3 viii);
an interleukin that acts as both a pro-inflammatory cytokine and an anti-inflammatory myokine;
a compound that acts as a hormone regulating bile acid synthesis and activates FGF and MAPK pathway as defined in claim 8 vi);
an activator of cAMP-dependent pathways as defined in claim 9;
a YAP inhibitor; and
a compound with biliary acid potency, wherein:
a) the YAP inhibitor is selected from the group consisting of:
Verteporfin 3-[(23S,24R)-14-ethenyl-5-(3-methoxy-3-oxopropyl)-22,23-bis(methoxycarbonyl)-4,10,15,24-tetramethyl-25,26,27,28-tetraazahexacyclo[16.6.1.13,6.18,11.113,16.019,24]octacosa-1,3,5,7,9,11(27),12,14,16,18(25),19,21-dodecaen-9-yl]propanoic acid,
LPA [(2R)-2-hydroxy-3-phosphonooxypropyl] (Z)-octadec-9-enoate,
S1P [(E,2S,3R)-2-amino-3-hydroxyoctadec-4-enyl] dihydrogen phosphate, Thrombin (2S)-2-[[(2S)-1-[(2S)-5-amino-2-[[2-[[(2S)-6-amino-2-[[2-[[(2S)-2-[[(2S)-4-amino-2-[[(2S)-2-[[(2S,3R)-2-[[(2S)-2-[[(2S,3R)-2-amino-3-hydroxybutanoyl]amino]-3-(1H-indol-3-yl)propanoyl]amino]-3-hydroxybutanoyl]amino]propanoyl]amino]-4-oxobutanoyl]amino]-3-methylbutanoyl]amino]acetyl]amino]hexanoyl]amino]acetyl]amino]-5-oxopentanoyl]pyrrolidine-2-carbonyl]amino]-3-hydroxypropanoic acid, Epinephrine 4-[(1R)-1-hydroxy-2-(methylamino)ethyl]benzene-1,2-diol, Glucagon (2S)-2-[[(2S,3R)-2-[[2-[[(2S)-5-amino-2-[[(2S)-2-[[(2S)-2-amino-3-(1H-imidazol-5-yl)propanoyl]amino]-3-hydroxypropanoyl]amino]-5-oxopentanoyl]amino]acetyl]amino]-3-hydroxybutanoyl]amino]-3-phenylpropanoic acid,
Dihydrexidine(6aR,12bS)-5,6,6a, 7,8, 12b-hexahydrobenzo[a]phenanthridine-10,11-diol,
Dobutamine4-[2-[4-(4-hydroxyphenyl)butan-2-ylamino]ethyl]benzene-1,2-diol, DasatinibN-(2-chloro-6-methylphenyl)-2-[[6-[4-(2-hydroxyethyl)piperazin-1-yl]-2-methylpyrimidin-4-yl]amino]-1,3-thiazole-5-carboxamide,
Latrunculin A (4R)-4-[(1R,4S,5Z,7E,11Z,15R,17R)-17-hydroxy-4,11-dimethyl-13-oxo-14,18-dioxabicyclo[13.3.1]nonadeca-5,7,11-trien-17-yl]-1,3-thiazolidin-2-one,
Latrunculin B (4R)-4-[(1R,4S,5Z,9Z,13R,15R)-15-hydroxy-4,9-dimethyl-11-oxo-12,16-dioxabicyclo[11.3.1]heptadeca-5,9-dien-15-yl]-1,3-thiazolidin-2-one,
Cytochalasin D(3S,3aR,4S,6S,6aR,7E,10S,12R,13E,15R,15aR)-3-Benzyl-6,12-dihydroxy-4,10,12-trimethyl-5-methylene-1,11-dioxo-2,3,3a,4,5,6,6a,9,10,11,12,15-dodecahydro-1H-cycloundeca[d]isoindol-15-yl acetate
Blebbistatin 3a-hydroxy-6-methyl-1-phenyl-2,3-dihydropyrrolo[2,3-b]quinolin-4-one, ML7 1-(5-iodonaphthalen-1-yl)sulfonyl-1,4-diazepane,
Botulinum C3 4-N-(3-chloro-7-methoxyacridin-1-yl)-1-N,1-N-diethylpentane-1,4-diamine;dihydrochloride,
Y27632 4-[(1R)-1-aminoethyl]-N-pyridin-4-ylcyclohexane-1-carboxamide,
and derivatives thereof, optionally wherein the YAP inhibitor is Verteporfin 3-[(23S,24R)-14-ethenyl-5-(3-methoxy-3-oxopropyl)-22,23-bis(methoxycarbonyl)-4,10,15,24-tetramethyl-25,26,27,28-tetraazahexacyclo[16.6.1.13,6.18,11.113,16.019,24]octacosa-1,3,5,7,9,11(27),12,14,16,18(25),19,21-dodecaen-9-yl]propanoic acid; and
b) the compound with biliary acid potency comprises one or more or all of the following activities: activating the nuclear farnesoid X receptor, increasing cAMP and activating the PKC signaling pathway, optionally wherein the compound with biliary acid potency is selected from the group consisting of:
Taurocholic acid 2-[[(4R)-4-[(3R,5S,7R,8R,9S,10S,12S,13R,14S,17R)-3,7,12-trihydroxy-10,13-dimethyl-2,3,4,5,6,7,8,9,11,12,14,15,16,17-tetradecahydro-1H-cyclopenta[a]phenanthren-17-yl]pentanoyl]amino]ethanesulfonic acid,
Cholic acid (4R)-4-[(3R,5S,7R,8R,9S,10S,12S,13R,14S,17R)-3,7,12-trihydroxy-10,13-dimethyl-2,3,4,5,6,7,8,9,11,12,14,15,16,17-tetradecahydro-1H-cyclopenta[a]phenanthren-17-yl]pentanoic acid,
Chenodeoxycholic acid (4R)-4-[(3R,5S,7R,8R,9S,10S,13R,14S,17R)-3,7-dihydroxy-10,13-dimethyl-2,3,4,5,6,7,8,9,11,12,14,15,16,17-tetradecahydro-1H-cyclopenta[a]phenanthren-17-yl]pentanoic acid,
Glycocholic acid 2-[[(4R)-4-[(3R,5S,7R,8R,9S,10S,12S,13R,14S,17R)-3,7,12-trihydroxy-10,13-dimethyl-2,3,4,5,6,7,8,9,11,12,14,15,16,17-tetradecahydro-1H-cyclopenta[a]phenanthren-17-yl]pentanoyl]amino]acetic acid,
Deoxycholic acid (4R)-4-[(3R,5R,8R,9S,10S,12S,13R,14S,17R)-3,12-dihydroxy-10,13-dimethyl-2,3,4,5,6,7,8,9,11,12,14,15,16,17-tetradecahydro-1H-cyclopenta[a]phenanthren-17-yl]pentanoic acid,
Glycochenodeoxycholic acid 2-[[(4R)-4-[(3R,5S,7R,8R,9S,10S,13R,14S,17R)-3,7-dihydroxy-10,13-dimethyl-2,3,4,5,6,7,8,9,11,12,14,15,16,17-tetradecahydro-1H-cyclopenta[a]phenanthren-17-yl]pentanoyl]amino]acetic acid,
Taurochenodeoxycholic acid 2-[[(4R)-4-[(3R,5S,7R,8R,9S,10S,13R,14S,17R)-3,7-dihydroxy-10,13-dimethyl-2,3,4,5,6,7,8,9,11,12,14,15,16,17-tetradecahydro-1H-cyclopenta[a]phenanthren-17-yl]pentanoyl]amino]ethanesulfonic acid,
Lithocholic acid (4R)-4-[(3R,5R,8R,9S,10S,13R,14S,17R)-3-hydroxy-10,13-dimethyl-2,3,4,5,6,7,8,9,11,12,14,15,16,17-tetradecahydro-1H-cyclopenta[a]phenanthren-17-yl]pentanoic acid, and
derivatives thereof, optionally wherein the compound with biliary acid potency is Taurocholic acid 2-[[(4R)-4-[(3R,5S,7R,8R,9S,10S,12S,13R,14S,17R)-3,7,12-trihydroxy-10,13-dimethyl-2,3,4,5,6,7,8,9,11,12,14,15,16,17-tetradecahydro-1H-cyclopenta[a]phenanthren-17-yl]pentanoyl]amino]ethanesulfonic acid; and
wherein the compound with bilary acid potency is at a concentration of between about 1 µM to 1 mM, or about 10 µM to 0.8 mM, or about 50 µM to 0.6 mM, or about 100 µM to 0.4 mM, or about 150 µM to 0.2 mM, or about 5, 15, 20, 40, 60, 80, 90, 100, 150, 250, 350, 450, 550, 650, 750, 850 µM;
or
ii) wherein the component(s) promoting survival of the hepatic organoid is selected from the group consisting of:
an activator of STAT3, GAB1 mediated cell adhesion and AKT/PI3K signaling pathway;
a glycosaminoglycan; and
an activator of AKT/PI3K signaling pathway and MAPK signaling pathway, wherein the activator of STAT3, GAB1 mediated cell adhesion and AKT/PI3K signaling pathway is as defined in claim 3 i).

13. The method of any one of claims 3 to 12, wherein the hepatic (liver) organoids comprise more than one liver specific cell type selected from the group consisting of hepatocytes and cholangiocytes; and optionally further comprise liver specific endothelial cells (LSEC), stellate cells, hepatic myofibroblast and/or hepatoblasts..

14. The method of any one of claims 3 to 13, wherein the early hepatic progenitor expresses any one or more or at least two, or at least three, or at least four, or at least five, or at least six, or 1 or 2 or 3 or 4 or 5 or 6 or 7 or all, of the following markers: SOX9 (NCBI: 6662), CK19 (NCBI: 3880), CK18 (NCBI: 3875), HNF4a (NCBI: 3172), PROX1 (NCBI: 5629), ONECUT1 (NCBI: 3175), AFP (NCBI: 174), TBX3 (NCBI:6926); and/or wherein the late hepatic progenitor expresses any one or more or at least two, or at least three, or at least four, or at least five, or at least six, or between 1 or 2 or 3 to 4 or 5 or 6 or 7 or all, or all of the following markers: CK19 (NCBI: 3880), CK18 (NCBI: 3875), HNF4a (NCBI: 3172), PROX1 (NCBI: 5629), ONECUT1 (NCBI: 3175), AFP (NCBI: 174), TBX3 (NCBI:6926), ALB (NCBI: 213).

15. A hepatic (liver) organoid obtained by a method of any one of claims 3 to 14 comprising at least two cell types selected from the group consisting of hepatocytes and cholangiocytes; wherein
the hepatocytes
a) express albumin (ALB);
b) express the hepatocyte markers HNF4a (NCBI: 3172), FAH (NCBI: 2184), TAT (NCBI: 6898), GCK (NCBI: 2645), TTR (NCBI: 7276), MET (NCBI: 4233), GLUl/MGAM (NCBI: 8972), FAHD2A (NCBI: 51011), HNF1B (NCBI: 6928), HNF1A (NCBI: 6927), CYP3A4 (NCBI: 1576), CYP2C9 (NCBI: 1559), CYP2C19 (NCBI: 1557), CYP1A2 (NCBI: 1544), CYP2E1 (NCBI: 1571), CYP2D6 (NCBI: 1565), CYP3A7 (NCBI: 1551), CYP1A1 (NCBI: 1543), CYP3A5 (NCBI: 1577), CYP27A1 (NCBI: 1593), MRP2 (NCBI:1244), NTCP (NCBI: 6554), OATP1B3 (NCBI: 28234), UGT2B7 (NCBI: 7364), UGT2B15 (NCBI: 7366), UGT1A1 (NCBI: 54658), CEBP (NCBI: 1050), KRT8 (NCBI: 3856), NOTCH2 (NCBI: 4853) and CYP2B6 (NCBI: 1555); and
c) do not express the cholangiocyte marker Cytokeratin 7 (CK7); and
d) the organoids are capable of performing liver functions and exhibit a spatially organized structure observed in liver.

## Patentansprüche

1. Leberorganoid, umfassend mindestens zwei Zelltypen, der ausgewählt ist aus der Gruppe bestehend aus Hepatozyten und Cholangiozyten, wobei
die Hepatozyten
a) Albumin (ALB) exprimieren;
b) die Hepatozyten-Marker HNF4a (NCBI: 3172), FAH (NCBI: 2184), TAT (NCBI: 6898), GCK (NCBI: 2645), TTR (NCBI: 7276), MET (NCBI: 4233), GLU1/MGAM (NCBI: 8972), FAHD2A (NCBI: 51011), HNF1B (NCBI: 6928), HNF1A (NCBI: 6927), CYP3A4 (NCBI: 1576), CYP2C9 (NCBI: 1559), CYP2C19 (NCBI: 1557), CYP1A2 (NCBI: 1544), CYP2E1 (NCBI: 1571), CYP2D6 (NCBI: 1565), CYP3A7 (NCBI: 1551), CYP1A1 (NCBI: 1543), CYP3A5 (NCBI: 1577), CYP27A1 (NCBI: 1593), MRP2 (NCBI: 1244), NTCP (NCBI: 6554), OATP1B3 (NCBI: 28234), UGT2B7 (NCBI: 7364), UGT2B15 (NCBI: 7366), UGT1A1 (NCBI: 54658), CEBP (NCBI: 1050), KRT8 (NCBI: 3856), NOTCH2 (NCBI: 4853) und CYP2B6 (NCBI: 1555) exprimieren; und
c) den Cholangiozytenmarker Cytokeratin 7 (CK7) nicht exprimieren; und
d) die Organoide in der Lage sind, Leberfunktionen durchzuführen und eine räumlich organisierte Struktur aufweisen, wie sie in der Leber vorzufinden ist.

2. Leberorganoid nach Anspruch 1, ferner umfassend mindestens einen Zelltyp, der ausgewählt ist aus der Gruppe bestehend aus leberspezifischen Endothelzellen (LSEC), Stellatzellen, hepatischen Myofibroblasten und Hepatoblasten, wobei:
die Cholangiozyten CK7 exprimieren, aber nicht Albumin (ALB) exprimieren; oder
die Hepatoblasten mindestens einen Marker exprimieren, der ausgewählt ist aus der Gruppe bestehend aus SOX9 (NCBI: 6662), CK19 (NCBI: 3880), CK18 (NCBI: 3875), HNF4a (NCBI: 3172), PROX1 (NCBI: 5629), ONECUT1 (NCBI: 3175), AFP (NCBI: 174) und ALB (NCBI: 213); oder
die leberspezifischen Endothelzellen (LSEC) mindestens einen Marker exprimieren, der ausgewählt ist aus der Gruppe bestehend aus CD45, CD80, CD86, CD11c, VAP1, STAB1 und CD31, wobei die CD31-Expression hauptsächlich in dem Zytoplasma und nicht auf der Zelloberfläche ist; oder
die Stellatzellen mindestens einen Marker exprimieren, der ausgewählt ist aus der Gruppe bestehend aus GFAP, VIM, LHX2, LRAT, PDGFRb, HAND2, ICAM-1, VCAM-1 und N-CAM-1; oder
die hepatischen Myofibroblasten einen Marker exprimieren, der ausgewählt ist aus der Gruppe bestehend aus COL1A1 und α-SMA; oder
die parenchymatösen Zelltypen von derselben Stammzelle abstammen; oder
die Zellen in Suspension ohne Verwendung extrazellulärer Matrizen kultiviert werden.

3. Verfahren zum Ableiten und Beibehalten eines hepatischen (Leber-)Organoids, wobei das Verfahren zum Ableiten und Beibehalten eines hepatischen (Leber-)Organoids Folgendes umfasst:
a) Kultivieren einer Endoderm-Stammzelle in einem ersten Zellkulturmedium, um frühen Lebervorläufer zu erlangen;
b) Überführen und Kultivieren der unter a) erlangten Zellen in einem Suspensionskultursystem in einem zweiten Zellkulturmedium, um späten hepatischen Vorläufer zu erlangen; und
c) Kultivieren der unter b) erhaltenen späten hepatischen Vorläufer in einem Suspensionskultursystem in einem dritten Zellkulturmedium, um hepatischen (Leber-)Organoid zu erlangen,
wobei das erste Medium Folgendes umfasst:
einen Aktivator von STAT3, GAB1-vermittelter Zelladhäsion und AKT/PI3K-Signalweg;
ein Molekül, das Repressor von NFκB-Aktivität ist und Aktivator einer Kinase von mitogen-aktiviertem Protein (MAP) ERK, p38 und JNK ist;
einen TGF-β-Hemmer und/oder SMAD2/3-Hemmer;
einen Aktivator für WNT-Signalübertragung;
ein Steroid;
mindestens ein Molekül(e), die Phosphorylierung von SMAD1, SMAD5 und SMAD8 induzieren und die MAPK-Signalübertragung aktivieren; und
ein Molekül, das den FGF- und MAPK-Weg aktiviert, wobei:
i) der Aktivator von STAT3, GAB 1-vermittelter Zelladhäsion und AKT/PI3K-Signalweg in dem ersten Medium ein Hepatozyten-Wachstumsfaktor (HGF) in einer Konzentration zwischen etwa 2 ng/ml bis 5 µg/ml ist, oder zwischen etwa 5 ng/ml bis 5 µg/ml, oder zwischen etwa 10 ng/ml bis 4 µg/ml, oder zwischen etwa 15 ng/ml bis 3 µg/ml, oder zwischen etwa 20 ng/ml bis 2 µg/ml, oder etwa 5, 18, 20, 25, 28, 30, 50, 60, 70 ng/ml, oder etwa 1, 2,5, 3,5 oder 4,5 µg/ml; und
ii) das Molekül, das ein Repressor von NFκB-Aktivität und ein Aktivator einer Kinase von mitogen-aktiviertem Protein (MAP) ERK, p38 und JNK in dem ersten Medium ist, eine Verbindung ist, die ausgewählt ist aus der Gruppe, bestehend aus Nikotinamid, Nikotinsäure, 5-Fluornikotinamid, Isonikotinsäurehydrazid und Nikethamid; in einer Konzentration zwischen etwa 0,1 mM bis 1,0 M oder zwischen etwa 2 mM bis 0,8 M oder zwischen etwa 4 mM bis 0,6 M oder zwischen etwa 6 mM bis 0,4 M oder zwischen etwa 8 mM bis 0,2 M oder zwischen etwa 10 mM bis 800 mM oder zwischen etwa 50 mM bis 500 mM oder etwa 3, 5, 9, 15, 20, 30, 50, 80, 100, 120, 150, 200, 250, 300, 350, 400 oder 450 mM; und
iii) der TGF-β-Hemmer in dem ersten Medium ausgewählt ist aus der Gruppe, bestehend aus A83-013 (6-Methyl-2-pyridinyl)-N-phenyl-4-(4-chinolinyl)-1H-pyrazol-1-carbothioamid,
A 77-01 4-(3-(6-Methylpyridin-2-yl)-1H-pyrazol-4-yl)-chinolin,
SD-208 2-(5-Chlor-2-fluorophenyl)-N-pyridin-4-ylpteridin-4-amin, LY2157299 4-[2-(6-Methylpyridin-2-yl)-5,6-dihydro-4H-pyrrolo[1,2-b]pyrazol-3-yl]chinolin-6-carboxamid,
SB 431542 4-[4-(1,3-Benzodioxol-5-yl)-5-pyridin-2-yl-1H-imidazol-2-yl]benzamid, GW788388 N-(Oxan-4-yl)-4-[4-(5-pyridin-2-yl-1H-pyrazol-4-yl)pyridin-2-yl]benzamid,
SB5051242-[4-(1,3-benzodioxol-5-yl)-2-tert-butyl-1H-imidazol-5-yl]-6-methylpyridin,
SB525334 6-[2-tert-Butyl-5-(6-methylpyridin-2-yl)-1H-imidazol-4-yl]chinoxalin, IN 1130 2-[3-[(4-Amino-2-methylpyrimidin-5-yl)methyl]-4-methyl-1,3-thiazol-3-ium-5-yl]ethanol,
ITD 1 (6,6-Dimethyl-5,7-dihydroimidazo[2,l-b][1,3]thiazol-4-ium-3-yl)methyl N,N'-dicyclohexylcarbamimidothioat,
LY2109761 4-[2-[4-(2-pyridin-2-yl-5,6-dihydro-4H-pyrrolo[1,2-b]pyrazol-3yl)chinolin-7-yl]oxyethyl]morpholin,
K02288 3-[6-Amino-5-(3,4,5-trimethoxyphenyl)pyridin-3-yl]phenol, TGF-β-RI-Kinase-Hemmer [3-(Pyridin-2-yl)-4-(4-quinonyl)]-1H-pyrazol] und Derivate davon; und
iv) wobei der TGF-β-Hemmer in dem ersten Medium in einer Konzentration zwischen etwa 0,5 nM bis 20 µM, oder 100 µM bis 10 µM, oder 250 nM bis 5 µM, oder 400 nM bis 2,5 µM, oder 0,5 nM bis 1 µM, oder 0,5 nM bis 0,5 µM, oder zwischen etwa 1,5 nM bis 0,4 µM, oder zwischen etwa 10 nM bis 0,3 µM oder zwischen etwa 30 nM bis 0,2 µM oder zwischen etwa 40 nM bis 0,1 µM oder zwischen etwa 50 nM bis 85 nM oder etwa 1, 5, 15, 25, 30, 35, 45, 50, 65, 75, 130, 150, 170, 250, 350 oder 450 nM; oder etwa 0,5, 0,7, 0,9, 1, 1,5, 2, 2,5, 3, 3,5, 4, 4,5, 5, 5,5, 6, 8, 9, 10, 12, 14, 16, 18 oder 20 µM ist; oder
v) der SMAD2/3-Hemmer ausgewählt ist aus der Gruppe, bestehend aus einem Smad2/3-Phosphorylierungshemmer und siRNA, die die mRNA des SMAD2- und SMAD3-Transkripts targetiert; und
vi) der WNT-Signalaktivator in dem ersten Medium ein Hemmer von Glycogen-Synthase-Kinase-3 (GSK3) ist; und der WNT-Signalaktivator in dem ersten Medium in einer Konzentration zwischen etwa 0,1 µM bis 10 µM, oder zwischen etwa 0,5 µM bis 8 µM, oder zwischen etwa 1 µM bis 7 µM, oder zwischen etwa 2 µM bis 6 µM, oder zwischen etwa 3 µM bis 5 µM, oder etwa 0,2, 0,3, 0,4, 3, 4, 4,5, 5, 6, 7,5, 8,5, 9, 9,5 oder 10 µM ist; und
vii) das Steroid in dem ersten Medium ein Kortikosteroid ist; und das Steroid in einer Konzentration zwischen etwa 0,5 µM und 200 µM oder zwischen etwa 1,5 µM bis 150 µM oder zwischen etwa 5 µM bis 100 µM oder zwischen etwa 10 µM bis 90 µM oder zwischen etwa 20 µM bis 80 µM oder zwischen etwa 30 µM bis 70 µM oder zwischen etwa 40 µM bis 60 µM oder etwa 2, 8, 15, 25, 30, 35, 45, 65, 75, 110, 130, 140, 160, 170 oder 190 µM ist; oder
viii) mindestens ein Molekül(e) in dem ersten Medium, das/diePhosphorylierung von SMAD1, SMAD5 und SMAD8 induzieren und MAPK-Signalisierung aktivieren, ausgewählt sind aus der Gruppe, bestehend aus BMP4, BMP2, BMP3, BMP5, BMP6 und BMP7; und das/die mindestens eine Molekül(e) in dem ersten Medium, das/die Phosphorylierung von SMAD1, SMAD5 und SMAD8 induzieren und MAPK-Signalübertragung aktivieren, in einer Konzentration zwischen etwa 2 ng/ml bis 5 µg/ml, oder zwischen etwa 5 ng/ml bis 5 µg/ml, oder zwischen etwa 10 ng/ml bis 4 µg/ml, oder zwischen etwa 15 ng/ml bis 3 µg/ml, oder zwischen etwa 20 ng/ml bis 2 µg/ml, oder etwa 5, 18, 20, 25, 28, 30, 50, 60, 70 ng/ml, oder etwa 1, 2,5, 3,5 oder 4,5 µg/ml sind; und
ix) das Molekül in dem ersten Medium, das den FGF- und MAPK-Weg aktiviert, ausgewählt ist aus der Gruppe, bestehend aus FGF7, FGF1, FGF3, FGF10 und FGF22.

4. Verfahren zum Ableiten und Beibehalten eines hepatischen (Leber-)Organoids nach Anspruch 3, wobei das erste Medium ferner einen Aktivator von AKT/PI3K-Signalweg und MAPK-Signalweg umfasst, wobei der Aktivator eine Verbindung ist, die ausgewählt ist aus der Gruppe, bestehend aus einem epidermalen Wachstumsfaktor (EGF), Amphiregulin (AR), Epigen (EPG), transformierendem Wachstumsfaktor alpha (TGFα), Betacellulin (BTC), Epiregulin (EPR), heparinbindenden EGF-ähnlichem Wachstumsfaktor (HB-EGF) und Neuregulin (NRG); und die Verbindung in einer Konzentration zwischen etwa 5 ng/ml bis 5 µg/ml, oder zwischen etwa 20 ng/ml bis 4 µg/ml, oder zwischen etwa 30 ng/ml bis 3 µg/ml, oder zwischen etwa 40 ng/ml bis 2 µg/ml, oder zwischen etwa 45 ng/ml und 500 ng/ml, oder zwischen etwa 50 ng/ml und 300 ng/ml, oder etwa 35, 40, 45, 50, 60, 70, 90, 100, 150, 200, 250, 300, 400, 450, 600, 700 oder 800 ng/ml ist.

5. Verfahren zum Ableiten und Beibehalten eines hepatischen (Leber-)Organoids nach Anspruch 3 oder 4, wobei das erste Medium ferner einen Aktivator von cAMP-abhängigen Signalwegen und/oder Protein-Kinase-A-Signalweg umfasst; wobei der Aktivator eine Verbindung ist, die ausgewählt ist aus der Gruppe, bestehend aus Dibutyryl-cAMP (dbCAMP), Forskolin ((3R,4aR,5S,6S,6aS, 10S, 10aR, 10bS)-6,10,10b-Trihydroxy-3,4a,7,7,10a-pentamethyl-1-oxo-3- vinyldodecahydro-1H-benzo[f]chromen-5-yl-acetat), Koffein, Theophyllin, Choleratoxin und Pertussistoxin; und die Verbindung in einer Konzentration zwischen etwa 20 ng/ml bis 1 µg/ml, oder zwischen etwa 10 ng/ml bis 0,8 µg/ml, oder zwischen etwa 15 ng/ml und 0,6 µg/ml, oder zwischen etwa
20 ng/ml bis 2 µg/ml, oder etwa 5, 18, 20, 25, 28, 30, 50, 60, 70 ng/ml, oder etwa 1, 2,5, 3,5 oder 4,5 µg/ml ist.

6. Verfahren zum Ableiten und Beibehalten eines hepatischen (Leber-)Organoids nach einem der Ansprüche 3 bis 5, wobei das erste Medium ferner einen Hemmer von Histon-Deacetylase (HDACs) umfasst; wobei der Hemmer eine Verbindung ist, die ausgewählt ist aus der Gruppe, bestehend aus Valporinsäure (VPA), Natriumbutyrat, Vorinotstat, Panobinostat, Trichostatin A, Mocetinostat, BG45 (N-(2-Aminophenyl)-2-pyrazincarboxamid), 4SC-202 ((E)-N-(2-Aminophenyl)-3-(1-((4-(1-methyl-1H-pyrazol-4-yl)phenyl)sulfonyl)-1H-pyrrol-3-yl)acrylamid), Belinostat, Scriptaid (6-(1,3-Dioxo-1H-benzo[de]isochinolin-2(3H)-yl)-N-hydroxyhexanamid), M344 (4-(Dimethylamino)-N-[7-(hydroxyamino)-7-oxoheptyl]benzamid), Dacinostat ((E)-N-Hydroxy-3-[4-[[2-hydroxyethyl-[2-(1H-indol-3-yl)ethyl]amino]methyl]phenyl]prop-2-enamid), Abexinostat, CUDC-101 (7-(4-(3-Ethynylphenylamino)-7-methoxychinazolin-6-yloxy)-N-hydroxyheptanamid), CUDC-907 (N-Hydroxy-2-(((2-(6-methoxypyridin-3-yl)-4-morpholinothieno[3,2-d]pyrimidin-6-yl)methyl)(methyl)amino)pyrimidin-5-carboxamid), und AR-42 ((S)-N-Hydroxy-4-(3-methyl-2-phenylbutanamido)benzamid); und die Verbindung in einer Konzentration zwischen etwa 0,1 µM bis 5 mM oder zwischen etwa 0,3 µM bis 4 mM oder zwischen etwa 0,6 µM bis 3 mM oder zwischen etwa 0,8 µM bis 2 mM oder zwischen etwa 1 mM bis 1,5 mM oder etwa 0,2, 0,4, 0,7, 0,9, 5, 10, 20, 50, 70 oder 90 µM oder etwa 1,5, 2,5, 3,5 mM ist.

7. Verfahren zum Ableiten und Beibehalten eines hepatischen (Leber-)Organoids nach Anspruch 3 bis 6, wobei das erste Medium ferner einen Aktivator des Notch-Rezeptors umfasst; wobei der Aktivator eine Verbindung ist, die ausgewählt ist aus der Gruppe, bestehend aus Jagged1-Protein (homo sapiens, auch bekannt als AGS; AHD; AWS; HJ1; CD339; JAGL1; JAG1), Jagged2 (NCBI 3714), Delta-like1 (NCBI 28514), Delta-like3 (NCBI 10683), und Delta-like4 (NCBI 54567); und die Verbindung in einer Konzentration zwischen etwa 10 nM und 100 µM, oder zwischen etwa 50 nM und 80 µM, oder zwischen etwa 100 nM und 60 µM, oder zwischen etwa 500 nM und 40 µM, oder zwischen etwa 800 nM und 20 µM, oder zwischen etwa 900 nM und 10 µM, oder etwa 20, 40, 60, 80 nM oder etwa 1, 1.5, 15, 30, 50, 60, 90 oder 100 µM ist.

8. Verfahren nach einem der Ansprüche 3 bis 7, wobei das zweite Zellkulturmedium Folgendes umfasst:
einen Aktivator von STAT3, GAB1-vermittelter Zelladhäsion und AKT/PI3K-Signalweg;
ein Molekül, das Repressor von NFκB-Aktivität ist und Aktivator einer Kinase von mitogen-aktiviertem Protein (MAP) ERK, p38 und JNK ist;
einen TGF-β-Hemmer und/oder SMAD2/3-Hemmer;
ein Steroid;
ein Molekül, das Phosphorylierung von SMAD1 und SMAD5 induziert und die MAPK-Signalübertragung aktiviert; und
ein Molekül, das Gallensäuresynthese reguliert und einen FGF- und MAPK-Weg aktiviert, und
eine Komponente zum Induzieren einer Differenzierung von spätem hepatischem Vorläufer, wobei die Komponente ein YAP-Hemmer ist:
i) wobei der Aktivator von STAT3, GAB 1-vermittelter Zelladhäsion und AKT/PI3K-Signalweg wie definiert in Anspruch 3 i) ist; oder
ii) wobei das Molekül, das ein Repressor von NFκB-Aktivität und ein Aktivator einer Kinase von mitogen-aktiviertem Protein (MAP) ERK, p38 und JNK ist, wie definiert in Anspruch 3 ii) ist; oder
iii) wobei der TGF-β-Hemmer und/oder SMAD2/3-Hemmer wie definiert in den Ansprüchen 3 hi) bis 3 v) ist; oder
iv) wobei das Steroid wie definiert in Anspruch 3 vii) ist; oder
v) wobei das Molekül, das Phosphorylierung von SMAD1, SMAD5 und SMAD8 induziert und MAPK-Signalisierung aktiviert, wie definiert in Anspruch 3 viii) ist; oder
vi) wobei das Molekül, das Gallensäuresynthese reguliert und FGF- und MAPK-Weg aktiviert, ausgewählt ist aus der Gruppe, bestehend aus FGF 19, FGF1, FGF2, FGF4, FGF6, FGF8, FGF9, FGF16, FGF17, FGF18, FGF20 und FGF23;
und
wobei das Molekül, das Gallensäuresynthese reguliert und FGF- und MAPK-Weg aktiviert, in einer Konzentration zwischen etwa 1 ng/ml bis 1 µg/ml, oder zwischen etwa 5 ng/ml bis 0,8 µg/ml, oder zwischen etwa 10 ng/ml bis 0,6 µg/ml, oder zwischen etwa 50 ng/ml und 0,5 µg/ml, oder zwischen etwa 150 ng/ml und 1 µg/ml, oder etwa 5, 20, 50, 100, 200, 250, 300, 400, 500 ng/ml, oder etwa 1, 0,8, 0,7 oder 0,9 µg/ml ist.

9. Verfahren nach Anspruch 8, wobei das zweite Zellkulturmedium ferner eine Komponente zum Fördern eines Überlebens von späten hepatischen Vorläufern umfasst, wobei die Komponente ausgewählt ist aus einer oder zwei oder drei oder allen der folgenden Komponenten:
einem Aktivator von AKT/PI3K-Signalweg und MAPK-Signalweg, wobei der Aktivator eine Verbindung ist, die ausgewählt ist aus der Gruppe, bestehend aus einem epidermalen Wachstumsfaktor (EGF), Amphiregulin (AR), Epigen (EPG), transformierendem Wachstumsfaktor alpha (TGFα), Betacellulin (BTC), Epiregulin (EPR), heparinbindenden EGF-ähnlichem Wachstumsfaktor (HB-EGF) und Neuregulin (NRG); und die Verbindung in einer Konzentration zwischen etwa 5 ng/ml bis 5 µg/ml, oder zwischen etwa 20 ng/ml bis 4 µg/ml, oder zwischen etwa 30 ng/ml bis 3 µg/ml, oder zwischen etwa 40 ng/ml bis 2 µg/ml, oder zwischen etwa 45 ng/ml und 500 ng/ml, oder zwischen etwa 50 ng/ml und 300 ng/ml, oder etwa 35, 40, 45, 50, 60, 70, 90, 100, 150, 200, 250, 300, 400, 450, 600, 700 oder 800 ng/ml ist.
einem Aktivator von cAMP-abhängigen Signalwegen oder einem Aktivator von Protein-Kinase-A-Signalweg, wobei der Aktivator eine Verbindung ist, die ausgewählt ist aus der Gruppe, bestehend aus Dibutyryl-cAMP (dbCAMP), Forskolin
((3R,4aR,5S,6S,6aS,10S, 10aR, 10bS)-6,10,10b-Trihydroxy-3,4a,7,7,10a-pentamethyl-1-oxo-3-vinyldodecahydro-1H-benzo[f]chromen-5-ylacetat), Koffein, Theophyllin, Choleratoxin und Pertussistoxin; und die Verbindung in einer Konzentration zwischen etwa 20 ng/ml bis 1 µg/ml oder zwischen etwa 10 ng/ml bis 0,8 µg/ml, oder zwischen etwa 15 ng/ml bis 0,6 µg/ml, oder zwischen etwa 20 ng/ml bis 2 µg/ml, oder etwa 5, 18, 20, 25, 28, 30, 50, 60, 70 ng/ml, oder etwa 1, 2,5, 3,5 oder 4,5 µg/ml ist;
einem Aktivator des Notch-Rezeptors, wobei der Aktivator eine Verbindung ist, die ausgewählt ist aus der Gruppe, bestehend aus Jagged1-Protein (homo sapiens, auch bekannt als AGS; AHD; AWS; HJ1; CD339; JAGL1; JAG1), Jagged2 (NCBI 3714), Delta-like1 (NCBI 28514), Delta-like3 (NCBI 10683), und Delta-like4 (NCBI 54567); und die Verbindung in einer Konzentration zwischen etwa 10 nM und 100 µM, oder zwischen etwa 50 nM und 80 µM, oder zwischen etwa 100 nM und 60 µM, oder zwischen etwa 500 nM und 40 µM, oder zwischen etwa 800 nM und 20 µM, oder zwischen etwa 900 nM und 10 µM, oder etwa 20, 40, 60, 80 nM oder etwa 1, 1.5, 15, 30, 50, 60, 90 oder 100 µM ist; und
einem Hemmer von Histon-Deacetylase (HDACs), wobei der Hemmer eine Verbindung ist, die ausgewählt ist aus der Gruppe, bestehend aus Valporinsäure (VPA), Natriumbutyrat, Vorinotstat, Panobinostat, Trichostatin A, Mocetinostat, BG45 (N-(2-Aminophenyl)-2-pyrazincarboxamid), 4SC-202 ((E)-N-(2-Aminophenyl)-3-(1-((4-(1-methyl-1H-pyrazol-4-yl)phenyl)sulfonyl)-1H-pyrrol-3-yl)acrylamid), Belinostat, Scriptaid (6-(1,3-Dioxo-1H-benzo[de]isochinolin-2(3H)-yl)-N-hydroxyhexanamid), M344 (4-(Dimethylamino)-N-[7-(hydroxyamino)-7-oxoheptyl]benzamid), Dacinostat ((E)-N-Hydroxy-3-[4-[[2-hydroxyethyl-[2-(1H-indol-3-yl)ethyl]amino]methyl]phenyl]prop-2-enamid), Abexinostat, CUDC-101 (7-(4-(3-Ethynylphenylamino)-7-methoxychinazolin-6-yloxy)-N-hydroxyheptanamid), CUDC-907 (N-Hydroxy-2-(((2-(6-methoxypyridin-3-yl)-4-morpholinothieno[3,2-d]pyrimidin-6-yl)methyl)(methyl)amino)pyrimidin-5-carboxamid), und AR-42 ((S)-N-Hydroxy-4-(3-methyl-2-phenylbutanamido)benzamid); und die Verbindung in einer Konzentration zwischen etwa 0,1 µM bis 5 mM oder zwischen etwa 0,3 µM bis 4 mM oder zwischen etwa 0,6 µM bis 3 mM oder zwischen etwa 0,8 µM bis 2 mM oder zwischen etwa 1 mM bis 1,5 mM oder etwa 0,2, 0,4, 0,7, 0,9, 5, 10, 20, 50, 70 oder 90 µM oder etwa 1,5, 2,5, 3,5 mM ist.

10. Verfahren nach Anspruch 8 oder Anspruch 9, wobei das zweite Zellkulturmedium ferner eine Komponente zum Induzieren einer Bildung von spätem hepatischem Vorläufer umfasst, wobei die Komponente ein WNT-Signalaktivator ist, wobei der WNT-Signalaktivator in Ansprüchen 3 bis vi) definiert ist.

11. Verfahren nach einem der Ansprüche 3 bis 5, wobei das dritte Zellkulturmedium Folgendes umfasst:
einen TGF-β-Hemmer und/oder SMAD2/3-Hemmer, wie definiert in Ansprüchen 3 iii) bis 3 v); ein pleiotropes Zytokin, das zu der Interleukin-6-Gruppe von Zytokinen gehört, wobei das pleiotrope Zytokin, das zu der Interleukin-6-Gruppe von Zytokin-Signalisierung gehört, ausgewählt ist aus Oncostatin M (OSM) oder Leukemie-hemmendem Faktor (LIF; NCBI: 3976) oder Cardiotrophin-l/CT-1 (NCBI: 1489) oder Rezeptor für ciliären neurotrophen Faktor (CNTF; NCBI: 1271), IL-11 oder IL-31, und wobei das pleiotrope Zytokin in einer Konzentration von zwischen etwa 0,1 ng/ml bis 1 µg/ml, oder zwischen etwa 10 ng/ml bis 0,8 µg/ml, oder zwischen etwa 15 ng/ml bis 0,6 µg/ml, oder zwischen etwa 20 ng/ml bis 2 µg/ml, oder etwa 5, 18, 20, 25, 28, 30, 50, 60, 70 ng/ml, oder etwa 1, 2,5, 3,5 oder 4,5 µg/ml ist; einen Hemmer von γ-Sekretase; und ein Steroid, wie definiert in Anspruch 3 vii), wobei:
der Hemmer von γ-Sekretase ausgewählt ist aus der Gruppe, bestehend aus Verbindung E (C-E) (2S)-2-[[2-(3,5-Difluorphenyl)acetyl]amino]-N-[(3S)-1-methyl-2-oxo-5-phenyl-3H-1,4-benzodiazepin-3-yl]propanamid, Dibenzazepin (DBZ): (2S)-2-[[2-(3,5-difluorphenyl)acetyl]amino]-N-[(7S)-5-methyl-6-oxo-7H-benzo[d][1]benzazepin-7-yl]propanamid, DAPT: tert-Butyl-(2S)-2-[[[(2S)-2-[[2-(3,5-Difluorphenyl)-acetyl]-amino]-propanoyl]-amino]-2-phenylacetat, Begacestat: 5-Chlor-N-[(2S)-4,4,4-trifluor-1-hydroxy-3-(trifluormethyl)-butan-2-yl]-thiophen-2-sulfonamid, und Flurizan: (2R)-2-(3-Fluor-4-phenylphenyl)propansäure; wobei der Hemmer der γ-Sekretase in einer Konzentration zwischen etwa 10 nM bis 5 µM oder zwischen etwa 100 nM bis 4 µM oder zwischen etwa 200 nM bis 3,5 µM, oder zwischen etwa 300 nM bis 3 µM, oder zwischen etwa 400 nM bis 2,5 µM, oder zwischen etwa 450 nM bis 2 µM, oder zwischen etwa 500 nM bis 1,5 µM, oder etwa 50, 90, 150, 250, 350, 450, 480, 500, 650, oder 700 nM ist.

12. Verfahren nach einem der Ansprüche 3 bis 5, wobei das dritte Zellkulturmedium mindestens eine oder zwei oder drei oder vier oder fünf oder sechs Komponente(n), die die Reifung des Leberorganoids fördern, und/oder mindestens eine oder zwei oder drei Komponente(n), die das Überleben von hepatischen Organoiden fördern, umfasst:
i) wobei die Komponente(n), die ein Reifen des hepatischen Organoids fördern, ausgewählt sind aus der Gruppe bestehend aus:
einer Verbindung, die Phosphorylierung von SMAD1, SMAD5 und SMAD8 induziert und MAPK-Signalweg aktiviert, wie definiert in Anspruch 3 viii);
einem Interleukin, das sowohl als entzündungsförderndes Zytokin als auch als entzündungshemmendes Myokin wirkt;
einer Verbindung, die als ein Gallensäuresynthese-regulierendes Hormon wirkt und FGF- und MAPK-Stoffwechselweg aktiviert, wie definiert in Anspruch 8 vi);
einen Aktivator von cAMP-abhängigen Signalwegen, wie definiert in Anspruch 9;
einen YAP-Hemmer; und
eine Verbindung mit biliärer Säurestärke, wobei:
a) der YAP-Hemmer ausgewählt ist aus der Gruppe, bestehend aus:
Verteporfin 3-[(23S,24R)-14-ethenyl-5-(3-methoxy-3-oxopropyl)-22,23-bis(methoxycarbonyl)-4,10,15,24-tetramethyl-25,26,27,28-tetraazahexacyclo [16.6.1.13,6.18,11.113,16.019,24]octacosa-1,3,5,7,9,11(27),12,14,16,18(25),19,21-dodecaen-9-yl]propansäure,
LPA [(2R)-2-Hydroxy-3-phosphonooxypropyl](Z)-octadec-9-enoat,
S1P [(E,2S,3R)-2-amino-3-hydroxyoctadec-4-enyl]dihydrogenphosphat,
Thrombin (2S)-2-[[(2S)-1-[(2S)-5-amino-2-[[2-[[(2S)-6-amino-2-[[2-[[(2S)-2-[[(2S)-4-amino-2-[[(2S)-2-[[(2S,3R)-2-[[(2S)-2-[[(2S,3R)-2-amino-3-hydroxybutanoyl]amino]-3-(1H-indol-3-yl)propanoyl]amino]-3-hydroxybutanoyl]amino]propanoyl]amino]-4-oxobutanoyl]amino]-3-methylbutanoyl]amino]acetyl]amino]hexanoyl]amino]acetyl]amino]-5-oxopentanoyl]pyrrolidin-2-carbonyl]amino]-3-hydroxypropansäure,
Epinephrin 4-[(1R)-1-Hydroxy-2-(methylamino)ethyl]benzol-1,2-diol, Glucagon (2S)-2-[[(2S,3R)-2-[[2-[[(2S)-5-Amino-2-[[(2S)-2-[[(2S)-2-amino-3-(1H-imidazol-5-yl)propanoyl]amino]-3-hydroxypropanoyl]amino]-5-oxopentanoyl]amino]acetyl]amino]-3-hydroxybutanoyl]amino]-3-phenylpropansäure,
Dihydrexidin (6aR,12bS)-5,6,6a,7,8,12b-hexahydrobenzo[a]phenanthridin-10,11-diol,
Dobutamin4 [2-[4-(4-Hydroxyphenyl)butan-2-ylamino]ethyl]benzol-1,2-diol, Dasatinib N-(2-Chlor-6-methylphenyl)-2-[[6-[4-(2-hydroxyethyl)piperazin-1-yl]-2-methylpyrimidin-4-yl]amino]-1,3-thiazol-5-carboxamid,
Latrunculin A (4R)-4-[(1R,4S,5Z,7E,11Z,15R,17R)-17-hydroxy-4,11-dimethyl-13-oxo-14,18-dioxabicyclo[13.3.1]nonadeca-5,7,11-trien-17-yl]-1,3-thiazolidin-2-on,
Latrunculin B (4R)-4-[(1R,4S,5Z,9Z,13R,15R)-15-Hydroxy-4,9-dimethyl-11-oxo-12,16-dioxabicyclo[11.3.1]heptadeca-5,9-dien-15-yl]-1,3-thiazolidin-2-on,
Cytochalasin D (3S,3aR,4S,6S,6aR,7E, 10S, 12R, 13E, 15R, 15aR)-3-Benzyl-6,12-dihydroxy-4,10,12-trimethyl-5-methylen-1,11-dioxo-2,3,3a,4,5,6,6a,9,10,11,12,15-dodecahydro-1H-cycloundeca[d]isoindol-15-yl-acetat
Blebbistatin 3a-Hydroxy-6-methyl-1-phenyl-2,3-dihydropyrrolo[2,3-b]chinolin-4-on, ML7 1-(5-Jodnaphthalin-1-yl)sulfonyl-1,4-diazepan,
Botulinum C3 4-N-(3-Chlor-7-methoxyacridin-1-yl)-1-N,1-N-Diethylpentan-1,4-diamin; Dihydrochlorid,
Y27632 4-[(1R)-1-Aminoethyl]-N-pyridin-4-yl-cyclohexan-1-carboxamid und Derivaten davon, optional wobei der YAP-Hemmer
Verteporfin 3-[(23S,24R)-14-ethenyl-5-(3-methoxy-3-oxopropyl)-22,23-bis(methoxycarbonyl)-4,10,15,24-tetramethyl-25,26,27,28-tetraazahexacyclo[16.6.1.13,6.18,11.113,16.019,24]octacosa-1,3,5,7,9,11(27),12,14,16,18(25),19,21-dodecaen-9-yl]propansäure ist; and
b) die Verbindung mit Gallensäure-Potenz eine oder mehrere oder alle der folgenden Aktivitäten umfasst: Aktivieren des nuklearen Farnesoid-X-Rezeptors, Erhöhen von cAMP und Aktivieren des PKC-Signalwegs, optional wobei die Verbindung mit Gallensäure-Potenz ausgewählt ist aus der Gruppe, bestehend aus:
Taurocholsäure 2-[[(4R)-4-[3R,5S,7R,8R,9S,10S,12S,13R,14S,17R)-3,7,12- trihydroxy-10,13-d 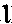 methyl-2,3,4,5,6,7,8,9,11,12,14,15,16,17-tetradecahydro-1H-cyclopenta[a]phenanthren-17-yl]pentanoyl]amino]ethansulfonsäure,
Cholsäure (4R)-4-[(3R,5S,7R,8R,9S,10S,12S,13R,14S,17R)- 3,7,12-trihydroxy-10,13- d 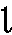 methyl-2,3,4,5,6,7,8,9,11,12,14,15,16,17-tetradecahydro-1H-cyclopenta[a]phenanthren-17-yl]pentansäure,
Chenodeoxycholsäure (4R)-4-[(3R,5S,7R,8R,9S, 10S, 13R, 14S, 17R)-3,7-Dihydroxy-10,13-dimethyl-2,3,4,5,6,7,8,9,11,12,14,15,16,17-tetradecahydro-1H-cyclopenta[a]phenanthren-17-yl]pentansäure,
Glycocholsäure 2-[[(4R)-4-[(3R,5S,7R,8R,9S,10S,12S,13R,14S,17R)-3,7,12-trihydroxy-10,13-dimethyl-2,3,4,5,6,7,8,9,11,12,14,15,16,17-tetradecahydro-1H-cyclopenta[a]phenanthren-17-yl]pentanoyl]amino]essigsäure,
Deoxycholsäure (4R)-4-[(3R,5R,8R,9S,10S,12S,13R,14S,17R)-3,12-dihydroxy-10,13-dimethyl-2,3,4,5,6,7,8,9,11,12,14,15,16,17-tetradecahydro-1H-cyclopenta[a]phenanthren-17-yl]pentansäure,
Glycochenodeoxycholsäure 2-[[(4R)-4-[(3R,5S,7R,8R,9S, 10S, 13R, 14S, 17R)-3,7-Dihydroxy-10,13-dimethy-1,2,3,4,5,6,7,8,9,11,12,14,15,16,17-tetradecahydro-1H-cyclopenta[a]phenanthren-17-yl]pentanoyl]amino]essigsäure,
Taurochenodeoxycholsäure 2-[[(4R)-4-[(3R,5S,7R,8R,9S, 10S, 13R, 14S, 17R)-3,7-Dihydroxy-10,13-dimethy-1,2,3,4,5,6,7,8,9,11,12,14,15,16,17-tetradecahydro-1H-cyclopenta[a]phenanthren-17-yl]pentanoyl]amino]ethansulfonsäure,
Lithocholsäure (4R)-4-[(3R,5R,8R,9S,10S,13R,14S,17R)-3-Hydroxy-10,13-dimethyl-2,3,4,5,6,7,8,9,11,12,14,15,16,17-tetradecahydro-1H-cyclopenta[a]phenanthren-17-yl]pentansäure und
Derivate davon, optional wobei die Verbindung mit Gallensäure-Potenz Taurocholsäure 2-[[(4R)-4-[(3R,5S,7R,8R,9S,10S,12S,13R,14S,17R)-3,7,12-trihydroxy-10,13-dimethyl-2,3,4,5,6,7,8,9,11,12,14,15,16,17-tetradecahydro-1H-cyclopenta[a]phenanthren-17-yl]pentanoyl]amino]ethansulfonsäure ist; und
wobei die Verbindung mit bilärer Säurepotenz in einer Konzentration zwischen etwa 1 µM bis 1 mM oder etwa 10 µM bis 0,8 mM oder etwa 50 µM bis 0,6 mM oder etwa 100 µM bis 0,4 mM oder etwa 150 µM bis 0,2 mM oder etwa 5, 15, 20, 40, 60, 80, 90, 100, 150, 250, 350, 450, 550, 650, 750, 850 µM ist;
oder
ii) wobei die Komponente(n), die das Überleben des Leberorganoids fördern, ausgewählt sind aus der Gruppe, bestehend aus:
einen Aktivator von STAT3, GAB1-vermittelter Zelladhäsion und AKT/PI3K-Signalweg;
einem Glykosaminoglykan; und
einem Aktivator von AKT/PI3K-Signalweg und MAPK-Signalweg, wobei der Aktivator von STAT3, GAB1-vermittelter Zelladhäsion und AKT/PI3K-Signalweg wie definiert in Anspruch 3 i) ist.

13. Verfahren nach einem der Ansprüche 3 bis 12, wobei die hepatischen (Leber-)Organoide mehr als einen leberspezifischen Zelltyp umfassen, der ausgewählt ist aus der Gruppe, bestehend aus Hepatozyten und Cholangiozyten, und optional ferner leberspezifische Endothelzellen (LSEC), Stellatzellen, hepatische Myofibroblasten und/oder Hepatoblasten umfassen.

14. Verfahren nach einem der Ansprüche 3 bis 13, wobei der frühe hepatische Vorläufer einen oder mehrere oder mindestens zwei oder mindestens drei oder mindestens vier oder mindestens fünf oder mindestens sechs oder 1 oder 2 oder 3 oder 4 oder 5 oder 6 oder 7 oder alle der folgenden Marker exprimiert: SOX9 (NCBI: 6662), CK19 (NCBI: 3880), CK18 (NCBI: 3875), HNF4a (NCBI: 3172), PROX1 (NCBI: 5629), ONECUT1 (NCBI: 3175), AFP (NCBI: 174), TBX3 (NCBI:6926); und/oder wobei der späte hepatische Vorläufer einen oder mehrere oder mindestens zwei oder mindestens drei oder mindestens vier oder mindestens fünf oder mindestens sechs oder zwischen 1 oder 2 oder 3 bis 4 oder 5 oder 6 oder 7 oder alle oder alle der folgenden Marker exprimiert: CK19 (NCBI: 3880), CK18 (NCBI: 3875), HNF4a (NCBI: 3172), PROX1 (NCBI: 5629), ONECUT1 (NCBI: 3175), AFP (NCBI: 174), TBX3 (NCBI:6926), ALB (NCBI: 213).

15. Hepatisches (Leber-)Organoid, das durch ein Verfahren nach einem der Ansprüche 3 bis 14 erlangt wird, umfassend mindestens zwei Zelltypen, die ausgewählt sind aus der Gruppe bestehend aus Hepatozyten und Cholangiozyten, wobei
die Hepatozyten
a) Albumin (ALB) exprimieren;
b) die Hepatozyten-Marker HNF4a (NCBI: 3172), FAH (NCBI: 2184), TAT (NCBI: 6898), GCK (NCBI: 2645), TTR (NCBI: 7276), MET (NCBI: 4233), GLU1/MGAM (NCBI: 8972), FAHD2A (NCBI: 51011), HNF1B (NCBI: 6928), HNF1A (NCBI: 6927), CYP3A4 (NCBI: 1576), CYP2C9 (NCBI: 1559), CYP2C19 (NCBI: 1557), CYP1A2 (NCBI: 1544), CYP2E1 (NCBI: 1571), CYP2D6 (NCBI: 1565), CYP3A7 (NCBI: 1551), CYP1A1 (NCBI: 1543), CYP3A5 (NCBI: 1577), CYP27A1 (NCBI: 1593), MRP2 (NCBI:1244), NTCP (NCBI: 6554), OATP1B3 (NCBI: 28234), UGT2B7 (NCBI: 7364), UGT2B15 (NCBI: 7366), UGT1A1 (NCBI: 54658), CEBP (NCBI: 1050), KRT8 (NCBI: 3856), NOTCH2 (NCBI: 4853) und CYP2B6 (NCBI: 1555) exprimieren; und
c) den Cholangiozytenmarker Cytokeratin 7 (CK7) nicht exprimieren; und
d) die Organoide in der Lage sind, Leberfunktionen durchzuführen und eine räumlich organisierte Struktur aufweisen, wie sie in der Leber vorzufinden ist.

## Revendications

1. Organoïde hépatique comprenant au moins deux types cellulaires choisis dans le groupe constitué des hépatocytes et des cholangiocytes, dans lequel
les hépatocytes
a) expriment l'albumine (ALB) ;
b) expriment les marqueurs des hépatocytes HNF4a (NCBI : 3172), FAH (NCBI : 2184), TAT (NCBI: 6898), GCK (NCBI: 2645), TTR (NCBI: 7276), MET (NCBI: 4233), GLU1/MGAM (NCBI : 8972), FAHD2A (NCBI : 51011), HNF1B (NCBI : 6928), HNF1A (NCBI : 6927), CYP3A4 (NCBI : 1576), CYP2C9 (NCBI : 1559), CYP2C19 (NCBI : 1557), CYP1A2 (NCBI : 1544), CYP2E1 (NCBI : 1571), CYP2D6 (NCBI : 1565), CYP3A7 (NCBI : 1551), CYP1A1 (NCBI : 1543), CYP3A5 (NCBI : 1577), CYP27A1 (NCBI : 1593), MRP2 (NCBI :1244), NTCP (NCBI : 6554), OATP1B3 (NCBI : 28234), UGT2B7 (NCBI : 7364), UGT2B15 (NCBI : 7366), UGT1A1 (NCBI : 54658), CEBP (NCBI: 1050), KRT8 (NCBI : 3856), NOTCH2 (NCBI : 4853) et CYP2B6 (NCBI : 1555) ; et
c) n'exprime pas le marqueur des cholangiocytes Cytokeratin 7 (CK7) ; et
d) les organoïdes sont capables de réaliser des fonctions hépatiques et présentent une structure spatialement organisée observée dans le foie.

2. Organoïde hépatique de la revendication 1 comprenant en outre au moins un type cellulaire choisi dans le groupe constitué de cellules endothéliales spécifiques du foie (LSEC), de cellules stellaires, de myofibroblastes hépatiques et d'hépatoblastes, dans lequel :
les cholangiocytes expriment CK7 mais n'expriment pas l'albumine (ALB) ; ou
les hépatoblastes expriment au moins un marqueur choisi dans le groupe constitué de SOX9 (NCBI : 6662), CK19 (NCBI : 3880), CK18 (NCBI : 3875), HNF4a (NCBI : 3172), PROX1 (NCBI : 5629), ONECUT1 (NCBI : 3175), AFP (NCBI : 174) et ALB (NCBI : 213) ; ou
les cellules endothéliales spécifiques du foie (LSEC) expriment au moins un marqueur choisi dans le groupe constitué de CD45, CD80, CD86, CD11c, VAP1, STAB1 et CD31, l'expression de CD31 étant principalement dans le cytoplasme et non sur la surface cellulaire ; ou
les cellules stellaires expriment au moins un marqueur choisi dans le groupe constitué de GFAP, VIM, LHX2, LRAT, PDGFRb, HAND2, ICAM-1, VCAM-1 et N-CAM-1 ; ou
les myofibroblastes hépatiques expriment un marqueur choisi dans le groupe constitué de COL1A1 et α-SMA ; ou
les types cellulaires parenchymateux proviennent de la même cellule souche ; ou
les cellules sont cultivées en suspension sans utilisation de matrices extracellulaires.

3. Procédé de dérivation et de maintien d'un organoïde hépatique (foie), ledit procédé de dérivation et de maintien d'un organoïde hépatique (foie) comprenant :
a) la culture d'une cellule souche d'endoderme dans un premier milieu cellulaire pour obtenir un progéniteur hépatique précoce ;
b) le transfert et la culture des cellules obtenues sous a) dans une système de culture en suspension dans un deuxième milieu de culture cellulaire pour obtenir un progéniteur hépatique tardif ; et
c) la culture des progéniteurs hépatiques tardifs obtenus sous b) dans un système de culture en suspension dans un troisième milieu de culture cellulaire pour obtenir un organoïde hépatique (foie)
ledit premier milieu comprenant :
un activateur de STAT3, de l'adhérence cellulaire médiée par GAB1 et de la voie de signalisation AKT/PI3K ;
une molécule qui est un répresseur de l'activité NFκB et de l'activateur des protéines kinases ERK, p38 et JNK activées par un mitogène (MAP) ;
un inhibiteur de TGF-β et/ou un inhibiteur de SMAD2/3 ;
un activateur de signalisation WNT ;
un stéroïde ;
au moins une molécule induisant la phosphorylation de SMAD1, SMAD5 et SMAD8 et activant la signalisation MAPK ; et
une molécule activant la voie FGF et MAPK , dans laquelle :
i) l'activateur de STAT3, de l'adhérence cellulaire médiée par GAB1 et de la voie de signalisation AKT/PI3K dans le premier milieu est un facteur de croissance des hépatocytes (HGF) à une concentration comprise entre environ 2 ng/ml et 5 µg/ml, ou entre environ 5 ng/ml et 5 µg/ml, ou entre environ 10 ng/ml et 4 µg/ml, ou entre environ 15 ng/ml et 3 µg/ml, ou entre environ 20 ng/ml et 2 µg/ml, ou environ 5, 18, 20, 25, 28, 30, 50, 60, 70 ng/ml, ou environ 1, 2,5, 3,5 ou 4,5 µg/ml ; et
ii) la molécule qui est un répresseur de l'activité NFκB et de l'activateur des protéines kinases ERK, p38 et JNK activées par un mitogène (MAP) dans le premier milieu est un composé choisi dans le groupe constitué du nicotinamide, de l'acide nicotinique, du 5-fluoronicotinamide, de l'hydrazide d'acide isonicotinique et du nikethamide ; à une concentration comprise entre environ 0,1 mM et 1 M, ou entre environ 2 mM et 0,8 M, ou entre environ 4 mM et 0,6 M, ou entre environ 6 mM et 0,4 M, ou entre environ 8 mM et 0.2 M, ou entre environ 10 mM et 800 mM, ou entre environ 50 mM et 500 mM, ou environ 3, 5, 9, 15, 20, 30, 50, 80, 100, 120, 150, 200, 250, 300, 350, 400 ou 450 mM ; et
iii) l'inhibiteur de TGF-β dans le premier milieu est choisi dans le groupe constitué de
A83-013-(6-Méthyl-2-pyridinyl)-N-phényl-4-(4-quinolinyl)-1H-pyrazole-1-carbothioamide,
A 77-01 4-(3-(6-méthylpyridin-2-yl)-1H-pyrazol-4-yl) quinoline,
SD-208 2-(5-chloro-2-fluorophényl)-N-pyridin-4-ylpteridin-4-amine, LY2157299 4-[2-(6-méthylpyridin-2-yl)-5,6-dihydro-4H-pyrrolo[1,2-b]pyrazol-3-yl]quinoline-6-carboxamide,
SB 431542 4-[4-(1,3-benzodioxol-5-yl)-5-pyridin-2-yl-1H-imidazol-2-yl]benzamide, GW788388 N-(oxan-4-yl)-4-[4-(5-pyridin-2-yl-1H-pyrazol-4-yl) pyridin-2-yl]benzamide,
SB505124 2-[4-(1,3-benzodioxol-5-yl)-2-tert-butyl-1H-imidazol-5-yl]-6-méthylpyridine,
SB525334 6-[2-tert-butyl-5-(6-méthylpyridin-2-yl)-1H-imidazol-4-yl]quinoxaline, IN 1130 2-[3-[(4-amino-2-méthylpyrimidin-5-yl)méthyl]-4-méthyl-1,3-thiazol-3-ium-5-yl]éthanol,
ITD 1 (6,6-diméthyl-5,7-dihydroimidazo[2,1-b][1,3]thiazol-4-ium-3-yl)méthyl N,N'-dicyclohexylcarbamimidothioate,
LY2109761 4-[2-[4-(2-pyridin-2-yl-5,6-dihydro-4H-pyrrolo[1,2-b]pyrazol-3-yl)quinolin-7-yl]oxyéthyl]morpholine,
K02288 3-[6-amino-5-(3,4,5-triméthoxyphényl)pyridin-3-yl]phénol,
inhibiteur de la kinase TGF-β RI [3-(Pyridin-2-yl)-4-(4-quinonyl)]-1H-pyrazole] et leurs dérivés ; et
iv) dans lequel l'inhibiteur de TGF-β dans le premier milieu est à une concentration comprise entre environ 0,5 nM et 20 µM, ou 100 µM et 10 µM, ou 250 nM et 5 µM, ou 400 nM et 2,5 µM, ou 0,5 nM et 1 µM, ou 0,5 nM et 0,5 µM, ou entre environ 1,5 nM et 0.4 µM, ou entre environ 10 nM et 0,3 µM, ou entre environ 30 nM et 0,2 µM, ou entre environ 40 nM et 0,1 µM, ou entre environ 50 nM et 85 nM, ou environ 1, 5, 15, 25, 30, 35, 45, 50, 65, 75, 130, 150, 170, 250, 350 ou 450 nM ; ou environ 0,5, 0,7, 0,9, 1, 1,5, 2, 2,5, 3, 3,5, 4, 4,5, 5, 5,5, 6, 8, 9, 10, 12, 14, 16, 18 ou 20 µM ; ou
v) l'inhibiteur de SMAD23 est choisi dans le groupe constitué d'un inhibiteur de phosphorylation de Smad2/3 et un ARNsi ciblant l'ARNm de la transcription de SMAD2 et SMAD3 ; et
vi) l'activateur de signalisation WNT dans le premier milieu est un inhibiteur de glycogène synthase kinase 3 (GSK3) ; et l'activateur de signalisation WNT dans le premier milieu est à une concentration comprise entre environ 0,1 µM et 10 µM, ou entre environ 0,5 µM et 8 µM, ou entre environ 1 µM et 7 µM, ou entre environ 2 µM et 6 µM, ou entre environ 3 µM et 5 µM, ou environ 0,2, 0,3, 0,4, 3, 4, 4,5, 5, 6, 7,5, 8,5, 9, 9,5 ou 10 µM ; et
vii) le stéroïde dans le premier milieu est un corticostéroïde ; et le stéroïde est à une concentration comprise entre environ 0,5 µM et 200 µM, ou entre environ 1,5 µM et 150 µM, ou entre environ 5 µM et 100 µM, ou entre environ 10 µM et 90 µM, ou entre environ 20 µM et 80 µM, ou entre environ 30 µM et 70 µM, ou entre environ 40 µM et 60 µM, ou environ 2, 8, 15, 25, 30, 35, 45, 65, 75, 110, 130, 140, 160, 170 ou 190 µM ; ou
viii) au moins une molécule dans le premier milieu induisant la phosphorylation de SMAD1, SMAD5 et SMAD8 et activant la signalisation MAPK est choisie dans le groupe constitué de BMP4, BMP2, BMP3, BMP5, BMP6 et BMP7 ; et ladite au moins une molécule dans le premier milieu induisant la phosphorylation de SMAD1, SMAD5 et SMAD8 et activant la signalisation MAPK est à une concentration comprise entre environ 2 ng/ml et 5 µg/ml, ou entre environ 5 ng/ml et 5 µg/ml, ou entre environ 10 ng/ml et 4 µg/ml, ou entre environ 15 ng/ml et 3 µg/ml, ou entre environ 20 ng/ml et 2 µg/ml, ou environ 5, 18, 20, 25, 28, 30, 50, 60, 70 ng/ml, ou environ 1, 2,5, 3,5 ou 4,5 µg/ml ; et
ix) la molécule dans le premier milieu activant la voie FGF et MAPK est choisie dans le groupe constitué de FGF7, FGF1, FGF3, FGF10 et FGF22.

4. Procédé de dérivation et de maintien d'un organoïde hépatique (foie) selon la revendication 3, dans lequel le premier milieu comprend en outre un activateur de la voie de signalisation AKT/PI3K et de la voie de signalisation MAPK, ledit activateur étant un composé choisi dans le groupe constitué d'un facteur de croissance épidermique (EGF), de l'amphiréguline (AR), de l'épigène (EPG), du facteur de croissance transformant alpha (TGFα), de la betacelluline (BTC), de l'épiréguline (EPR), du facteur de croissance de type EGF se liant à l'héparine (HB-EGF) et de la neuréguline (NRG) ; et le composé est à une concentration comprise entre environ 5 ng/ml et 5 µg/ml, ou entre environ 20 ng/ml et 4 µg/ml, ou entre environ 30 ng/ml et 3 µg/ml, ou entre environ 40 ng/ml et 2 µg/ml, ou entre environ 45 ng/ml et 500 ng/ml, ou entre environ 50 ng/ml et 300 ng/ml, ou environ 35, 40, 45, 50, 60, 70, 90, 100, 150, 200, 250, 300, 400, 450, 600, 700 ou 800 ng/ml.

5. Procédé de dérivation et de maintien d'un organoïde hépatique (foie) selon la revendication 3 ou la revendication 4, dans lequel le premier milieu comprend en outre un activateur des voies dépendantes de l'AMPc et/ou de la voie de signalisation de la protéine kinase A ; ledit activateur étant un composé choisi dans le groupe constitué du dibutyryl-AMPc (dbAMPc), de la forskoline ((3R,4aR,5S,6S,6aS,10S,10aR,10bS)-6,10,10b-trihydroxy-3,4a,7,7,10a-pentaméthyl-1-oxo-3-vinyldodécahydro-1H-benzo[f]chromen-5-yl acétate), de la caféine, de la théophylline, de la toxine cholérique et de la toxine pertussique ; et le composé est à une a concentration comprise entre environ 20 ng/ml et 1 µg/ml, ou entre environ 10 ng/ml et *0,8* µg/ml, ou entre environ 15 ng/ml et 0,6 µg/ml, ou entre environ 20 ng/ml et 2 µg/ml, ou environ 5, 18, 20, 25, 28, 30, 50, 60, 70 ng/ml, ou environ 1, 2,5, 3,5 ou 4,5 µg/ml.

6. Procédé de dérivation et de maintien d'un organoïde hépatique (foie) selon les revendications 3 à 5, dans lequel le premier milieu comprend en outre un inhibiteur des histones désacétylases (HDAC) ; ledit inhibiteur étant un composé choisi dans le groupe constitué de l'acide valporique (VPA), du butyrate de sodium, du vorinotstat, du panobinostat, de la trichostatine A, du mocétinostat, du BG45 (N-(2-aminophényl)-2-pyrazinecarboxamide), du 4SC-202 ((E)-N-(2-aminophényl)-3-(1-((4-(1-méthyl-1H-pyrazol-4-yl)phényl)sulfonyl)-1H-pyrrol-3-yl)acrylamide), du bélinostat, du scriptaid (6-(1,3-Dioxo-1H-benzo[de]isoquinolin-2(3H)-yl)-N-hydroxyhexanamide), du M344 (4-(diméthylamino)-N-[7-(hydroxyamino)-7-oxoheptyl]benzamide), du dacinostat ((E)-N-hydroxy-3-[4-[[2-hydroxyéthyl-[2-(1H-indol-3-yl)éthyl]amino]méthyl]phényl]prop-2-énamide), de l'abexinostat, du CUDC-101(7-(4-(3-éthynylphénylamino)-7-méthoxyquinazolin-6-yloxy)-N-hydroxyheptanamide), du CUDC-907 (N-hydroxy-2-(((2-(6-méthoxypyridin-3-yl)-4-morpholinothiéno[3,2-d]pyrimidin-6-yl)méthyl)(méthyl)amino)pyrimidine-5-carboxamide) et de l'AR-42 ((S)-N-hydroxy-4-(3-méthyl-2-phénylbutanamido)benzamide) ; et le composé est à une concentration comprise entre environ 0,1 µM et 5 mM, ou entre environ 0,3 µM et 4 mM, ou entre environ 0,6 µM et 3 mM, ou entre environ 0,8 µM et 2 mM, ou entre environ 1 mM et 1,5 mM, ou environ 0,2, 0,4, 0,7, 0,9, 5, 10, 20, 50, 70 ou 90 µM, ou environ 1,5, 2,5, 3,5 mM.

7. Procédé de dérivation et de maintien d'un organoïde hépatique (foie) selon les revendications 3 à 6, dans lequel le premier milieu comprend en outre un activateur du récepteur Notch ; ledit activateur étant un composé choisi dans le groupe constitué de la protéine Jagged1 (homo sapiens, également connue sous le nom AGS, AHD, AWS, HJ1, CD339, JAGL1, JAG1), Jagged2 (NCBI 3714), Delta-like1 (NCBI 28514), Delta-like3 (NCBI 10683) et Delta-like4 (NCBI 54567) ; et le composé est à une concentration comprise entre environ 10 nM et 100 µM, ou entre environ 50 nM et 80 µM, ou entre environ 100 nM et 60 µM, ou entre environ 500 nM et 40 µM, ou entre environ 800 nM et 20 µM, ou entre environ 900 nM et 10 µM, ou environ 20, 40, 60, 80 nM ou environ 1, 1,5, 15, 30, 50, 60, 90 ou 100 µM.

8. Procédé de l'une quelconque des revendications 3 à 7, dans lequel le deuxième milieu de culture cellulaire comprend :
un activateur de STAT3, de l'adhérence cellulaire médiée par GAB1 et de la voie de signalisation AKT/PI3K ;
une molécule qui est un répresseur de l'activité NFκB et de l'activateur des protéines kinases ERK, p38 et JNK activées par un mitogène (MAP) ;
un inhibiteur de TGF-β et/ou un inhibiteur de SMAD2/3 ;
un stéroïde ;
une molécule induisant la phosphorylation de SMAD1 et SMAD5 et activant la signalisation MAPK ; et
une molécule qui régule la synthèse des acides biliaires et active une voie FGF et MAPK, et
un composant destiné à induire la différenciation des progéniteurs hépatiques tardifs, ledit composant étant un inhibiteur de YAP :
i) dans lequel l'activateur de STAT3, de l'adhérence cellulaire médiée par GAB1 et de la voie de signalisation AKT/PI3K est tel que défini dans la revendication 3 i) ; ou
ii) dans lequel la molécule qui est un répresseur de l'activité NFκB et de l'activateur des protéines kinases ERK, p38 et JNK activées par un mitogène (MAP) est telle que définie dans la revendication 3 ii) ; ou
iii) dans lequel l'inhibiteur de TGF-β et/ou l'inhibiteur de SMAD2/3 est tel que défini dans les revendications 3 iii) à 3 v) ; ou
iv) dans lequel le stéroïde est tel que défini dans la revendication 3 vii) ; ou
v) dans lequel la molécule induisant la phosphorylation de SMAD1, SMAD5 et SMAD8 et activant la signalisation MAPK est telle que définie dans la revendication 3 viii) ; ou
vi) dans lequel la molécule qui régule la synthèse des acides biliaires et active la voie FGF et MAPK est choisie dans le groupe constitué de FGF 19, FGF1, FGF2, FGF4, FGF6, FGF8, FGF9, FGF16, FGF17, FGF18, FGF20 et FGF23 ;
et
dans lequel la molécule qui régule la synthèse des acides biliaires et active la voie FGF et MAPK est à une concentration comprise entre environ 1 ng/ml et 1 µg/ml, ou entre environ 5 ng/ml et 0,8 µg/ml, ou entre environ 10 ng/ml et 0,6 µg/ml, ou entre environ 50 ng/ml et *0,5* µg/ml, ou entre environ 150 ng/ml et 1 µg/ml, ou environ 5, 20, 50, 100, 200, 250, 300, 400, 500 ng/ml, ou environ 1, 0,8, 0,7 ou 0,9 µg/ml.

9. Procédé de la revendication 8, dans lequel le deuxième milieu de culture cellulaire comprend en outre un composant pour favoriser la survie de progéniteurs hépatiques tardifs, ledit composant étant choisi parmi un ou deux ou trois ou l'ensemble des composants suivants :
un activateur de la voie de signalisation AKT/PI3K et de la voie de signalisation MAPK, ledit activateur étant un composé choisi dans le groupe constitué d'un facteur de croissance épidermique (EGF), de l'amphiréguline (AR), de l'épigène (EPG), du facteur de croissance transformant alpha (TGFα), de la betacelluline (BTC), de l'épiréguline (EPR), du facteur de croissance de type EGF se liant à l'héparine (HB-EGF) et de la neuréguline (NRG) ; et le composé est à une concentration comprise entre environ 5 ng/ml et 5 µg/ml, ou entre environ 20 ng/ml et 4 µg/ml, ou entre environ 30 ng/ml et 3 µg/ml, ou entre environ 40 ng/ml et 2 µg/ml, ou entre environ 45 ng/ml et 500 ng/ml, ou entre environ 50 ng/ml et 300 ng/ml, ou environ 35, 40, 45, 50, 60, 70, 90, 100, 150, 200, 250, 300, 400, 450, 600, 700 ou 800 ng/ml ;
un activateur des voies dépendantes de l'AMPc ou un activateur de la voie de signalisation de la protéine kinase A, ledit activateur étant un composé choisi dans le groupe constitué du dibutyryl-AMPc (dbAMPc), de la forskoline ((3R,4aR,5S,6S,6aS,10S,10aR,10bS)-6,10,10b-trihydroxy-3,4a,7,7,10a-pentaméthyl-1-oxo-3-vinyldodécahydro-1H-benzo[f]chromen-5-yl acétate), de la caféine, de la théophylline, de la toxine cholérique et de la toxine pertussique ; et le composé est à une concentration comprise entre environ 20 ng/ml et 1 µg/ml, ou entre environ 10 ng/ml et 0,8 µg/ml, ou entre environ 15 ng/ml et 0,6 µg/ml, ou entre environ 20 ng/ml et 2 µg/ml, ou environ 5, 18, 20, 25, 28, 30, 50, 60, 70 ng/ml, ou environ 1, 2,5, 3,5 ou 4,5 µg/ml ;
un activateur du récepteur Notch, ledit activateur étant un composé choisi dans le groupe constitué de la protéine Jagged1 (homo sapiens, également connue sous le nom AGS, AHD, AWS, HJ1, CD339, JAGL1, JAG1), Jagged2 (NCBI 3714), Delta-like1 (NCBI 28514), Delta-like3 (NCBI 10683) et Delta-like4 (NCBI 54567) ; et le composé est à une concentration comprise entre environ 10 nM et 100 µM, ou entre environ 50 nM et 80 µM, ou entre environ 100 nM et 60 µM, ou entre environ 500 nM et 40 µM, ou entre environ 800 nM et 20 µM, ou entre environ 900 nM et 10 µM, ou environ 20, 40, 60, 80 nM ou environ 1, 1,5, 15, 30, 50, 60, 90 ou 100 µM ; et
un inhibiteur des histones désacétylases (HDAC), l'inhibiteur étant un composé choisi dans le groupe constitué de l'acide valporique (VPA), du butyrate de sodium, du vorinotstat, du panobinostat, de la trichostatine A, du mocétinostat, du BG45 (N-(2-aminophényl)-2-pyrazinecarboxamide), du 4SC-202 ((E)-N-(2-aminophényl)-3-(1-((4-(1-méthyl-1H-pyrazol-4-yl)phényl)sulfonyl)-1H-pyrrol-3-yl)acrylamide), du belinostat, du scriptaid (6-(1,3-Dioxo-1H-benzo[de]isoquinolin-2(3H)-yl)-N-hydroxyhexanamide), du M344 (4-(diméthylamino)-N-[7-(hydroxyamino)-7-oxoheptyl]benzamide), du dacinostat ((E)-N-hydroxy-3-[4-[[2-hydroxyéthyl-[2-(1H-indol-3-yl)éthyl]amino]méthyl]phényl]prop-2-énamide), de l'abexinostat, du CUDC-101 (7-(4-(3-éthynylphénylamino)-7-méthoxyquinazolin-6-yloxy)-N-hydroxyheptanamide), du CUDC-907 (N-hydroxy-2-(((2-(6-méthoxypyridin-3-yl)-4-morpholinothiéno[3,2-d]pyrimidin-6-yl)méthyl)(méthyl)amino)pyrimidine-5-carboxamide) et de l'AR-42 ((S)-N-hydroxy-4-(3-méthyl-2-phénylbutanamido)benzamide) ; et le composé est à une concentration comprise entre environ 0,1 µM et 5 mM, ou entre environ 0,3 µM et 4 mM, ou entre environ 0,6 µM et 3 mM, ou entre environ 0,8 µM et 2 mM, ou entre environ 1 mM et 1,5 mM, ou environ 0,2, 0,4, 0,7, 0,9, 5, 10, 20, 50, 70 ou 90 µM, ou environ 1,5, 2,5, 3,5 mM.

10. Procédé de la revendication 8 ou la revendication 9, dans lequel le deuxième milieu de culture cellulaire comprend en outre un composant pour induire la formation de progéniteurs hépatiques tardifs, ledit composant étant un activateur de signalisation WNT, ledit activateur de signalisation WNT étant défini dans la revendication 3 vi).

11. Procédé de l'une quelconque des revendications 3 à 5, dans lequel le troisième milieu de culture cellulaire comprend :
un inhibiteur de TGF-β et/ou un inhibiteur de SMAD2/3 tel que défini dans les revendications 3 iii) à 3 v) ; une cytokine pléiotropique qui appartient au groupe interleukine 6 de cytokines, ladite cytokine pléiotropique qui appartient au groupe interleukine 6 de signalisation des cytokines étant choisie parmi l'oncostatine M (OSM) ou le facteur inhibiteur de la leucémie (LIF ; NCBI : 3976), ou la cardiotrophin-1/CT-1 (NCBI : 1489), ou le récepteur du facteur neurotrophique ciliaire (CNTF ; NCBI : 1271), IL-11 ou IL-31, et la cytokine pléiotropique est à une concentration comprise entre environ 0,1 ng/ml et 1 µg/ml, ou entre environ 10 ng/ml et 0,8 µg/ml, ou entre environ 15 ng/ml et 0,6 µg/ml, ou entre environ 20 ng/ml et 2 µg/ml, ou environ 5, 18, 20, 25, 28, 30, 50, 60, 70 ng/ml, ou environ 1, 2,5, 3,5 ou 4,5 µg/ml ;
un inhibiteur de la γ-sécrétase ; et
un stéroïde tel que défini dans la revendication 3 vii) :
ledit inhibiteur de la γ-sécrétase étant choisi dans le groupe constitué du composé E (CE) (2S)-2-[[2-(3,5-difluorophényl)acétyl]amino]-N-[(3S)-1-méthyl-2-oxo-5-phényl-3H-1,4-benzodiazepin-3-yl]propanamide, de la dibenzazepine (DBZ) : (2S)-2-[[2-(3,5-difluorophényl)acétyl]amino]-N-[(7S)-5-méthyl-6-oxo-7H-benzo[d][1]benzazepin-7-yl]propanamide, du DAPT : (2S)-2-[[(2S)-2-[[2-(3,5-difluorophényl)acétyl]amino]propanoyl]amino]-2-phénylacetate de tert-butyle, du begacestat : 5-chloro-N-[(2S)-4,4,4-trifluoro-1-hydroxy-3-(trifluorométhyl)butan-2-yl]thiophène-2-sulfonamide et du flurizan : acide (2R)-2-(3-fluoro-4-phénylphényl) propanoïque ; l'inhibiteur de la γ-sécrétase étant à une concentration comprise entre environ 10 nM et 5 µM, ou entre environ 100 nM et 4 µM, ou entre environ 200 nM et 3,5 µM, ou entre environ 300 nM et 3 µM, ou entre environ 400 nM et 2,5 µM, ou entre environ 450 nM et 2 µM, ou entre environ 500 nM et 1,5 µM, ou environ 50, 90, 150, 250, 350, 450, 480, 500, 650 ou 700 nM.

12. Procédé de l'une quelconque des revendications 3 à 5, dans lequel le troisième milieu de culture cellulaire comprend au moins un ou deux ou trois ou quatre ou cinq ou six composant(s) favorisant la maturation de l'organoïde hépatique et/ou au moins un ou deux ou trois composant(s) favorisant la survie des organoïdes hépatiques :
i) le ou les composant(s) favorisant la maturation de l'organoïde hépatique étant choisis dans le groupe constitué de :
un composé induisant la phosphorylation de SMAD1, SMAD5 et SMAD8 et activant la signalisation MAPK tel que défini dans la revendication 3 viii) ;
une interleukine agissant à la fois comme une cytokine pro-inflammatoire et une myokine anti-inflammatoire ;
un composé agissant comme une hormone de régulation de la synthèse des acides biliaires et activant la voie FGF et MAPK tel que défini dans la revendication 8 vi) ;
un activateur des voies dépendantes de l'AMPc tel que défini dans la revendication 9 ;
un inhibiteur de YAP ; et
un composé avec activité des acides biliaires :
a) l'inhibiteur de YAP étant choisi dans le groupe constitué de :
Vertéporfine acide 3-[(23S,24R)-14-éthényl-5-(3-méthoxy-3-oxopropyl)-22,23-bis(méthoxycarbonyl)-4,10,15,24-tétraméthyl-25,26,27,28-tétraazahexacyclo[16.6.1.13,6.18,11.113,16.019,24]octacosa-1,3,5,7,9,11(27),12,14,16,18(25),19,21-dodécaèn-9-yl]propanoïque,
LPA [(2R)-2-hydroxy-3-phosphonooxypropyl](Z)-octadéc-9-énoate,
S1P [(E,2S,3R)-2-amino-3-hydroxyoctadec-4-enyl] dihydrogénophosphate, Thrombine acide (2S)-2-[[(2S)-1-[(2S)-5-amino-2-[[2-[[(2S)-6-amino-2-[[2-[[(2S)-2-[[(2S)-4-amino-2-[[(2S)-2-[[(2S,3R)-2-[[(2S)-2-[[(2S,3R)-2-amino-3-hydroxybutanoyl]amino]-3-(1H-indol-3-yl)propanoyl]amino]-3-hydroxybutanoyl]amino]propanoyl]amino]-4-oxobutanoyl]amino]-3-méthylbutanoyl]amino]acétyl]amino]hexanoyl]amino]acétyl]amino]-5-oxopentanoyl]pyrrolidine-2-carbonyl]amino]-3-hydroxypropanoïque, Épinéphrine 4-[(1R)-1-hydroxy-2-(méthylamino)éthyl]benzène-1,2-diol, Glucagon acide (2S)-2-[[(2S,3R)-2-[[2-[[(2S)-5-amino-2-[[(2S)-2-[[(2S)-2-amino-3-(1H-imidazol-5-yl)propanoyl]amino]-3-hydroxypropanoyl]amino]-5-oxopentanoyl]amino]acétyl]amino]-3-hydroxybutanoyl]amino]-3-phénylpropanoïque,
Dihydrexidine(6aR,12bS)-5,6,6a,7,8,12b-hexahydrobenzo[a]phénanthridine-10,11-diol,
Dobutamine4-[2-[4-(4-hydroxyphényl)butan-2-ylamino]éthyl]benzène-1,2-diol, DasatinibN-(2-chloro-6-méthylphényl)-2-[[6-[4-(2-hydroxyéthyl)pipérazin-1-yl]-2-méthylpyrimidin-4-yl]amino]-1,3-thiazole-5-carboxamide,
Latrunculine A (4R)-4-[(1R,4S,5Z,7E,11Z,15R,17R)-17-hydroxy-4,11-diméthyl-13-oxo-14,18-dioxabicyclo[13.3.1]nonadéca-5,7,11-trien-17-yl]-1,3-thiazolidin-2-one,
Latrunculine B (4R)-4-[(1R,4S,5Z,9Z,13R,15R)-15-hydroxy-4,9-diméthyl-11-oxo-12,16-dioxabicyclo[11.3.1]heptadéca-5,9-dien-15-yl]-1,3-thiazolidin-2-one,
Cytochalasine D(3S,3aR,4S,6S,6aR,7E,10S,12R,13E,15R,15aR)-3-Benzyl-6,12-dihydroxy-4,10,12-triméthyl-5-méthylène-1,11-dioxo-2,3,3a,4,5,6,6a,9,10,11,12,15-dodécahydro-1H-cycloundéca[d]isoindol-15-yl acétate
Blebbistatine 3a-hydroxy-6-méthyl-1-phényl-2,3-dihydropyrrolo[2,3-b]quinolin-4-one, ML7 1-(5-iodonaphthalèn-1-yl)sulfonyl-1,4-diazepane,
Botulinum C3 4-N-(3-chloro-7-méthoxyacridin-1-yl)-1-N,1-N-diéthylpentane-1,4-diamine;dihydrochlorydrate,
Y27632 4-[(1R)-1-aminoéthyl]-N-pyridin-4-ylcyclohexane-1-carboxamide,
et leurs dérivés, l'inhibiteur de YAP étant éventuellement Vertéporfine acide 3-[(23S,24R)-14-éthényl-5-(3-méthoxy-3-oxopropyl)-22,23-bis(méthoxycarbonyl)-4,10,15,24-tétraméthyl-25,26,27,28-tétraazahexacyclo[16.6.1.13,6.18,11.113,16.019,24]octacosa-1,3,5,7,9,11(27),12,14,16,18(25),19,21-dodécaèn-9-yl]propanoïque ; et
b) le composé avec activité des acides biliaires comprend une ou plusieurs ou l'ensemble des activités suivantes : activation du récepteur nucléaire farnésoïde X, augmentation de l'AMPc et activation de la voie de signalisation PKC, ledit composé avec activité des acides biliaires étant éventuellement choisi dans le groupe constitué de :
Acide taurocholique acide 2-[[(4R)-4-[(3R,5S,7R,8R,9S,10S,12S,13R,14S,17R)-3,7,12-trihydroxy-10,13-diméthyl-2,3,4,5,6,7,8,9,11,12,14,15,16,17-tétradécahydro-1H-cyclopenta[a]phénanthrèn-17-yl]pentanoyl]amino]éthanesulfonique,
Acide cholique (4R)-4-[(3R,5S,7R,8R,9S,10S,12S,13R,14S,17R)-3,7,12- tπhydroxy-10,13-d 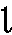 méthyl-2,3,4,5,6,7,8,9,11,12,14,15,16,17-tétradécahydro-1H-cyclopenta[a]phéenanthrèn-17-yl]pentanoïque,
Acide chénodésoxycholique (4R)-4-[(3R,5S,7R,8R,9S,10S,13R,14S,17R)-3,7-dihydroxy-10,13-diméthyl-2,3,4,5,6,7,8,9,11,12,14,15,16,17-tétradécahydro-1H-cyclopenta[a]phénanthrèn-17-yl]pentanoïque,
Acide glycocholique 2-[[(4R)-4-[(3R,5S,7R,8R,9S,10S,12S,13R,14S,17R)-3,7,12-trihydroxy-10,13-diméthyl-2,3,4,5,6,7,8,9,11,12,14,15,16,17-tétradécahydro-1H-cyclopenta[a]phénanthrèn-17-yl]pentanoyl]amino]acétique,
Acide désoxycholique (4R)-4-[(3R,5R,8R,9S,10S,12S,13R,14S,17R)-3,12-dihydroxy-10,13-diméthyl-2,3,4,5,6,7,8,9,11,12,14,15,16,17-tétradécahydro-1H-cyclopenta[a]phénanthrèn-17-yl]pentanoïque,
Acide glycochénodésoxycholique 2-[[(4R)-4-[(3R,5S,7R,8R,9S,10S,13R,14S,17R)-3,7-dihydroxy-10,13-diméthyl-2,3,4,5,6,7,8,9,11,12,14,15,16,17-tétradécahydro-1H-cyclopenta[a]phénanthrèn-17-yl]pentanoyl]amino]acetique, Acide taurochénodésoxycholique 2-[[(4R)-4-[(3R,5S,7R,8R,9S,10S,13R,14S,17R)-3,7-dihydroxy-10,13-diméthyl-2,3,4,5,6,7,8,9,11,12,14,15,16,17-tétradécahydro-1H-cyclopenta[a]phénanthrèn-17-yl]pentanoyl]amino]éthanesulfonique,
Acide lithocholique (4R)-4-[(3R,5R,8R,9S,10S,13R,14S,17R)-3 hydroxy-10,13-diméthyl-2,3,4,5,6,7,8,9,11,12,14,15,16,17-tétradécahydro-1H-cyclopenta[a]phénanthrèn-17-yl]pentanoïque, et
leurs dérivés, le composé avec activité des acides biliaires étant éventuellement l'acide taurocholique 2-[[(4R)-4-[(3R,5S,7R,8R,9S,10S,12S,13R,14S,17R)-3,7,12-trihydroxy-10,13-diméthyl-2,3,4,5,6,7,8,9,11,12,14,15,16,17-tétradécahydro-1H-cyclopenta[a]phénanthrèn-17-yl]pentanoyl]amino]éthanesulfonique ; et
le composé avec activité des acides biliaires étant à une concentration comprise entre environ 1 µM et 1 mM, ou environ 10 µM et 0,8 mM, ou environ 50 µM et 0,6 mM, ou environ 100 µM et 0,4 mM, ou environ 150 µM et 0,2 mM, ou environ 5, 15, 20, 40, 60, 80, 90, 100, 150, 250, 350, 450, 550, 650, 750, 850 µM ;
ou
ii) le ou les composant(s) favorisant la survie de l'organoïde hépatique étant choisi(s) dans le groupe constitué de :
un activateur de STAT3, de l'adhérence cellulaire médiée par GAB1 et de la voie de signalisation AKT/PI3K ;
un glycosaminoglycane ; et
un activateur de la voie de signalisation AKT/PI3K et de la voie de signalisation MAPK, dans lequel l'activateur de STAT3, de l'adhérence cellulaire médiée par GAB1 et de la voie de signalisation AKT/PI3K est tel que défini dans la revendication 3 i).

13. Procédé de l'une quelconque des revendications 3 à 12, dans lequel les organoïdes hépatiques (foie) comprennent plus d'un type de cellule spécifique du foie choisi dans le groupe constitué des hépatocytes et des cholangiocytes ; et comprennent en outre éventuellement des cellules endothéliales spécifiques du foie (LSEC), des cellules stellaires, des myofibroblastes hépatiques et/ou des hépatoblastes.

14. Procédé de l'une quelconque des revendications 3 à 13, dans lequel le progéniteur hépatique précoce exprime un ou plusieurs ou au moins deux, ou au moins trois, ou au moins quatre, ou au moins cinq, ou au moins six, ou 1 ou 2 ou 3 ou 4 ou 5 ou 6 ou 7 ou l'ensemble des marqueurs suivants : SOX9 (NCBI : 6662), CK19 (NCBI : 3880), CK18 (NCBI : 3875), HNF4a (NCBI : 3172), PROX1 (NCBI : 5629), ONECUT1 (NCBI : 3175), AFP (NCBI : 174), TBX3 (NCBI:6926) ; et/ou dans lequel le progéniteur hépatique tardif exprime un ou plusieurs ou au moins deux, ou au moins trois, ou au moins quatre, ou au moins cinq, ou au moins six, ou entre 1 ou 2 ou 3 à 4 ou 5 ou 6 ou 7 ou tous, ou l'ensemble des marqueurs suivants : CK19 (NCBI : 3880), CK18 (NCBI : 3875), HNF4a (NCBI : 3172), PROX1 (NCBI : 5629), ONECUT1 (NCBI : 3175), AFP (NCBI : 174), TBX3 (NCBI:6926), ALB (NCBI : 213).

15. Organoïde hépatique (foie) obtenu par un procédé de l'une quelconque des revendications 3 à 14, comprenant aux moins deux types cellulaires choisis dans le groupe constitué des hépatocytes et des cholangiocytes ; dans lequel
les hépatocytes
a) expriment l'albumine (ALB) ;
b) expriment les marqueurs des hépatocytes HNF4a (NCBI : 3172), FAH (NCBI : 2184), TAT (NCBI: 6898), GCK (NCBI: 2645), TTR (NCBI: 7276), MET (NCBI: 4233), GLU1/MGAM (NCBI : 8972), FAHD2A (NCBI : 51011), HNF1B (NCBI : 6928), HNF1A (NCBI : 6927), CYP3A4 (NCBI : 1576), CYP2C9 (NCBI : 1559), CYP2C19 (NCBI : 1557), CYP1A2 (NCBI : 1544), CYP2E1 (NCBI : 1571), CYP2D6 (NCBI : 1565), CYP3A7 (NCBI : 1551), CYP1A1 (NCBI : 1543), CYP3A5 (NCBI : 1577), CYP27A1 (NCBI : 1593), MRP2 (NCBI : 1244), NTCP (NCBI : 6554), OATP1B3 (NCBI : 28234), UGT2B7 (NCBI : 7364), UGT2B15 (NCBI : 7366), UGT1A1 (NCBI : 54658), CEBP (NCBI: 1050), KRT8 (NCBI : 3856), NOTCH2 (NCBI : 4853) et CYP2B6 (NCBI : 1555) ; et
c) n'expriment pas le marqueur des cholangiocytes Cytokeratin 7 (CK7) ; et
d) les organoïdes sont capables de réaliser des fonctions hépatiques et présentent une structure spatialement organisée observée dans le foie.
